# EUROPEAN PATENT APPLICATION

(11) **EP 4 837 638 A2**
(43) Date of publication of application: **11.11.2026**
(21) Application number: 26208177.1
(22) Date of filing: 11.03.2024
(51) Int. Cl.: A61P 35/00

(54) **TRANS-CYCLOOCTENE WITH IMPROVED T-LINKER**

(30) Priority: 10.03.2023 EP 23161339; 13.03.2023 EP 23161604; 22.05.2023 EP 23174613; 13.12.2023 EP 23216318; 15.12.2023 EP 23217334
(62) Document number(s) of the earlier application(s) in accordance with Art. 76 EPC: 24712333.4 / 4 676 542
(71) Applicant: Tagworks Pharmaceuticals B.V., 6525 ED Nijmegen (NL)
(72) Inventor: ROSSIN, Raffaella, 6525 ED Nijmegen (NL); ROBILLARD, Marc Stefan, 6525 ED Nijmegen (NL); KLEIJN, Laurens Henri Johan, 6525 ED Nijmegen (NL); VERSTEEGEN, Ronny Mathieu, 6525 ED Nijmegen (NL)
(74) Representative: V.O.

(57) **Abstract**

Disclosed herein include trans-cyclooctenes (TCOs) that may have improved properties, for example in clinical use. In certain embodiments, the TCOs may have improved *in vitro* and *in vivo* properties as compared to other TCOs. The disclosure also pertains to *in vivo* and *in vitro* methods of using said trans-cyclooctenes, as well as medical uses thereof, methods for making said TCOs, and compositions and/or combinations comprising said TCOs.

## Description

### Technological field

The disclosure disclosed herein relates to *trans*-cyclooctenes (TCOs) with improved properties. Compositions and combinations comprising the TCOs of the disclosure, as well as methods for using same are provided as well.

### Background

In the field of bioorthogonal chemistry the ligation between TCOs and dienes, in particular tetrazines, has been studied in depth. While the ligation works well both *in vitro* as *in vivo,* identifying optimal compounds for clinical use remains a research focus.

Along these lines, it is desired to identify new TCOs with overall good *in vitro* and *in vivo* properties, *e.g.* one or more of: fast blood clearance rate, high uptake at the target site (*e*.*g*. a tumor), low off-target uptake, a good metabolism profile, good stability, good reactivity with tetrazines, and/or high payload release (especially *in vivo*).

There is thus a need for new TCOs that address one or more of the abovementioned problems and/or desires.

### Summary

The disclosure relates to at least the following embodiments:
Embodiment 1. A compound or a salt, hydrate, or solvate thereof; wherein said compound has a structure according to Formula (1): 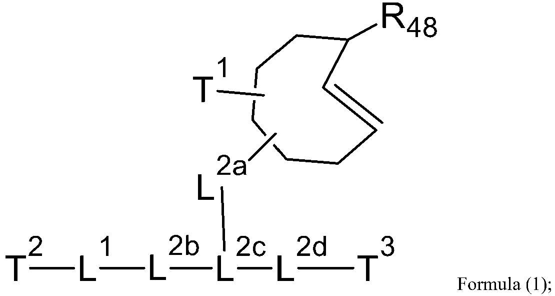 wherein **L¹** is selected from the group consisting of linear or branched C₄-C₁₂ alkylene, C₃-C₈ (hetero)cycloalkylene, C₆-C₁₂ arylene, and C₄-C₁₁ heteroarylene; **L^{2a}**, **L^{2b}**, and **L^{2d}** are each independently a linker; **L^{2c}** is selected from the group consisting of C₁-C₈ (hetero)alkanetriyl, C₅-C₆
   (hetero)arenetriyl, C₃-C₇ cycloalkanetriyl, and C₂-C₇ heterocycloalkanetriyl; **T¹** is selected from the group consisting of - OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and - Si(T^{1A})₃;
   each **T^{1A}** is independently selected from the group consisting of hydrogen, (hetero)alkyl, (hetero)alkenyl, (hetero)alkynyl, (hetero)aryl, and an amino acid residue; **T²** is a bioconjugation moiety or a group -L³-C^{B}; wherein **L³** is a residue of a bioconjugation moiety, and **C^{B}** is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof; **T³** is a polymer; and **R₄₈** is selected from the group consisting of -OH, -O-acetyl, -O-C₁₋₄ alkyl, halogen, active carbonate, and a releasable group; and preferably L¹ is linear or branched C₄-C₁₂ alkylene, more preferably L¹ is linear or branched C₄-C₁₀ alkylene, and most preferably L¹ is linear C₅-C₆ alkylene; preferably L^{2a}, L^{2b}, and L^{2d} are each independently a linker containing at most twenty atoms; more preferably L^{2a}, L²⁸, and L^{2d} are each independently selected from the group consisting of -C(O)NL^{2T}-, -NL^{2T}C(O)-, -O-, -S-, -NL^{2T}-, -N=N-, and -C(O)-; wherein L^{2T} is hydrogen or methyl, preferably L^{2T} is hydrogen; preferably L^{2c} is C₁-C₈ (hetero)alkanetriyl, more preferably L^{2c} is C₁-C₈ alkanetriyl, and most preferably L^{2c} is C₄-C₆ alkanetriyl; preferably T¹ is - OT^{1A}; and most preferably T¹ is -OH; preferably T^{1A} is hydrogen or methyl, more preferably T^{1A} is hydrogen; preferably T² is maleimidyl, N-hydroxysuccinimidyl, or -L³-C^{B}; preferably L³ is a residue of a maleimidyl moiety or a residue of an N-hydroxysuccinimidyl moiety; preferably C^{B} is a protein, more preferably C^{B} is an antibody or a diabody, even more preferably C^{B} is a diabody, and most preferably C^{B} is AVP0458 consisting of two monomers, wherein each of the two monomers has an amino acid sequence according to SEQ ID NO: 1; preferably T³ is a polymer comprising a polyethylene glycol moiety; and preferably R₄₈ is a releasable group.
Embodiment 2. The compound according to Embodiment 1, or a salt, hydrate, or solvate thereof; wherein said compound is according to Formula (2): 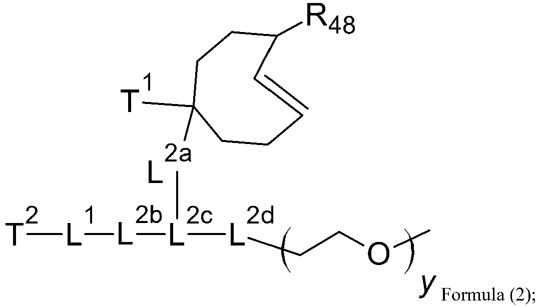 wherein
   y is an integer in a range of from 1 to 50; preferably y is an integer in a range of from 2 to 45; more preferably y is an integer in a range of from 10 to 40, more preferably in a range of from 12 to 37, even more preferably in a range of from 15 to 35, more preferably still in a range of from 20 to 30, and most preferably in a range of from 23 to 25.
Embodiment 3. The compound according to any one of the preceding Embodiments, or a salt, hydrate, or solvate thereof; wherein said compound is according to Formula (3): 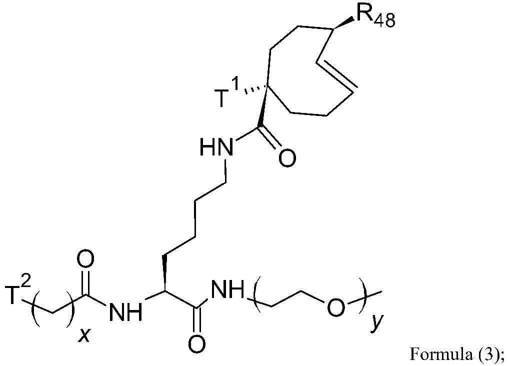 wherein y is as defined in Embodiment 2; x is an integer in a range of from 4 to 12; preferably x is an integer in a range of from 4 to 8, more preferably x is an integer in a range of from 4 to 6.
Embodiment 4. The compound according to any one of the preceding Embodiments, or a salt, hydrate, or solvate thereof; wherein R⁴⁸ is a releasable group, and said releasable group is
   -O-CO-C^{A}; wherein C^{A} is a drug; preferably the drug is linked to the moiety -O-CO- via a secondary or tertiary nitrogen atom that is part of the drug, forming a carbamate; preferably the drug is monomethyl auristatin E (MMAE), exatecan, or an exatecan derivative, more preferably the drug is MMAE.
Embodiment 5. The compound according to any one of the preceding Embodiments, or a salt, hydrate, or solvate thereof; wherein T² is selected from the group consisting of 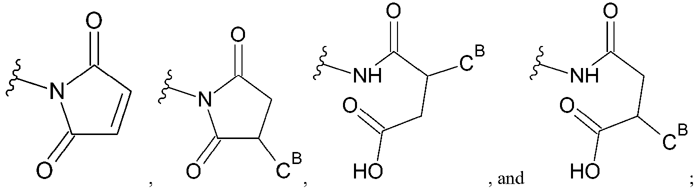 wherein
   C^{B} is a protein; preferably C^{B} is an antibody or a diabody, more preferably a diabody, and most preferably AVP0458 consisting of two monomers, wherein each of the two monomers has an amino acid sequence according to SEQ ID NO: 1; preferably C^{B} is linked to the remainder of T² via S or N that is part of C^{B}, more preferably S.
Embodiment 6. The compound according to any one of the preceding Embodiments, or a salt, hydrate, or solvate thereof; wherein said compound is: 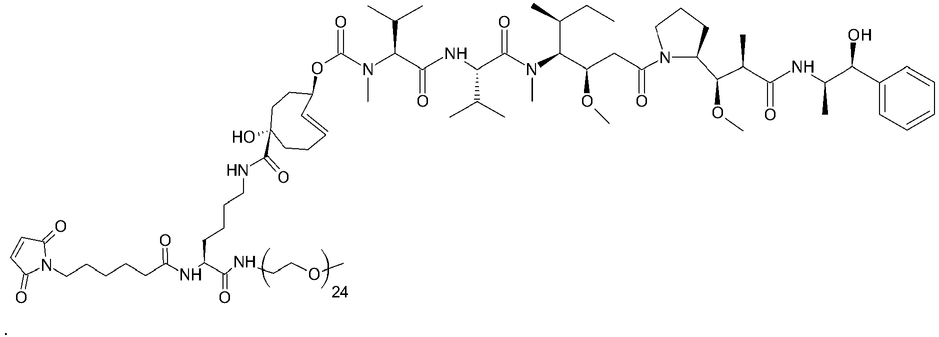
Embodiment 7. The compound according to any one of the preceding Embodiments, or a salt, hydrate, or solvate thereof; wherein said compound is 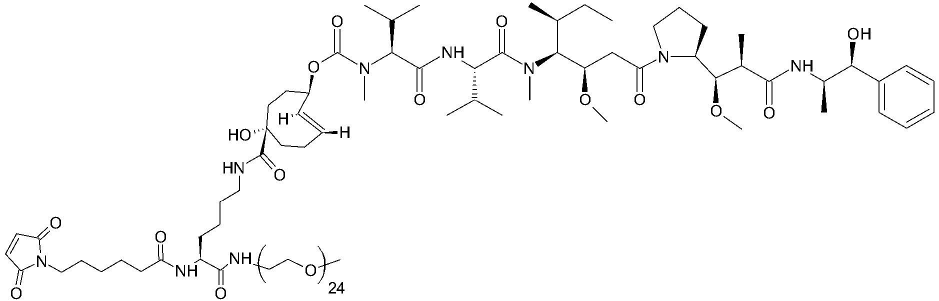 or 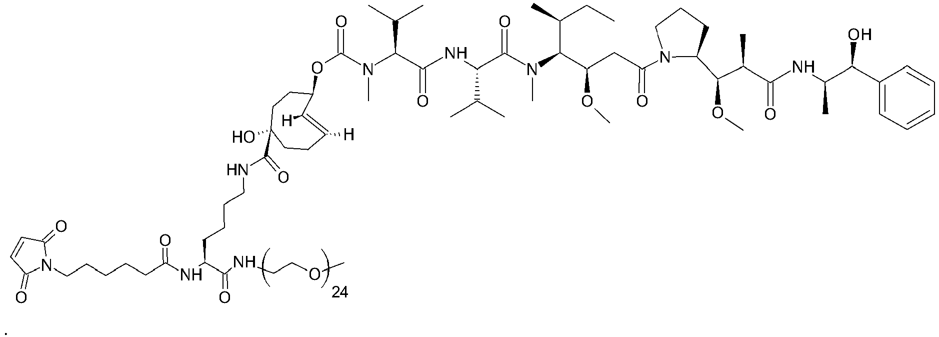
Embodiment 8. The compound according to any one of Embodiments 1 to 5, or a salt, hydrate, or solvate thereof; wherein said compound is: 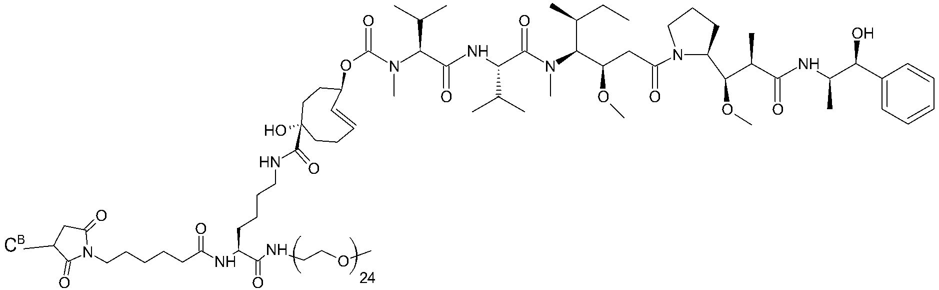 wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine.
Embodiment 9. The compound according to Embodiment 8, or a salt, hydrate, or solvate thereof; wherein said compound is: 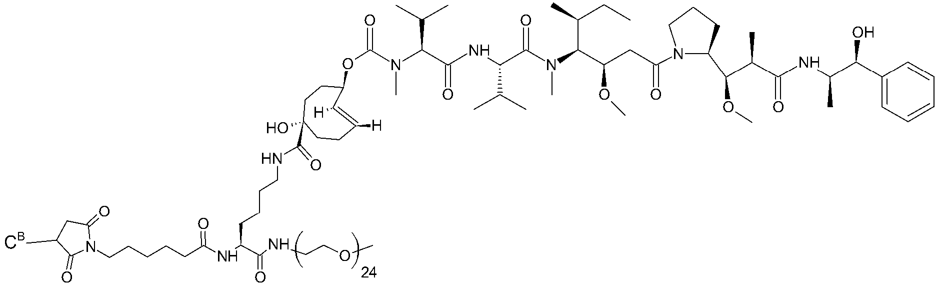 or 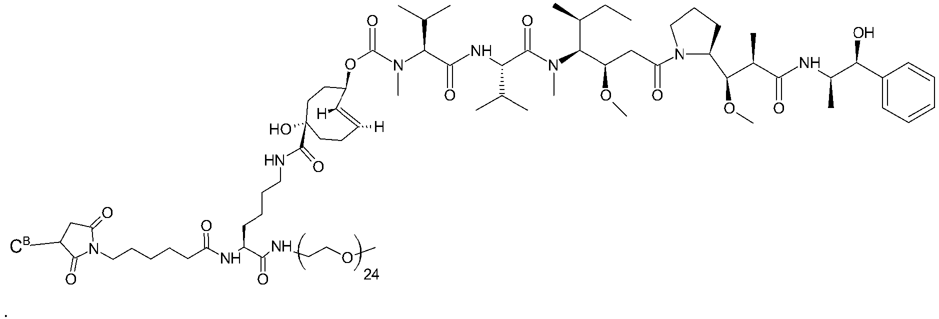
Embodiment 10. A compound or a salt, hydrate, or solvate thereof; wherein said compound comprises an eight-membered non-aromatic cyclic mono-alkenylene moiety, wherein said moiety comprises a non-vinylic carbon atom, wherein said non-vinylic carbon atom is substituted with at least one structure according to Formula (A): 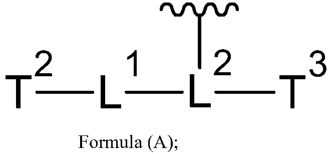 wherein
   L¹ and L² are each independently a linker; and
   T² and T³ are organic moieties.
Embodiment 11. A conjugate, or a salt, hydrate, or solvate thereof, wherein the conjugate comprises a protein conjugated to at least one compound according to Formula (1) as defined in any one of Embodiments 1 to 9, wherein L¹, L^{2a}, L^{2b}, L^{2c}, L^{2d}, T¹, T³, and R⁴⁸ are as defined in any one of Embodiments 1 to 9, and wherein T² is a residue of a bioconjugation moiety, and said protein and said compound are conjugated via T²; preferably the protein is a diabody or an antibody; more preferably the protein is a diabody; and most preferably the protein is AVP0458 consisting of two monomers, wherein each of the two monomers has an amino acid sequence according to SEQ ID NO: 1; preferably the protein is conjugated to at most 12 of said compounds; more preferably the protein is conjugated to at most 8 of said compounds, most preferably the protein is conjugated to at most 4 of said compounds; preferably said protein and said compound are conjugated via T² and a residue of a sulfhydryl of said protein, a residue of a hydroxyl of said protein, or a residue of an amine of said protein; more preferably said protein and said compound are conjugated via T² and a residue of a sulfhydryl of said protein; preferably T² is a residue of a maleimidyl moiety or a residue of an N-hydroxysuccinimidyl moiety; more preferably T² is a residue of a maleimidyl moiety.
Embodiment 12. The conjugate according to Embodiment 11, or a salt, hydrate, or solvate thereof, wherein the conjugate is 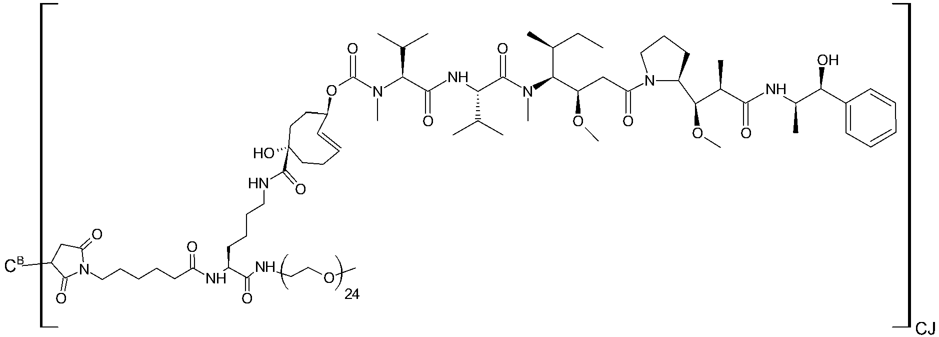 wherein CJ is in a range of from 1 to 12; wherein C^{B} is AVP0458 consisting of two monomers, wherein each of the two monomers has an amino acid sequence according to SEQ ID NO: 1; preferably CJ is of from 2 to 10, more preferably of from 2.5 to 8, even more preferably of from 3 to 6, even more preferably still of from 3.5 to 4, and most preferably about 4; preferably C^{B} is linked to each maleimidyl group via a sulfur atom, preferably the sulfur atom is part of a cysteine.
Embodiment 13. The conjugate according to Embodiment 12, or a salt, hydrate, or solvate thereof, wherein the conjugate is 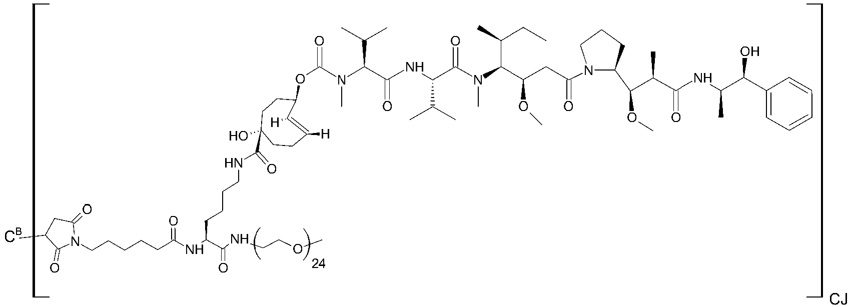 or 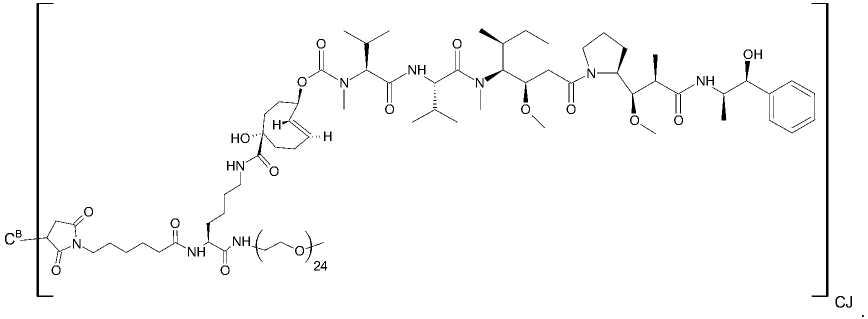
Embodiment 14. A composition comprising:
   (a) a compound according to any one of Embodiments 1 to 10, or the salt, hydrate, or solvate thereof; and/or
   (b) the conjugate according to any one of Embodiments 11 to 13, or the salt, hydrate, or solvate thereof; preferably the composition is a pharmaceutical composition.
Embodiment 15. A composition according to Embodiment 14, wherein said composition comprises:
   (a) a compound according to any one of Embodiments 1 to 10, or the salt, hydrate, or solvate thereof; and
   (b) the enantiomer of said compound, or the salt, hydrate, or solvate thereof; preferably said composition is a racemic mixture of (a) and (b).
Embodiment 16. A combination of
   (A1) a compound according to any one of Embodiments 1 to 10, or the salt, hydrate, or solvate thereof;
   (A2) a conjugate according to any one of Embodiments 11 to 13, or the salt, hydrate, or solvate thereof; and/or
   (A3) a composition according to Embodiment 14 or 15; with
   (B) a diene or a salt, solvate, or hydrate thereof; preferably the diene is a tetrazine.
Embodiment 17. The combination according to Embodiment 16, wherein the diene is selected from the group consisting of: 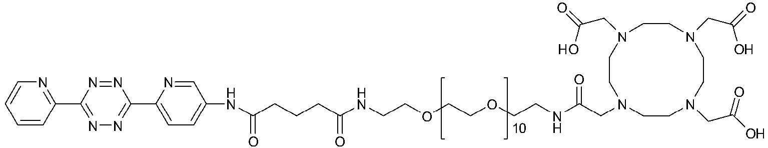 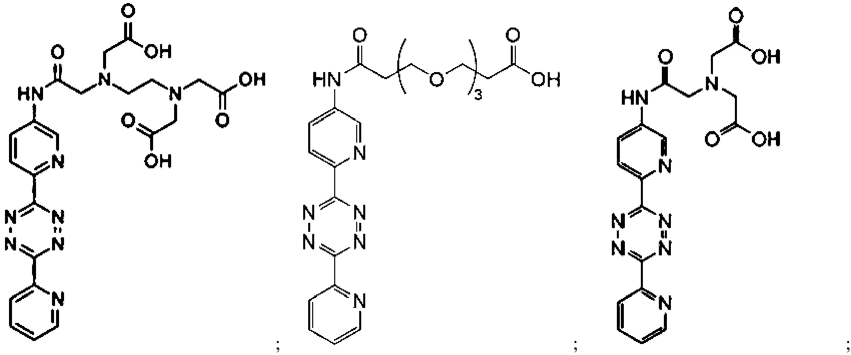 and 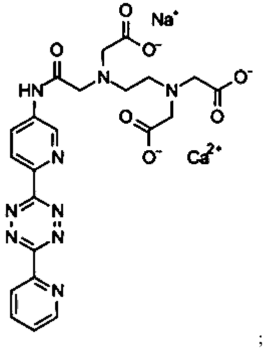 or a salt, hydrate, and/or solvate thereof.
Embodiment 18. The compound according to any one of Embodiments 1 to 10, or the salt, hydrate, or solvate thereof; the conjugate according to any one of Embodiments 11 to 13, or the salt, hydrate, or solvate thereof; the composition according to any one of Embodiments 14 to 15; or the combination according to any one of Embodiments 16 to 17; for use as a medicament.
Embodiment 19. The compound according to any one of Embodiments 1 to 10, or the salt, hydrate, or solvate thereof; the conjugate according to any one of Embodiments 11 to 13, or the salt, hydrate, or solvate thereof; the composition according to any one of Embodiments 14 to 15; or the combination according to any one of Embodiments 16 to 17; for use in the treatment of a disease in a subject, preferably the subject is a human; preferably the disease is cancer.
Embodiment 20. A method of treating a disease in a subject, wherein said method comprises the step of administering to said subject:
   (a) the compound according to any one of Embodiments 1 to 10, or the salt, hydrate, or solvate thereof;
   (b) the conjugate according to any one of Embodiments 11 to 13, or the salt, hydrate, or solvate thereof;
   (c) the composition according to any one of Embodiments 14 to 15; and/or
   (d) the combination according to any one of Embodiments 16 to 17; preferably the subject is a human; preferably the disease is cancer.
Embodiment 21. A non-therapeutic method for reacting:
   (ia) the compound according to any one of Embodiments 1 to 10, or the salt, hydrate, or solvate thereof;
   (iia) the conjugate according to any one of Embodiments 11 to 13, or the salt, hydrate, or solvate thereof; and/or
   (iiia) the composition according to any one of Embodiments 14 to 15;
   with a diene or a salt, solvate, or hydrate thereof,
   wherein said method comprises the step of contacting (ia), (iia), or (iiia) with said diene or salt, solvate, or hydrate thereof, preferably said non-therapeutic method is an *in vitro* method; and preferably said diene is a tetrazine.
Embodiment 22. A non-therapeutic use of:
   (a) the compound according to any one of Embodiments 1 to 10, or the salt, hydrate, or solvate thereof;
   (b) the conjugate according to any one of Embodiments 11 to 13, or the salt, hydrate, or solvate thereof;
   (c) the composition according to any one of Embodiments 14 to 15; and/or
   (d) the combination according to any one of Embodiments 16 to 17; in a click reaction.
Embodiment 23. A method for synthesizing a compound according to any one of Embodiments 1 to 10, or a salt, hydrate, or solvate thereof; wherein said method comprises
   (A) coupling a compound of Formula (R) to a compound of Formula (S): 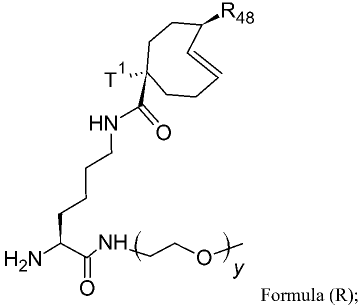 wherein R₄₈, T¹, and y are as defined in any one of Embodiments 1 to 10; 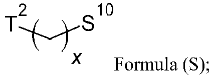 wherein T², and x, are as defined in any one of Embodiments 1 to 10, and S¹⁰ is -COOH or an active ester, preferably S¹⁰ is -COOH;
      or
   (B) coupling a compound of Formula (T) to a compound of Formula (U): 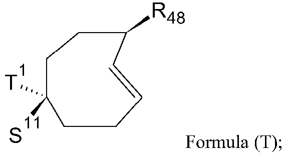 wherein R₄₈, and T¹ are as defined in any one of Embodiments 1 to 10; and S¹¹ is - COOH or an active ester, preferably S¹¹ is an active ester; 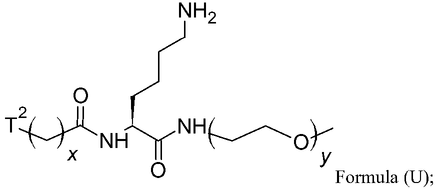 wherein T², x, and y are as defined in any one of Embodiments 1 to 10.
Embodiment 24. A method for synthesizing a conjugate according to any one of Embodiments 11 to 13, or a salt, hydrate, or solvate thereof; wherein said method comprises the step of coupling a protein to a compound according to any one of Embodiments 1 to 10, or a salt, hydrate, or solvate thereof; wherein in said compound T² is a bioconjugation moiety; wherein preferably in said protein disulfide bonds have been reduced.

### Detailed Description

As an example from the field of bioorthogonal chemistry, WO 2022/197182 describes AVP0458-**22-PEG24**, herein referred to as compound **1.** Compound **1** is an AVP0458 diabody modified with four TCO-containing moieties, and has the following structure:

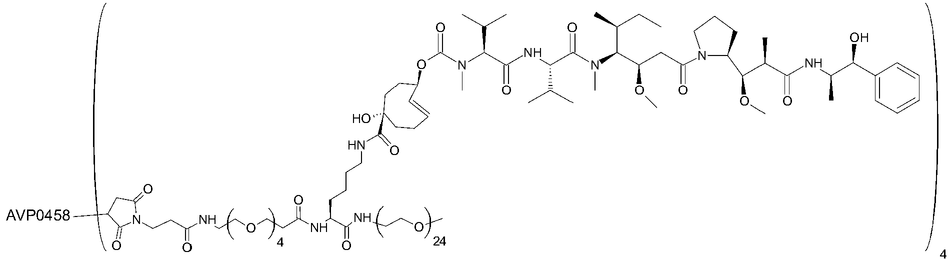

However, the inventors have identified, for the first time, that certain properties of compound 1 may be improved.

First, it was found that while compound 1 has a good clearance from blood, further improvements are desired. Compound 1 has a half-life in the blood of healthy mice of 4.22 hours, and 48 hours after injection in healthy mice 1.14% ID/g of compound **1** in the blood of said mice was observed. Based on this, it is desired that new TCOs be provided that show faster clearance rates as compared to compound 1.

Second, it was found that while compound **1** shows good tumor uptake of 18.42 %ID/g in LS174T xenograft bearing mice, further improvements are desired. It is therefore also desired that TCOs with a higher tumor uptake be provided.

Third, it was observed that compound **1** shows uptake at off-target sites, *e*.*g*. non-tumour sites such as the heart, lung, etc. It is also desired that TCOs be provided with a lower off-target uptake.

Fourth, it was observed that compound **1** shows a metabolism profile that can be improved. Thus, it is also desired that TCOs be provided showing a better metabolism profile.

Some aspects and embodiments of the disclosure are therefore, in a broad sense, based on the judicious insight that TCOs of the disclosure may meet one or more of the aforementioned desires. In particular, it was surprisingly found that replacing the PEG4 moiety of compound 1 may result in a higher clearance rate, higher tumor uptake, lower off-target uptake, a better metabolism profile, and/or further improved *in vitro* and *in vivo* properties.

Especially the combination of a faster clearance rate and a higher tumor uptake is surprising, since this means that the faster clearance from blood is not due to *e*.*g*. excretion from the body. Instead, the compounds of the disclosure may be quickly taken up in the tumour. Even more advantageously the uptake of compounds of the disclosure in off-target tissues may be much lower as compared to the uptake in the tumour. This means that a higher percentage of payload may be released at the desired target site, and that the trigger to activate this payload release (typically a diene, *e.g.* a tetrazine) may be administered at an earlier point in time, shortening the entire procedure. Thus, the compounds of the disclosure may also result in a higher convenience for patients, as fewer and/or less severe side-effects may be expected as well as a shorter treatment time.

Preferred embodiments of the disclosure are further described below, also in relation to a List of Clauses below. All of these embodiments, regardless of whether said embodiments are disclosed in the general part of the description or as part of the List of Clauses, can be combined as long as said embodiments are not mutually exclusive.

### Compounds of the disclosure

The compounds of the disclosure are according to Clause 1 as defined below, and preferably according to Formula (1) as defined above. It is understood that any compounds as provided herein may be in a form, formulation or solution in which the compound is present as a salt, solvate or hydrate of the compound. Accordingly, whereever herein a compound or genus of compounds are provided, or reference is made to "compound of the disclosure" or "compounds of the disclosure", it will be understood that also the salt, hydrate, or solvate of said compound(s) are included by such a statement even if the terms salt, hydrate or solvate are not specifically mentioned in each instance. In certain embodiments, a compound of the disclosure is purified or in a form or state in which it is not present as a salt, or as a hydrate, or as a solvate of the compound; however, unless specifically indicated as such it is intended to be assumed that any compound herein may be in the form of a salt, hydrate or solvate.

Preferred embodiments of the compounds of the disclosure are further described below in relation to several Formulae and variables.

### Formulae

Preferably, the compound of the disclosure is according to a Formula selected from the group consisting of Formula (1), Formula (2), Formula (3), Formula (B), Formula (C), Formula (D), Formula (E), Formula (F), Formula (G), Formula (H), Formula (I), Formula (J), Formula (K), Formula (L), Formula (M), Formula (N), Formula (O), Formula (P), and Formula (Q).

Preferably, the compound of the disclosure is according to Formula (B). More preferably, the compound of the disclosure is according to Formula (C). Even more preferably, the compound of the disclosure is according to Formula (1). More preferably still, the compound of the disclosure is according to Formula (D). Even more preferably, the compound of the disclosure is according to Formula (E). Yet more preferably, the compound of the disclosure is according to Formula (F). Still more preferably, the compound of the disclosure is according to Formula (G). More preferably still, the compound of the disclosure is according to Formula (2). Even more preferably still, the compound of the disclosure is according to Formula (H). Yet more preferably still, the compound of the disclosure is according to Formula (I). Even more preferably, the compound of the disclosure is according to Formula (J). More preferably still, the compound of the disclosure is according to Formula (K). Even more preferably still, the compound of the disclosure is according to Formula (L). Yet more preferably, the compound of the disclosure is according to Formula (M). Even more preferably still, the compound of the disclosure is according to Formula (N). Still more preferably, the compound of the disclosure is according to Formula (O). Yet more preferably, the compound of the disclosure is according to Formula (3). Still more preferably, the compound of the disclosure is according to Formula (P). Even more preferably, the compound of the disclosure is according to Formula (Q).
Formula (1) is: 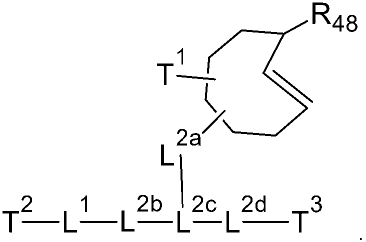
Formula (2) is: 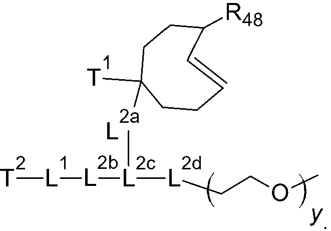
Formula (3) is: 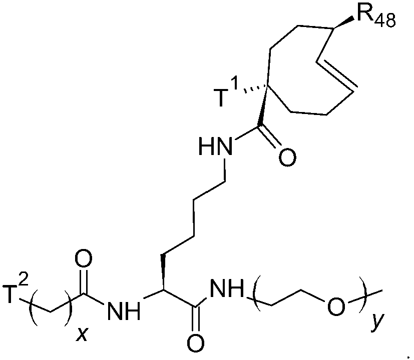
Formula (B) is: 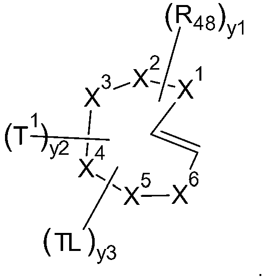
Formula (C) is: 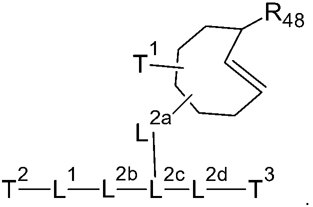
Formula (D) is: 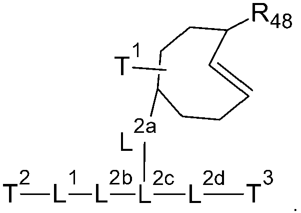
Formula (E) is: 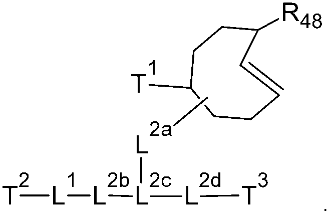
Formula (F) is: 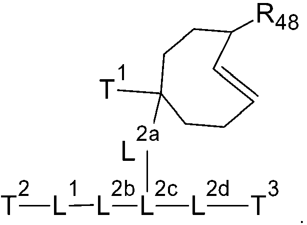
Formula (G) is: 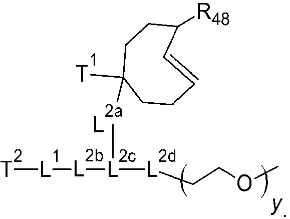
Formula (H) is: 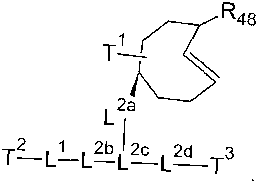
Formula (I) is: 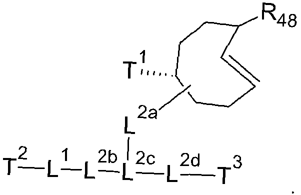
Formula (J) is: 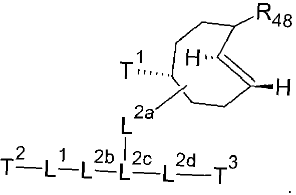
Formula (K) is: 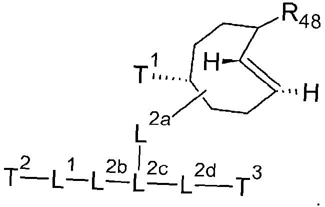
Formula (L) is: 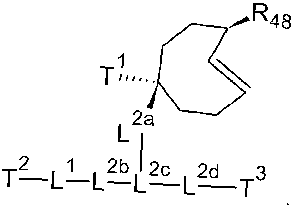
Formula (M) is: 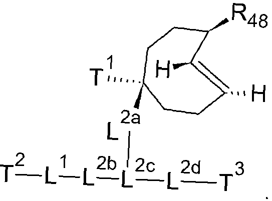
Formula (N) is: 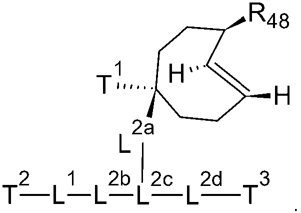
Formula (O) is: 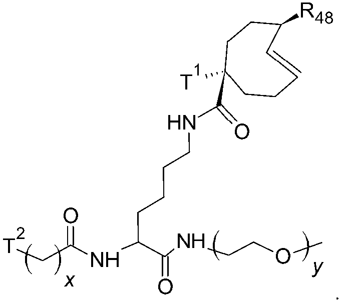
Formula (P) is: 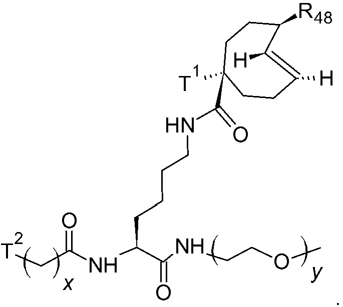
Formula (Q) is:

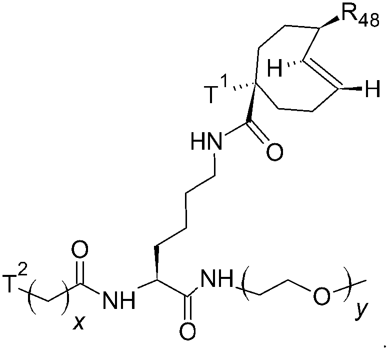

### L¹

L¹ is a linker. Preferably, L¹ is according to Radical Group 2 as defined herein.

Preferably, L¹ contains of from 1 to 100 atoms, preferably of from 2 to 75 atoms, more preferably of from 3 to 60 atoms, even more preferably of from 4 to 50 atoms, more preferably still of from 5 to 40 atoms, yet more preferably of from 6 to 35 atoms, even more preferably of from 7 to 30 atoms, more preferably still of from 8 to 25 atoms, even more preferably of from 9 to 22 atoms, and most preferably of from 10 to 20 atoms. Preferably, L¹ contains about 15 atoms.

More preferably, L¹ is selected from the group consisting of linear or branched C₁-C₁₂ (hetero)alkylene, C₃-C₈ (hetero)cycloalkylene, C₆-C₁₂ arylene, and C₄-C₁₁ heteroarylene.
More preferably than the foregoing, L¹ is selected from the group consisting of linear or branched C₁-C₁₂ alkylene, C₃-C₈ (hetero)cycloalkylene, C₆-C₁₂ arylene, and C₄-C₁₁ heteroarylene. More preferably than the foregoing, L¹ is selected from the group consisting of linear or branched C₂-C₁₂ alkylene, C₃-C₈ (hetero)cycloalkylene, C₆-C₁₂ arylene, and C₄-C₁₁ heteroarylene. More preferably than the foregoing, L¹ is selected from the group consisting of linear or branched C₃-C₁₂ alkylene, C₃-C₈ (hetero)cycloalkylene, C₆-C₁₂ arylene, and C₄-C₁₁ heteroarylene. More preferably than the foregoing, L¹ is selected from the group consisting of linear or branched C₄-C₁₂ alkylene, C₃-C₈ (hetero)cycloalkylene, C₆-C₁₂ arylene, and C₄-C₁₁ heteroarylene. More preferably than the foregoing, L¹ is a linear or branched C₁-C₁₂ alkylene. More preferably than the foregoing, L¹ is a linear or branched C₂-C₁₂ alkylene, viz. linear C₂ alkylene, linear or branched C₃ alkylene, linear or branched C₄ alkylene, linear or branched C₅ alkylene, linear or branched C₆ alkylene, linear or branched C₇ alkylene, linear or branched C₈ alkylene, linear or branched C₉ alkylene, linear or branched C₁₀ alkylene, linear or branched C₁₁ alkylene, or linear or branched C₁₂ alkylene. More preferably than the foregoing, L¹ is a linear or branched C₃-C₁₂ alkylene. More preferably than the foregoing, L¹ is a linear or branched C₄-C₁₂ alkylene. More preferably than the foregoing, L¹ is a linear or branched C₄-C₁₁ alkylene. More preferably than the foregoing, L¹ is a linear or branched C₄-C₁₀ alkylene. More preferably than the foregoing, L¹ is a linear or branched C₄-C₉ alkylene. More preferably than the foregoing, L¹ is a linear or branched C₄-C₈ alkylene. More preferably than the foregoing, L¹ is a linear or branched C₄-C₇ alkylene. More preferably than the foregoing, L¹ is a linear or branched C₄-C₆ alkylene. More preferably than the foregoing, L¹ is a linear or branched C₅ alkylene. More preferably than the foregoing, L¹ is a linear C₁-C₁₂ alkylene. More preferably than the foregoing, L¹ is a linear C₂-C₁₂ alkylene, viz. linear C₂ alkylene, linear C₃ alkylene, linear C₄ alkylene, linear C₅ alkylene, linear C₆ alkylene, linear C₇ alkylene, linear C₈ alkylene, linear C₉ alkylene, linear C₁₀ alkylene, linear C₁₁ alkylene, or linear C₁₂ alkylene. More preferably than the foregoing, L¹ is a linear C₃-C₁₂ alkylene. More preferably than the foregoing, L¹ is a linear C₄-C₁₂ alkylene. More preferably than the foregoing, L¹ is a linear C₄-C₁₁ alkylene. More preferably than the foregoing, L¹ is a linear C₄-C₁₀ alkylene. More preferably than the foregoing, L¹ is a linear C₄-C₉ alkylene. More preferably than the foregoing, L¹ is a linear C₄-C₈ alkylene. More preferably than the foregoing, L¹ is a linear C₄-C₇ alkylene. More preferably than the foregoing, L¹ is a linear C₄-C₆ alkylene. Most preferably, L¹ is a linear C₅ alkylene.

L¹ can be substituted or unsubistuted. Preferably, L¹ is unsubstituted. Most preferably, L¹ is an unsubstituted, linear C₅ alkylene.

Without wishing to be bound by theory, the inventors believe that the linker L¹ of compounds of Formula (1) of the present disclosure may provide a faster blood clearance rate, while maintaining a high uptake at the target site of the compound of Formula (1). Still without wishing to be bound by theory, an advantage of linkers L¹ having a length as defined in claim 1, in particular linear C₄-C₁₂ alkylene, may be that sufficient distance between the moiety C^{B} and the *trans-*cyclooctene can be achieved, so that the double bond of the *trans-cyclooctene* may readily react with a diene. Still without wishing to be bound by theory, an advantage of linkers L¹ having a length as defined in claim 1, in particular linear C₄-C₁₂ alkylene, may be that they are not too long, so that they may still be shielded by moiety C^{B} which may prevent e.g. deactivation. An advantage of relatively short alkylene linkers, such as C₄-C₆ alkylene, may be that their solubility is also higher than for relatively long alkylene linkers.

### L²

L² is a linker. Preferably, L² is according to Radical Group 2 as defined herein.

Preferably, L² contains of from 1 to 200 atoms, preferably of from 2 to 150 atoms, more preferably of from 3 to 100 atoms, even more preferably of from 4 to 90 atoms, more preferably still of from 5 to 80 atoms, yet more preferably of from 6 to 70 atoms, even more preferably of from 7 to 60 atoms, more preferably still of from 8 to 50 atoms, even more preferably of from 9 to 45 atoms, and most preferably of from 10 to 35 atoms.

Preferably, L² is selected from the group consisting of linear or branched C₁-C₁₂ (hetero)alkanetriyl, C₃-C₈ (hetero)cycloalkanetriyl, C₆-C₁₂ arenetriyl, and C₄-C₁₁ heteroarenetriyl. More preferably than the foregoing, L² is a linear or branched C₁-C₁₂ (hetero)alkanetriyl. More preferably than the foregoing, L² is a linear or branched C₁-C₁₂ heteroalkanetriyl. More preferably than the foregoing, L² is a branched C₁-C₁₂ (hetero)alkanetriyl. More preferably than the foregoing, L² is a branched C₁-C₁₂ heteroalkanetriyl. More preferably than the foregoing, L² is a branched C₃-C₁₁ heteroalkanetriyl. More preferably than the foregoing, L² is a branched C₆-C₁₀ heteroalkanetriyl. More preferably than the foregoing, L² is a branched C₈ heteroalkanetriyl. More preferably than the foregoing, L² is a branched C₈ heteroalkanetriyl substituted with up to five =O groups. More preferably than the foregoing, L² is a branched C₈ heteroalkanetriyl substituted with three =O groups. More preferably than the foregoing, L² is a branched C₈ heteroalkanetriyl containing up to five -NH- groups. More preferably than the foregoing, L² is a branched C₈ heteroalkanetriyl containing three -NH- groups. More preferably than the foregoing, L² is a branched C₈ heteroalkanetriyl containing three -NH- groups, and wherein the C₈ heteroalkanetriyl is substituted with three =O groups.
More preferably, L² is: 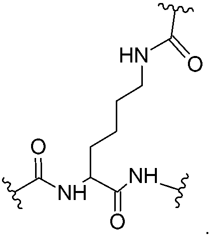
Even more preferably, L² is: 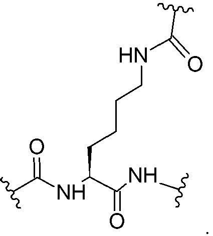
More preferably still, L² is: 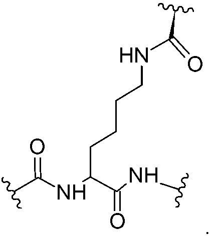
Most preferably, L² is:

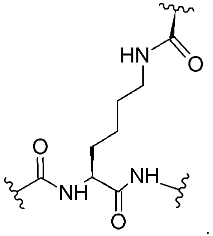

In preferred embodiments, L² has the following structure: 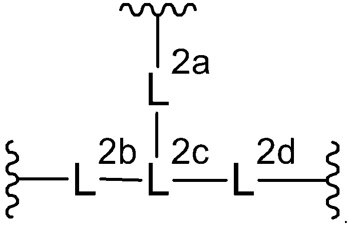 Herein, L^{2a}, L²⁸, L^{2c}, and L^{2d} are each independently a linker. Preferably, L^{2a}, L^{2b}, L^{2c}, and L^{2d} are each independently according to Radical Group 2 as defined herein.

### L^{2a}

L^{2a} is a linker. Preferably, L^{2a} is according to Radical Group 2 as defined herein. More preferably, L^{2a} is a linker containing at most twenty atoms. More preferably than the foregoing, L^{2a} is a linker containing at most fifteen atoms. More preferably than the foregoing, L^{2a} is a linker containing at most ten atoms. More preferably than the foregoing, L^{2a} is a linker containing at most five atoms. More preferably than the foregoing, L^{2a} is selected from the group consisting of -C(O)NL^{2T}-, -NL^{2T}C(O)-, - O-, -S-, -NL^{2T}-, -N=N-, and -C(O)-; wherein L^{2T} is hydrogen or methyl. More preferably than the foregoing, L^{2a} is selected from the group consisting of -C(O)NL^{2T}-, and -NL^{2T}C(O)-. More preferably than the foregoing, L^{2a} is selected from the group consisting of -C(O)NH-, and -NHC(O)-. Most preferably, L^{2a} is -NHC(O)-.

### L^{2b}

L^{2b} is a linker. Preferably, L^{2b} is according to Radical Group 2 as defined herein. More preferably, L^{2b} is a linker containing at most twenty atoms. More preferably than the foregoing, L^{2d} is a linker containing at most fifteen atoms. More preferably than the foregoing, L^{2d} is a linker containing at most ten atoms. More preferably than the foregoing, L^{2d} is a linker containing at most five atoms. More preferably than the foregoing, L^{2d} is selected from the group consisting of -C(O)NL^{2T}-, -NL^{2T}C(O)-, -O-, -S-, -NL^{2T}-, -N=N-, and -C(O)-; wherein L^{2T} is hydrogen or methyl. More preferably than the foregoing, L^{2d} is selected from the group consisting of -C(O)NL^{2T}-, and -NL^{2T}C(O)-. More preferably than the foregoing, L^{2d} is selected from the group consisting of -C(O)NH-, and -NHC(O)-. Most preferably, L^{2b} is -NHC(O)-.

### L^{2c}

L^{2c} is a linker. Preferably, L^{2c} is according to Radical Group 2 as defined herein. More preferably than the foregoing, L^{2c} is a linker comprising at most 50 atoms. More preferably than the foregoing, L^{2c} is a linker comprising at most 40 atoms. More preferably than the foregoing, L^{2c} is a linker comprising at most 30 atoms. More preferably than the foregoing, L^{2c} is a linker comprising at most 20 atoms. More preferably than the foregoing, L^{2c} is a linker comprising at most 15 atoms. More preferably than the foregoing, L^{2c} is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl. More preferably than the foregoing, L^{2c} is C₁-C₈ (hetero)alkanetriyl. More preferably than the foregoing, L^{2c} is C₁-C₈ alkanetriyl. More preferably than the foregoing, L^{2c} is C₂-C₇ alkanetriyl. More preferably than the foregoing, L^{2c} is C₃-C₆ alkanetriyl. More preferably than the foregoing, L^{2c} is C₄-C₅ alkanetriyl. More preferably than the foregoing, L^{2c} is C₅ alkanetriyl. Most preferably, L^{2c} is >CH-CH₂-CH₂-CH₂-CH₂-.

### L^{2d}

L^{2d} is a linker. Preferably, L^{2d} is according to Radical Group 2 as defined herein. More preferably than the foregoing, L^{2d} is a linker containing at most twenty atoms. More preferably than the foregoing, L^{2d} is a linker containing at most fifteen atoms. More preferably than the foregoing, L^{2d} is a linker containing at most ten atoms. More preferably than the foregoing, L^{2d} is a linker containing at most five atoms. More preferably than the foregoing, L^{2d} is selected from the group consisting of - C(O)NL^{2T}-, -NL^{2T}C(O)-, -O-, -S-, -NL^{2T}-, -N=N-, and -C(O)-; wherein L^{2T} is hydrogen or methyl. More preferably than the foregoing, L^{2d} is selected from the group consisting of -C(O)NL^{2T}-, and -NL^{2T}C(O)-. More preferably than the foregoing, L^{2d} is selected from the group consisting of -C(O)NH-, and -NHC(O)-. Most preferably, L^{2d} is -C(O)NH-.

### T¹

T¹ is according to Radical Group 1, Radical Group 3, Radical Group 4, or Radical Group 5, as defined herein. Preferably, each T¹ is independently according to Radical Group 1 as defined herein.

More preferably, each T¹ is independently selected from the group consisting of -OT^{1A}, hydrogen, C₁-C₁₂ (hetero)alkyl, C₆ aryl, C₄-C₅ heteroaryl, C₃-C₆ (hetero)cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃. Even more preferably, each T¹ is independently selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, - ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃. Yet more preferably, each T¹ is independently selected from the group consisting of -OT^{1A}, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, - O-C(O)T^{1A} -C(O)N(T^{1A})₂, -N(T^{1A})₂-CO-T^{1A}, and -Si(T^{1A})₃. More preferably still, T¹ is -OT^{1A}.

Most preferably, T¹ is -OH.

As used herein, each T^{1A} is independently selected from the group consisting of hydrogen, (hetero)alkyl, (hetero)alkenyl, (hetero)alkynyl, (hetero)aryl, and an amino acid residue. More preferably, each T^{1A} is independently selected from the group consisting of hydrogen, C₁-C₆ (hetero)alkyl, C₁-C₆ (hetero)alkenyl, C₁-C₆ (hetero)alkynyl, C₂-C₅ heteroaryl, phenyl, and an amino acid residue. Even more preferably, each T^{1A} is independently selected from the group consisting of hydrogen, C₁-C₄ (hetero)alkyl, C₁-C₄ (hetero)alkenyl, C₁-C₄ (hetero)alkynyl, C₃-C₅ heteroaryl, phenyl, an aspartic acid residue, a glutamic acid residue, and a glycine residue. Even more preferably, each T^{1A} is independently selected from the group consisting of hydrogen, C₁-C₃ alkyl, an aspartic acid residue, a glutamic acid residue, and a glycine residue. Most preferably, T^{1A} is hydrogen.

Preferably, T¹ is in an axial position. Without wishing to be bound by theory, the inventors believe that in that case and when R₄₈ is a releasable group, when the compound of the disclosure reacts with a diene, T¹ aids in releasing the payload. This results in optimal release yields and/or release kinetics.

### T²

T² is an organic moiety. Preferably, T² is according to any one of Radical Group 1, Radical Group 3, or Radical Group 5, as defined herein, or wherein T² is a group -L³-C^{B}. More preferably, T² is a bioconjugation moiety, a residue of a bioconjugation moiety, or a group
-L³-C^{B}. More preferably, T² is a bioconjugation moiety, or a group -L³-C^{B}.

In preferred embodiments, T² is a bioconjugation moiety. These embodiments typically relate to compounds that can be coupled to *e.g.* a protein. More preferably, T² is according to Radical Group 1f as defined herein. Residues of these bioconjugation moieties are known in the art. More preferably, T² is N-maleimidyl. In these embodiments, it is most preferred that T² is:

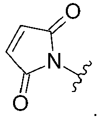

In other preferred embodiments, T² is a residue of a bioconjugation moiety. These embodiments typically relate to conjugates of the disclosure, wherein T² links to *e.g.* a protein. Such residues are well-known to the skilled person. In these embodiments, it is most preferred that T² is: 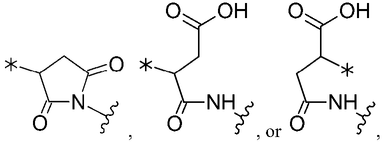 wherein the asterisk indicates a bond to the protein, and the wiggly line denotes a bond to the rest of the compound of the disclosure.

In other preferred embodiments, T² is a group -L³-C^{B}. These embodiments relate to when T² itself comprises a Construct B (C^{B}), which is usually a protein. C^{B} is as defined herein. L³ is according to Radical Group 2. Preferably, L³ is a residue of a bioconjugation moiety. More preferably, L³ is a residue of an N-maleimidyl moiety or a residue of an N-hydroxy-succinimidyl moiety. In these embodiments, it is preferred that T² is selected from the group consisting of 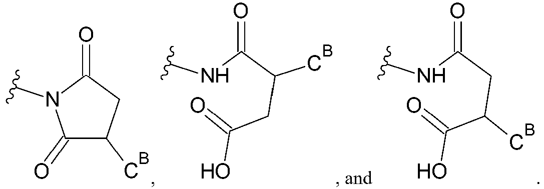 In these embodiments, it is most preferred that T² is:

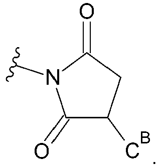

For the moiety -L³-C^{B}, it is preferred that L³ and a sulfur atom, secondary nitrogen atom, or tertiary nitrogen atom, preferably a sulfur atom, of C^{B} together form any one of the following structures -L³-C^{B}. 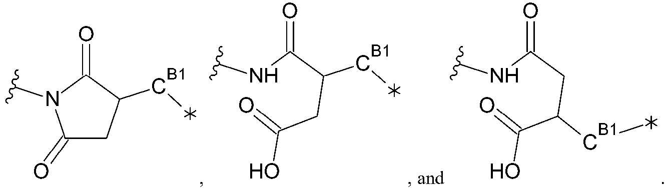 wherein C^{B1} indicates S, secondary N, or tertiary N that is part of C^{B}, preferably S; the wiggly lines indicates a bond to moiety L¹, and the asterisk indicates a bond to the remainder of C^{B}, preferably AVP0458.

### C^{B}

C^{B} is according to Radical Group 4 or Radical Group 5, as defined herein. Preferably, C^{B} is a targeting agent as defined herein. Preferably, C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof. More preferably, C^{B} is a protein. Even more preferably, C^{B} is an antibody or a diabody. More preferably still, C^{B} is a diabody.

An antibody is a protein generated by the immune system that is capable of recognizing and binding to a specific antigen. While antibodies or immunoglobulins derived from IgG antibodies are particularly well-suited for use in this disclosure, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)₂, Fab', Fab'-SH, F(ab')₂, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), chimeric antibodies, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Canc. Gen. Prot. 2013 10, 1-18], and [BioDrugs 2014, 28, 331-343], the contents of which are hereby incorporated by reference. "Antibody fragment" refers to at least a portion of the variable region of the immunoglobulin that binds to its target, i.e. the antigen-binding region. Other embodiments use antibody mimetics as Drug D^{D} or Targeting Agent T^{T}, such as but not limited to Affimers, Anticalins, Avimers, Alphabodies, Affibodies, DARPins, and multimers and derivatives thereof; reference is made to [Trends in Biotechnology 2015, 33, 2, 65], the contents of which is hereby incorporated by reference. For the avoidance of doubt, in the context of this disclosure the term "antibody" is meant to encompass all of the antibody variations, fragments, derivatives, fusions, analogs and mimetics outlined in this paragraph, unless specified otherwise.

Preferably, an antibody is selected from the group consisting of AVP0458, CC49, 3F8, abagovomab, abciximab, abituzumab, abrezekimab, abrilumab, actoxumab, adalimumab, adecatumumab, aducanumab, afasevikumab, afelimomab, alacizumab pegol, alemtuzumab, alirocumab, altumomab pentetate, amatuximab, amivantamab, anatumomab mafenatox, andecaliximab, anetumab ravtansine, anifrolumab, ansuvimab, anrukinzumab, apolizumab, aprutumab ixadotin, arcitumomab, ascrinvacumab, aselizumab, atezolizumab, atidortoxumab, atinumab, atoltivimab, atoltivimab, maftivimab, odesivimab, atorolimumab, avelumab, azintuxizumab vedotin, bamlanivimab, bapineuzumab, basiliximab, bavituximab, BCD-100, bebtelovimab, bectumomab, bedinvetmab, begelomab, belantamab mafodotin, belimumab, bemarituzumab, benralizumab, berlimatoxumab, bermekimab, bersanlimab, bertilimumab, besilesomab, bevacizumab, bezlotoxumab, biciromab, bimagrumab, bimekizumab, birtamimab, bivatuzumab, bleselumab, blinatumomab, blontuvetmab, blosozumab, bococizumab, brazikumab, brentuximab vedotin, briakinumab, brodalumab, brolucizumab, brontictuzumab, burosumab, cabiralizumab, camidanlumab tesirine, camrelizumab, canakinumab, cantuzumab mertansine, cantuzumab ravtansine, caplacizumab, casirivimab, capromab, carlumab, carotuximab, catumaxomab, cBR96-doxorubicin immunoconjugate, cedelizumab, cemiplimab, cergutuzumab amunaleukin, certolizumab pegol, cetrelimab, cetuximab, cibisatamab, cilgavimab, cirmtuzumab, citatuzumab bogatox, cixutumumab, clazakizumab, clenoliximab, clivatuzumab tetraxetan, codrituzumab, cofetuzumab pelidotin, coltuximab ravtansine, conatumumab, concizumab, cosfroviximab, crenezumab, crizanlizumab, crotedumab, CR6261, cusatuzumab, dacetuzumab, daclizumab, dalotuzumab, dapirolizumab pegol, daratumumab, dectrekumab, demcizumab, denintuzumab mafodotin, denosumab, depatuxizumab mafodotin, derlotuximab biotin, detumomab, dezamizumab, dinutuximab, dinutuximab beta, diridavumab, divozilimab, domagrozumab, donanemab, dorlimomab aritox, dostarlimab, drozitumab, DS-8201, duligotuzumab, dupilumab, durvalumab, dusigitumab, duvortuxizumab, ecromeximab, eculizumab, edobacomab, edrecolomab, efalizumab, efungumab, eldelumab, elezanumab, elgemtumab, elotuzumab, elsilimomab, emactuzumab, emapalumab, emibetuzumab, emicizumab, enapotamab vedotin, enavatuzumab, enfortumab vedotin, enlimomab pegol, enoblituzumab, enokizumab, enoticumab, ensituximab, epcoritamab, epitumomab cituxetan, epratuzumab, eptinezumab, erenumab, erlizumab, ertumaxomab, etaracizumab, etesevimab, etigilimab, etrolizumab, evinacumab, evolocumab, exbivirumab, fanolesomab, faralimomab, faricimab, farletuzumab, fasinumab, FBTA05, felvizumab, fezakinumab, fibatuzumab, ficlatuzumab, figitumumab, firivumab, flanvotumab, fletikumab, flotetuzumab, fontolizumab, foralumab, foravirumab, fremanezumab, fresolimumab, frovocimab, frunevetmab, fulranumab, futuximab, galcanezumab, galiximab, gancotamab, ganitumab, gantenerumab, gatipotuzumab, gavilimomab, gedivumab, gemtuzumab ozogamicin, gevokizumab, gilvetmab, gimsilumab, girentuximab, glembatumumab vedotin, glofitamab, golimumab, gomiliximab, gosuranemab, guselkumab, ianalumab, ibalizumab, sintilimab, ibritumomab tiuxetan, icrucumab, idarucizumab, ifabotuzumab, igovomab, iladatuzumab vedotin, imalumab, imaprelimab, imciromab, imdevimab, imgatuzumab, inclacumab, indatuximab ravtansine, indusatumab vedotin, inebilizumab, infliximab, intetumumab, inolimomab, inotuzumab ozogamicin, ipilimumab, iomab-B, iratumumab, isatuximab, iscalimab, istiratumab, itolizumab, ixekizumab, keliximab, labetuzumab, lacnotuzumab, ladiratuzumab vedotin, lampalizumab, lanadelumab, landogrozumab, laprituximab emtansine, larcaviximab, lebrikizumab, lecanemab, lemalesomab, lendalizumab, lenvervimab, lenzilumab, lerdelimumab, leronlimab, lesofavumab, letolizumab, lexatumumab, libivirumab, lifastuzumab vedotin, ligelizumab, loncastuximab tesirine, losatuxizumab vedotin, lilotomab satetraxetan, lintuzumab, lirilumab, lodelcizumab, lokivetmab, lorvotuzumab mertansine, lucatumumab, lulizumab pegol, lumiliximab, lumretuzumab, lupartumab, lupariumab amadotin, lutikizumab, maftivimab, mapatumumab, margetuximab, marstacimab, maslimomab, mavrilimumab, matuzumab, mepolizumab, metelimumab, milatuzumab, minretumomab, mirikizumab, mirvetuximab soravtansine, mitumomab, modotuximab, mogamulizumab, monalizumab, morolimumab, mosunetuzumab, motavizumab, moxetumomab pasudotox, muromonab-CD3, nacolomab tafenatox, namilumab, naptumomab estafenatox, naratuximab emtansine, namatumab, natalizumab, navicixizumab, navivumab, naxitamab, nebacumab, necitumumab, nemolizumab, NEOD001, nerelimomab, nesvacumab, netakimab, nimotuzumab, nirsevimab, nivolumab, nofetumomab merpentan, obiltoxaximab, obinutuzumab, ocaratuzumab, ocrelizumab, odesivimab, odulimomab, ofatumumab, olaratumab, oleclumab, olendalizumab, olokizumab, omalizumab, omburtamab, OMS721, onartuzumab, ontuxizumab, onvatilimab, opicinumab, oportuzumab monatox, oregovomab, orticumab, otelixizumab, otilimab, otlertuzumab, oxelumab, ozanezumab, ozoralizumab, pagibaximab, palivizumab, pamrevlumab, panitumumab, pankomab, panobacumab, parsatuzumab, pascolizumab, pasotuxizumab, pateclizumab, patritumab, PDR001, pembrolizumab, pemtumomab, perakizumab, pertuzumab, pexelizumab, pidilizumab, pinatuzumab vedotin, pintumomab, placulumab, pozelimab, prezalumab, plozalizumab, pogalizumab, polatuzumab vedotin, ponezumab, porgaviximab, prasinezumab, prezalizumab, priliximab, pritoxaximab, pritumumab, PRO 140, quilizumab, racotumomab, radretumab, rafivirumab, ralpancizumab, ramucirumab, ranevetmab, ranibizumab, raxibacumab, ravagalimab, ravulizumab, refanezumab, regavirumab, regdanvimab, relatlimab, remtolumab, reslizumab, retifanlimab, rilotumumab, rinucumab, risankizumab, rituximab, rivabazumab pegol, robatumumab, Rmab, roledumab, romilkimab, romosozumab, rontalizumab, rosmantuzumab, rovalpituzumab tesirine, rovelizumab, rozanolixizumab, ruplizumab, SA237, sacituzumab govitecan, samalizumab, samrotamab vedotin, sarilumab, satralizumab, satumomab pendetide, secukinumab, selicrelumab, seribantumab, setoxaximab, setrusumab, sevirumab, sibrotuzumab, SGN-CD19A, SHP647, sifalimumab, siltuximab, simtuzumab, siplizumab, sirtratumab vedotin, sirakumab, sofituzumab vedotin, solanezumab, solitomab, sonepcizumab, sontuzumab, sotrovimab, spartalizumab, spesolimab, stamulumab, sulesomab, suptavumab, sutimlimab, suvizumab, suvratoxumab, tabalumab, tacatuzumab tetraxetan, tadocizumab, tafasitamab, talacotuzumab, talizumab, talquetamab, tamtuvetmab, tanezumab, taplitumomab paptox, tarextumab, tavolimab, teclistamab, tefibazumab, telimomab aritox, telisotuzumab, telisotuzumab vedotin, tenatumomab, teneliximab, teplizumab, tepoditamab, teprotumumab, tesidolumab, tetulomab, tezepelumab, TGN1412, tibulizumab, tildrakizumab, tigatuzumab, timigutuzumab, timolumab, tiragol, umab, tiragotumab, tislelizumab, tisotumab vedotin, tixagevimab, TNX-650, tocilizumab, tomuzotuximab, toralizumab, tosatoxumab, tositumomab, tovetumab, tralokinumab, trastuzumab, trastuzumab duocarmazine, trastuzumab emtansine, TRBS07, tregalizumab, tremelimumab, trevogrumab, tucotuzumab celmoleukin, tuvirumab, ublituximab, ulocuplumab, urelumab, urtoxazumab, ustekinumab, utomilumab, vadastuximab talirine, vanalimab, vandoriuzumab vedotin, vantictumab, vanucizumab, vapaliximab, varisacumab, varlilumab, vatelizumab, vedolizumab, veltuzumab, vepalimomab, vesencumab, vilobelimab, visilizumab, vobarilizumab, volociximab, vonlerolizumab, vopratelimab, vorsetuzumab mafodotin, votumumab, vunakizumab, xentuzumab, XMAB-5574, zalutumumab, zanolimumab, zatuximab, zenocutuzumab, ziralimumab, zolbetuximab, and zolimomab aritox.

Preferably, C^{B} is selected from the group consisting of AVP0458, CC49, insulin, transferrin, fibrinogen-gamma fragment, thrombospondin, claudin, apolipoprotein E, Affibody molecules such as for example ABY-025, Ankyrin repeat proteins, ankyrin-like repeat proteins, interferons, e.g. alpha, beta, and gamma interferon, interleukins, lymphokines, colony stimulating factors and protein growth factor, such as tumor growth factor, e.g. alpha, beta tumor growth factor, platelet-derived growth factor (PDGF), uPAR targeting protein, apolipoprotein, LDL, annexin V, endostatin, and angiostatin. Examples of peptides as targeting agents include LHRH receptor targeting peptides, EC-1 peptide, RGD peptides, HER2-targeting peptides, PSMA targeting peptides, somatostatin-targeting peptides, bombesin. Other examples of targeting agents include lipocalins, such as anticalins. One particular embodiment uses Affibodies^{™} and multimers and derivatives.

More preferably, C^{B} is AVP0458 or CC49. Most preferably, C^{B} is AVP0458 consisting of two monomers, wherein each of the two monomers has an amino acid sequence according to SEQ ID NO: 1.

Preferably, C^{B} is linked to the remainder of the compound of the disclosure or the conjugate of the disclosure via S or N that is part of C^{B}. More preferably, C^{B} is linked to the remainder of the compound of the disclosure or the conjugate of the disclosure via S that is part of C^{B}.

### AVP0458

As used herein, AVP0458 refers to a TAG72-binding diabody derived from the CC49 antibody. AVP0458 is a diabody consisting of two monomers, each monomer having an amino acid sequence according to SEQ ID NO: 1: 
SEQ ID NO:1 (amino acid sequence of AVP0458 diabody monomer):
SVQLQQSDAELVKPGASVKISCKASGYTFTDHAIHWVKQNPEQGLEWIGYFSPGNDDFKYNERFKGKATLTADKSS
STAYLQLNSLTSEDSAVYFCTRSLNMAYWGQGTSVTVSSGGGGSDIVMTQSCSSCPVSVGEKVTLSCKSSQSLLYS
GNQKNYLAWYQQKPGQSPKLLIYWASTRESGVPDRFTGSGSGTDFTLSISSVETEDLAVYYCQQYYSYPLTFGAGT
KLVLKR
 Herein, the underlining indicates the cysteines that are preferably modified with or linked to a compound of the disclosure or the remainder thereof if AVP0458 is itself part of the compound of the disclosure.

Thus, in SEQ ID NO: 1 it is preferred that at least one of the underligned cysteines, more preferably both underlined cysteines, is modified with or linked to a compound according to the disclosure. In other words: it is preferred that the sulfur atom of the underlined cysteines is coupled to a moiety T² as defined herein, preferably T² is the residue of an N-maleimidyl group.

### Targeting Agent

A Targeting Agent, T^{T}, binds to a Primary Target. A "primary target" as used in the present disclosure can be any molecule, which is present in an organism, tissue or cell. Preferably, a "primary target" relates to a target for a targeting agent for therapy, imaging, theranostics, diagnostics, or in vitro studies.

In order to allow specific targeting of the above-listed Primary Targets, the Targeting Agent T^{T} can comprise compounds including but not limited to antibodies, antibody derivatives, antibody fragments, antibody (fragment) fusions (e.g. bi-specific and tri-specific mAb fragments or derivatives), proteins, peptides, e.g. octreotide and derivatives, VIP, MSH, LHRH, chemotactic peptides, cell penetrating peptide, membrane translocation moiety, bombesin, elastin, peptide mimetics, organic compounds, inorganic compounds, carbohydrates, monosaccharides, oligosacharides, polysaccharides, oligonucleotides, aptamers, viruses, whole cells, phage, drugs, polymers, liposomes, chemotherapeutic agents, receptor agonists and antagonists, cytokines, hormones, steroids, toxins. Examples of organic compounds envisaged within the context of the present disclosure are, or are derived from, dyes, compounds targeting CAIX and PSMA, estrogens, e.g. estradiol, androgens, progestins, corticosteroids, methotrexate, folic acid, and cholesterol. Examples of Targeting Agents of protein nature include insulin, transferrin, fibrinogen-gamma fragment, thrombospondin, claudin, apolipoprotein E, Affibody molecules such as for example ABY-025, Ankyrin repeat proteins, ankyrin-like repeat proteins, interferons, e.g. alpha, beta, and gamma interferon, interleukins, lymphokines, colony stimulating factors and protein growth factor, such as tumor growth factor, e.g. alpha, beta tumor growth factor, platelet-derived growth factor (PDGF), uPAR targeting protein, apolipoprotein, LDL, annexin V, endostatin, and angiostatin. Examples of peptides as targeting agents include LHRH receptor targeting peptides, EC-1 peptide, RGD peptides, HER2-targeting peptides, PSMA targeting peptides, somatostatin-targeting peptides, bombesin. Other examples of targeting agents include lipocalins, such as anticalins. One particular embodiment uses Affibodies^{™} and multimers and derivatives.

In one embodiment antibodies are used as the T^{T}. While antibodies or immunoglobulins derived from IgG antibodies are particularly well-suited for use in this disclosure, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)₂, Fab', Fab'-SH, F(ab')₂, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), chimeric antibodies, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Canc. Gen. Prot. 2013 10, 1-18], and [BioDrugs 2014, 28, 331-343], the contents of which are hereby incorporated by reference. "Antibody fragment" refers to at least a portion of the variable region of the immunoglobulin that binds to its target, i.e. the antigen-binding region. Other embodiments use antibody mimetics as T^{T}, such as but not limited to Affimers, Anticalins, Avimers, Alphabodies, Affibodies, DARPins, and multimers and derivatives thereof; reference is made to [Trends in Biotechnology 2015, 33, 2, 65], the contents of which is hereby incorporated by reference. For the avoidance of doubt, in the context of this disclosure the term "antibody" is meant to encompass all of the antibody variations, fragments, derivatives, fusions, analogs and mimetics outlined in this paragraph, unless specified otherwise.

Preferably the T^{T} is selected from antibodies and antibody derivatives such as antibody fragments, fragment fusions, proteins, peptides, peptide mimetics, organic molecules, dyes, fluorescent molecules, enzyme substrates.

Preferably the T^{T} being an organic molecule has a molecular weight of less than 2000 Da, more preferably less than 1500 Da, more preferably less than 1000 Da, even more preferably less than 500 Da.

In another preferred embodiment the T^{T} is selected from antibody fragments, fragment fusions, and other antibody derivatives that do not contain a Fc domain.

In another embodiment the T^{T} is a polymer and accumulates at the Primary Target by virtue of the EPR effect. Typical polymers used in this embodiment include but are not limited to polyethyleneglycol (PEG), poly(N-(2-hydroxypropyl)methacrylamide) (HPMA), polylactic acid (PLA), polylactic-glycolic acid (PLGA), polyglutamic acid (PG), polyvinylpyrrolidone (PVP), poly(1-hydroxymethylethylene hydroxymethyl-formal (PHF). Other examples are copolymers of a polyacetal/polyketal and a hydrophilic polymer selected from the group consisting of polyacrylates, polyvinyl polymers, polyesters, polyorthoesters, polyamides, oligopeptides, polypeptides and derivatives thereof. Other examples are oligopeptides, polypeptides, glycopolysaccharides, and polysaccharides such as dextran and hyaluronan. In addition reference is made to [G. Pasut, F.M. Veronese, Prog. Polym. Sci. 2007, 32, 933-961]. In some embodiments the T^{T} can be a cell penetrating moiety, such as cell penetrating peptide. In other embodiments, the T^{T} is a polymer, particle, gel, biomolecule or another above listed T^{T} moiety and is locally injected to create a local depot of Prodrug, which can subsequently be activated by the Activator. In another embodiment the targeting agent T^{T} is a solid material such as but not limited to polymer, metal, ceramic, wherein this solid material is or is comprised in a cartridge, reservoir, depot, wherein preferably said cartridge, reservoir, depot is used for drug release in vivo. In some embodiments, the targeting agent T^{T} also acts as a Drug, which may be denoted as D^{D}.

### T³

T³ is an organic moiety. Preferably, T³ is according to any one of Radical Group 1, Radical Group 3, or Radical Group 5, as defined herein. More preferably, T³ is according to Radical Group 3, as defined herein. Even more preferably, T³ is a polymer. More preferably still, T³ is a polymer comprising a polyethylene glycol moiety.

More preferably, T³ comprises a moiety -(CH₂CH₂-O-)_{y}-T⁴. Herein, y is an integer in a range of from 1 to 50, preferably y is an integer in a range of from 2 to 45, more preferably y is an integer in a range of from 10 to 40, more preferably in a range of from 12 to 37, even more preferably in a range of from 15 to 35, more preferably still in a range of from 20 to 30, even more preferably in a range of from 23 to 25, and most preferably y is 24. This definition and these preferences for y also apply to compounds of Formula (2), Formula (3), Formula (G), Formula (O), Formula (P), and Formula (Q), wherein y is used as well.

T⁴ is according to Radical Group 1, Radical Group 3, Radical Group 4, or Radical Group 5 as defined herein. Preferably, T⁴ is according to Radical Group 1. More preferably, T⁴ is according to Radical Group 1a. More preferably, T⁴ is according to Radical Group 1b. More preferably, T⁴ is according to Radical Group 1c. More preferably, T⁴ is according to Radical Group 1d. Even more preferably, T⁴ is according to Radical Group 1e. Most preferably, T⁴ is methyl.

Even more preferably, T³ is a moiety -(CH₂CH₂-O-)_{y}-T⁴. Most preferably, T³ is a moiety -(CH₂CH₂-O-)₂₄-CH₃.

### Variables of Formula (B)

In Formula (B), R₄₈ and T¹ are as defined herein. In Formula (B), TL is a structure according to Formula (A) as defined in any one of Clauses 1-128, and preferably TL is as defined in any one of Clauses 216-227.

In Formula (B), y1 is an integer of from 0 to 4, preferably an integer of from 1 to 2, most preferably y1 is 1.

In Formula (B), y2 is an integer of from 0 to 5, preferably an integer of from 1 to 4, more preferably an integer of from 1 to 3, even more preferably an integer of from 1 to 2, and most preferably y2 is 1.

In Formula (B), y3 is an integer of from 1 to 5, preferably an integer of from 1 to 4, more preferably an integer of from 1 to 3, even more preferably an integer of from 1 to 2, and most preferably y3 is 1..

In Formula (B), each of X¹, X², X³, X⁴, X⁵, and X⁶ is independently selected from the group consisting of a substituted or unsubstituted carbon atom, a nitrogen atom, or an oxygen atom, provided that if one of X¹, X², X³, X⁴, X⁵, and X⁶ is a nitrogen atom or an oxygen atom, an adjacent X¹, X², X³, X⁴, X⁵, and X⁶ is not a nitrogen atom or an oxygen atom.

Preferably, each of X¹, X², X³, X⁴, X⁵, and X⁶ is independently a substituted or unsubstituted carbon atom. More preferably, X¹ and/or X⁶ are independently a carbon atom substituted with R₄₈. Even more preferably, X¹ is a carbon atom substituted with R₄₈, and most preferably, X¹ is -CHR₄₈-. More preferably still, X¹ is -CHR₄₈-, and X⁴ is -CT¹TL-. Preferably, X², X³, X⁵, and X⁶ are unsubstituted carbon atoms, more preferably -CH₂-.

### Variable x in Formulae (3), (O), (P), and (Q)

In Formula (3), Formula (O), Formula (P), and Formula (Q), x is an integer in a range of from 4 to 12; preferably x is an integer in a range of from 4 to 8, more preferably x is an integer in a range of from 4 to 6, and most preferably x is 5.

### R₄₈

R₄₈ is selected from the group consisting of -OH, -O-acetyl, -O-C₁₋₄ alkyl, halogen, active carbonate, and a releasable group.

Preferably, R₄₈ is a substituent on an allylic carbon of a compound of the disclosure.

Preferably, R₄₈ is in the axial position. This preference holds especially when R₄₈ is a releasable group. Having the releasable group in an axial position results in better release of the payload as compared to having the releasable group in an equatorial position.

Preferably, group R₄₈ is a releasable group. Such releasable groups are well-known and have a clear meaning in the art. Especially in the context of click-to-release reactions, as in the present disclosure, the skilled person would immediately recognize that a releasable group on the allylic carbon of a *trans-*cyclooctene (viz. R₄₈ in Formula (1)) refers to a group that may be released from the *trans-*cyclooctene upon contacting the *trans-*cyclooctene with an activator such as a diene.

In preferred embodiments, the releasable group is -(Y¹-C(=Y²))ᵢ-(S^{P})ⱼ-C^{A}. Therein, each of Y¹ and Y² are independently selected from O, and S; preferably Y¹ and Y² are O.
For the releasable group, j is 0 or 1; preferably j is 0; and i is 0 or 1; preferably i is 1. If i is 0, -(S^{P})ⱼ-C^{A} is connected to the remainder of the compound via O or S, that is part of -(S^{P})ⱼ-C^{A}. On the other hand, if i is 1, -(S^{P})ⱼ-C^{A} is connected to -C(=Y²)-via O, S, a secondary N, or a tertiary N, that is part of -(S^{P})ⱼ-C^{A}. Preferably, if i is 1, -(S^{P})ⱼ-C^{A} is connected to -C(=Y²)- via a secondary N, or a tertiary N, that is part of -(S^{P})ⱼ-C^{A}.

More preferably, the releasable group is -(O-C(=Y²))ᵢ-(S^{P})ⱼ-C^{A}. More preferably, the
releasable group is -(Y¹-C(-O))ᵢ-(S^{P})ⱼ-C^{A}. More preferably, the releasable group is -(O-C(-O))ᵢ-(S^{P})ⱼ-C^{A}. More preferably, the releasable group is -O-C(-O)-(S^{P})ⱼ-C^{A}.

Most preferably, the releasable group is -O-C(=O)-C^{A}.

C^{A} is Construct A, which is a payload. Preferably, C^{A} is an organic molecule or an inorganic molecule. More preferably, C^{A} is a drug. Preferably, C^{A} is monomethyl auristatin E (MMAE), exatecan, or an exatecan derivative. Most preferably, C^{A} is monomethyl auristatin E (MMAE).

Preferably, C^{A} is linked to the moiety -(Y¹-C(=Y²))ᵢ-, preferably -O-C(=O)-, via a secondary or tertiary nitrogen atom that is part of C^{A}, forming a carbamate. Preferably, C^{A} is monomethyl auristatin E (MMAE) linked to the moiety -(Y¹-C(=Y²))ᵢ-, preferably -O-C(=O)-, via a secondary or tertiary nitrogen atom that is part of MMAE, forming a carbamate; or C^{A} is exatecan or an exatecan derivative linked to the moiety -(Y¹-C(=Y²))ᵢ-, preferably -O-C(=O)-, via a primary or secondary nitrogen atom that is part of exatecan, forming a carbamate.More preferably, C^{A} is monomethyl auristatin E (MMAE) linked to the moiety -(Y¹-C(=Y²))ᵢ-, preferably -O-C(=O)-, via a secondary or tertiary nitrogen atom that is part of MMAE, forming a carbamate.

Preferably, group R₄₈ is: 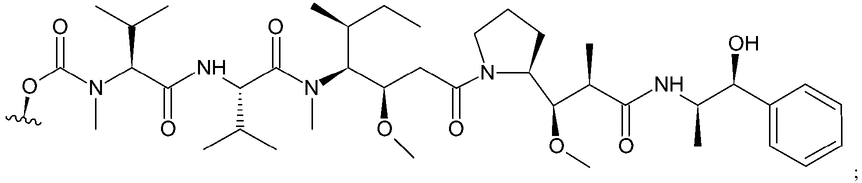 or 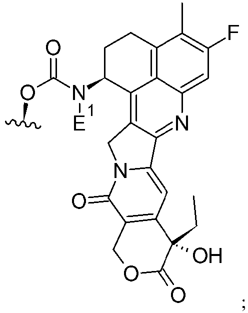 wherein E¹ is -H or -CH₃, preferably E¹ is -H.

Most preferably, group R₄₈ is:

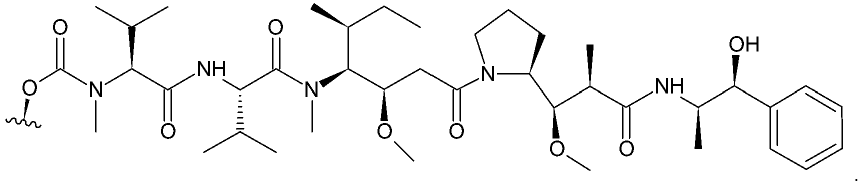

S^{P} is a spacer, of which preferred embodiments are defined below. Preferably, when S^{P} is part of a releasable group, S^{P} is a self-immolative linker, which is herein also referred to as L^{C}. Such self-immolative linkers are well-known in the art, and preferred embodiments of self-immolative linkers are defined below. If the spacer in the releasable group is a self-immolative linker, upon reaction of a compound of the disclosure with a diene, initially a construct -L^{C}-C^{A} is released. Thereafter, the self-immolative linker self-immolates and releases the payload C^{A}.

### Drugs

Drugs that can be used in a compound of Formula (1) are pharmaceutically active compounds. Preferably the pharmaceutically active compound is selected from the group consisting of cytotoxins, antiproliferative/antitumor agents, antiviral agents, antibiotics, anti-inflammatory agents, chemosensitizing agents, radio sensitizing agents, immunomodulators, immunosuppressants, immunostimulants, anti-angiogenic factors, and enzyme inhibitors. Preferably these pharmaceutically active compounds are selected from the group consisting of antibodies, antibody derivatives, antibody fragments, proteins, aptamers, oligopeptides, oligonucleotides, oligo saccharides, carbohydrates, as well as peptides, peptoids, steroids, toxins, hormones, cytokines, and chemokines. Most preferably, the drug is a protein, a toxin, a chelating moiety, monomethyl auristatin E, or doxorubicin; wherein preferably the chelating moiety comprises a radionuclide. Preferably these drugs are low to medium molecular weight compounds, preferably organic compounds (e.g. about 200 to about 2500 Da, preferably about 300 to about 1750 Da, more preferably about 300 to about 1000 Da). Exemplary cytotoxic drug types for use as conjugates to the Trigger and to be released upon IEDDA reaction with the Activator, for example for use in cancer therapy, include but are not limited to DNA damaging agents, DNA crosslinkers, DNA binders, DNA alkylators, DNA intercalators, DNA cleavers, microtubule stabilizing and destabilizing agents, topoisomerases inhibitors, radiation sensitizers, antimetabolites, natural products and their analogs, peptides, oligonucleotides, enzyme inhibitors such as dihydrofolate reductase inhibitors and thymidylate synthase inhibitors. Examples include but are not limited to colchinine, vinca alkaloids, anthracyclines (e.g. doxorubicin, epirubicin, idarubicin, daunorubicin), camptothecins, taxanes, taxols, vinblastine, vincristine, vindesine, calicheamycins, tubulysins, tubulysin M, cryptophycins, methotrexate, methopterin, aminopterin, dichloromethotrexate, irinotecans, enediynes, amanitins, deBouganin, dactinomycines, CC1065 and its analogs, duocarmycins, maytansines, maytansinoids, dolastatins, auristatins, pyrrolobenzodiazepines and dimers (PBDs), indolinobenzodiazepines and dimers, pyridinobenzodiazepines and dimers, mitomycins (e.g. mitomycin C, mitomycin A, caminomycin), melphalan, leurosine, leurosideine, actinomycin, tallysomycin, lexitropsins, bleomycins, podophyllotoxins, etoposide, etoposide phosphate, staurosporin, esperamicin, the pteridine family of drugs, SN-38 and its analogs, platinum-based drugs, cytotoxic nucleosides. Other exemplary drug classes are angiogenesis inhibitors, cell cycle progression inhibitors, P13K/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, and RNA polymerase inhibitors. In some embodiments, the drug is an auristatin. Examples of auristatins include dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ. MMAE is a preferred auristatin. Suitable auristatins are also described in U.S. Publication Nos. 2003/0083263, 2011/0020343, and 2011/0070248; PCT Application Publication Nos. WO09/117531, WO2005/081711, WO04/010957; WO02/088172 and WO01/24763, and U.S. Patent Nos. 7,498,298; 6,884,869; 6,323,315; 6,239,104; 6,124,431; 6,034,065; 5,780,588; 5,767,237; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,879,278; 4,816,444; and 4,486,414, the disclosures of which are incorporated herein by reference in their entirety. Exemplary drugs include the dolastatins and analogues thereof including: dolastatin A ( U.S. Pat No. 4,486,414), dolastatin B (U.S. Pat No. 4,486,414), dolastatin 10 (U.S. Pat No. 4,486,444, 5,410,024, 5,504,191, 5,521,284, 5,530,097, 5,599,902, 5,635,483, 5,663,149, 5,665,860, 5,780,588, 6,034,065, 6,323,315), dolastatin 13 (U.S. Pat No. 4,986,988), dolastatin 14 (U.S. Pat No. 5,138,036), dolastatin 15 (U.S. Pat No. 4,879,278), dolastatin 16 (U.S. Pat No. 6,239,104), dolastatin 17 (U.S. Pat No. 6,239,104), and dolastatin 18 (U.S. Pat No. 6,239,104), each patent incorporated herein by reference in their entirety. Exemplary maytansines, maytansinoids, such as DM-1 and DM-4, or maytansinoid analogs, including maytansinol and maytansinol analogs, are described in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 5,208,020; 5,416,064; 5,475,092; 5,585,499; 5,846,545; 6,333,410; 6,441,163; 6,716,821 and 7,276,497. Other examples include mertansine and ansamitocin. Pyrrolobenzodiazepines (PBDs), which expressly include dimers and analogs, include but are not limited to those described in [Denny, Exp. Opin. Ther. Patents, 10(4):459-474 (2000)], [Hartley et al., Expert Opin Investig Drugs. 2011, 20(6):733-44], Antonow et al., Chem Rev. 2011, 111(4), 2815-64]. Calicheamicins include, e.g. enediynes, esperamicin, and those described in U.S. Patent Nos. 5,714,586 and 5,739,116. Examples of duocarmycins and analogs include CC1065, duocarmycin SA, duocarmycin A, duocarmycin B1, duocarmycin B2, duocarmycin C1, duocarmycin C2, duocarmycin D, DU-86, KW-2189, adozelesin, bizelesin, carzelesin, seco-adozelesin, CPI, CBI. Other examples include those described in, for example, US Patent No. 5,070,092; 5,101,092; 5,187,186; 5,475,092; 5,595,499; 5,846,545; 6,534,660; 6,548,530; 6,586,618; 6,660,742; 6,756,397; 7,049,316; 7,553,816; 8,815,226; US20150104407; 61/988,011 filed may 2, 2014 and 62/010,972 filed June 11, 2014; the disclosure of each of which is incorporated herein in its entirety. Exemplary vinca alkaloids include vincristine, vinblastine, vindesine, and navelbine, and those disclosed in U.S. Publication Nos. 2002/0103136 and 2010/0305149, and in U.S. Patent No. 7,303,749, the disclosures of which are incorporated herein by reference in their entirety. Exemplary epothilone compounds include epothilone A, B, C, D, E, and F, and derivatives thereof. Suitable epothilone compounds and derivatives thereof are described, for example, in U.S. Patent Nos. 6,956,036; 6,989,450; 6,121,029; 6,117,659; 6,096,757; 6,043,372; 5,969,145; and 5,886,026; and WO97/19086; WO98/08849; WO98/22461; WO98/25929; WO98/38192; WO99/01124; WO99/02514; WO99/03848; WO99/07692; WO99/27890; and WO99/28324; the disclosures of which are incorporated herein by reference in their entirety. Exemplary cryptophycin compounds are described in U.S. Patent Nos. 6,680,311 and 6,747,021; the disclosures of which are incorporated herein by reference in their entirety. Exemplary platinum compounds include cisplatin, carboplatin, oxaliplatin, iproplatin, ormaplatin, tetraplatin. Exemplary DNA binding or alkylating drugs include CC-1065 and its analogs, anthracyclines, calicheamicins, dactinomycines, mitromycines, pyrrolobenzodiazepines, indolinobenzodiazepines, pyridinobenzodiazepines and the like. Exemplary microtubule stabilizing and destabilizing agents include taxane compounds, such as paclitaxel, docetaxel, tesetaxel, and carbazitaxel; maytansinoids, auristatins and analogs thereof, vinca alkaloid derivatives, epothilones and cryptophycins. Exemplary topoisomerase inhibitors include camptothecin and camptothecin derivatives, camptothecin analogs and non-natural camptothecins, such as, for example, CPT-11, SN-38, topotecan, 9-aminocamptothecin, rubitecan, gimatecan, karenitecin, silatecan, lurtotecan, exatecan, diflometotecan, belotecan, lurtotecan and S39625. Other camptothecin compounds that can be used in the present disclosure include those described in, for example, J. Med. Chem., 29:2358-2363 (1986); J. Med. Chem., 23:554 (1980); J. Med Chem., 30:1774 (1987). Angiogenesis inhibitors include, but are not limited to, MetAP2 inhibitors, VEGF inhibitors, PIGF inhibitors, VGFR inhibitors, PDGFR inhibitors, MetAP2 inhibitors. Exemplary VGFR and PDGFR inhibitors include sorafenib, sunitinib and vatalanib. Exemplary MetAP2 inhibitors include fumagillol analogs, meaning compounds that include the fumagillin core structure. Exemplary cell cycle progression inhibitors include CDK inhibitors such as, for example, BMS-387032 and PD0332991; Rho-kinase inhibitors such as, for example, AZD7762; aurora kinase inhibitors such as, for example, AZD1152, MLN8054 and MLN8237; PLK inhibitors such as, for example, BI 2536, BI6727, GSK461364, ON-01910; and KSP inhibitors such as, for example, SB 743921, SB 715992, MK-0731, AZD8477, AZ3146 and ARRY-520. Exemplary P13K/m-TOR/AKT signalling pathway inhibitors include phosphoinositide 3-kinase (P13K) inhibitors, GSK-3 inhibitors, ATM inhibitors, DNA-PK inhibitors and PDK-1 inhibitors. Exemplary P13 kinases are disclosed in U.S. Patent No. 6,608,053, and include BEZ235, BGT226, BKM120, CAL263, demethoxyviridin, GDC-0941, GSK615, IC87114, LY294002, Palomid 529, perifosine, PF-04691502, PX-866, SAR245408, SAR245409, SF1126, Wortmannin, XL147 and XL765. Exemplary AKT inhibitors include, but are not limited to AT7867. Exemplary MAPK signaling pathway inhibitors include MEK, Ras, JNK, B-Raf and p38 MAPK inhibitors. Exemplary MEK inhibitors are disclosed in U.S. Patent No. 7,517,944 and include GDC-0973, GSK1120212, MSC1936369B, AS703026, RO5126766 and RO4987655, PD0325901, AZD6244, AZD8330 and GDC-0973. Exemplary B-raf inhibitors include CDC-0879, PLX-4032, and SB590885. Exemplary B p38 MAPK inhibitors include BIRB 796, LY2228820 and SB 202190. Exemplary receptor tyrosine kinases inhibitors include but are not limited to AEE788 (NVP-AEE 788), BIBW2992 (Afatinib), Lapatinib, Erlotinib (Tarceva), Gefitinib (Iressa), AP24534 (Ponatinib), ABT-869 (linifanib), AZD2171, CHR-258 (Dovitinib), Sunitinib (Sutent), Sorafenib (Nexavar), and Vatalinib. Exemplary protein chaperon inhibitors include HSP90 inhibitors. Exemplary inhibitors include 17AAG derivatives, BIIB021, BIIB028, SNX-5422, NVP-AUY-922 and KW-2478. Exemplary HDAC inhibitors include Belinostat (PR₄₈101), CUDC-101, Droxinostat, ITF2357 (Givinostat, Gavinostat), JNJ-26481585, LAQ824 (NVP-LAQ824, Dacinostat), LBH-589 (Panobinostat), MC1568, MGCD0103 (Mocetinostat), MS-275 (Entinostat), PCI-24781, Pyroxamide (NSC 696085), SB939, Trichostatin A and Vorinostat (SAHA). Exemplary PARP inhibitors include iniparib (BSI 201), olaparib (AZD-2281), ABT-888 (Veliparib), AG014699, CEP9722, MK 4827, KU-0059436 (AZD2281), LT-673, 3-aminobenzamide, A-966492, and AZD2461. Exemplary Wnt/Hedgehog signalling pathway inhibitors include vismodegib, cyclopamine and XAV-939. Exemplary RNA polymerase inhibitors include amatoxins. Exemplary amatoxins include alpha-amanitins, beta amanitins, gamma amanitins, eta amanitins, amanullin, amanullic acid, amanisamide, amanon, and proamanullin. Exemplary immunomodulators are APRIL, cytokines, including IL-2, IL-7, IL-10, IL12, IL-15, IL-21, TNF, interferon gamma, GMCSF, NDV-GMCSF, and agonists and antagonists of STING, agonists and antagonists of TLRs including TLR1/2, TLR3, TLR4, TLR7/8, TLR9, TLR12, agonists and antagonists of GITR, CD3, CD28, CD40, CD74, CTLA4, OX40, PD1, PDL1, RIG, MDA-5, NLRP1, NLRP3, AIM2, IDO, MEK, cGAS, and CD25, NKG2A. Other exemplary drugs include puromycins, topetecan, rhizoxin, echinomycin, combretastatin, netropsin, estramustine, cemadotin, discodermolide, eleutherobin, mitoxantrone, pyrrolobenzimidazoles (PBI), gamma-interferon, Thialanostatin (A) and analogs, CDK11, immunotoxins, comprising e.g. ricin A, diphtheria toxin, cholera toxin. In exemplary embodiments of the disclosure, the drug moiety is a mytomycin compound, a vinca alkaloid compound, taxol or an analogue, an anthracycline compound, a calicheamicin compound, a maytansinoid compound, an auristatin compound, a duocarmycin compound, SN38 or an analogue, a pyrrolobenzodiazepine compound, a indolinobenzodiazepine compound, a pyridinobenzodiazepine compound, a tubulysin compound, a non-natural camptothecin compound, a DNA binding drug, a kinase inhibitor, a MEK inhibitor, a KSP inhibitor, a P13 kinase inhibitor, a topoisomerase inhibitor, or analogues thereof. In one preferred embodiment the drug is a non-natural camptothecin compound, vinca alkaloid, kinase inhibitor, (e.g. P13 kinase inhibitor: GDC-0941 and PI-103), MEK inhibitor, KSP inhibitor, RNA polymerase inhibitor, PARP inhibitor, docetaxel, paclitaxel, doxorubicin, dolastatin, calicheamicins, SN38, pyrrolobenzodiazepines, pyridinobenzodiazepines, indolinobenzodiazepines, DNA binding drugs, maytansinoids DM1 and DM4, auristatin MMAE, CC1065 and its analogs, camptothecin and its analogs, SN-38 and its analogs. In another preferred embodiment the drug is selected from DNA binding drugs and microtubule agents, including pyrrolobenzodiazepines, indolinobenzodiazepines, pyridinobenzodiazepines, maytansinoids, maytansines, auristatins, tubulysins, duocarmycins, anthracyclines, taxanes. In another preferred embodiment the drug is selected from colchinine, vinca alkaloids, tubulysins, irinotecans, an inhibitory peptide, amanitin and deBouganin. In another preferred embodiment the drug is a radioactive moiety, said moiety comprising a radioactive isotope for radiation therapy. A radionuclide used for therapy is preferably an isotope selected from the group consisting of ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹¹¹In, ¹²¹Sn, ¹²⁷Te, ¹³¹T, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac, and ²²⁷Th. When the radioactive moiety is intended to comprise a metal, such as ¹⁷⁷Lu, such radiometal is preferably provided in the form of a chelate. In such a case the radioactive moiety preferably comprises a structural moiety capable of forming a coordination complex with such a metal. A good example hereof are macrocylic lanthanide(III) chelates derived from 1,4,7,10-tetraazacyclododecane-1,4,7,10-tetraacetic acid (H₄dota). Preferably, the structural moiety capable of forming a coordination complex with such a metal is a chelating moiety as defined herein. In other embodiments the radioactive moiety comprises a prosthetic group (i.e. a phenol) that is bound by a non-metal radionuclide, such as ¹³¹I. Drugs optionally include a (portion of a) membrane translocation moiety (e.g. adamantine, poly-lysine/arginine, TAT, human lactoferrin) and/or a targeting agent (against e.g. a tumor cell receptor) optionally linked through a stable or labile linker. Exemplary references include: Trends in Biochemical Sciences, 2015,. 40, 12, 749; J. Am. Chem. Soc. 2015, 137, 12153-12160; Pharmaceutical Research, 2007, 24, 11, 1977. It will further be understood that, in addition to one or more targeting agents (or C^{B}) that may be attached to the Trigger or Linker L^{C} a targeting agent T^{T} may optionally be attached to a drug, optionally via a spacer S^{P}. Alternatively, it will be further understood that the targeting agent (or C^{B}) may comprise one or more additional drugs which are bound to the targeting agent by other types of linkers, e.g. cleavable by proteases, pH, thiols, or by catabolism. It will be understood that chemical modifications may also be made to the desired compound in order to make reactions of that compound more convenient for purposes of preparing conjugates of the disclosure. Drugs containing an amine functional group for coupling to the Trigger include mitomycin-C, mitomycin-A, daunorubicin, doxorubicin, aminopterin, actinomycin, bleomycin, 9-amino camptothecin, N8-acetyl spermidine, 1-(2 chloroethyl)1,2-dimethanesulfonyl hydrazide, tallysomycin, cytarabine, dolastatins (including auristatins) and derivatives thereof. Drugs containing a hydroxyl function group for coupling to the Trigger include etoposide, camptothecin, taxol, esperamicin, 1,8-dihydroxy-bicyclo[7.3.1]trideca-4-9-diene-2,6-diyne-13-one (U.S. Pat No. 5,198,560), podophyllotoxin, anguidine, vincristine, vinblastine, morpholine-doxorubicin, n-(5,5-diacetoxy-pentyl)doxorubicin, and derivatives thereof. Drugs containing a sulfhydryl functional group for coupling to the Trigger include esperamicin and 6-mecaptopurine, and derivatives thereof.

### Log P

In preferred embodiments the Log P of compounds of Formula (1) have a value in a range of from 2.0 and -2.0, more preferably in a range of from 1.0 and -1.0.

In embodiments where it is required that a compound as disclosed herein, in particular a diene, has an extracellular volume of distribution it is preferred that the Log P of said compound is at most 2, preferably at most 1, more preferably at most 0, even more preferably at most -1. In embodiments where it is required that a compound as disclosed herein, in particular a diene, has an intracellular volume of distribution it is preferred that the Log P of the Activator is at least -1, preferably at least 0, more preferably at least 1, even more preferably at least 2.

### Molecular weight

For a compound of Formula (1) wherein T² is a bioconjugation moiety, it is preferred that the molecular weight of said compound is at most 5 kDa, more preferably at most 4 kDa, even more preferably at most 3.5 kDa, more preferably stil at most 3 kDa, and most preferably at most 2.5 kDa. For a compound of Formula (1) wherein T² is a group -L³-C^{B}, it is preferred that the molecular weight of said compound is at most 100 kDa, more preferably at most 85 kDa, even more preferably at most 75 kDa, more preferably stil at most 65 kDa, and most preferably at most 62.5 kDa.

### Spacers S^{P}

All linkers as used herein may each independently be a spacer S^{P}. As the skilled person is aware, the specific structure of a spacer used in either a dienophile or diene as described herein does not typically influence whether the payload is released. However, in some cases specific spacers are preferred. For example, if a payload is to be released, the spacer between e.g. the allylic carbon of the eight-membered non-aromatic cyclic mono-alkenylene moiety and the payload is preferably a self-immolative linker. Such a linker, which is typically referred to as L^{C} herein, ensures that upon release of the end of the linker connected to said allylic carbon, a further rearrangement or reaction takes place, after which the payload is decoupled from the linker L^{C}. Below, first spacers in general are discussed, and thereafter the more specific self-immolative linkers.

In general, a spacer S^{P} as used herein is a moiety according to RG2, more preferably any one of the preferred and/or specific embodiments thereof.

Preferably, a spacer S^{P} consists of one or multiple Spacer Units S^{U} arranged linearly and/or branched and may be connected to one or more C^{B} moieties and/or one or more L^{C} or T^{R} moieties. The Spacer may be used to connect C^{B} to one T^{R} (Example A below; with reference to Formula 5a and 5b: f, e, a = 1) or more T^{R} (Example B and C below; with reference to Formula 5a and 5b: f, e = 1, a ≥ 1), but it can also be used to modulate the properties, e.g. pharmacokinetic properties, of the C^{B}-T^{R}-C^{A} conjugate (Example D below; with reference to Formula 5a and 5b: one or more of c,e,g,h ≥ 1). Thus a Spacer unit does not necessarily connect two entities together, it may also be bound to only one component, e.g. the T^{R} or L^{C}. Alternatively, the Spacer may comprise a Spacer Unit linking C^{B} to T^{R} and in addition may comprise another Spacer Unit that is only bound to the Spacer and serves to modulate the properties of the conjugate (Example F below; with reference to Formula 5a and 5b: e ≥ 1). The Spacer may also consist of two different types of S^{U} constructs, e.g. a PEG linked to a peptide, or a PEG linked to an alkylene moiety (Example E below; with reference to Formula 5a and 5b: e ≥ 1). For the sake of clarity, Example B depicts a S^{U} that is branched by using a multivalent branched S^{U}. Example C depicts a S^{U} that is branched by using a linear S^{U} polymer, such as a peptide, whose side chain residues serve as conjugation groups. 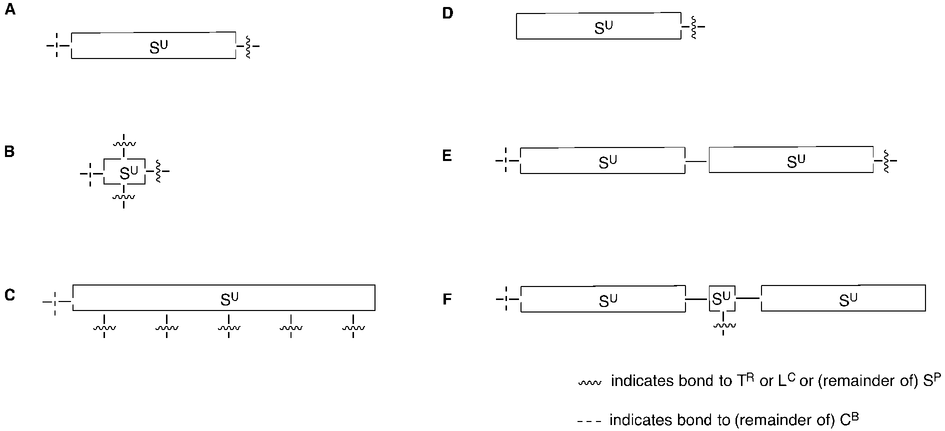 The Spacer may be bound to the Activator in similar designs such as depicted in above examples A- F.

Each individual spacer unit S^{U} may be independently selected from the group of radicals according to RG2. The Spacer Units include but are not limited to amino acids, nucleosides, nucleotides, and biopolymer fragments, such as oligo- or polypeptides, oligo- or polypeptoids, or oligo- or polylactides, or oligo- or poly-carbohydrates, varying from 2 to 200, particularly 2 to 113, preferably 2 to 50, more preferably 2 to 24 and more preferably 2 to 12 repeating units. Preferred biopolymer S^{U} are peptides. Preferably each S^{U} comprises at most 50 carbon atoms, more preferably at most 25 carbon atoms, more preferably at most 10 carbon atoms. In some embodiments the S^{U} is independently selected from the group consisting of (CH₂)ᵣ, (C₃-C₈ carbocyclo), O-(CH₂)ᵣ, arylene, (CH₂)ᵣ-arylene, arylene-(CH₂)ᵣ, (CH₂)ᵣ -(C₃-C₈ carbocyclo), (C₃-C₈ carbocyclo)-(CH₂)ᵣ, (C₃-C₈ heterocyclo), (CH₂)ᵣ -(C₃-C₈ heterocyclo), (C₃-C₈ heterocyclo)-(CH₂)ᵣ, -(CH₂)ᵣC(O)NR'(CH₂)ᵣ, (CH₂CH₂O)ᵣ, (CH₂CH₂O)ᵣCH₂,(CH₂)ᵣC(O)NR'(CH₂ CH₂O)ᵣ, (CH₂)ᵣC(O)NR'(CH₂CH₂O)ᵣCH₂, (CH₂CH₂O)ᵣ C(O)NR'(CH₂CH₂O)ᵣ, (CH₂CH₂O)ᵣ C(O)NR'(CH₂CH₂O)ᵣCH₂, (CH₂CH₂O)ᵣC(O)NR'CH₂; wherein r is independently an integer from 1 -10. As used herein, each R'is independently selected from the group consisting of radicals according to RG1. Preferably, R'is hydrogen. Other examples of Spacer Units S^{U} are linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, - OCH₂CH₃, OCH₂CH₂CO₂H. Other polymeric Spacer Units are polymers and copolymers such as poly-(2-oxazoline), poly(N-(2-hydroxypropyl)methacrylamide) (HPMA), polylactic acid (PLA), polylactic-glycolic acid (PLGA), polyglutamic acid (PG), dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used as S^{U} are cellulose, amylose, dextran, dextrin, levan, fucoidan, carrageenan, inulin, pectin, amylopectin, glycogen, lixenan, agarose, hyaluronan, chondroitinsulfate, dermatansulfate, keratansulfate, alginic acid and heparin. In yet other exemplary embodiments, the polymeric S^{U} comprises a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Preferred polymeric S^{U} are PEG, HPMA, PLA, PLGA, PVP, PHF, dextran, oligopeptides, and polypeptides. In some embodiments, polymers used in a S^{U} have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa. Other exemplary S^{U} are dendrimers, such as poly(propylene imine) (PPI) dendrimers, PAMAM dendrimers, and glycol-based dendrimers. The S^{U} of the disclosure expressly include but are not limited to conjugates prepared with commercially available cross-linker reagents such as BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, sulfo-SMPB, and SVSB, DTME, BMB, BMDB, BMH, BMOE, BM(PEO)₃ and BM(PEO)₄. To construct a branching Spacer one may use a S^{U} based on one or several natural or non-natural amino acids, amino alcohol, aminoaldehyde, or polyamine residues or combinations thereof that collectively provide the required functionality for branching. For example serine has three functional groups, i.e. acid, amino and hydroxyl groups and may be viewed as a combined amino acid an aminoalcohol residue for purpose of acting as a branching S^{U}. Other exemplary amino acids are lysine and tyrosine. In some embodiments, the Spacer consists of one Spacer Unit, therefore in those cases S^{P} equals S^{U}. Preferably the Spacer consists of two, three or four Spacer Units. In some embodiments, S^{P} has a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, or from 500 dalton to 5 kDa. In some embodiments, the S^{P} has a mass of no more than 5000 daltons, no more than 4000 daltons, no more than 3000 daltons, no more than 2000 daltons, no more than 1000 daltons, no more than 800 daltons, no more than 500 daltons, no more than 300 daltons, no more than 200 daltons. In some aspects the S^{P} has a mass from 100 daltons, from 200 daltons, from 300 daltons to 5000 daltons. In some aspects of the S^{P} has a mass from 30, 50, or 100 daltons to 1000 daltons, from about 30, 50, or 100 daltons to 500 daltons.

Preferably, S^{P} comprises a moiety RG2a, RG2b, RG2c, or a residue of RG1f, as described herein. Preferably, said RG2a, RG2b, RG2c, or a residue of RG1f connects the S^{P} to C^{B}, L^{C}, or T^{R}.

### Self-immolative linkers L^{C}

L^{C} is an optional self-immolative linker, which may consist of multiple units arranged linearly and/or branched. The possible L^{C} structures, their use, position and ways of attachment of linkers L^{C}, C^{A} and the T^{R} (the Trigger, *i.e.* the *trans-*cyclooctene moiety) are known to the skilled person, see for example [Papot et al., Anticancer Agents Med. Chem., 2008, 8, 618-637]. Nevertheless, preferred but non-limiting examples of self-immolative linkers L^{C} are benzyl-derivatives, such as those drawn below. There are two main self-immolation mechanisms: electron cascade elimination and cyclization-mediated elimination. The preferred example below on the left functions by means of the cascade mechanism, wherein the bond between the allylic carbon of the Trigger and the -O- or -S- attached to said carbon is cleaved, and an electron pair of Y^{C1}, for example an electron pair of NR⁶, shifts into the benzyl moiety resulting in an electron cascade and the formation of 4-hydroxybenzyl alcohol, CO₂ and the liberated payload. The preferred example in the middle functions by means of the cyclization mechanism, wherein cleavage of the bond to the NR⁶ on the side of the Trigger leads to nucleophilic attack of the amine on the carbonyl, forming a 5-ring 1,3-dimethylimidazolidin-2-one and liberating the payload. The preferred example on the right combines both mechanisms. This linker will degrade not only into CO₂ and one unit of 4-hydroxybenzyl alcohol (when Y^{C1} is O), but also into one 1,3-dimethylimidazolidin-2-one unit. 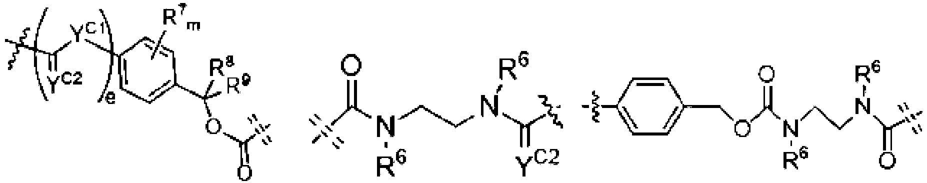 wherein the wiggly line indicates a bond to -O- or -S- on the allylic position of the trans-cyclooctene, and the double dashed line indicates a bond to C^{A}.

By substituting the benzyl groups of aforementioned self-immolative linkers L^{C}, it is possible to tune the rate of release of the payload, caused by either steric and/or electronic effects on the cyclization and/or cascade release. Synthetic procedures to prepare such substituted benzyl-derivatives are known to the skilled person (see for example [Greenwald et al, J. Med. Chem., 1999, 42, 3657-3667] and [Thornthwaite et al, Polym. Chem., 2011, 2, 773-790]. Some preferred substituted benzyl-derivatives with different release rates are drawn below.

Self-immolative linkers that undergo cyclization include but are not limited to substituted and unsubstituted aminobutyric acid amide, appropriately substituted bicyclo[2.2.1] and bicyclo[2.2.2] ring system, 2-aminophenylpropionic acid amides, and trimethyl lock-based linkers, see e.g. [Chem. Biol. 1995, 2, 223], [J. Am. Chem. Soc. 1972, 94, 5815], [J. Org. Chem. 1990, 55, 5867], the contents of which are hereby incorporated by reference. Further preferred examples of L^{C} can be found in WO2009017394(A1), US7375078, WO2015038426A1, WO2004043493, Angew. Chem. Int. Ed. 2015, 54, 7492 - 7509, the contents of which are hereby incorporated by reference.

Preferably the L^{C} has a mass of no more than 1000 daltons, no more than 500 daltons, no more than 400 daltons, no more than 300 daltons, or from 10, 50 or 100 to 1000 daltons, from 10, 50, 100 to 400 daltons, from 10, 50, 100 to 300 daltons, from 10, 50, 100 to 200 daltons, e.g., 10-1000 daltons, such as 50-500 daltons, such as 100 to 400 daltons.

A person skilled in the art will know that one L^{C} may be connected to another L^{C} that is bound to C^{A}, wherein upon reaction of the Activator with the Trigger T^{R}, L^{C}-L^{C}-C^{A} is released from the T^{R}, leading to self-immolative release of both L^{C} moietes and the payload. With respect to the L^{C} formulas disclosed herein, the L^{C} linking the T^{R} to the other L^{C} then does not release the payload but an L^{C} that is bound via Y^{C1} and further links to C^{A}. The skilled person will acknowledge that this principle also holds for further linkers L^{C} linked to L^{C}, *e.g.* L^{C}-L^{C}-L^{C}-L^{C}-C^{A}.

Preferably, if the releasable group contains a self-immolative linker, the releasable group is according to any one of Group I, Group II, Group III, and Group IV as shown below. In the structures depicted for said Groups, only bonds to Construct A and an atom (typically oxygen) on the allylic position of the eight-membered non-aromatic cyclic mono-alkenylene moiety (preferably a *trans-cyclooctene* ring) are shown for reasons of clarity, but said Construct A and said atom are part of the releasable group.

Releasable groups according to Group I are 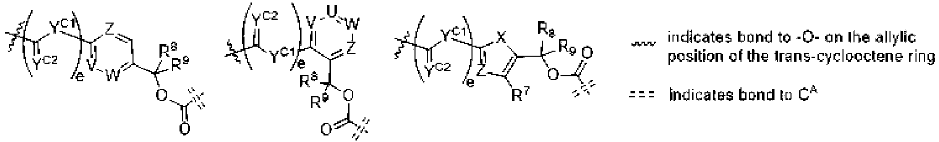 , wherein the wiggly line may also indicate a bond to -S- on the allylic position of the trans-cyclooctene, wherein U, V, W, Z are each independently selected from the group consisting of -CR⁷-, and -N-, wherein e is 0 or 1, wherein X is selected from the group consisting of -O-, -S- and -NR⁶-, wherein preferably each R⁸ and R⁹ are independently selected from the group consisting of hydrogen, C₁-C₄ (hetero)alkyl, C₂-C₄ (hetero)alkenyl, and C₄₋₆ (hetero)aryl; wherein for R⁸ and R⁹ the (hetero)alkyl, (hetero)alkenyl, and (hetero)aryl are optionally substituted with a moiety selected from the group consisting of -Cl, -F, -Br, -I, -OH, -NH₂, =O, -SH, -SO₃H, -PO₃H, -PO₄H₂ and -NO₂ and preferably contain at most two heteroatoms selected from the group consisting of -O-, -S-, -NH-, -P-, and -Si-, wherein the N, S, and P atoms are optionally oxidized. Preferably, for releasable groups of Group I both R⁸ and R⁹ are hydrogen.

The releasable group according to Group II is 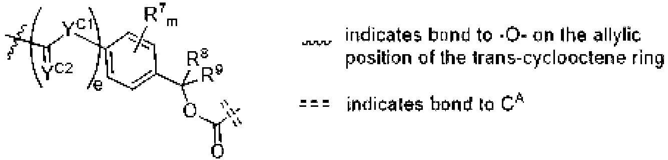 , wherein the wiggly line may also indicate a bond to -S- on the allylic position of the trans-cyclooctene, wherein m is an integer between 0 and 2, preferably m is 0, wherein e is 0 or 1. Preferably, for releasable groups of Group II both R⁸ and R⁹ are hydrogen. Preferably, for releasable groups of Group II R⁷ is methyl or isopropyl. Optionally, R⁶, R⁷, R⁸, R⁹ comprised in said Group I, and II, are -(S^{P})ᵢ-C^{B}.

For all releasable groups according to Group I and Group II Y^{C1} is selected from the group consisting of -O-, -S-, and -NR⁶-, preferably -NR⁶-. For all linkers according to Group I, and Group II, Y^{C2} is selected from the group consisting of O and S, preferably O.

Releasable groups according to Group III are 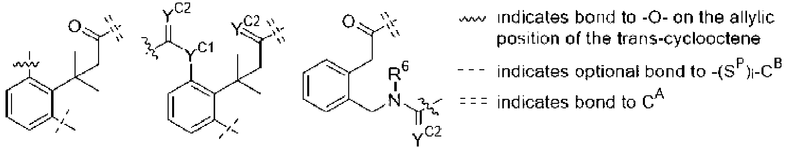 , wherein the wiggly line may also indicate a bond to -S- on the allylic position of the trans-cyclooctene.

Releasable groups according to Group IV are 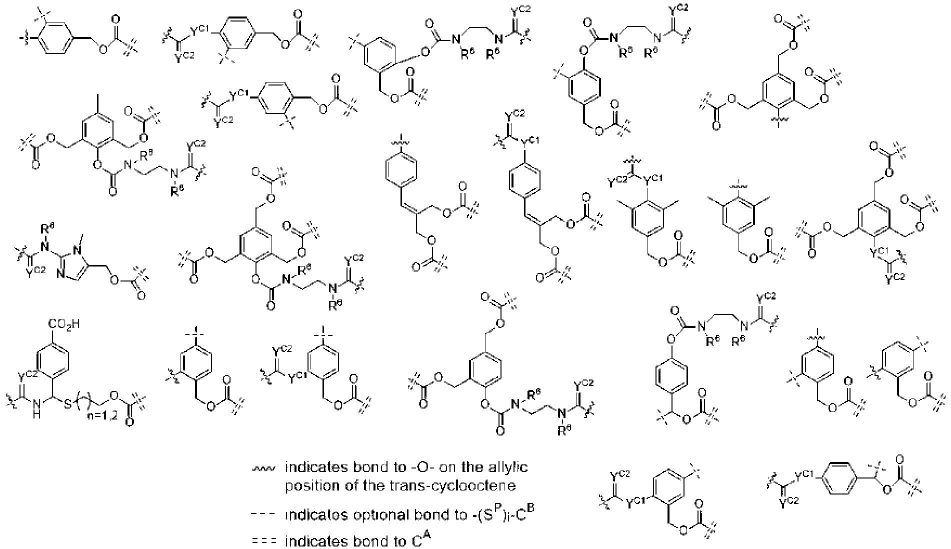 , wherein the wiggly line may also indicate a bond to -S- on the allylic position of the trans-cyclooctene.

Preferably, R⁶, R⁷, R⁸, R⁹ are according to RG1 or any preferred embodiment thereof. Preferably, R⁶, R⁷, R⁸, R⁹ as used herein are not substituted. Most preferably, R⁶, R⁷, R⁸, R⁹ as used herein are hydrogen.

### Conjugates of the disclosure

The disclosure also relates to a conjugate, or a salt, hydrate, or solvate thereof, wherein the conjugate comprises a protein conjugated to at least one compound according to the disclosure wherein T² is a residue of a bioconjugation moiety, and said protein and said compound are conjugated via T². Thus, the conjugate of the disclosure is to be understood as a compound of the disclosure (wherein T² was originally a bioconjugation moiety) linked to a protein via T², wherein due to the coupling of said compound and said protein, T² in the conjugate of the disclosure is the residue of a bioconjugation moiety, preferably the residue of an N-maleimidyl group, viz.: 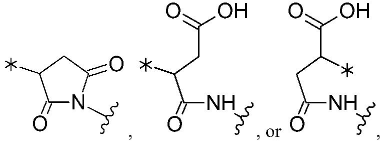 wherein the asterisk indicates a bond to the protein, and the wiggly line denotes a bond to the rest of the compound of the disclosure.

In the conjugate of the disclosure, the protein is preferably a diabody or an antibody, more preferably a diabody, and most preferably the protein is AVP0458 consisting of two monomers, wherein each of the two monomers has an amino acid sequence according to SEQ ID NO: 1.

Preferably, in the conjugate of the disclosure the protein and the compound of the disclosure are conjugated via T² and a residue of a sulfhydryl of said protein, a residue of a hydroxyl of said protein, or a residue of an amine of said protein; more preferably via T² and a residue of a sulfhydryl of said protein. Preferably, the residue of the sulfhydryl group of said protein is part of a cysteine residue of said protein.

Preferably, the conjugate of the disclosure is 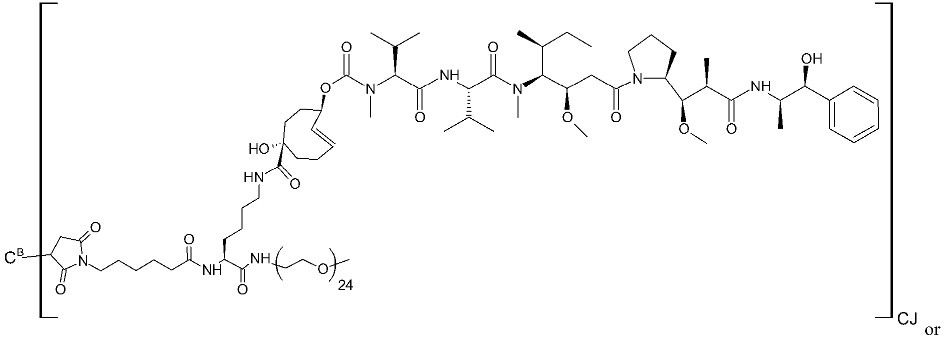 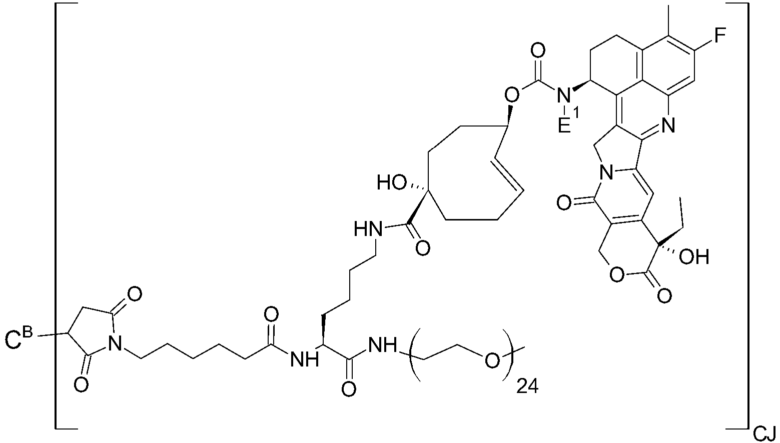 wherein E¹ is -H or -CH₃, preferably E¹ is -H.

More preferably, the conjugate of the disclosure is

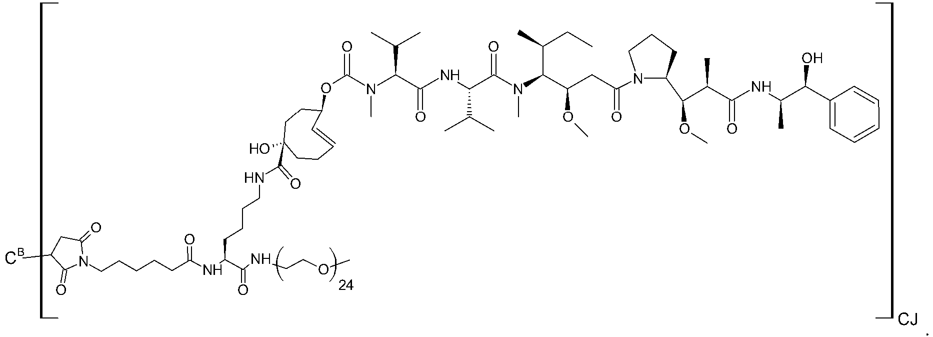

In relation to conjugates of the disclosure, CJ is in a range of from 1 to 12, preferably CJ is of from 2 to 10, more preferably of from 2.5 to 8, even more preferably of from 3 to 6, and most preferably of from 3.5 to 4. It will be understood that for individual conjugates, CJ is typically an integer, and is most preferably about 4. When measuring CJ for multiple conjugates, however, an average number may be obtained, which is not necessarily an integer. As CJ is commonly determined for multiple conjugates, CJ in relation to the disclosure typically refers to an average number.

More preferably, the conjugate is: 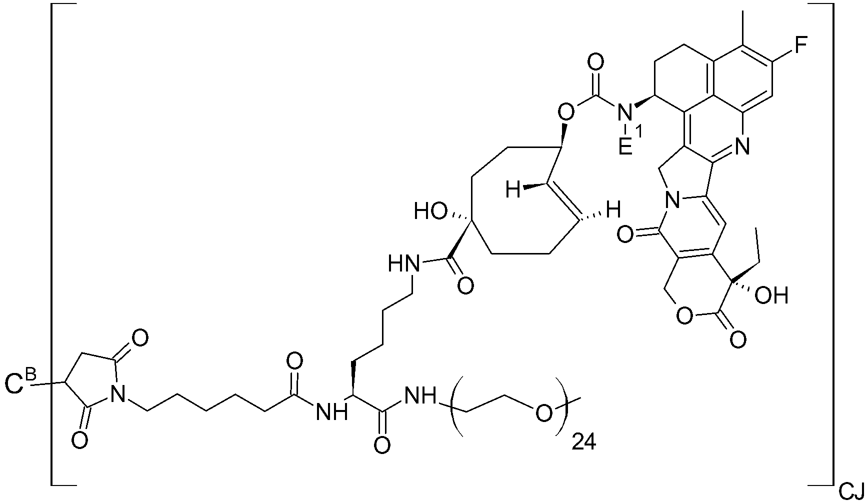 wherein E¹ is -H or -CH₃, preferably E¹ is -H.

More preferably, the conjugate is: 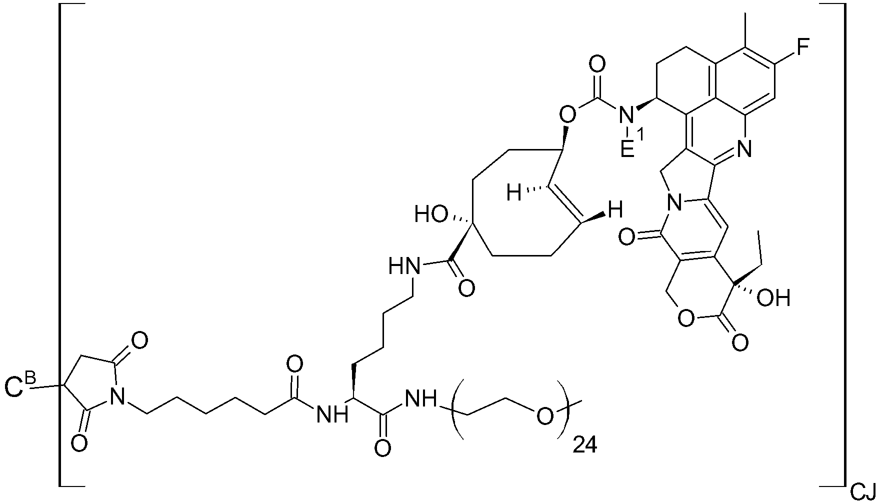 wherein E¹ is -H or -CH₃, preferably E¹ is -H.

More preferably, the conjugate is:

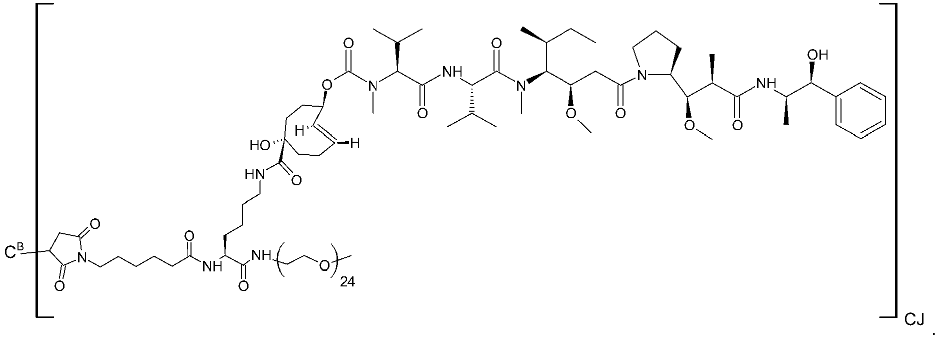

More preferably, the conjugate is:

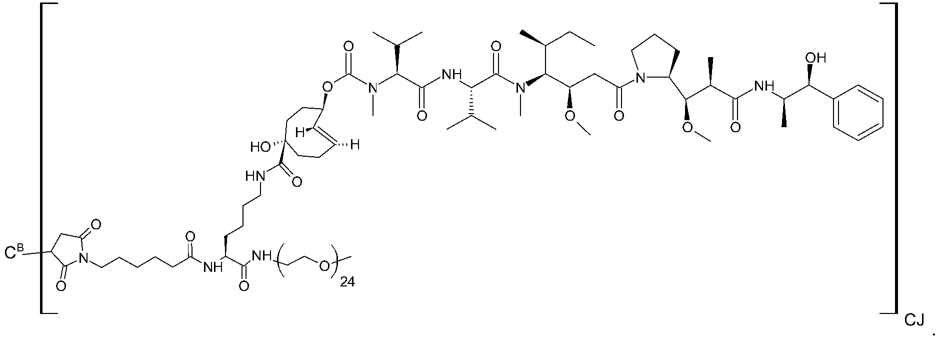

### Compositions of the disclosure

The disclosure also pertains to a composition comprising a compound according to the disclosure, or the salt, hydrate, or solvate thereof. Preferably, the composition is a pharmaceutical composition. Preferably, the composition of the disclosure further comprises a pharmaceutically acceptable carrier. It is also preferred that if a salt of a compound of the disclosure is included in the composition of the disclosure, a pharmaceutically acceptable salt is used.

### Combinations of the disclosure

The disclosure also relates to a combination of (A1) a compound according to the disclosure, or the salt, hydrate, or solvate thereof; (A2) a conjugate according to the disclosure, or the salt, hydrate, or solvate thereof; and/or (A3) a composition according to the disclosure; with (B) a diene or a salt, solvate, or hydrate thereof. It will be understood that herein, a compound according to the disclosure is a dienophile and/or comprises a dienophile moiety, and may be called a "Trigger". The diene may be referred to as an "Activator".

Preferably, the combination is of (A1) and (B). Preferably, the combination is of (A2) and (B). Preferably, the combination is of (A3) and (B). Preferably, the combination is of (A1), (A2), and (B). Preferably, the combination is of (A1), (A3), and (B). Preferably, the combination is of (A2), (A3), and (B). Preferably, the combination is of (A1), (A2), (A3), and (B).

Preferably, the combination of the disclosure is a kit. More preferably, the combination of the disclosure is a kit wherein (A1), (A2), and/or (A3) is/are physically separated from (B).

Preferably the diene is a tetrazine. More preferably, the diene is selected from the group consisting of: 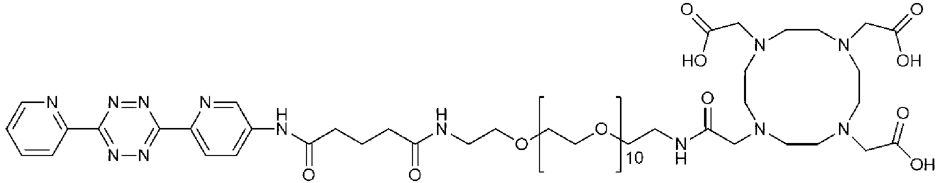 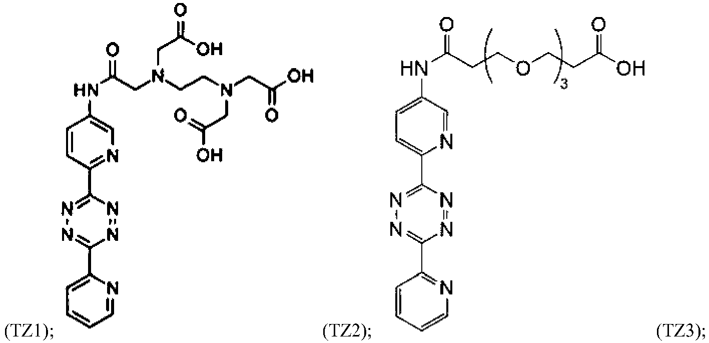 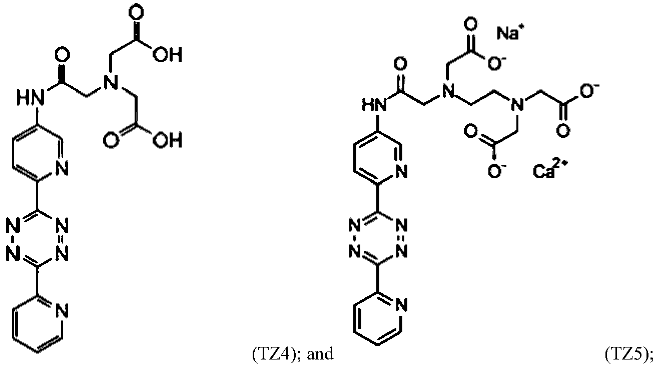 or a salt, hydrate, and/or solvate thereof.

Preferably, the diene is (TZ1) or a salt, hydrate, and/or solvate thereof. More preferably, the diene is (TZ2) or a salt, hydrate, and/or solvate thereof. More preferably, the diene is (TZ3) or a salt, hydrate, and/or solvate thereof. More preferably, the diene is (TZ4) or a salt, hydrate, and/or solvate thereof. Most preferably, the diene is (TZ5) or a salt, hydrate, and/or solvate thereof.

(TZ1) is the best-studied tetrazine for *in vivo* use in literature, and the most promising candidate for clinical use. Reference is made to, *inter alia,* Rossin et al., Angew. Chem. Int. Ed. 2010, volume 49, pages 3375-3378; Rossin et al., J. Nucl. Med. 2013, volume 54; pages 1989-1995; Rossin et al., Bioconjugate Chem. 2013, volume 24, pages 1210-1217; Rossin et al., Mol. Pharm. 2014, volume 11, pages 3090-3096; Van Duijnhoven et al., J. Nucl. Med. 2015, volume 56, pages 1422-1428; Edem et al. Molecules 2020, volume 25, page 463; Rossin et al., Bioconjugate Chem. 2016, volume 27, pages 1697-1706; Rossin et al., Nature Commun. 2018, volume 9, article 1484; WO 2020/256546 (in particular Example 5 at pages 294-296).

However, the inventors have identified several hitherto unknown disadvantages of (TZ1). These problems mainly arise when using (TZ1) *in vivo* as an activator for the payload release from an eight-membered non-aromatic cyclic mono-alkenylene moiety (such as a *trans*-cyclooctene), which require higher doses than when using (TZ1) for radioimaging and/or radiotherapy.

First, it was found that compound (TZ1) strongly inhibits the physiologically relevant enzymes cyclooxygenase (COX-1), acetyl cholinesterase (ACES), monoamine oxidase (MAO-B), and calcium channel L-type, dihydropyridine. Each of these proteins is important in maintaining health in a subject, and undesired inhibition of these enzymes and/or transporter may lead to side-effects.

Second, it was found that (TZ1) has a relatively low maximum tolerated dose (MTD) in mice of about 39 µmol/kg.

Furthermore, the synthesis of (TZ1) comprises many steps, while it is preferred that tetrazines are used that can be synthesized in fewer steps.

Finally, it is desired that tetrazines with overall good *in vitro* and *in vivo* properties be provided, *i*.*e*. one or more of: good stability, good reactivity with and/or high payload release from *trans*-cyclooctenes (especially *in vivo*), low membrane permeability, low cell toxicity, and low genotoxicity.

It was found that (TZ2), (TZ3), (TZ4), and in particular (TZ5) overcome one or more of these disadvantages of (TZ1). Therefore, combinations with at least one of (TZ2), (TZ3), (TZ4), and (TZ5) are preferred over combinations comprising (TZ1), and combinations with (TZ5) are most preferred.

### Non-therapeutic methods using and uses for using compounds of the disclosure

In some embodiments, the disclosure pertains to non-therapeutic methods and non-therapeutic uses. Preferably, the dienophile used therein is as described in relation to the combination of the disclosure.

For the non-therapeutic method of the disclosure it is preferred that the compound of the disclosure (viz. (ia)), the conjugate of the disclosure (viz. (iia)), and/or the composition of the disclosure (viz. (iiia)), and the diene are further contacted with a solvent. The skilled person is aware of suitable solvents for a reaction between a *trans-*cyclooctene (TCO) and a tetrazine. Preferably, the solvent comprises water, and more preferably the solvent is water.

For the non-therapeutic use, the click reaction is preferably a bioorthogonal click reaction. Preferably, the click reaction is performed *in vitro,* although non-therapeutic reactions *in vivo* can be carried out as well.

### Medical use

The disclosure also relates to a compound of the disclosure, or the salt, hydrate, or solvate thereof; the conjugate of the disclosure, or the salt, hydrate, or solvate thereof; the composition of the disclosure; or the combination of the disclosure; for use in the treatment of a disease in a subject.

The disclosure also pertains to a method of treating a disease in a subject, wherein said method comprises the step of administering to said subject: (a) the compound according to the disclosure, or the salt, hydrate, or solvate thereof; (b) the conjugate according to the disclosure, or the salt, hydrate, or solvate thereof; (c) the composition according to the disclosure; and/or
(d) the combination according to the disclosure.

Use of (a) a compound according to the disclosure, or the salt, hydrate, or solvate thereof; (b) a conjugate according to the disclosure, or the salt, hydrate, or solvate thereof;
(c) a composition according to the disclosure; and/or (d) a combination according to the disclosure; for the manufacture of a medicament for the treatment of a disease in a subject.

In relation to the medical use, preferably the subject is a human. Preferably, the disease is cancer.

### Methods of synthesizing compounds of the disclosure

The disclosure also relates to a method for synthesizing a compound of the disclosure, wherein said method comprises coupling a compound of Formula (R) to a compound of Formula (S): 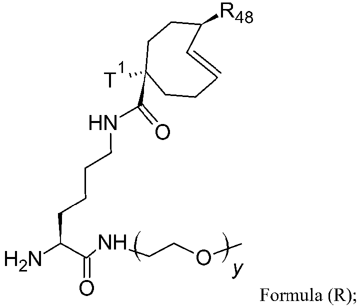 wherein R₄₈, T¹, and y are as defined herein; 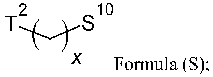 wherein T², and x, are as defined herein, and S¹⁰ is -COOH or an active ester, preferably S¹⁰ is - COOH. Preferably, in Formula (S) x is an integer of from 4 to 6, and most preferably x is 5.

In the method for synthesizing a compound of the disclosure, when S¹⁰ is -COOH, it is preferred that the compound of Formula (S) is contacted with at least one coupling reagent, preferably in the presence of a base, preferably a non-nucleophilic base. Preferred non-nucleophilic bases are N,N-diisopropylethylamine (DIPEA), 1,8-diazabicycloundec-7-ene (DBU), and 1,5-diazabicyclo(4.3.0)non-5-ene (DBN). Preferably, the at least one coupling reagent is as defined in Clause 583.

The skilled person is aware of suitable conditions to carry out a coupling reaction between a compound of Formula (R) and a compound of Formula (S).

Preferably, the coupling is carried out at a temperature of from -20°C to 80°C, more preferably of from 0°C to 60°C, even more preferably of from 4°C to 50°C, more preferably still of from 10°C to 40°C, and most preferably of from 15°C to 30°C.

Preferably, the coupling is carried out in the presence of a solvent, wherein preferably the solvent is an organic solvent.

The disclosure also relates to an alternative method for synthesizing a compound of the disclosure, wherein said method comprises coupling a compound of Formula (T) to a compound of Formula (U): 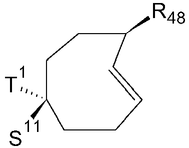 Formula (T); wherein T¹ and R₄₈ are as defined herein; and S¹¹ is -COOH or an active ester, preferably S¹¹ is an active ester, more preferably S¹¹ is selected from the group consisting of -C(O)O-N-succinimidyl, -C(O)O-pentafluorophenyl, -C(O)O-tetrafluorophenyl, -C(O)O-4-nitrophenyl, and -C(O)Cl; even more preferably, S¹¹ is -C(O)O-N-succinimidyl, or -C(O)O-pentafluorophenyl; and most preferably, S¹¹ is -C(O)O-pentafluorophenyl. 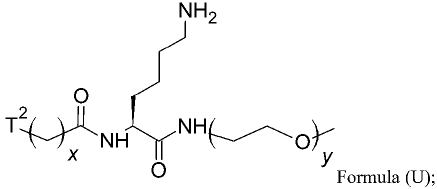 wherein T², x, and y, are as defined herein.
Preferably, in Formula (U) x is an integer of from 4 to 6, and most preferably x is 5.

In the alternative method for synthesizing a compound of the disclosure, when S¹¹ is -COOH, it is preferred that the compound of Formula (S) is contacted with at least one coupling reagent, preferably in the presence of a base, preferably a non-nucleophilic base. Preferred non-nucleophilic bases are N,N-diisopropylethylamine (DIPEA), 1,8-diazabicycloundec-7-ene (DBU), and 1,5-diazabicyclo(4.3.0)non-5-ene (DBN). Preferably, the at least one coupling reagent is as defined in Clause 583.

The skilled person is aware of suitable conditions to carry out a coupling reaction between a compound of Formula (T) and a compound of Formula (U). Preferably, the coupling is carried out at a temperature of from -20°C to 80°C, more preferably of from 0°C to 60°C, even more preferably of from 4°C to 50°C, more preferably still of from 10°C to 40°C, and most preferably of from 15°C to 30°C. Preferably, the coupling is carried out in the presence of a solvent, wherein preferably the solvent is an organic solvent.

### Methods of synthesizing conjugates of the disclosure

The disclosure also pertains to a method for synthesizing a conjugate of the disclosure, wherein said method comprises the step of coupling a protein to a compound of the disclosure, or a salt, hydrate, or solvate thereof; wherein in said compound T² is a bioconjugation moiety; wherein preferably in said protein disulfide bonds have been reduced.

As T² in the compound of the disclosure is preferably a bioconjugation moiety that can react with a sulfhydryl group, such as an N-maleimidyl group, it is preferred that the protein contains free sulfhydryl groups. Typically, such sulfhydryl groups can be obtained by reducing disulfide bonds present in the protein. To that end, it is preferred that the protein has been contacted with a reducing agent prior to the coupling. Preferably, the reducing agent is selected from the group consisting of dithiothreitol (DTT), and tris-2-carboxyethylphosphine hydrochloride (TCEP).

If the protein is contacted with a reducing agent prior to the coupling, the reducing agent is preferably DTT. Additionally or alternatively, the formation of free sulfhydryl groups on the protein can also be performed *in situ.* To that end, preferably the coupling is carried out in the presence of a reducing agent. In that case, it is preferred to use a reducing agent that does not contain free sulfhydryl groups itself. Thus, if the coupling is carried out in the presence of a reducing agent, it is preferred that the reducing agent is TCEP.

The skilled person is aware of suitable conditions to carry out the method of synthesizing a conjugate of the disclosure.

Prefeably, the coupling is carried out at a temperature of from 0°C to 40°C, more preferably of from 1°C to 30°C, more preferably still of from 2°C to 20°C, even more preferably of from 4°C to 10°C, and most preferably at about 4°C.

Preferably, the coupling is carried out in an aqueous solution, preferably the aqueous solution is an aqueous buffer solution.

Preferably the coupling is carried out at a pH of from 6.0 to 8.5, preferably of from 6.2 to 8.0, more preferably of from 6.4 to 7.8, even more preferably of from 6.5 to 7.4, more preferably still of from 6.6 to 7.0, and most preferably at a pH of about 6.8.

The present disclosure is herein described with respect to particular embodiments, but the disclosure is not limited thereto but only by the claims. Where an indefinite or definite article is used when referring to a singular noun e.g. "a" or "an", "the", this includes a plural of that noun unless something else is specifically stated.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present disclosure, the only relevant components of the device are A and B.

The compounds herein may occur in different tautomeric forms. The compounds according to the disclosure are meant to include all tautomeric forms, unless stated otherwise. When the structure of a compound is depicted as a specific tautomer, it is to be understood that the disclosure of the present application is not limited to that specific tautomer, unless stated otherwise.

The compounds herein may occur in different enantiomeric forms. The compounds according to the disclosure are meant to include all enantiomeric forms, unless stated otherwise. When the structure of a compound is depicted as a specific enantiomer, it is to be understood that the disclosure of the present application is not limited to that specific enantiomer, unless stated otherwise.

Unless stated otherwise, the compounds of the disclosure and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

Unless stated otherwise, if in this disclosure reference is made to a molecular structure, such as "compound", "diene", "tetrazine", and the like, it will be understood that such a molecular structure may also be in its salt, hydrate, and/or solvate form.

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

Herein, reference is made to "alkyl", and the like. The number of carbon atoms that these groups have, excluding the carbon atoms comprised in any optional substituents according to Radical Group 1, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

A cycloalkyl group is a cyclic alkyl group. Unsubstituted cycloalkyl groups comprise at least three carbon atoms and have the general formula CₙH₂ₙ-₁. Optionally, the cycloalkyl groups are substituted by one or more substituents further specified in this document. Examples of cycloalkyl groups include cyclopropyl, cyclobutyl, cyclopentyl and cyclohexyl.

An alkenyl group comprises one or more carbon-carbon double bonds, and may be linear or branched. Unsubstituted alkenyl groups comprising one C-C double bond have the general formula CₙH₂ₙ₋₁. Unsubstituted alkenyl groups comprising two C-C double bonds have the general formula CₙH₂ₙ₋₃. An alkenyl group may comprise a terminal carbon-carbon double bond and/or an internal carbon-carbon double bond. A terminal alkenyl group is an alkenyl group wherein a carbon-carbon double bond is located at a terminal position of a carbon chain. An alkenyl group may also comprise two or more carbon-carbon double bonds. Examples of an alkenyl group include ethenyl, propenyl, isopropenyl, t-butenyl, 1,3-butadienyl, 1,3-pentadienyl, etc. Unless stated otherwise, an alkenyl group may optionally be substituted with one or more, independently selected, substituents according to Radical Group 1.

A cycloalkenyl group is a cyclic alkenyl group. An unsubstituted cycloalkenyl group comprising one double bond has the general formula CₙH₂ₙ₋₃. Optionally, a cycloalkenyl group is substituted by one or more substituents further specified in this document. An example of a cycloalkenyl group is cyclopentenyl.

An alkynyl group comprises one or more carbon-carbon triple bonds, and may be linear or branched. Unsubstituted alkynyl groups comprising one C-C triple bond have the general formula CₙH₂ₙ₋₃. An alkynyl group may comprise a terminal carbon-carbon triple bond and/or an internal carbon-carbon triple bond. A terminal alkynyl group is an alkynyl group wherein a carbon-carbon triple bond is located at a terminal position of a carbon chain. An alkynyl group may also comprise two or more carbon-carbon triple bonds. Unless stated otherwise, an alkynyl group may optionally be substituted with one or more, independently selected, substituents according to Radical Group 1. Examples of an alkynyl group include ethynyl, propynyl, isopropynyl, t-butynyl, etc.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl.

An aryl group refers to an aromatic hydrocarbon ring system that comprises six to twenty-four carbon atoms, more preferably six to twelve carbon atoms, and may include monocyclic and polycyclic structures. When the aryl group is a polycyclic structure, it is preferably a bicyclic structure. Optionally, the aryl group may be substituted by one or more substituents further specified in this document. Examples of aryl groups are phenyl and naphthyl. Preferably, an aryl group is phenyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-tert-butylphenyl.

Preferably, heteroaryl groups comprise five to sixteen carbon atoms and contain between one to five heteroatoms. Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and an alkylheteroaryl group, and (hetero)arylalkyl is meant to include an arylalkyl group and a heteroarylalkyl group. A C₂-C₂₄ (hetero)aryl group is thus to be interpreted as including a C₂-C₂₄ heteroaryl group and a C₆-C₂₄ aryl group. Similarly, a C₃-C₂₄ alkyl(hetero)aryl group is meant to include a C₇-C₂₄ alkylaryl group and a C₃-C₂₄ alkylheteroaryl group, and a C₃-C₂₄ (hetero)arylalkyl is meant to include a C₇-C₂₄ arylalkyl group and a C₃-C₂₄ heteroarylalkyl group.

In general, when (hetero) is placed before a group, it refers to both the variant of the group without the prefix hetero- as well as the group with the prefix hetero-. Herein, the prefix hetero- denotes that the group contains one or more heteroatoms selected from the group consisting of O, N, S, P, and Si. Preferably, the one or more heteroatoms is selected from the group consisting of O, N, S, and P. It will be understood that for any compound containing a heteroatom, the N, S, and P atoms are optionally oxidized and the N atoms are optionally quaternized. Preferably, up to two heteroatoms are consecutive, such as in for example -CH₂-NH-OCH₃ and -CH₂-O-Si(CH₃)₃. More preferably, however, the heteroatoms are not directly bound to one another.

Examples of heteroalkyls include -CH₂CH₂-O-CH₃, -CH₂CH₂-NH-CH₃, -CH₂CH₂-S(O)-CH₃, -CH₌CH-O-CH₃, CH₂CH₂-NH₂, CH₂CH₂-SH, -CH₂CH₂-OH, -CH₂CH₂-COOH, -CH₂C(O)H, -C(O)HCH₃, and -Si(CH₃)₃. Preferably, a C₁-C₄ heteroalkyl contains at most 2 heteroatoms.

Herein, it will be understood that when the prefix hetero- is used for combinations of groups, the prefix hetero- only refers to the one group before it is directly placed. For example, heteroarylalkyl denotes the combination of a heteroaryl group and an alkyl group, not the combination of a heteroaryl and a heteroalkyl group.

Herein, the prefix cyclo- denotes that groups are cyclic. It will be understood that when the prefix cyclo- is used for combinations of groups, the prefix cyclo- only refers to the one group before it is directly placed. For example, cycloalkylalkenylene denotes the combination of a cycloalkylene group (see the definition of the suffix -ene below) and an alkenylene group, not the combination of a cycloalkylene and a cycloalkenylene group. In general, when (cyclo) is placed before a group, it refers to both the variant of the group without the prefix cyclo- as well as the group with the prefix cyclo-.

Herein, the suffix -ene denotes divalent groups, i.e. that the group is linked to at least two other moieties. An example of an alkylene is propylene (-CH₂-CH₂-CH₂-), which is linked to another moiety at both termini. It is understood that if a group with the suffix -ene is substituted at one position with -H, then this group is identical to a group without the suffix. For example, an alkylene attached to an -H is identical to an alkyl group. I.e. propylene, -CH₂-CH₂-CH₂-, attached to an -H at one terminus, -CH₂-CH₂-CH₂-H, is logically identical to propyl, -CH₂-CH₂-CH₃.

Herein, when combinations of groups are listed with the suffix -ene, it refers to a divalent group, i.e. that the group is linked to at least two other moieties, wherein each group of the combination contains one linkage to one of these two moieties. As such, for example alkylarylene is understood as a combination of an arylene group and an alkylene group. An example of an alkylarylene group is -phenyl-CH₂-, and an example of an arylalkylene group is -CH₂-phenyl-.

Herein, the suffix -triyl denotes trivalent groups, i.e. that the group is linked to at least three other moieties. An example of an arenetriyl is depicted below: 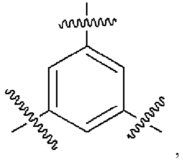 wherein the wiggly lines denote bonds to different groups of the main compound.

It is understood that if a group with the suffix -triyl is substituted at one position with -H, then this group is identical to a divalent group with the suffix -ene. For example, an arenetriyl substituted with -H is identical to an arylene group. Similarly, it is understood that if a group with the suffix -triyl is substituted at two positions with -H, then this group is identical to a monovalent group. For example, an arenetriyl substituted with two -H is identical to an aryl group.

Unless indicated otherwise, a hetero group may contain a heteroatom at non-terminal positions or at one or more terminal positions. In this case, "terminal" refers to the terminal position within the group, and not necessarily to the terminal position of the entire compound. For example, C₂ heteroalkylene may refer to -NH-CH₂-CH₂-, -CH₂-NH-CH₂-, and -CH₂-CH₂-NH-. For example, C₂ heteroalkyl may refer to -NH-CH₂-CH₃, -CH₂-NH-CH₃, and -CH₂-CH₂-NH₂.

Herein, it is understood that cyclic compounds (i.e. aryl, cycloalkyl, cycloalkenyl, etc.) are understood to be monocyclic, polycyclic or branched. It is understood that the number of carbon atoms for cyclic compounds not only refers to the number of carbon atoms in one ring, but that the carbon atoms may be comprised in multiple rings. These rings may be fused to the main ring or substituted onto the main ring. For example, C₁₀ aryl optionally containing heteroatoms may refer to *inter alia* a naphthyl group (fused rings) or to *e.g.* a bipyridyl group (substituted rings, both containing an N atom).

Unless stated otherwise, any group disclosed herein that is not cyclic is understood to be linear or branched. In particular, (hetero)alkyl groups, (hetero)alkenyl groups, (hetero)alkynyl groups, (hetero)alkylene groups, (hetero)alkenylene groups, (hetero)alkynylene groups, and the like are linear or branched, unless stated otherwise.

As used herein, unless stated otherwise all of the following groups: (hetero)alkyl, (hetero)alkenyl, (hetero)alkynyl, (hetero)cycloalkyl, (hetero)cycloalkenyl, (hetero)cycloalkynyl, (hetero)aryl, (hetero)alkylene, (hetero)alkenylene, (hetero)alkynylene, (hetero)cycloalkylene, (hetero)cycloalkenylene, (hetero)cycloalkynylene, (hetero)arylene, (hetero)alkanetriyl, (hetero)cycloalkanetriyl, arenetriyl, heteroarenetriyl, combinations thereof, and the like, can be substituted or unsubstituted; preferably these groups are unsubstituted. If said groups are substituted, said groups preferably contain up to 4, more preferably up to 3, more preferably still up to 2, and most preferably 1 substituent according to Radical Group 1 as defined herein.

The general term "sugar" is herein used to indicate a monosaccharide, for example glucose (Glc), galactose (Gal), mannose (Man) and fucose (Fuc). The term "sugar derivative" is herein used to indicate a derivative of a monosaccharide sugar, i.e. a monosaccharide sugar comprising substituents and/or functional groups. Examples of a sugar derivative include amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (ldoA). A sugar may be without further substitution, and then it is understood to be a monosaccharide. A sugar may be further substituted with at one or more of its hydroxyl groups, and then it is understood to be a disaccharide or an oligosaccharide. A disaccharide contains two monosaccharide moieties linked together. An oligosaccharide chain may be linear or branched, and may contain from 3 to 10 monosaccharide moieties.

The term "amino acid" is used herein in its normal scientific meaning. In particular, amino acids in relation to the disclosure comprise both natural and unnatural amino acids. Preferably, amino acids as used herein are selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, azidolysine, beta-alanine (bAla), 4-aminomethyl phenylalanine (Amf), 4-guanidine phenylalanine (Gnf), 4-aminomethyl-N-isopropyl phenylalanine (Iaf), 3-pyridyl alanine (Pya), 4-piperidyl alanine (Ppa), 4-aminomethyl cyclohexyl alanine (Ama), 4-aminocyclohexyl alanine (Aca), ornithine (Orn), citrulline, hydroxylysine (Hyl), allo-hydroxylysine (aHyl), 6-N-methyllysine (MeLys), desmosine (Des), isodesmosine (Ide), 2-aminoadipic acid (Aad), 3-aminoadipic acid (bAad), 2-aminobutyric acid (Abu), 4-aminobutyric acid (4Abu), 6-aminohexonic acid (Acp), 2-aminoheptanoic acid (Ahe), 2-aminoisobutyric acid (Aib), 3-aminoisobutyric acid (bAib), 2-aminopimelic acid (Apm), 2,4-diaminobutyric acid (Dbu), 2,2'-diaminopimelic acid (Dpm), 2-3-diaminopropionic acid (Dpr), N-ethylglycine (EtGly), N-ethylasparagine (EtAsn), 3-hydroxyproline (3Hyp), 4-hydroxyproline (4Hyp), allo-isoleucine (AIle), sarcosine (MeGly), N-methylisoleucine (MeIle), N-methylvaline (MeVal), norvaline (Nva), and norleucine (Nle).

The term "protein" is herein used in its normal scientific meaning. Herein, polypeptides comprising about 10 or more amino acids are considered proteins. A protein may comprise natural, but also unnatural amino acids. The term "protein" herein is understood to comprise antibodies and antibody fragments.

The term "peptide" is herein used in its normal scientific meaning. Herein, peptides are considered to comprise a number of amino acids in a range of from 2 to 9.

The term "peptoid" is herein used in its normal scientific meaning.

A spacer is herein defined as a moiety that connects two or more elements of a compound. The terms "spacer" and "linker" are used herein interchangeably. Typically, a spacer is herein denoted as S^{P}, and the more specific self-immolative linkers as L^{C}. It will be understood that when herein, it is stated that "each individual S^{P} is linked at all ends to the remainder of the structure" this refers to the fact that the spacer S^{P} connects multiple moieties within a structure, and therefore the spacer has multiple ends by defintion. The spacer S^{P} may be linked to each individual moiety via different or identical moieties that may be each individually selected. Typically, these linking moieties are to be seen to be part of spacer S^{P} itself. In case the spacer S^{P} links two moieties within a structure, "all ends" should be interpreted as "both ends". As an example, if the spacer connects a trans-cylooctene moiety to a Construct B, then "the remainder of the molecule" refers to the trans-cylooctene moiety and Construct B, while the connecting moieties between the spacer and the trans-cyclooctene moiety and Construct B (i.e. at both ends) may be individually selected.

As used herein, an organic molecule is defined as a molecule comprising a C-H bond. Organic compound and organic molecule are used synonymously.

As used herein, an inorganic molecule is defined as any molecule not being an organic molecule, *i.e.* not comprising a C-H bond. It will be understood that "inorganic molecule" typically also comprises hydrogen, -COOH, etc.

As used herein, a "small molecule" is preferably a small organic molecule. In general, a small molecule has a molecular weight of at most 2 kDa, more preferably at most 1 kDa, more preferably at most 750 Da, more preferably at most 500 Da, and most preferably at most 300 Da. Preferably, a small molecule has a molecular weight of at least 15 Da, more preferably at least 50 Da, more preferably at least 75 Da, and most preferably at least 100 Da.

As used herein, "particle" is preferably defined as a microparticle or a nanoparticle.

The term "salt thereof" means a compound formed when an acidic proton, typically a proton of an acid, is replaced by a cation, such as a metal cation or an organic cation and the like. The term "salt thereof" also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted salt" means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

As used herein, the term "solvate" refers to a compound that apart from a main molecule (e.g. a compound of the disclosure, a diene, and the like) further includes a stoichiometric or non-stoichiometric amount of solvent bound to said main molecule by non-covalent intermolecular forces. In particular, the term "solvate" may refer to a crystalline compound, the crystal lattice structure of which contains one or more molecules of the solvent.

As used herein, the term "hydrate" refers to a compound that apart from a main molecule (e.g. a compound of the disclosure, a diene, and the like) further includes a stoichiometric or non-stoichiometric amount of water bound to said main molecule by non-covalent intermolecular forces. In particular, the term "hydrate" may refer to a crystalline compound, the crystal lattice structure of which contains one or more molecules of water.

The logarithm of the partition-coefficient, i.e. Log P, is herein used as a measure of the hydrophobicity of a compound. Typically, the Log P is defined as $log \left(\frac{{\left[Solute\right]}_{octanol}^{un - ionized}}{{\left[Solute\right]}_{water}^{un - ionized}}\right)$ The skilled person is aware of methods to determine the partition-coefficient of compounds without undue experimentation. Alternatively, the skilled person knows that software is available to reliably estimate the Log P value, for example as a function within ChemDraw^{®} software or online available tools.

The unified atomic mass unit or Dalton is herein abbreviated to Da. The skilled person is aware that Dalton is a regular unit for molecular weight and that 1 Da is equivalent to 1 g/mol (grams per mole).

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example,
-((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂- before the individual R₅₁ and R₅₂ moieties are selected, rather than first selecting moieties R₅₁ and R₅₂ and then writing out the formula.

As used herein, "activated carboxylic acid" and "active ester" may be used interchangeably. As the skilled person is aware, an "activated carboxylic acid" or an "active ester" is a derivative of a carboxylic acid (-C(O)OH) of which the -OH moiety has been exchanged for a better leaving group. Preferred activated carboxylic acids or active esters are selected from the group consisting of -C(O)O-N-succinimidyl, -C(O)O-pentafluorophenyl, -C(O)O-tetrafluorophenyl, -C(O)O-4-nitrophenyl, and -C(O)Cl. More preferably, the activated carboxylic acid or active ester is -C(O)O-N-succinimidyl, or - C(O)O-pentafluorophenyl.

As the skilled person is aware, an "active carbonate" is a derivative of a carbonate (-O-C(O)-OH) of which the -OH moiety has been exchanged for a better leaving group. Preferred active carbonates are -OC(O)O-N-succinimidyl, -OC(O)O-pentafluorophenyl, -OC(O)O-tetrafluorophenyl, -OC(O)O-4-nitrophenyl, and -OC(O)Cl. More preferably, the active carbonate is -OC(O)O-N-succinimidyl, or -OC(O)O-pentafluorophenyl.

As used herein, a "drug" refers to a pharmaceutical agent. As such, "drug", "pharmaceutical agent", "therapeutic agent", and "medicine" can typically be used interchangeably. Preferred drugs in relation to the disclosure are monomethyl auristatin E (MMAE), exatecan, and exatecan derivatives. Preferably, exatecan and exatecan derivatives have the following structure: 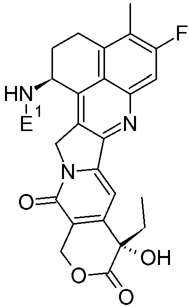 wherein E¹ is -H, or an optionally substituted C₁-C₄ alkyl group. It will be understood that when E¹ is -H, said structure is exatecan. Preferably, E¹ is -H, -CH₃, or -C(O)-CH₂-OH. If E¹ is - H or -CH₃, then the exatecan or exatecan derivative is preferably linked to the remainder of R₄₈ via the nitrogen atom to which E¹ is attached. If E¹ is C(O)-CH₂-OH, then the exatecan derivative is preferably linked to the remainder of R₄₈ via the oxygen atom that is part of the hydroxyl group of E¹. More preferably, E¹ is -H or -CH₃. Most preferably, E¹ is -H.
Most preferably, the drug is monomethyl auristatin E (MMAE).

### Radical groups (RG)

### Radical Group 1: terminal groups

For Radical Group 1 (RG1), the radical is selected from the group consisting of -H, -Cl, -F, -Br, -I, -OH, -NH₂, - COOH, -CONH₂, -CN, -N₃, -NCS, -SCN, -SO₃H, -PO₃H, -PO₄H₂, -NO₂, -CF₃, -CF₂H, -CFH₂, =O, =NH, -SH, -SO₂H, -(S^{P})ᵢ-C^{B}, (hetero)alkyl, (hetero)alkenyl, (hetero)alkynyl, (hetero)cycloalkyl, (hetero)cycloalkenyl, (hetero)cycloalkynyl, (hetero)aryl, and combinations thereof. Herein, S^{P} is a spacer as defined herein, C^{B} is Construct B as defined herein, and i is an integer in a range of from 0 to 4, preferably i is 0 or 1.

For RG1, "combinations thereof" in particular refers to (hetero)alkylcycloalkyl, (hetero)alkylcycloalkenyl, (hetero)alkylcycloalkynyl, (hetero)cycloalkylalkyl, (hetero)cycloalkenylalkyl, (hetero)cycloalkynylalkyl, (hetero)alkenylcycloalkyl, (hetero)alkenylcycloalkenyl, (hetero)alkenylcycloalkynyl, (hetero)cycloalkylalkenyl, (hetero)cycloalkenylalkenyl, (hetero)cycloalkynylalkenyl, (hetero)alkynylcycloalkyl, (hetero)alkynylcycloalkenyl, (hetero)alkynylcycloalkynyl, (hetero)cycloalkylalkynyl, (hetero)cycloalkenylalkynyl, (hetero)cycloalkynylalkynyl, (hetero)arylalkyl, (hetero)arylalkenyl, (hetero)arylalkynyl, alkyl(hetero)aryl, alkenyl(hetero)aryl, alkynyl(hetero)aryl, cycloalkyl(hetero)aryl, cycloalkenyl(hetero)aryl, cycloalkynyl(hetero)aryl, (hetero)arylcycloalkyl, (hetero)arylcycloalkenyl, and (hetero)arylcycloalkynyl. In addition, "combinations thereof" in relation to RG1 also refers to *e.g.* an alkyl group substituted with one or more -Cl and/or -OH groups. As such, RG1 also comprises radicals such as -NH-CH₂-COOH (a glycine residue), which is a combination of a heteroalkyl and -COOH.

Preferably, for RG1 the radical is selected from the group RG1a consisting of -H, -Cl, -F, -Br, -I, -OH, -NH₂, - COOH, -CONH₂, -SO₃H, -PO₃H, -PO₄H₂, -NO₂, -CF₃, =O, =NH, -SH, -(S^{P})ᵢ-C^{B}, C₁-C₂₄ (hetero)alkyl, C₂-C₂₄ (hetero)alkenyl, C₂-C₂₄ (hetero)alkynyl, C₃-C₂₄ cycloalkyl, C₂-C₂₄ heterocycloalkyl, C₅-C₂₄ cycloalkenyl, C₃-C₂₄ heterocycloalkenyl, C₇-C₂₄ cycloalkynyl, C₅-C₂₄ (hetero)cycloalkynyl, C₆-C₂₄ aryl, C₂-C₂₄ heteroaryl, and combinations thereof.

More preferably, for RG1 the radical is selected from the group RG1b consisting of -H, -Cl, -F, -Br, -I, -OH, -NH₂,
-COOH, -CONH₂, -SO₃H, -PO₃H, -PO₄H₂, -NO₂, -CF₃, =O, =NH, -SH, -(S^{P})ᵢ-C^{B}, C₁-C₁₂ (hetero)alkyl, C₂-C₁₂ (hetero)alkenyl, C₂-C₁₂ (hetero)alkynyl, C₃-C₁₂ cycloalkyl, C₂-C₁₂ heterocycloalkyl, C₅-C₁₂ cycloalkenyl, C₃-C₁₂ heterocycloalkenyl, C₇-C₁₂ cycloalkynyl, C₅-C₁₂ (hetero)cycloalkynyl, C₆-C₁₂ aryl, C₂-C₁₂ heteroaryl, and combinations thereof.

Even more preferably, for RG1 the radical is selected from the group RGlc consisting of -H, -Cl, -F, -Br, -I, -OH, - NH₂, -COOH, -CONH₂, -SO₃H, -PO₃H, -PO₄H₂, -NO₂, -CF₃, =O, =NH, -SH, -(S^{P})ᵢ-C^{B}, C₁-C₈ (hetero)alkyl, C₂-C₈ (hetero)alkenyl, C₂-C₈ (hetero)alkynyl, C₃-C₈ cycloalkyl, C₂-C₈ heterocycloalkyl, C₅-C₈ cycloalkenyl, C₃-C₈ heterocycloalkenyl, C₇-C₈ cycloalkynyl, C₅-C₈ (hetero)cycloalkynyl, C₆-C₈ aryl, C₂-C₈ heteroaryl, and combinations thereof.

More preferably still, for RG1 the radical is selected from the group RG1d consisting of -H, -Cl, -F, -Br, -I, -OH, - NH₂, -COOH, -CONH₂, -SO₃H, -PO₃H, -PO₄H₂, -NO₂, -CF₃, =O, =NH, -SH, -(S^{P})ᵢ-C^{B}, C₁-C₆ (hetero)alkyl, C₂-C₆ (hetero)alkenyl, C₂-C₆ (hetero)alkynyl, C₃-C₆ cycloalkyl, C₂-C₆ heterocycloalkyl, C₅-C₇ cycloalkenyl, C₃-C₅ heterocycloalkenyl, Cs cycloalkynyl, C₆-C₇ (hetero)cycloalkynyl, phenyl, C₃-C₅ heteroaryl, and combinations thereof.

Most preferably, for RG1 the radical is selected from the group RGle consisting of -H, -Cl, -F, -Br, -I, -OH, -NH₂, -COOH, -CONH₂, -SO₃H, -PO₃H, -PO₄H₂, -NO₂, -CF₃, =O, =NH, -SH, -(S^{P})ᵢ-C^{B}, C₁-C₃ (hetero)alkyl, C₃-C₆ cycloalkyl, C₂-C₅ heterocycloalkyl, phenyl, C₄-C₅ heteroaryl, and combinations thereof.

In some embodiments, for RG1 the radical is a conjugation moiety, which is a chemical group that can be used for binding, conjugation or coupling of a Construct, such as Construct-B, or a Spacer, or another molecule or construct of interest. The person skilled in the art is aware of the myriad of strategies that are available for the chemoselective or -unselective or enzymatic coupling or conjugation of one molecule or construct to another.

In some embodiments, RG1 is a moiety that allows conjugation to a protein comprising natural and/or non-natural amino acids. Moieties suitable for conjugation are known to the skilled person. Conjugation strategies are for example found in [O. Boutureira, G.J.L. Bernardes, Chem. Rev., 2015, 115, 2174-2195].

If RG1 is a conjugation moiety, it is preferably selected from the group RG1f consisting of N-maleimidyl, halogenated N-alkylamido, sulfonyloxy N-alkylamido, vinyl sulfone, (activated) carboxylic acids, active ester, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (oxa)norbornadiene, (imino)sydnones, methylsulfonyl phenyloxadiazole, aminooxy, 2-amino benzamidoxime, ethynylphosphonamidates, reactive in the Pictet-Spengler ligation and hydrazine- Pictet-Spengler (HIPS) ligation, DNA intercalators, tetrazine, trans-cyclooctene, and photocrosslinkers. More preferably, RG1f is N-maleimidyl.

In other embodiments RG1f is selected from the group consisting of hydroxyl, amine, halogens, vinyl pyridine, disulfide, pyridyl disulfide, sulfonyloxy, mercaptoacetamide, anhydride, sulfonylated hydroxyacetamido, sulfonyl chlorides, thiosemicarbazone, hydrazine carboxylate, and arylhydrazide. In yet other embodiments RG1f is a group that can be connected to another group by means of an enzyme, for example sortase or Tubulin tyrosine ligase.

### Radical Group 2: connecting groups

For Radical Group 2 (RG2), the radical is selected from the group consisting of (hetero)alkylene, (hetero)alkenylene, (hetero)alkynylene, (hetero)cycloalkylene, (hetero)cycloalkenylene, (hetero)cycloalkynylene, (hetero)arylene, amino acid, peptide, protein, polymer, oligonucleotide, nucleotide, carbohydrate, RG2a, RG2b, RG2c, and combinations thereof.

The radicals from RG2 are optionally attached to one or more radicals according to RG1. Thus, RG2 also covers *e.g.* -NH-CH(CH₂OH)-C(O)- *(i.e.* a serine residue), which is a heteroalkylene attached to -OH and =O.

For RG2, "combinations thereof" in particular, but not exclusively, refers to alkyl(hetero)arylene, (hetero)arylalkylene, (hetero)arylalkenylene, (hetero)arylalkynylene, alkenyl(hetero)arylene, and alkynyl(hetero)arylene.

Preferably, for RG2 the radical is selected from the group consisting of C₁-C₂₄ (hetero)alkylene, C₂-C₂₄ (hetero)alkenylene, C₂-C₂₄ (hetero)alkynylene, C₃-C₂₄ cycloalkylene, C₂-C₂₄ heterocycloalkylene, C₅-C₂₄ cycloalkenylene, C₃-C₂₄ heterocycloalkenylene, C₇-C₂₄ cycloalkynylene, C₅-C₂₄ (hetero)cycloalkynylene, C₆-C₂₄ arylene, C₂-C₂₄ heteroarylene, amino acid, peptide, protein, polymer, oligonucleotide, nucleotide, carbohydrate, RG2a, RG2b, RG2c, and combinations thereof.

More preferably, for RG2 the radical is selected from the group consisting of C₁-C₁₂ (hetero)alkylene, C₂-C₁₂ (hetero)alkenylene, C₂-C₁₂ (hetero)alkynylene, C₃-C₁₂ cycloalkylene, C₂-C₁₂ heterocycloalkylene, C₅-C₁₂ cycloalkenylene, C₃-C₁₂ heterocycloalkenylene, C₇-C₁₂ cycloalkynylene, C₅-C₁₂ (hetero)cycloalkynylene, C₆-C₁₂ arylene, C₂-C₁₂ heteroarylene, amino acid, peptide, protein, polymer, oligonucleotide, nucleotide, carbohydrate, RG2a, RG2b, RG2c, and combinations thereof.

Even more preferably, for RG2 the radical is selected from the group consisting of C₁-C₈ (hetero)alkylene, C₂-C₈ (hetero)alkenylene, C₂-C₈ (hetero)alkynylene, C₃-C₈ cycloalkylene, C₂-C₈ heterocycloalkylene, C₅-C₈ cycloalkenylene, C₃-C₈ heterocycloalkenylene, C₇-C₈ cycloalkynylene, C₅-C₈ (hetero)cycloalkynylene, C₆-C₈ arylene, C₂-C₈ heteroarylene, amino acid, peptide, protein, polymer, oligonucleotide, nucleotide, carbohydrate, RG2a, RG2b, RG2c, and combinations thereof.

More preferably still, for RG2 the radical is selected from the group consisting of C₁-C₆ (hetero)alkylene, C₂-C₆ (hetero)alkenylene, C₂-C₆ (hetero)alkynylene, C₃-C₆ cycloalkylene, C₂-C₆ heterocycloalkylene, C₅-C₇ cycloalkenylene, C₃-C₅ heterocycloalkenylene, C₈ cycloalkynylene, C₆-C₇ (hetero)cycloalkynylene, phenylene, C₃-C₅ heteroarylene, amino acid, peptide, protein, polymer, oligonucleotide, nucleotide, carbohydrate, RG2a, RG2b, RG2c, and combinations thereof.

Even more preferably still, for RG2 the radical is selected from the group consisting of C₁-C₃ (hetero)alkylene, C₃-C₆ cycloalkylene, C₂-C₅ heterocycloalkylene, phenylene, C₄-C₅ heteroarylene, amino acid, peptide, protein, polymer, oligonucleotide, nucleotide, carbohydrate, RG2a, RG2b, RG2c, and combinations thereof.

RG2a is selected from the group consisting of -O-, -S-, -SS-, -NR₄-, -N=N-, -C(O)-, -C(O)NR₄-, -OC(O)-, -C(O)O-, -OC(O)O-, -OC(O)NR₄-, -NR₄C(O)-, -NR₄C(O)O-, -NR₄C(O)NR₄-, -SC(O)-, -C(O)S-, -SC(O)O-, -OC(O)S-, -SC(O)NR₄-, - NR₄C(O)S-, -S(O)-, -S(O)₂-, -OS(O)₂-, -S(O₂)O-, -OS(O)₂O-, -OS(O)₂NR₄-, -NR₄S(O)₂O-, -C(O)NR₄S(O)₂NR₄-, - OC(O)NR₄S(O)₂NR₄-, -OS(O)-, -OS(O)O-, -OS(O)NR₄-, -ONR₄C(O)-, -ONR₄C(O)O-, -ONR₄C(O)NR₄-, -NR₄OC(O)-, - NR₄OC(O)O-, -NR₄OC(O)NR₄-, -ONR₄C(S)-, -ONR₄C(S)O-, -ONR₄C(S)NR₄-, -NR₄OC(S)-, -NR₄OC(S)O-, - NR₄OC(S)NR₄-, -OC(S)-, -C(S)O-, -OC(S)O-, -OC(S)NR₄-, -NR₄C(S)-, -NR₄C(S)O-, -SS(O)₂-, -S(O)₂S-, -OS(O₂)S-, - SS(O)₂O-, -NR₄OS(O)-, -NR₄OS(O)O-, -NR₄OS(O)NR₄-, -NR₄OS(O)₂-, -NR₄OS(O)₂O-, -NR₄OS(O)₂NR₄-, -ONR₄S(O)-, - ONR₄S(O)O-, -ONR₄S(O)NR₄-, -ONR₄S(O)₂O-, -ONR₄S(O)₂NR₄-, -ONR₄S(O)₂-, -OP(O)(R₄)₂-, -SP(O)(R₄)₂-, and - NR₄P(O)(R₄)₂-.
Herein, R₄ is according to RG1, preferably R₄ is hydrogen or methyl, more preferably R₄ is hydrogen.

Preferably, RG2a is selected from the group consisting of -O-, -S-, -SS-, -NR₄-, -N=N-, -C(O)-, -C(O)NR₄-, - OC(O)-, -C(O)O-, -OC(O)NR₄-, -NR₄C(O)-, -NR₄C(O)O-, -NR₄C(O)NR₄-, -SC(O)-, -C(O)S-, -SC(O)O-, -OC(O)S-, - SC(O)NR₄-, -NR₄C(O)S-, -S(O)-, -S(O)₂-, -C(O)NR₄S(O)₂NR₄-, -OC(O)NR₄S(O)₂NR₄-, -OC(S)-, -C(S)O-, -OC(S)O-, - OC(S)NR₄-, -NR₄C(S)-, -NR₄C(S)O-, and -SS(O)₂-.

More preferably, for RG2 the radical is RG2b or RG2c, most preferably RG2b.

RG2b is selected from the group consisting of 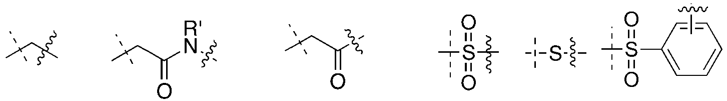 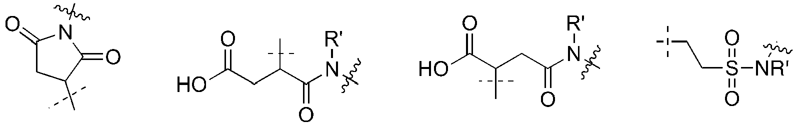 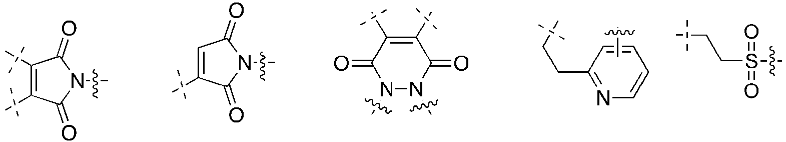 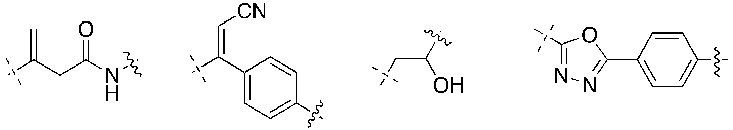 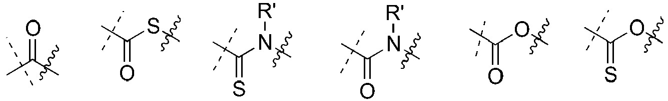 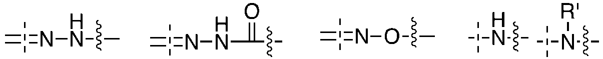 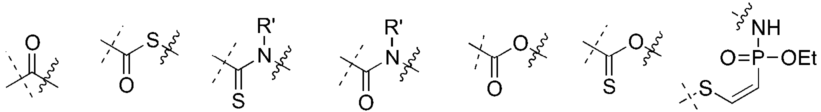 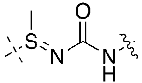 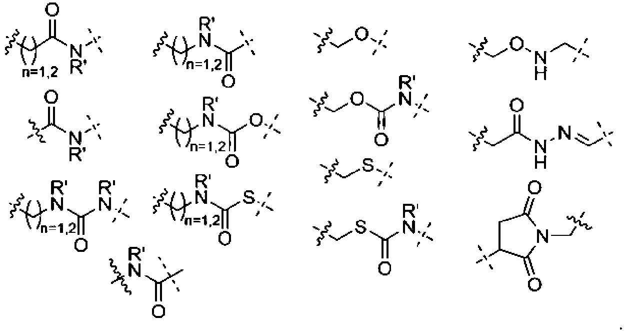 Therein, R' is a radical according to RG1, preferably R' is hydrogen or C₁₋₃ alkyl. The dashed and wiggly lines denote bonds to the other parts of the molecule.

RG2c is selected from the group consisting of 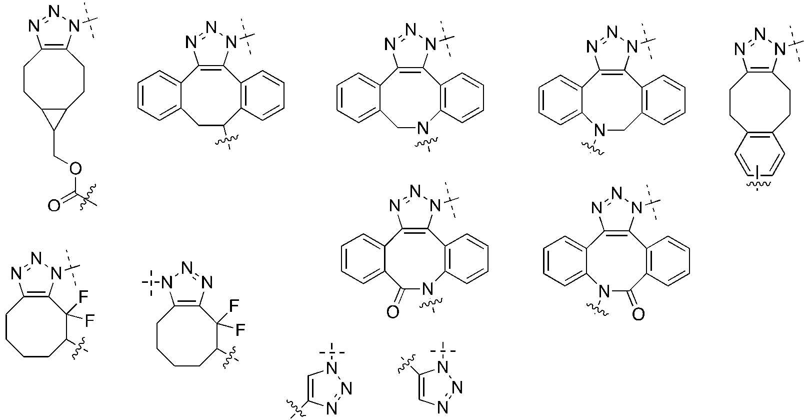 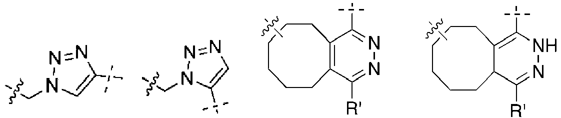 Therein, R' is a radical according to RG1, preferably R' is hydrogen or C₁₋₃ alkyl. The dashed and wiggly lines denote bonds to the other parts of the molecule.

### Radical Group 3: organic molecule

For Radical Group 3 (RG3) the radical is an organic molecule selected from the group consisting of a nucleic acid, a peptide, a protein, a carbohydrate, an aptamer, a hormone, a toxin, a steroid, a cytokine, a lipid, a small organic molecule as defined herein, a polymer, LNA, PNA, an amino acid, a peptoid, a chelating moiety, a molecule comprising a radionuclide, a fluorescent dye, a phosphorescent dye, a drug, a resin, a bead, an organic particle, a gel, an organic surface, an organometallic compound, a cell, and combinations thereof.

Preferably, for RG3 the radical is a a nucleic acid, a peptide, a protein, a carbohydrate, a lipid, a polymer, an amino acid, a chelating moiety, a drug, or a gel.

As used herein, a nucleic acid is preferably selected from the group consisting of an oligonucleotide, a polynucleotide, DNA, and RNA.

As used herein, a protein is preferably an antibody or a diabody. A preferred antibody is CC49, and a preferred diabody is AVP0458.

As used herein, a carbohydrate is preferably selected from the group consisting of a monosaccharide, an oligosaccharide, and a polysaccharide.

As used herein, a polymer is typically selected from the group consisting of polyethyleneglycol (PEG), poly(N-(2-hydroxypropyl)methacrylamide) (HPMA), polylactic acid (PLA), polylactic-glycolic acid (PLGA), polyglutamic acid (PG), polyvinylpyrrolidone (PVP), poly(1-hydroxymethylethylene hydroxymethyl-formal (PHF), copolymers of a polyacetal/polyketal and a hydrophilic polymer selected from the group consisting of polyacrylates, polyvinyl polymers, polyesters, polyorthoesters, polyamides, oligopeptides, polypeptides and derivatives thereof, oligopeptides, polypeptides, glycopolysaccharides, and polysaccharides such as dextran and hyaluronan. Preferably, a polymer as used herein is polyethylene glycol (PEG).

As used herein, a resin is preferably a polystyrene resin or an agarose resin.

As used herein, an organic particle is preferably a liposome or a polymersome.

As used herein, a chelating moiety is preferably selected from the group consisting of DTPA (diethylenetriaminepentaacetic acid), DOTA (1,4,7,10- tetraazacyclododecane-N,N',N",N"-tetraacetic acid), NOTA (1,4,7-triazacyclononane-N,N',N"-triacetic acid), TETA (1,4,8,11-tetraazacyclotetradecane-N,N',N",N'-tetraacetic acid), OTTA (N1-(*p*-isothiocyanatobenzyl)-diethylenetriamine-N₁,N₂,N₃,N₃-tetraacetic acid), deferoxamine or DFA (N'-[5-[[4-[[5-(acetylhydroxyamino)pentyl]amino]-1,4-dioxobutyl]hydroxyamino]pentyl]-N-(5-aminopentyl)-N-hydroxybutanediamide) or HYNIC (hydrazinonicotinamide), EDTA (ethylenediaminetetraacetic acid), OTAM, TACN, sarcophagine, and 3,4-HOPO-based chelators.

More preferably, herein a chelating moiety is selected from the group consisting of 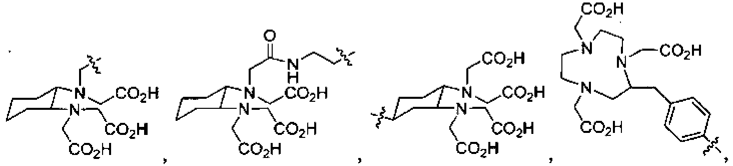 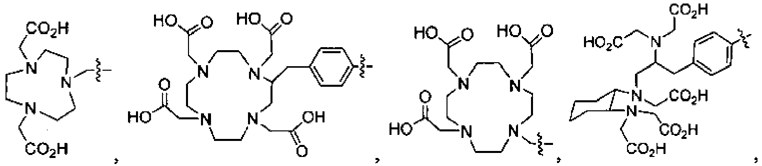 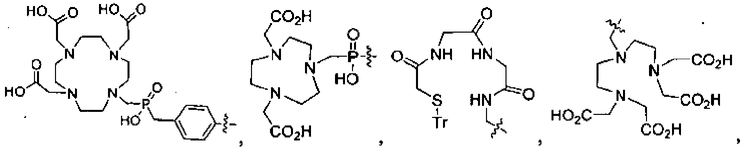 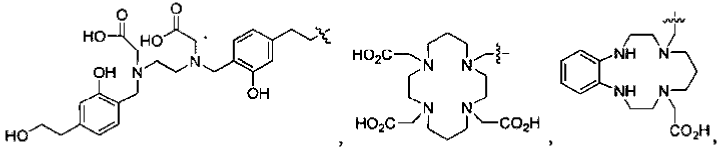 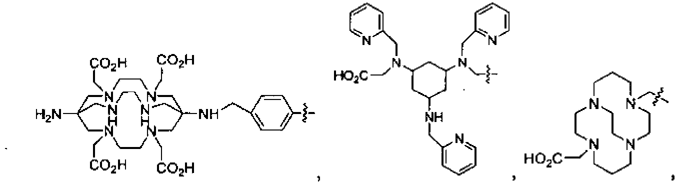 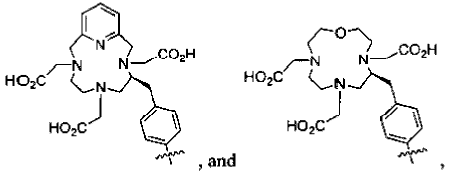 wherein the wiggly line denotes a bond to the remaining part of the molecule, optionally bound via -C(O)NH-, wherein the chelator moieties according to said group optionally chelate a metal, wherein the metal is preferably selected from the group consisting of ⁴⁴Sc,⁶²Cu, ⁶⁴Cu, ⁶⁶Ga, ⁶⁷Ga, ⁶⁷Cu, ⁶⁸Ga, ⁸⁶Y, ⁸⁹Zr, ⁹⁰Y, ^{99m}Tc, ¹¹¹In, ¹⁶⁶Ho, ¹⁷⁷Lu, ¹⁸⁶Re, ¹⁸⁸Re, ²¹¹Bi, ²¹²Bi, ²¹²Pb, ²¹³Bi, ²¹⁴Bi, and ²²⁵Ac.

### Radical Group 4: inorganic molecule

For Radical Group 4 (RG4), the radical is an inorganic molecule selected from the group consisting of an inorganic surface, an inorganic particle, an allotrope of carbon, an inorganic drug, a radionuclide, and combinations thereof.

As used herein, an inorganic surface is preferably selected from the group consisting of chips, wafers, metal such as gold, and silica-based surfaces such as glass.

As used herein, an inorganic particle is preferably selected from the group consisting of beads, silica-based particles, polymer-based materials, and iron oxide particles. Preferably, a bead is a magnetic bead or a gold bead.

As used herein, an allotrope of carbon is preferably selected from the group consisting of fullerenes such as Buckminsterfullerene; graphite, graphene, diamond, Lonsdaleite, Q-carbon, linearn acetylenic carbon, amorphous carbon, and carbon nanotubes.

As used herein, an inorganic drug is preferably cisplatin.

### Radical group 5: further terminal groups

For RG5 the radical is: 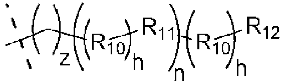 wherein the dashed line indicates a bond to the remaining part of the dienophile or diene.

For RG5, each R₁₀ is independently selected from RG2, preferably from RG2a.

For RG5, each R₁₁ is independently selected from RG2, preferably not being RG2a, RG2b, or RG2c.

For RG5, R₁₂ is selected from RG1 or RG3, preferably RG3, more preferably a protein, polymer, or chelating moiety.

Preferably, z is an integer in a range of from 0 to 12, preferably from 0 to 10, more preferably from 0 to 8, even more preferably from 1 to 6, most preferably from 2 to 4. Preferably, z is 0. In case the compound according to the disclosure comprises more than one moiety RG5, each z is independently selected.

Preferably, h is 0 or 1. In case the compound according to the disclosure comprises more than one moiety RG5, each h, z, and n is independently selected. Preferably, each n belonging to RG5 is an integer independently selected from a range of from 0 to 24, preferably from 1 to 12, more preferably from 1 to 6, even more preferably from 1 to 3. Preferably, n is 1. In other preferred embodiments n is an integer in the range from 12 to 24.

Preferably, z is 0, and n is 1. In other embodiments, z is 1, and n is 1. Preferably, the moiety RG5 has a molecular weight in a range of from 100 Da to 3000 Da, preferably, in a range of from 100 Da to 2000 Da, more preferably, in a range of from 100 Da to 1500 Da, even more preferably in a range of from 150 Da to 1500 Da. Even more preferably still, the moiety RG5 has a molecular weight in a range of from 150 Da to 1000 Da, most preferably in a range of from 200 Da to 1000 Da.

Preferably, RG5 is selected from the group RG5a consisting of: 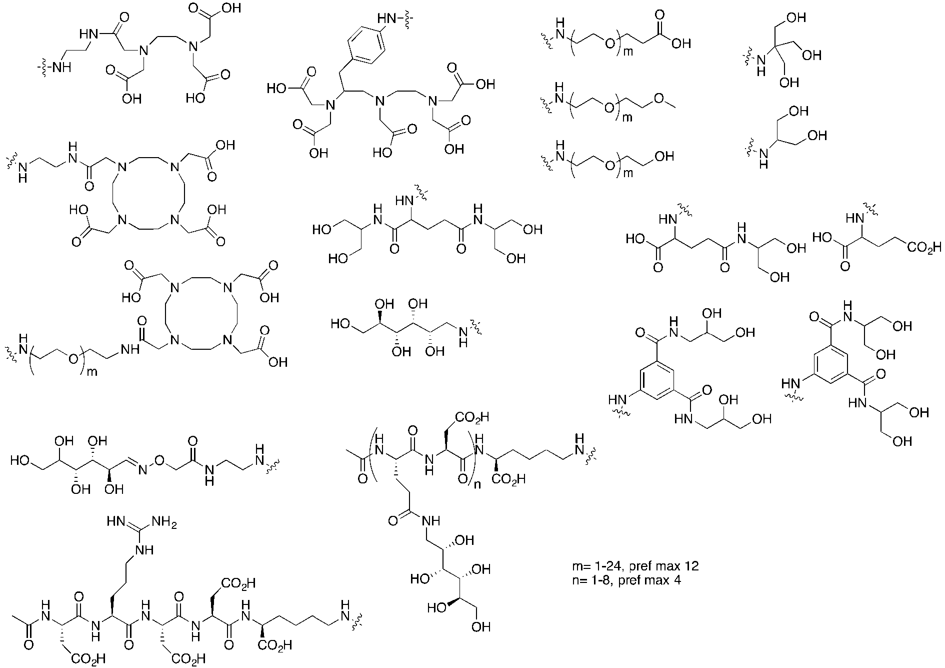 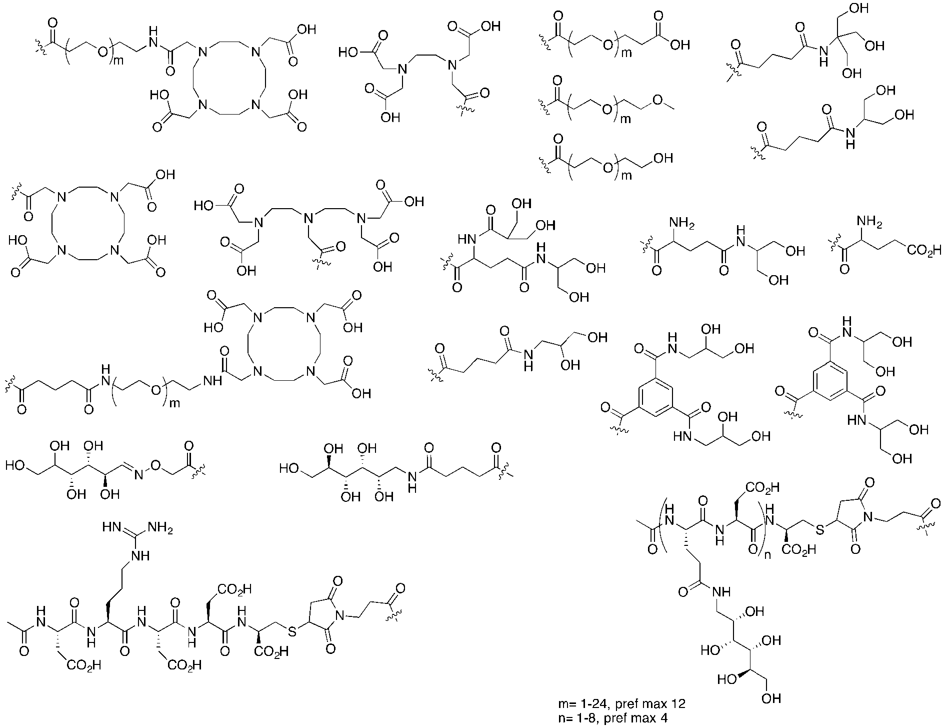 , wherein the wiggly line denotes a bond to the remainder of the molecule.

It is understood that when n is more than 1, -((R₁₀)ₕ-R₁₁)ₙ-(R₁₀)ₕ-R₁₂ may be preceded by a group -(R₁₀)ₕ-R₁₁- so as to form a group -(R₁₀)ₕ-R₁₁-((R₁₀)ₕ-R₁₁)ₙ-(R₁₀)ₕ-R₁₂. It is understood that this follows from the definition of how to write out the repeating units, i.e.
-((R₁₀)ₕ-R₁₁)₂- would first be written as -(R₁₀)ₕ-R₁₁-(R₁₀)ₕ-R₁₁- before R₁₀, h, and R₁₁ are independently selected.

### List of Clauses

The disclosure pertains to any one of the following clauses.
Clause 1. A compound or a salt, hydrate, or solvate thereof; wherein said compound comprises an eight-membered non-aromatic cyclic mono-alkenylene moiety, wherein said moiety comprises a non-vinylic carbon atom, wherein said non-vinylic carbon atom is substituted with at least one structure according to Formula (A): 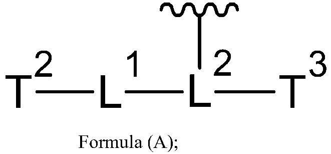 wherein
   L¹ and L² are each independently a linker; and T² and T³ are organic moieties.
Clause 2. A compound of Clause 1 or a salt, hydrate, or solvate thereof; wherein L¹ is according to Radical Group 2 as defined herein.
Clause 3. A compound of any one of Clauses 1-2 or a salt, hydrate, or solvate thereof; wherein L¹ is selected from the group consisting of linear or branched C₁-C₁₂ (hetero)alkylene, C₃-C₈ (hetero)cycloalkylene, C₆-C₁₂ arylene, and C₄-C₁₁ heteroarylene.
Clause 4. A compound of any one of Clauses 1-3 or a salt, hydrate, or solvate thereof; wherein L¹ is selected from the group consisting of linear or branched C₁-C₁₂ alkylene, C₃-C₈ (hetero)cycloalkylene, C₆-C₁₂ arylene, and C₄-C₁₁ heteroarylene.
Clause 5. A compound of any one of Clauses 1-4 or a salt, hydrate, or solvate thereof; wherein L¹ is selected from the group consisting of linear or branched C₂-C₁₂ alkylene, C₃-C₈ (hetero)cycloalkylene, C₆-C₁₂ arylene, and C₄-C₁₁ heteroarylene.
Clause 6. A compound of any one of Clauses 1-5 or a salt, hydrate, or solvate thereof; wherein L¹ is selected from the group consisting of linear or branched C₃-C₁₂ alkylene, C₃-C₈ (hetero)cycloalkylene, C₆-C₁₂ arylene, and C₄-C₁₁ heteroarylene.
Clause 7. A compound of any one of Clauses 1-6 or a salt, hydrate, or solvate thereof; wherein L¹ is selected from the group consisting of linear or branched C₄-C₁₂ alkylene, C₃-C₈ (hetero)cycloalkylene, C₆-C₁₂ arylene, and C₄-C₁₁ heteroarylene.
Clause 8. A compound of any one of Clauses 1-7 or a salt, hydrate, or solvate thereof; wherein L¹ is linear or branched C₁-C₁₂ alkylene.
Clause 9. A compound of any one of Clauses 1-8 or a salt, hydrate, or solvate thereof; wherein L¹ is linear or branched C₂-C₁₂ alkylene.
Clause 10. A compound of any one of Clauses 1-9 or a salt, hydrate, or solvate thereof; wherein L¹ is linear or branched C₃-C₁₂ alkylene.
Clause 11. A compound of any one of Clauses 1-10 or a salt, hydrate, or solvate thereof; wherein L¹ is linear or branched C₄-C₁₂ alkylene.
Clause 12. A compound of any one of Clauses 1-11 or a salt, hydrate, or solvate thereof; wherein L¹ is linear or branched C₄-C₁₁ alkylene.
Clause 13. A compound of any one of Clauses 1-12 or a salt, hydrate, or solvate thereof; wherein L¹ is linear or branched C₄-C₁₀ alkylene.
Clause 14. A compound of any one of Clauses 1-13 or a salt, hydrate, or solvate thereof; wherein L¹ is linear or branched C₄-C₉ alkylene.
Clause 15. A compound of any one of Clauses 1-14 or a salt, hydrate, or solvate thereof; wherein L¹ is linear or branched C₄-C₈ alkylene.
Clause 16. A compound of any one of Clauses 1-15 or a salt, hydrate, or solvate thereof; wherein L¹ is linear or branched C₄-C₇ alkylene.
Clause 17. A compound of any one of Clauses 1-16 or a salt, hydrate, or solvate thereof; wherein L¹ is linear or branched C₄-C₆ alkylene.
Clause 18. A compound of any one of Clauses 1-17 or a salt, hydrate, or solvate thereof; wherein L¹ is linear or branched C₅ alkylene.
Clause 19. A compound of any one of Clauses 1-8 or a salt, hydrate, or solvate thereof; wherein L¹ is linear C₁-C₁₂ alkylene.
Clause 20. A compound of any one of Clauses 1-9 or a salt, hydrate, or solvate thereof; wherein L¹ is linear C₂-C₁₂ alkylene.
Clause 21. A compound of any one of Clauses 1-10 or a salt, hydrate, or solvate thereof; wherein L¹ is linear C₃-C₁₂ alkylene.
Clause 22. A compound of any one of Clauses 1-11 or a salt, hydrate, or solvate thereof; wherein L¹ is linear C₄-C₁₂ alkylene.
Clause 23. A compound of any one of Clauses 1-12 or a salt, hydrate, or solvate thereof; wherein L¹ is linear C₄-C₁₁ alkylene.
Clause 24. A compound of any one of Clauses 1-13 or a salt, hydrate, or solvate thereof; wherein L¹ is linear C₄-C₁₀ alkylene.
Clause 25. A compound of any one of Clauses 1-14 or a salt, hydrate, or solvate thereof; wherein L¹ is linear C₄-C₉ alkylene.
Clause 26. A compound of any one of Clauses 1-15 or a salt, hydrate, or solvate thereof; wherein L¹ is linear C₄-C₈ alkylene.
Clause 27. A compound of any one of Clauses 1-16 or a salt, hydrate, or solvate thereof; wherein L¹ is linear C₄-C₇ alkylene.
Clause 28. A compound of any one of Clauses 1-17 or a salt, hydrate, or solvate thereof; wherein L¹ is linear C₄-C₆ alkylene.
Clause 29. A compound of any one of Clauses 1-28 or a salt, hydrate, or solvate thereof; wherein L¹ is linear C₅ alkylene.
Clause 30. A compound of any one of Clauses 1-29 or a salt, hydrate, or solvate thereof; wherein L² is according to Radical Group 2 as defined herein.
Clause 31. A compound of any one of Clauses 1-30 or a salt, hydrate, or solvate thereof; wherein L² contains of from 1 to 200 atoms, preferably of from 2 to 150 atoms, more preferably of from 3 to 100 atoms, even more preferably of from 4 to 90 atoms, more preferably still of from 5 to 80 atoms, yet more preferably of from 6 to 70 atoms, even more preferably of from 7 to 60 atoms, more preferably still of from 8 to 50 atoms, even more preferably of from 9 to 45 atoms, and most preferably of from 10 to 35 atoms.
Clause 32. A compound of any one of Clauses 1-31 or a salt, hydrate, or solvate thereof; wherein L² is selected from the group consisting of linear or branched C₁-C₁₂ (hetero)alkanetriyl, C₃-C₈ (hetero)cycloalkanetriyl, C₆-C₁₂ arenetriyl, and C₄-C₁₁ heteroarenetriyl.
Clause 33. A compound of any one of Clauses 1-32 or a salt, hydrate, or solvate thereof; wherein L² is a linear or branched C₁-C₁₂ (hetero)alkanetriyl.
Clause 34. A compound of any one of Clauses 1-33 or a salt, hydrate, or solvate thereof; wherein L² is a linear or branched C₁-C₁₂ heteroalkanetriyl.
Clause 35. A compound of any one of Clauses 1-33 or a salt, hydrate, or solvate thereof; wherein L² is a branched C₁-C₁₂ (hetero)alkanetriyl.
Clause 36. A compound of any one of Clauses 1-35 or a salt, hydrate, or solvate thereof; wherein L² is a branched C₁-C₁₂ heteroalkanetriyl.
Clause 37. A compound of any one of Clauses 1-36 or a salt, hydrate, or solvate thereof; wherein L² is a branched C₃-C₁₁ heteroalkanetriyl.
Clause 38. A compound of any one of Clauses 1-37 or a salt, hydrate, or solvate thereof; wherein L² is a branched C₆-C₁₀ heteroalkanetriyl.
Clause 39. A compound of any one of Clauses 1-38 or a salt, hydrate, or solvate thereof; wherein L² is a branched C₈ heteroalkanetriyl.
Clause 40. A compound of any one of Clauses 1-39 or a salt, hydrate, or solvate thereof; wherein L² is a branched C₈ heteroalkanetriyl substituted with up to five =O groups.
Clause 41. A compound of any one of Clauses 1-40 or a salt, hydrate, or solvate thereof; wherein L² is a branched C₈ heteroalkanetriyl substituted with three =O groups.
Clause 42. A compound of any one of Clauses 1-41 or a salt, hydrate, or solvate thereof; wherein L² is a branched C₈ heteroalkanetriyl containing up to five -NH- groups.
Clause 43. A compound of any one of Clauses 1-42 or a salt, hydrate, or solvate thereof; wherein L² is a branched C₈ heteroalkanetriyl containing three -NH- groups.
Clause 44. A compound of any one of Clauses 1-43 or a salt, hydrate, or solvate thereof; wherein L² is a branched C₈ heteroalkanetriyl containing three -NH- groups, and wherein the C₈ heteroalkanetriyl is substituted with three =O groups.
Clause 45. A compound of any one of Clauses 1-44 or a salt, hydrate, or solvate thereof; wherein L² is: 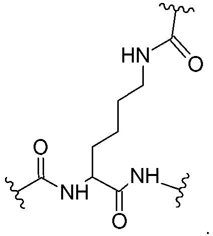
Clause 46. A compound of any one of Clauses 1-45 or a salt, hydrate, or solvate thereof; wherein L² is: 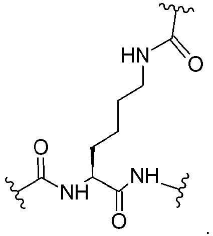
Clause 47. A compound of any one of Clauses 1-45 or a salt, hydrate, or solvate thereof; wherein L² is: 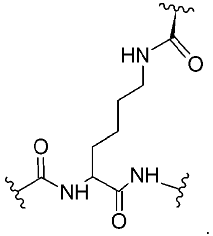
Clause 48. A compound of any one of Clauses 1-47 or a salt, hydrate, or solvate thereof; wherein L² is: 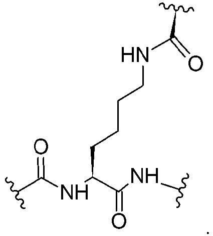
Clause 49. A compound of any one of Clauses 1-48 or a salt, hydrate, or solvate thereof; wherein T² is according to any one of Radical Group 1, Radical Group 3, or Radical Group 5, as defined herein, or wherein T² is a group -L³-C^{B}; wherein L³ is according to Radical Group 2, and C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof.
Clause 50. A compound of any one of Clauses 1-49 or a salt, hydrate, or solvate thereof; wherein T² is according to Radical Group 1 as defined herein.
Clause 51. A compound of any one of Clauses 1-50 or a salt, hydrate, or solvate thereof; wherein T² is according to Radical Group 1a as defined herein.
Clause 52. A compound of any one of Clauses 1-51 or a salt, hydrate, or solvate thereof; wherein T² is according to Radical Group 1b as defined herein.
Clause 53. A compound of any one of Clauses 1-52 or a salt, hydrate, or solvate thereof; wherein T² is according to Radical Group 1c as defined herein.
Clause 54. A compound of any one of Clauses 1-53 or a salt, hydrate, or solvate thereof; wherein T² is according to Radical Group 1d as defined herein.
Clause 55. A compound of any one of Clauses 1-54 or a salt, hydrate, or solvate thereof; wherein T² is according to Radical Group 1e as defined herein.
Clause 56. A compound of any one of Clauses 1-55 or a salt, hydrate, or solvate thereof; wherein T² is according to Radical Group 1f as defined herein.
Clause 57. A compound of any one of Clauses 1-56 or a salt, hydrate, or solvate thereof; wherein T² is N-maleimidyl.
Clause 58. A compound of any one of Clauses 1-57 or a salt, hydrate, or solvate thereof; wherein T² is: 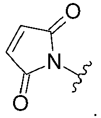
Clause 59. A compound of any one of Clauses 1-49 or a salt, hydrate, or solvate thereof; wherein T² is a group -L³-C^{B}.
Clause 60. A compound of any one of Clauses 1-49, and 59, or a salt, hydrate, or solvate thereof; wherein L³ is a residue of a bioconjugation moiety.
Clause 61. A compound of any one of Clauses 1-49, and 59-60, or a salt, hydrate, or solvate thereof; wherein L³ is a residue of an N-maleimidyl moiety or a residue of an N-hydroxysuccinimidyl moiety.
Clause 62. A compound of any one of Clauses 1-49, and 59-61, or a salt, hydrate, or solvate thereof; wherein T² is selected from the group consisting of 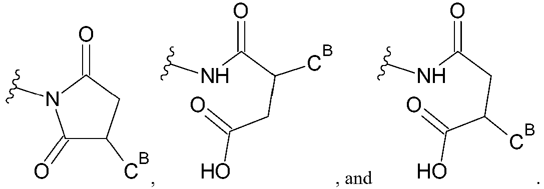
Clause 63. A compound of any one of Clauses 1-49, and 59-62, or a salt, hydrate, or solvate thereof; wherein T² is: 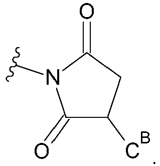
Clause 64. A compound of any one of Clauses 1-49, and 59-63, or a salt, hydrate, or solvate thereof; wherein C^{B} is a protein.
Clause 65. A compound of any one of Clauses 1-49, and 59-64, or a salt, hydrate, or solvate thereof; wherein C^{B} is an antibody or a diabody.
Clause 66. A compound of any one of Clauses 1-49, and 59-65, or a salt, hydrate, or solvate thereof; wherein C^{B} is a diabody.
Clause 67. A compound of any one of Clauses Clauses 1-49, and 59-66, or a salt, hydrate, or solvate thereof; wherein C^{B} is AVP0458 consisting of two monomers, wherein each of the two monomers has an amino acid sequence according to SEQ ID NO: 1.
Clause 68. A compound of any one of Clauses 1-49, and 59-67, or a salt, hydrate, or solvate thereof; wherein C^{B} is linked to the remainder of T² via S or N that is part of C^{B}.
Clause 69. A compound of any one of Clauses 1-49, and 59-68, or a salt, hydrate, or solvate thereof; wherein C^{B} is linked to the remainder of T² via S that is part of C^{B}.
Clause 70. A compound of any one of Clauses 1-69, or a salt, hydrate, or solvate thereof; wherein T³ is according to any one of Radical Group 1, Radical Group 3, or Radical Group 5, as defined herein.
Clause 71. A compound of any one of Clauses 1-70 or a salt, hydrate, or solvate thereof; wherein T³ is according to Radical Group 3, as defined herein.
Clause 72. A compound of any one of Clauses 1-71 or a salt, hydrate, or solvate thereof; wherein T³ is a polymer.
Clause 73. A compound of any one of Clauses 1-72 or a salt, hydrate, or solvate thereof; wherein T³ is a polymer comprising a polyethylene glycol moiety.
Clause 74. A compound of any one of Clauses 1-73 or a salt, hydrate, or solvate thereof; wherein T³ comprises a moiety - (CH₂CH₂-O-)_{y}-T⁴, wherein y is an integer in a range of from 1 to 50, and T⁴ is according to Radical Group 1, Radical Group 3, Radical Group 4, or Radical Group 5 as defined herein;
   preferably y is an integer in a range of from 10 to 40, more preferably in a range of from 12 to 37, even more preferably in a range of from 15 to 35, more preferably still in a range of from 20 to 30, even more preferably in a range of from 23 to 25, and most preferably y is 24.
Clause 75. A compound of Clause 74 or a salt, hydrate, or solvate thereof; wherein T³ is a moiety -(CH₂CH₂-O-)_{y}-T⁴.
Clause 76. A compound of any one of Clauses 74-75 or a salt, hydrate, or solvate thereof; wherein y is an integer in a range of from 10 to 40.
Clause 77. A compound of any one of Clauses 74-76 or a salt, hydrate, or solvate thereof; wherein y is an integer in a range of from 12 to 37.
Clause 78. A compound of any one of Clauses 74-77 or a salt, hydrate, or solvate thereof; wherein y is an integer in a range of from 15 to 35.
Clause 79. A compound of any one of Clauses 74-78 or a salt, hydrate, or solvate thereof; wherein y is an integer in a range of from 20 to 30.
Clause 80. A compound of any one of Clauses 74-79 or a salt, hydrate, or solvate thereof; wherein y is an integer in a range of from 23 to 25.
Clause 81. A compound of any one of Clauses 74-80 or a salt, hydrate, or solvate thereof; wherein y is 24.
Clause 82. A compound of any one of Clauses 74-81 or a salt, hydrate, or solvate thereof; wherein T⁴ is according to Radical Group 1.
Clause 83. A compound of any one of Clauses 74-82 or a salt, hydrate, or solvate thereof; wherein T⁴ is according to Radical Group 1a.
Clause 84. A compound of any one of Clauses 74-83 or a salt, hydrate, or solvate thereof; wherein T⁴ is according to Radical Group 1b.
Clause 85. A compound of any one of Clauses 74-84 or a salt, hydrate, or solvate thereof; wherein T⁴ is according to Radical Group 1c.
Clause 86. A compound of any one of Clauses 74-85 or a salt, hydrate, or solvate thereof; wherein T⁴ is according to Radical Group 1d.
Clause 87. A compound of any one of Clauses 74-86 or a salt, hydrate, or solvate thereof; wherein T⁴ is according to Radical Group 1e.
Clause 88. A compound of any one of Clauses 74-87 or a salt, hydrate, or solvate thereof; wherein T⁴ is methyl.
Clause 89. A compound of any one of Clauses 1-81 or a salt, hydrate, or solvate thereof; wherein T³ is a moiety -(CH₂CH₂-O-)₂₄-CH₃.
Clause 90. A compound of any one of Clauses 1-29, and 49-89 or a salt, hydrate, or solvate thereof; wherein Formula (A) is according to Formula (A1): 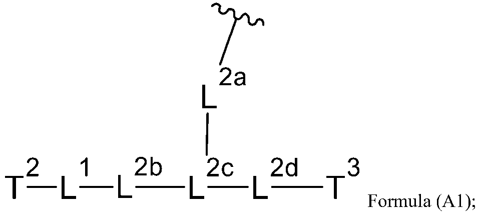
   wherein L¹ is according to any one of Clauses 1-29;
   wherein T² is according to any one of Clauses 1, and 49-69;
   wherein T³ is according to any one of Clauses 1, and 70-89;
   and L^{2a}, L²⁸, L^{2c}, and L^{2d} are each independently a linker.
Clause 91. A compound of Clause 90 or a salt, hydrate, or solvate thereof; wherein L^{2a}, L^{2b}, L^{2c}, and L^{2d} are each independently according to Radical Group 2 as defined herein.
Clause 92. A compound of any one of Clauses 90-91 or a salt, hydrate, or solvate thereof; wherein L^{2a} is a linker containing at most twenty atoms.
Clause 93. A compound of any one of Clauses 90-92 or a salt, hydrate, or solvate thereof; wherein L^{2a} is a linker containing at most fifteen atoms.
Clause 94. A compound of any one of Clauses 90-93 or a salt, hydrate, or solvate thereof; wherein L^{2a} is a linker containing at most ten atoms.
Clause 95. A compound of any one of Clauses 90-94 or a salt, hydrate, or solvate thereof; wherein L^{2a} is a linker containing at most five atoms.
Clause 96. A compound of any one of Clauses 90-95 or a salt, hydrate, or solvate thereof; wherein L^{2a} is selected from the group consisting of -C(O)NL^{2T}-, -NL^{2T}C(O)-, -O-, -S-,
   -NL^{2T}-, -N=N-, and -C(O)-; wherein L^{2T} is hydrogen or methyl.
Clause 97. A compound of Clause 96 or a salt, hydrate, or solvate thereof; wherein L^{2a} is selected from the group consisting of -C(O)NL^{2T}-, and -NL^{2T}C(O)-.
Clause 98. A compound of any one of Clauses 90-97 or a salt, hydrate, or solvate thereof; wherein L^{2a} is selected from the group consisting of -C(O)NH-, and -NHC(O)-.
Clause 99. A compound of any one of Clauses 90-98 or a salt, hydrate, or solvate thereof; wherein L^{2a} is -NHC(O)-.
Clause 100. A compound of any one of Clauses 90-99 or a salt, hydrate, or solvate thereof; wherein L^{2b} is a linker containing at most twenty atoms.
Clause 101. A compound of any one of Clauses 90-100 or a salt, hydrate, or solvate thereof; wherein L^{2b} is a linker containing at most fifteen atoms.
Clause 102. A compound of any one of Clauses 90-101 or a salt, hydrate, or solvate thereof; wherein L^{2d} is a linker containing at most ten atoms.
Clause 103. A compound of any one of Clauses 90-102 or a salt, hydrate, or solvate thereof; wherein L^{2b} is a linker containing at most five atoms.
Clause 104. A compound of any one of Clauses 90-103 or a salt, hydrate, or solvate thereof; wherein L^{2d} is selected from the group consisting of -C(O)NL^{2T}-, -NL^{2T}C(O)-, -O-, -S-,
   -NL^{2T}-, -N=N-, and -C(O)-; wherein L^{2T} is hydrogen or methyl.
Clause 105. A compound of Clause 104 or a salt, hydrate, or solvate thereof; wherein L^{2b} is selected from the group consisting of -C(O)NL^{2T}-, and -NL^{2T}C(O)-.
Clause 106. A compound of any one of Clauses 90-105 or a salt, hydrate, or solvate thereof; wherein L^{2d} is selected from the group consisting of -C(O)NH-, and -NHC(O)-.
Clause 107. A compound of any one of Clauses 90-106 or a salt, hydrate, or solvate thereof; wherein L^{2d} is -NHC(O)-.
Clause 108. A compound of any one of Clauses 90-107 or a salt, hydrate, or solvate thereof; wherein L^{2d} is a linker containing at most twenty atoms.
Clause 109. A compound of any one of Clauses 90-108 or a salt, hydrate, or solvate thereof; wherein L^{2d} is a linker containing at most fifteen atoms.
Clause 110. A compound of any one of Clauses 90-109 or a salt, hydrate, or solvate thereof; wherein L^{2d} is a linker containing at most ten atoms.
Clause 111. A compound of any one of Clauses 90-110 or a salt, hydrate, or solvate thereof; wherein L^{2d} is a linker containing at most five atoms.
Clause 112. A compound of any one of Clauses 90-111 or a salt, hydrate, or solvate thereof; wherein L^{2d} is selected from the group consisting of -C(O)NL^{2T}-, -NL^{2T}C(O)-, -O-, -S-,
   -NL^{2T}-, -N=N-, and -C(O)-; wherein L^{2T} is hydrogen or methyl.
Clause 113. A compound of Clause 112 or a salt, hydrate, or solvate thereof; wherein L^{2d} is selected from the group consisting of -C(O)NL^{2T}-, and -NL^{2T}C(O)-.
Clause 114. A compound of any one of Clauses 90-113 or a salt, hydrate, or solvate thereof; wherein L^{2d} is selected from the group consisting of -C(O)NH-, and -NHC(O)-.
Clause 115. A compound of any one of Clauses 90-114 or a salt, hydrate, or solvate thereof; wherein L^{2d} is -C(O)NH-.
Clause 116. A compound of any one of Clauses 90-115 or a salt, hydrate, or solvate thereof; wherein L^{2c} is a linker comprising at most 50 atoms.
Clause 117. A compound of any one of Clauses 90-116 or a salt, hydrate, or solvate thereof; wherein L^{2c} is a linker comprising at most 40 atoms.
Clause 118. A compound of any one of Clauses 90-117 or a salt, hydrate, or solvate thereof; wherein L^{2c} is a linker comprising at most 30 atoms.
Clause 119. A compound of any one of Clauses 90-118 or a salt, hydrate, or solvate thereof; wherein L^{2c} is a linker comprising at most 20 atoms.
Clause 120. A compound of any one of Clauses 90-119 or a salt, hydrate, or solvate thereof; wherein L^{2c} is a linker comprising at most 15 atoms.
Clause 121. A compound of any one of Clauses 90-120 or a salt, hydrate, or solvate thereof; wherein L^{2c} is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl.
Clause 122. A compound of any one of Clauses 90-121 or a salt, hydrate, or solvate thereof; wherein L^{2c} is C₁-C₈ (hetero)alkanetriyl.
Clause 123. A compound of any one of Clauses 90-122 or a salt, hydrate, or solvate thereof; wherein L^{2c} is C₁-C₈ alkanetriyl.
Clause 124. A compound of any one of Clauses 90-123 or a salt, hydrate, or solvate thereof; wherein L^{2c} is C₂-C₇ alkanetriyl.
Clause 125. A compound of any one of Clauses 90-124 or a salt, hydrate, or solvate thereof; wherein L^{2c} is C₃-C₆ alkanetriyl. Clause 126. A compound of any one of Clauses 90-125 or a salt, hydrate, or solvate thereof; wherein L^{2c} is C₄-C₅ alkanetriyl.
Clause 127. A compound of any one of Clauses 90-126 or a salt, hydrate, or solvate thereof; wherein L^{2c} is C₅ alkanetriyl.
Clause 128. A compound of any one of Clauses 90-127 or a salt, hydrate, or solvate thereof; wherein L^{2c} is >CH-CH₂-CH₂-CH₂-CH₂-.
Clause 129. A compound of any one of Clauses 1-128 or a salt, hydrate, or solvate thereof; wherein said non-vinylic carbon atom is substituted with at most one structure according to Formula (A).
Clause 130. A compound of any one of Clauses 1-129 or a salt, hydrate, or solvate thereof; wherein said non-vinylic carbon atom is a non-allylic carbon atom.
Clause 131. A compound of any one of Clauses 1-130 or a salt, hydrate, or solvate thereof; wherein said eight-membered non-aromatic cyclic mono-alkenylene moiety is substituted with at most three structures according to Formula (A).
Clause 132. A compound of any one of Clauses 1-131 or a salt, hydrate, or solvate thereof; wherein said eight-membered non-aromatic cyclic mono-alkenylene moiety is substituted with at most two structures according to Formula (A).
Clause 133. A compound of any one of Clauses 1-132 or a salt, hydrate, or solvate thereof; wherein said eight-membered non-aromatic cyclic mono-alkenylene moiety is substituted with at most one structure according to Formula (A).
Clause 134. A compound of any one of Clauses 1-133 or a salt, hydrate, or solvate thereof; wherein said eight-membered non-aromatic cyclic mono-alkenylene moiety comprises at most two heteroatoms.
Clause 135. A compound of any one of Clauses 1-134 or a salt, hydrate, or solvate thereof; wherein said eight-membered non-aromatic cyclic mono-alkenylene moiety comprises at most two heteroatoms, wherein the heteroatoms are N or O.
Clause 136. A compound of any one of Clauses 1-135 or a salt, hydrate, or solvate thereof; wherein said eight-membered non-aromatic cyclic mono-alkenylene moiety comprises at most one heteroatom, wherein the heteroatom is N or O.
Clause 137. A compound of any one of Clauses 1-136 or a salt, hydrate, or solvate thereof; wherein said eight-membered non-aromatic cyclic mono-alkenylene moiety comprises at most one heteroatom, wherein the heteroatom is N.
Clause 138. A compound of any one of Clauses 1-137 or a salt, hydrate, or solvate thereof; wherein said eight-membered non-aromatic cyclic mono-alkenylene moiety comprises at least five carbon atoms.
Clause 139. A compound of any one of Clauses 1-138 or a salt, hydrate, or solvate thereof; wherein said eight-membered non-aromatic cyclic mono-alkenylene moiety comprises at least six carbon atoms.
Clause 140. A compound of any one of Clauses 1-139 or a salt, hydrate, or solvate thereof; wherein said eight-membered non-aromatic cyclic mono-alkenylene moiety comprises at least seven carbon atoms.
Clause 141. A compound of any one of Clauses 1-140 or a salt, hydrate, or solvate thereof; wherein said eight-membered non-aromatic cyclic mono-alkenylene moiety is an all-carbon ring.
Clause 142. A compound of any one of Clauses 1-141 or a salt, hydrate, or solvate thereof; wherein said eight-membered non-aromatic cyclic mono-alkenylene moiety is further substituted with a moiety according to any one of Radical Group 1, Radical Group 3, Radical Group 4, or Radical Group 5, as defined herein.
Clause 143. A compound of any one of Clauses 1-142 or a salt, hydrate, or solvate thereof; wherein said eight-membered non-aromatic cyclic mono-alkenylene moiety is further substituted with at most 5 moieties according to any one of Radical Group 1, Radical Group 3, Radical Group 4, or Radical Group 5, as defined herein.
Clause 144. A compound of any one of Clauses 1-143 or a salt, hydrate, or solvate thereof; wherein said eight-membered non-aromatic cyclic mono-alkenylene moiety is further substituted with at most 4 moieties according to any one of Radical Group 1, Radical Group 3, Radical Group 4, or Radical Group 5, as defined herein.
Clause 145. A compound of any one of Clauses 1-144 or a salt, hydrate, or solvate thereof; wherein said eight-membered non-aromatic cyclic mono-alkenylene moiety is further substituted with at most 3 moieties according to any one of Radical Group 1, Radical Group 3, Radical Group 4, or Radical Group 5, as defined herein.
Clause 146. A compound of any one of Clauses 1-145 or a salt, hydrate, or solvate thereof; wherein said eight-membered non-aromatic cyclic mono-alkenylene moiety is further substituted with at most 2 moieties according to any one of Radical Group 1, Radical Group 3, Radical Group 4, or Radical Group 5, as defined herein.
Clause 147. A compound of any one of Clauses 1-146 or a salt, hydrate, or solvate thereof; wherein said eight-membered non-aromatic cyclic mono-alkenylene moiety is substituted with a group R₄₈, wherein R₄₈ is selected from the group consisting of -OH, -O-acetyl, -O-C₁₋₄ alkyl, halogen, active carbonate, and a releasable group;.
Clause 148. A compound of Clause 147 or a salt, hydrate, or solvate thereof; wherein said eight-membered non-aromatic cyclic mono-alkenylene moiety comprises at least one allylic carbon, and said at least one allylic carbon is substituted with said group R₄₈.
Clause 149. A compound of any one of Clauses 147-148 or a salt, hydrate, or solvate thereof; wherein said group R₄₈ is in the axial position.
Clause 150. A compound of any one of Clauses 147-149 or a salt, hydrate, or solvate thereof; wherein said group R₄₈ is a releasable group.
Clause 151. A compound of any one of Clauses 147-150 or a salt, hydrate, or solvate thereof; wherein said eight-membered non-aromatic cyclic mono-alkenylene moiety comprises at most one releasable group.
Clause 152. A compound of any one of Clauses 147-151 or a salt, hydrate, or solvate thereof; wherein said releasable group is -(Y¹-C(-Y²))ᵢ-(S^{P})ⱼ-C^{A}; wherein each of Y¹ and Y² are independently selected from O, and S; C^{A} is Construct A, which is a payload; S^{P} is a linker; j is 0 or 1; i is 0 or 1;
   if i is 0, -(S^{P})ⱼ-C^{A} is connected to the remainder of the compound via O or S, that is part of
      -(S^{P})ⱼ-C^{A};
   if i is 1, -(S^{P})ⱼ-C^{A} is connected to -C(=Y²)- via O, S, secondary N, or a tertiary N, that is part of -(S^{P})ⱼ-C^{A}.
Clause 153. A compound of any one of Clauses 147-152 or a salt, hydrate, or solvate thereof; wherein said releasable group is -(O-C(=Y²))ᵢ-(S^{P})ⱼ-C^{A}.
Clause 154. A compound of any one of Clauses 147-152 or a salt, hydrate, or solvate thereof; wherein said releasable group is -(Y¹-C(-O))ᵢ-(S^{P})ⱼ-C^{A}.
Clause 155. A compound of any one of Clauses 147-154 or a salt, hydrate, or solvate thereof; wherein said releasable group is -(O-C(-O))ᵢ-(S^{P})ⱼ-C^{A}.
Clause 156. A compound of any one of Clauses 147-155 or a salt, hydrate, or solvate thereof; wherein said releasable group is -O-C(-O)-(S^{P})ⱼ-C^{A}.
Clause 157. A compound of any one of Clauses 147-156 or a salt, hydrate, or solvate thereof; wherein S^{P} is according to Radical Group 2.
Clause 158. A compound of any one of Clauses 147-157 or a salt, hydrate, or solvate thereof; wherein S^{P} is a self-immolative linker.
Clause 159. A compound of any one of Clauses 147-158 or a salt, hydrate, or solvate thereof; wherein said releasable group is -O-C(-O)-C^{A}.
Clause 160. A compound of Clause 159 or a salt, hydrate, or solvate thereof; wherein C^{A} is linked to the moiety -O-C(=O)-via a secondary or tertiary nitrogen atom that is part of C^{A}, forming a carbamate.
Clause 161. A compound of any one of Clauses 147-160 or a salt, hydrate, or solvate thereof; wherein C^{A} is a drug, preferably monomethyl auristatin E (MMAE), exatecan, or an exatecan derivative.
Clause 162. A compound of any one of Clauses 147-161 or a salt, hydrate, or solvate thereof; wherein C^{A} is monomethyl auristatin E (MMAE).
Clause 163. A compound of any one of Clauses 1-162 or a salt, hydrate, or solvate thereof; wherein said eight-membered non-aromatic cyclic mono-alkenylene moiety comprises at least one allylic carbon, and said at least one allylic carbon is substituted with a group R₄₈, wherein said group R₄₈ is in the axial position, and wherein said group R₄₈ is -O-C(=O)-C^{A}; wherein C^{A} is a drug.
Clause 164. A compound of Clause 163 or a salt, hydrate, or solvate thereof; wherein C^{A} is monomethyl auristatin E (MMAE) linked to the moiety -O-C(=O)- via a secondary or tertiary nitrogen atom that is part of MMAE, forming a carbamate.
Clause 165. A compound of Clause 164 or a salt, hydrate, or solvate thereof; wherein C^{A} is monomethyl auristatin E (MMAE) linked to the moiety -O-C(=O)- via a tertiary nitrogen atom that is part of MMAE, forming a carbamate.
Clause 166. A compound of any one of Clauses 147-165 or a salt, hydrate, or solvate thereof; wherein said group R₄₈ is: 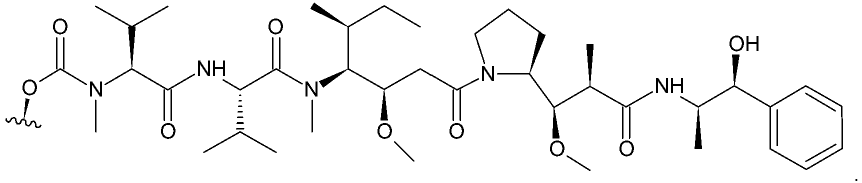
Clause 167. A compound of any one of Clauses 1-166 or a salt, hydrate, or solvate thereof; wherein said eight-membered non-aromatic cyclic mono-alkenylene moiety is substituted with at least one group T¹, wherein T¹ is according to Radical Group 1, Radical Group 3, Radical Group 4, or Radical Group 5, as defined herein.
Clause 168. A compound of Clause 167 or a salt, hydrate, or solvate thereof; wherein said eight-membered non-aromatic cyclic mono-alkenylene moiety is substituted with at most 5 groups T¹.
Clause 169. A compound of any one of Clauses 167-168 or a salt, hydrate, or solvate thereof; wherein said eight-membered non-aromatic cyclic mono-alkenylene moiety is substituted with at most 4 groups T¹.
Clause 170. A compound of any one of Clauses 167-169 or a salt, hydrate, or solvate thereof; wherein said eight-membered non-aromatic cyclic mono-alkenylene moiety is substituted with at most 3 groups T¹.
Clause 171. A compound of any one of Clauses 167-170 or a salt, hydrate, or solvate thereof; wherein said eight-membered non-aromatic cyclic mono-alkenylene moiety is substituted with at most 2 groups T¹.
Clause 172. A compound of any one of Clauses 167-171 or a salt, hydrate, or solvate thereof; wherein said eight-membered non-aromatic cyclic mono-alkenylene moiety is substituted with at most 1 group T¹.
Clause 173. A compound of any one of Clauses 167-172 or a salt, hydrate, or solvate thereof; wherein each T¹ is independently according to Radical Group 1 as defined herein.
Clause 174. A compound of any one of Clauses 167-173 or a salt, hydrate, or solvate thereof; wherein each T¹ is independently selected from the group consisting of -OT^{1A} , hydrogen, C₁-C₁₂ (hetero)alkyl, C₆ aryl, C₄-C₅ heteroaryl, C₃-C₆ (hetero)cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, - C(O)T^{1A}, -C(O)OT^{1A}, -O-C(O)T^{1A} -C(O)N(T^{1A})₂, -N(T^{1A})₂-CO-T^{1A}, and -Si(T^{1A})₃;
   each T^{1A} is independently selected from the group consisting of hydrogen, (hetero)alkyl, (hetero)alkenyl, (hetero)alkynyl, (hetero)aryl, and an amino acid residue;
   preferably each T^{1A} is independently selected from the group consisting of hydrogen, C₁-C₆ (hetero)alkyl, C₁-C₆ (hetero)alkenyl, C₁-C₆ (hetero)alkynyl, C₂-C₅ heteroaryl, phenyl, and an amino acid residue; even more preferably each T^{1A} is independently selected from the group consisting of hydrogen, C₁-C₄ (hetero)alkyl, C₁-C₄ (hetero)alkenyl, C₁-C₄ (hetero)alkynyl, C₃-C₅ heteroaryl, phenyl, an aspartic acid residue, a glutamic acid residue, and a glycine residue; even more preferably each T^{1A} is independently selected from the group consisting of hydrogen, C₁-C₃ alkyl, an aspartic acid residue, a glutamic acid residue, and a glycine residue; and most preferably T^{1A} is hydrogen..
Clause 175. A compound of Clauses 174 or a salt, hydrate, or solvate thereof; wherein each T¹ is independently selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃.
Clause 176. A compound of any one of Clauses 167-175 or a salt, hydrate, or solvate thereof; wherein T¹ is -OT^{1A}.
Clause 177. A compound of any one of Clauses 167-176 or a salt, hydrate, or solvate thereof; wherein T¹ is -OH.
Clause 178. A compound of any one of Clauses 167-177 or a salt, hydrate, or solvate thereof; wherein T¹ is in an axial position.
Clause 179. A compound of any one of Clauses 167-178 or a salt, hydrate, or solvate thereof; wherein T¹ is not a substituent on a vinylic carbon or an allylic atom of said eight-membered non-aromatic cyclic mono-alkenylene moiety.
Clause 180. A compound of any one of Clauses 1-179 or a salt, hydrate, or solvate thereof; wherein said eight-membered non-aromatic cyclic mono-alkenylene moiety comprises at least one allylic carbon, and said at least one allylic carbon is substituted with a group R₄₈, wherein said group R₄₈ is in the axial position, and wherein said group R₄₈ is -O-C(=O)-C^{A}; wherein C^{A} is a drug; and
   wherein said eight-membered non-aromatic cyclic mono-alkenylene moiety is substituted with at most one group T¹, wherein T¹ is -OH, and wherein T¹ is not a substituent on a vinylic carbon or an allylic atom of said eight-membered non-aromatic cyclic mono-alkenylene moiety.
Clause 181. A compound of Clause 1-180 or a salt, hydrate, or solvate thereof; wherein said group R₄₈ is: 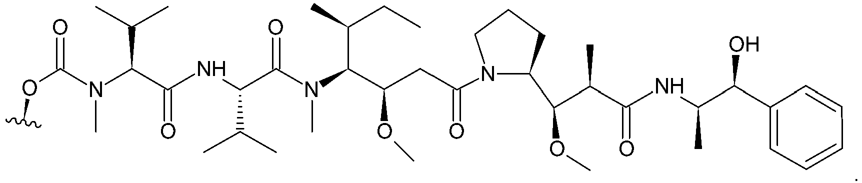
Clause 182. A compound of any one of Clauses 1-180 or a salt, hydrate, or solvate thereof; wherein said compound is according to Formula (B): 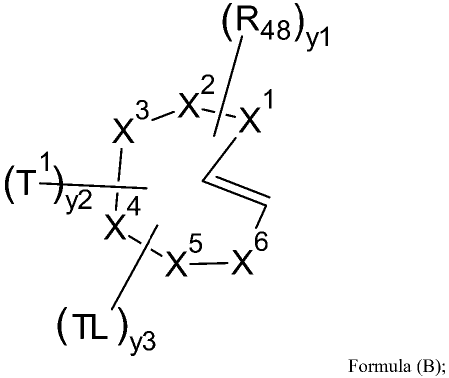 wherein
   R₄₈ is as defined in any one of Clauses 147-166;
   T¹ is as defined in any one of Clauses 167-179;
   TL is a structure according to Formula (A) as defined in any one of Clauses 1-128;
   y1 is an integer of from 0 to 4;
   y2 is an integer of from 0 to 5;
   y3 is an integer of from 1 to 5; and
   each of X¹, X², X³, X⁴, X⁵, and X⁶ is independently selected from the group consisting of a substituted or unsubstituted carbon atom, a nitrogen atom, or an oxygen atom, provided that if one of X¹, X², X³, X⁴, X⁵, and X⁶ is a nitrogen atom or an oxygen atom, an adjacent X¹, X², X³, X⁴, X⁵, and X⁶ is not a nitrogen atom or an oxygen atom.
Clause 183. A compound of Clause 182 or a salt, hydrate, or solvate thereof; wherein y1 is an integer of from 1 to 2.
Clause 184. A compound of any one of Clauses 182-183 or a salt, hydrate, or solvate thereof; wherein y1 is 1.
Clause 185. A compound of any one of Clauses 182-184 or a salt, hydrate, or solvate thereof; wherein y2 is an integer of from 1 to 4.
Clause 186. A compound of any one of Clauses 182-185 or a salt, hydrate, or solvate thereof; wherein y2 is an integer of from 1 to 3.
Clause 187. A compound of any one of Clauses 182-186 or a salt, hydrate, or solvate thereof; wherein y2 is an integer of from 1 to 2.
Clause 188. A compound of any one of Clauses 182-187 or a salt, hydrate, or solvate thereof; wherein y2 is 1.
Clause 189. A compound of any one of Clauses 182-188 or a salt, hydrate, or solvate thereof; wherein y3 is an integer of from 1 to 4.
Clause 190. A compound of any one of Clauses 182-189 or a salt, hydrate, or solvate thereof; wherein y3 is an integer of from 1 to 3.
Clause 191. A compound of any one of Clauses 182-190 or a salt, hydrate, or solvate thereof; wherein y3 is an integer of from 1 to 2.
Clause 192. A compound of any one of Clauses 182-191 or a salt, hydrate, or solvate thereof; wherein y3 is 1.
Clause 193. A compound of any one of Clauses 182-192 or a salt, hydrate, or solvate thereof; wherein each of X¹, X², X³, X⁴, X⁵, and X⁶ is independently a substituted or unsubstituted carbon atom.
Clause 194. A compound of any one of Clauses 182-193 or a salt, hydrate, or solvate thereof; wherein at least three of X¹, X², X³, X⁴, X⁵, and X⁶ are independently a substituted carbon atom.
Clause 195. A compound of any one of Clauses 182-194 or a salt, hydrate, or solvate thereof; wherein each substituted carbon atom is independently substituted with R₄₈, T¹, TL, and/or a moiety according to any one of Radical Group 1, Radical Group 3, Radical Group 4, or Radical Group 5.
Clause 196. A compound of any one of Clauses 182-195 or a salt, hydrate, or solvate thereof; wherein each substituted carbon atom is independently substituted with R₄₈, T¹, TL, and/or a moiety according to Radical Group 1.
Clause 197. A compound of any one of Clauses 182-196 or a salt, hydrate, or solvate thereof; wherein each substituted carbon atom is independently substituted with R₄₈, T¹, and/or TL.
Clause 198. A compound of any one of Clauses 182-197 or a salt, hydrate, or solvate thereof; wherein at most three of X¹, X², X³, X⁴, X⁵, and X⁶ are independently a substituted carbon atom.
Clause 199. A compound of any one of Clauses 182-198 or a salt, hydrate, or solvate thereof; wherein X¹ and/or X⁶ are independently a carbon atom substituted with R₄₈.
Clause 200. A compound of any one of Clauses 182-199 or a salt, hydrate, or solvate thereof; wherein one of X¹ and X⁶ is a carbon atom substituted with R₄₈.
Clause 201. A compound of any one of Clauses 182-200 or a salt, hydrate, or solvate thereof; wherein X¹ is a carbon atom substituted with R₄₈.
Clause 202. A compound of any one of Clauses 182-201 or a salt, hydrate, or solvate thereof; wherein X¹ is -CHR₄₈-.
Clause 203. A compound of any one of Clauses 182-202 or a salt, hydrate, or solvate thereof; wherein at least one of X², X³, X⁴, and X⁵ is independently a carbon atom substituted with T¹ and/or TL.
Clause 204. A compound of any one of Clauses 182-204 or a salt, hydrate, or solvate thereof; wherein one of X², X³, X⁴, and X⁵ is independently a carbon atom substituted with T¹ and/or TL.
Clause 205. A compound of any one of Clauses 182-204 or a salt, hydrate, or solvate thereof; wherein one of X², X³, X⁴, and X⁵ is independently a carbon atom substituted with T¹ and TL.
Clause 206. A compound of any one of Clauses 182-205 or a salt, hydrate, or solvate thereof; wherein X⁴ is a carbon atom substituted with T¹ and/or TL.
Clause 207. A compound of any one of Clauses 182-206 or a salt, hydrate, or solvate thereof; wherein X⁴ is a carbon atom substituted with T¹ and TL.
Clause 208. A compound of any one of Clauses 182-206 or a salt, hydrate, or solvate thereof; wherein X¹ is a carbon atom substituted with R₄₈, and X⁴ is a carbon atom substituted with T¹ and/or TL.
Clause 209. A compound of any one of Clauses 182-208 or a salt, hydrate, or solvate thereof; wherein X¹ is a carbon atom substituted with R₄₈, and X⁴ is a carbon atom substituted with T¹ and TL.
Clause 210. A compound of any one of Clauses 182-209 or a salt, hydrate, or solvate thereof; wherein X¹ is -CHR₄₈-, and X⁴ is -CT¹TL-.
Clause 211. A compound of any one of Clauses 182-210 or a salt, hydrate, or solvate thereof; wherein X², X³, X⁵, and X⁶ are unsubstituted carbon atoms.
Clause 212. A compound of any one of Clauses 182-211 or a salt, hydrate, or solvate thereof; wherein X², X³, X⁵, and X⁶ are -CH₂-.
Clause 213. A compound of any one of Clauses 182-212 or a salt, hydrate, or solvate thereof; wherein X¹ is -CHR₄₈-, X⁴ is - CT¹TL-, and X², X³, X⁵, and X⁶ are -CH₂-.
Clause 214. A compound of Clause 213 or a salt, hydrate, or solvate thereof; wherein R₄₈ is: 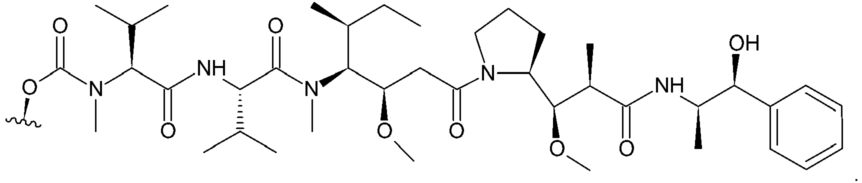
Clause 215. A compound of any one of Clauses 213-214 or a salt, hydrate, or solvate thereof; wherein T¹ is -OH.
Clause 216. A compound of any one of Clauses 213-215 or a salt, hydrate, or solvate thereof; wherein TL is 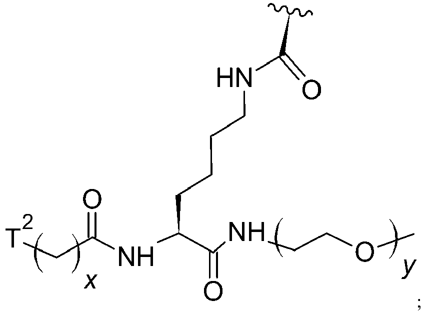 wherein
   x is an integer in a range of from 4 to 12;
   y is an integer in a range of from 15 to 35; and
   T² is selected from the group consisting of 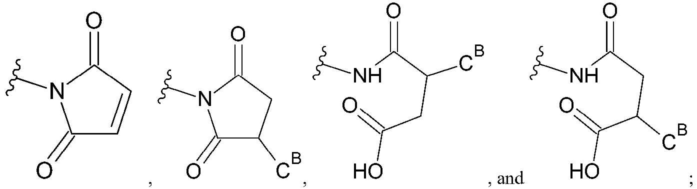 wherein
   C^{B} is a protein.
Clause 217. A compound of Clause 216 or a salt, hydrate, or solvate thereof; wherein
   x is an integer of from 4 to 6.
Clause 218. A compound of any one of Clauses 216-217 or a salt, hydrate, or solvate thereof; wherein x is 5.
Clause 219. A compound of any one of Clauses 216-218 or a salt, hydrate, or solvate thereof; wherein y is an integer of from 20 to 30.
Clause 220. A compound of any one of Clauses 216-219 or a salt, hydrate, or solvate thereof; wherein y is an integer of from 23 to 25.
Clause 221. A compound of any one of Clauses 216-220 or a salt, hydrate, or solvate thereof; wherein y is 24.
Clause 222. A compound of any one of Clauses 216-221 or a salt, hydrate, or solvate thereof; wherein T² is 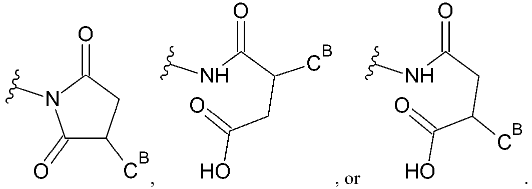
Clause 223. A compound of any one of Clauses 216-222 or a salt, hydrate, or solvate thereof; wherein T² is 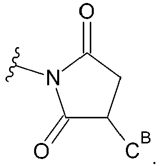
Clause 224. A compound of any one of Clauses 216-223 or a salt, hydrate, or solvate thereof; wherein C^{B} is AVP0458 consisting of two monomers, wherein each of the two monomers has an amino acid sequence according to SEQ ID NO: 1.
Clause 225. A compound of Clause 223 or a salt, hydrate, or solvate thereof; wherein C^{B} is linked to the maleimidyl group via a sulfur atom that is part of C^{B}, wherein the sulfur atom is part of a cysteine.
Clause 226. A compound of Clause 225 or a salt, hydrate, or solvate thereof; wherein C^{B} is AVP0458 consisting of two monomers, wherein each of the two monomers has an amino acid sequence according to SEQ ID NO: 1.
Clause 227. A compound of any one of Clauses 216-221 or a salt, hydrate, or solvate thereof; wherein T² is 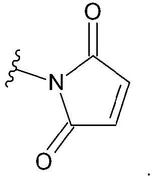
Clause 228. A compound of any one of Clauses 1-227 or a salt, hydrate, or solvate thereof; wherein said compound is according to Formula (C): 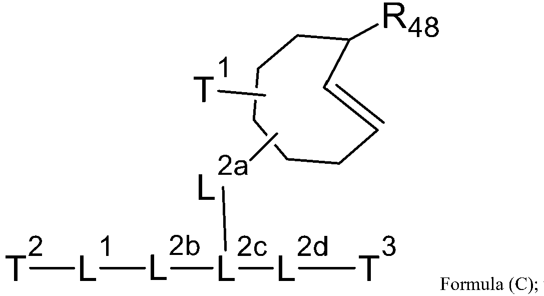 wherein
   R₄₈ is as defined in any one of Clauses 147-166;
   T¹ is as defined in any one of Clauses 167-179;
   T² is as defined in any one of Clauses 1, and 49-69;
   T³ is as defined in any one of Clauses 1, and 70-89;
   L¹ is as defined in any one of Clauses 1-29;
   L^{2a} is as defined in any one of Clauses 90-99;
   L²¹ is as defined in any one of Clauses 90, 91, and 100-107;
   L^{2c} is as defined in any one of Clauses 90, 91, and 116-128; and
   L^{2d} is as defined in any one of Clauses 90, 91, and 108-115.
Clause 229. A compound of Clause 228 or a salt, hydrate, or solvate thereof; wherein L¹ is selected from the group consisting of linear or branched C₄-C₁₂ alkylene, C₃-C₈ (hetero)cycloalkylene, C₆-C₁₂ arylene, and C₄-C₁₁ heteroarylene.
Clause 230. A compound of Clause 229 or a salt, hydrate, or solvate thereof; wherein L¹ is a linear or branched C₄-C₁₂ alkylene.
Clause 231. A compound of Clause 230 or a salt, hydrate, or solvate thereof; wherein L¹ is a linear or branched C₄-C₁₀ alkylene.
Clause 232. A compound of Clause 231 or a salt, hydrate, or solvate thereof; wherein L¹ is
   L¹ is a linear C₅-C₆ alkylene.
Clause 233. A compound of Clause 232 or a salt, hydrate, or solvate thereof; wherein L¹ is a linear C₅ alkylene.
Clause 234. A compound of Clause 233 or a salt, hydrate, or solvate thereof; wherein L¹ is a linear, unsubstituted C₅ alkylene.
Clause 235. A compound of any one of Clauses 228-234 or a salt, hydrate, or solvate thereof; wherein L^{2a}, L^{2b}, and L^{2d} are each independently a linker.
Clause 236. A compound of any one of Clauses 228-235 or a salt, hydrate, or solvate thereof; wherein L^{2a}, L^{2b}, and L^{2d} are each independently a linker containing at most twenty atoms.
Clause 237. A compound of any one of Clauses 228-236 or a salt, hydrate, or solvate thereof; wherein L^{2a}, L^{2b}, and L^{2d} are each independently selected from the group consisting of
   -C(O)NL^{2T}-, -NL^{2T}C(O)-, -O-, -S-, -NL^{2T}-, -N=N-, and -C(O)-; wherein L^{2T} is hydrogen or methyl.
Clause 238. A compound of Clause 237 or a salt, hydrate, or solvate thereof; wherein L^{2T} is hydrogen.
Clause 239. A compound of any one of Clauses 228-238 or a salt, hydrate, or solvate thereof; wherein L^{2a}, L^{2b}, and L^{2d} are each independently selected from the group consisting of
   -C(O)NH-, and -NHC(O)-.
Clause 240. A compound of any one of Clauses 228-239 or a salt, hydrate, or solvate thereof; wherein L^{2c} is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl.
Clause 241. A compound of any one of Clauses 228-240 or a salt, hydrate, or solvate thereof; wherein L^{2c} is C₁-C₈ (hetero)alkanetriyl.
Clause 242. A compound of any one of Clauses 228-241 or a salt, hydrate, or solvate thereof; wherein L^{2c} is C₂-C₈ alkanetriyl.
Clause 243. A compound of any one of Clauses 228-242 or a salt, hydrate, or solvate thereof; wherein L^{2c} is C₄-C₆ alkanetriyl.
Clause 244. A compound of any one of Clauses 228-243 or a salt, hydrate, or solvate thereof; wherein L^{2c} is C₅ alkanetriyl.
Clause 245. A compound of any one of Clauses 228-244 or a salt, hydrate, or solvate thereof; wherein L^{2c} is >CH-CH₂-CH₂-CH₂-CH₂-.
Clause 246. A compound of any one of Clauses 228-245 or a salt, hydrate, or solvate thereof; wherein T¹ is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃;
   each T^{1A} is independently selected from the group consisting of hydrogen, (hetero)alkyl, (hetero)alkenyl, (hetero)alkynyl, (hetero)aryl, and an amino acid residue;
   preferably each T^{1A} is independently selected from the group consisting of hydrogen, C₁-C₈ (hetero)alkyl, C₁-C₆ (hetero)alkenyl, C₁-C₆ (hetero)alkynyl, C₂-C₅ heteroaryl, phenyl, and an amino acid residue; even more preferably each T^{1A} is independently selected from the group consisting of hydrogen, C₁-C₄ (hetero)alkyl, C₁-C₄ (hetero)alkenyl, C₁-C₄ (hetero)alkynyl, C₃-C₅ heteroaryl, phenyl, an aspartic acid residue, a glutamic acid residue, and a glycine residue; even more preferably each T^{1A} is independently selected from the group consisting of hydrogen, C₁-C₃ alkyl, an aspartic acid residue, a glutamic acid residue, and a glycine residue; and most preferably T^{1A} is hydrogen.
Clause 247. A compound of any one of Clauses 228-246 or a salt, hydrate, or solvate thereof; wherein T¹ is -OT^{1A}.
Clause 248. A compound of any one of Clauses 246-247 or a salt, hydrate, or solvate thereof; wherein T^{1A} is hydrogen or methyl.
Clause 249. A compound of any one of Clauses 246-248 or a salt, hydrate, or solvate thereof; wherein T^{1A} is hydrogen.
Clause 250. A compound of any one of Clauses 228-249 or a salt, hydrate, or solvate thereof; wherein T¹ is -OH.
Clause 251. A compound of any one of Clauses 228-250 or a salt, hydrate, or solvate thereof; wherein T² is a bioconjugation moiety or a group -L³-C^{B}; wherein L³ is a residue of a bioconjugation moiety, and C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof.
Clause 252. A compound of Clause 251 or a salt, hydrate, or solvate thereof; wherein the bioconjugation moiety is selected from the group consisting of N-maleimidyl, halogenated N-alkylamido, sulfonyloxy N-alkylamido, vinyl sulfone, (activated) carboxylic acids, active ester, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (oxa)norbomadiene, (imino)sydnones, methylsulfonyl phenyloxadiazole, aminooxy, 2-amino benzamidoxime, ethynylphosphonamidates, reactive in the Pictet-Spengler ligation and hydrazine- Pictet-Spengler (HIPS) ligation, DNA intercalators, tetrazine, *trans-*cyclooctene*,* and photocrosslinkers.
Clause 253. A compound of Clause 252 or a salt, hydrate, or solvate thereof; wherein the bioconjugation moiety is selected from the group consisting of N-maleimidyl, halogenated N-alkylamido, sulfonyloxy N-alkylamido, vinyl sulfone, carboxylic acids, benzenesulfonyl halides, ester, carbonate, sulfonyl halide, thiol, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl, carbonyl halide, allenamide, 1,2-quinone, isothiocyanate, isocyanate, aldehyde, triazine, squaric acids, 2-imino-2-methoxyethyl, (oxa)norbornene, (oxa)norbomadiene, (imino)sydnones, methylsulfonyl phenyloxadiazole, aminooxy, 2-amino benzamidoxime, and ethynylphosphonamidates.
Clause 254. A compound of Clause 253 or a salt, hydrate, or solvate thereof; wherein the bioconjugation moiety is N-maleimidyl.
Clause 255. A compound of any one of Clauses 228-254 or a salt, hydrate, or solvate thereof; wherein T² is a bioconjugation moiety.
Clause 256. A compound of Clause 255 or a salt, hydrate, or solvate thereof; wherein T² is N-maleimidyl.
Clause 257. A compound of Clause 256 or a salt, hydrate, or solvate thereof; wherein T² is 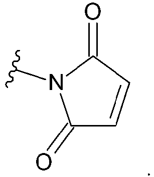
Clause 258. A compound of any one of Clauses 228-254 or a salt, hydrate, or solvate thereof; wherein T² is a group -L³-C^{B}.
Clause 259. A compound of any one of Clauses 228-254, and 258, or a salt, hydrate, or solvate thereof; wherein L³ is a residue of a maleimidyl moiety or a residue of an N-hydroxysuccinimidyl moiety.
Clause 260. A compound of any one of Clauses 228-254, and 258-259, or a salt, hydrate, or solvate thereof; wherein L³ is a residue of a maleimidyl moiety.
Clause 261. A compound of any one of Clauses 228-254, and 258-260, or a salt, hydrate, or solvate thereof; wherein T² is is selected from the group consisting of 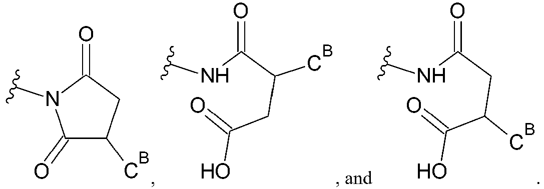
Clause 262. A compound of any one of Clauses 228-254, and 258-261, or a salt, hydrate, or solvate thereof; wherein C^{B} is a protein.
Clause 263. A compound of any one of Clauses 228-254, and 258-262, or a salt, hydrate, or solvate thereof; wherein C^{B} is an antibody or a diabody.
Clause 264. A compound of any one of Clauses 228-254, and 258-263, or a salt, hydrate, or solvate thereof; wherein C^{B} is a diabody.
Clause 265. A compound of any one of Clauses 228-254, and 258-264, or a salt, hydrate, or solvate thereof; wherein C^{B} is AVP0458 consisting of two monomers, wherein each of the two monomers has an amino acid sequence according to SEQ ID NO: 1.
Clause 266. A compound of any one of Clauses 228-254, and 258-265, or a salt, hydrate, or solvate thereof; wherein C^{B} is linked to the remainder of T² via a sulfur atom or nitrogen atom, wherein the sulfur atom or nitrogen atom is part of C^{B}.
Clause 267. A compound of any one of Clauses 228-254, and 258-266, or a salt, hydrate, or solvate thereof; wherein C^{B} is linked to the remainder of T² via a sulfur atom, wherein the sulfur atom is part of C^{B}.
Clause 268. A compound of any one of Clauses 228-254, and 258-267, or a salt, hydrate, or solvate thereof; wherein C^{B} is linked to the remainder of T² via a sulfur atom that is part of C^{B}, wherein the sulfur atom is part of a cysteine residue.
Clause 269. A compound of any one of Clauses 228-268, or a salt, hydrate, or solvate thereof; wherein T³ is a polymer.
Clause 270. A compound of any one of Clauses 228-269, or a salt, hydrate, or solvate thereof; wherein T³ is a polymer comprising a polyethylene glycol moiety.
Clause 271. A compound of any one of Clauses 228-270, or a salt, hydrate, or solvate thereof; wherein T³ comprises a moiety -(CH₂CH₂-O-)_{y}-T⁴, wherein y is an integer in a range of from 1 to 50, and T⁴ is according to Radical Group 1, Radical Group 3, Radical Group 4, or Radical Group 5 as defined herein; preferably y is an integer in a range of from 10 to 40, more preferably in a range of from 12 to 37, even more preferably in a range of from 15 to 35, more preferably still in a range of from 20 to 30, even more preferably in a range of from 23 to 25, and most preferably y is 24.
Clause 272. A compound of any one of Clauses 228-271, or a salt, hydrate, or solvate thereof; wherein T³ is a moiety - (CH₂CH₂-O-)_{y}-T⁴.
Clause 273. A compound of any one of Clauses 271-272, or a salt, hydrate, or solvate thereof; wherein y is an integer in a range of from 10 to 40.
Clause 274. A compound of any one of Clauses 271-273, or a salt, hydrate, or solvate thereof; wherein y is an integer in a range of from 12 to 37.
Clause 275. A compound of any one of Clauses 271-274, or a salt, hydrate, or solvate thereof; wherein y is an integer in a range of from 15 to 35.
Clause 276. A compound of any one of Clauses 271-275, or a salt, hydrate, or solvate thereof; wherein y is an integer in a range of from 20 to 30.
Clause 277. A compound of any one of Clauses 271-276, or a salt, hydrate, or solvate thereof; wherein y is an integer in a range of from 23 to 25.
Clause 278. A compound of any one of Clauses 271-277, or a salt, hydrate, or solvate thereof; wherein y is 24.
Clause 279. A compound of any one of Clauses 271-278, or a salt, hydrate, or solvate thereof; wherein T⁴ is methyl.
Clause 280. A compound of any one of Clauses 228-279, or a salt, hydrate, or solvate thereof; wherein T³ is a moiety - (CH₂CH₂-O-)₂₄-CH₃.
Clause 281. A compound of any one of Clauses 228-280, or a salt, hydrate, or solvate thereof; wherein R₄₈ is selected from the group consisting of -OH, -O-acetyl, -O-C₁₋₄ alkyl, halogen, active carbonate, and a releasable group.
Clause 282. A compound of any one of Clauses 228-281, or a salt, hydrate, or solvate thereof; wherein R₄₈ is a releasable group.
Clause 283. A compound of any one of Clauses 228-282, or a salt, hydrate, or solvate thereof; wherein said releasable group is -(Y¹-C(=Y²))ᵢ-(S^{P})ⱼ-C^{A}; wherein each of Y¹ and Y² are independently selected from O, and S; C^{A} is Construct A, which is a payload; S^{P} is a linker; j is 0 or 1; i is 0 or 1; if i is 0, -(S^{P})ⱼ-C^{A} is connected to the remainder of the compound via O or S, that is part of -(S^{P})ⱼ-C^{A}; if i is 1, -(S^{P})ⱼ-C^{A} is connected to -C(=Y²)- via O, S, secondary N, or a tertiary N, that is part of -(S^{P})ⱼ-C^{A}.
Clause 284. A compound of any one of Clauses 228-283, or a salt, hydrate, or solvate thereof; wherein said releasable group is -(O-C(-O))ᵢ-(S^{P})ⱼ-C^{A}.
Clause 285. A compound of any one of Clauses 228-284, or a salt, hydrate, or solvate thereof; wherein said releasable group is -O-C(=O)-(S^{P})ⱼ-C^{A}.
Clause 286. A compound of any one of Clauses 228-285, or a salt, hydrate, or solvate thereof; wherein S^{P} is according to Radical Group 2.
Clause 287. A compound of any one of Clauses 228-286, or a salt, hydrate, or solvate thereof; wherein S^{P} is a self-immolative linker.
Clause 288. A compound of any one of Clauses 228-287, or a salt, hydrate, or solvate thereof; wherein said releasable group is -O-C(=O)-C^{A}.
Clause 289. A compound of any one of Clauses 228-288, or a salt, hydrate, or solvate thereof; wherein C^{A} is linked to the moiety -O-C(=O)- via a secondary or tertiary nitrogen atom that is part of C^{A}, forming a carbamate.
Clause 290. A compound of any one of Clauses 228-289, or a salt, hydrate, or solvate thereof; wherein C^{A} is a drug.
Clause 291. A compound of any one of Clauses 228-290, or a salt, hydrate, or solvate thereof; wherein C^{A} is monomethyl auristatin E (MMAE).
Clause 292. A compound of any one of Clauses 228-291, or a salt, hydrate, or solvate thereof; wherein C^{A} is monomethyl auristatin E (MMAE) linked to the moiety -O-C(=O)- via a secondary or tertiary nitrogen atom that is part of MMAE, forming a carbamate.
Clause 293. A compound of any one of Clauses 228-292, or a salt, hydrate, or solvate thereof; wherein C^{A} is monomethyl auristatin E (MMAE) linked to the moiety -O-C(=O)- via a tertiary nitrogen atom that is part of MMAE, forming a carbamate.
Clause 294. A compound of any one of Clauses 228-293, or a salt, hydrate, or solvate thereof; wherein said group R₄₈ is in an axial position.
Clause 295. A compound of any one of Clauses 228-294, or a salt, hydrate, or solvate thereof; wherein said group R₄₈ is: 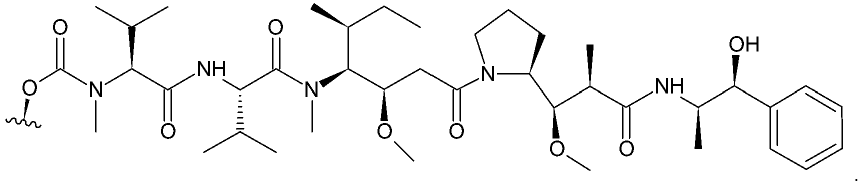
Clause 296. A compound of any one of Clauses 1-295, or a salt, hydrate, or solvate thereof; wherein said compound has a structure according to Formula (1): 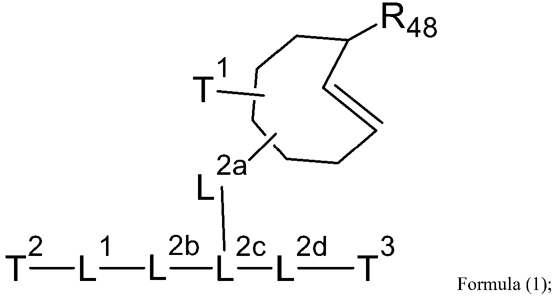 wherein
   L¹ is selected from the group consisting of linear or branched C₄-C₁₂ alkylene, C₃-C₈ (hetero)cycloalkylene, C₆-C₁₂ arylene, and C₄-C₁₁ heteroarylene; L^{2a}, L^{2b}, and L^{2d} are each independently a linker; L^{2c} is selected from the group consisting of C₁-C₈ (hetero)alkanetriyl, C₅-C₆ (hetero)arenetriyl, C₃-C₇ cycloalkanetriyl, and C₂-C₇ heterocycloalkanetriyl; T¹ is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A} )₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃; each T^{1A} is independently selected from the group consisting of hydrogen, (hetero)alkyl, (hetero)alkenyl, (hetero)alkynyl, (hetero)aryl, and an amino acid residue; T² is a bioconjugation moiety or a group -L³-C^{B}; wherein L³ is a residue of a bioconjugation moiety, and C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof; T³ is a polymer; and R₄₈ is selected from the group consisting of -OH, -O-acetyl, -O-C₁₋₄ alkyl, halogen, active carbonate, and a releasable group; and preferably L¹ is linear or branched C₄-C₁₂ alkylene, more preferably L¹ is linear or branched C₄-C₁₀ alkylene, and most preferably L¹ is linear C₅-C₆ alkylene; preferably L^{2a}, L^{2b}, and L^{2d} are each independently a linker containing at most twenty atoms; more preferably L^{2a}, L^{2b}, and L^{2d} are each independently selected from the group consisting of -C(O)NL^{2T}-, - NL^{2T}C(O)-, -O-, -S-, -NL^{2T}-, -N=N-, and -C(O)-; wherein L^{2T} is hydrogen or methyl, preferably L^{2T} is hydrogen; preferably L^{2c} is C₁-C₈ (hetero)alkanetriyl, more preferably L^{2c} is C₁-C₈ alkanetriyl, and most preferably L^{2c} is C₄-C₆ alkanetriyl; preferably T¹ is -OT^{1A}; and most preferably T¹ is -OH; preferably T^{1A} is hydrogen or methyl, more preferably T^{1A} is hydrogen; preferably T² is maleimidyl, N-hydroxysuccinimidyl, or -L³-C^{B}; preferably L³ is a residue of a maleimidyl moiety or a residue of an N-hydroxysuccinimidyl moiety; preferably C^{B} is a protein, more preferably C^{B} is an antibody or a diabody, even more preferably C^{B} is a diabody, and most preferably C^{B} is AVP0458 consisting of two monomers, wherein each of the two monomers has an amino acid sequence according to SEQ ID NO: 1; preferably T³ is a polymer comprising a polyethylene glycol moiety; and preferably R₄₈ is a releasable group.
Clause 297. A compound of Clause 296, or a salt, hydrate, or solvate thereof; wherein R₄₈ is in an axial position.
Clause 298. A compound of any one of Clauses 1-296, or a salt, hydrate, or solvate thereof; wherein said compound has a structure according to Formula (D): 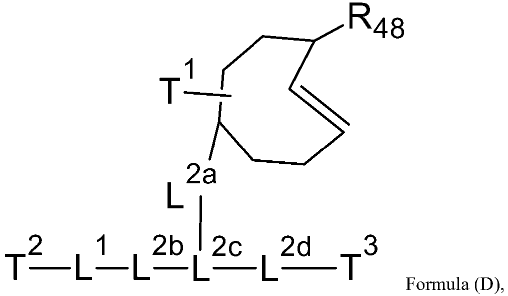 wherein R₄₈ is as defined in any one of Clauses 147-166, and 281-295; T¹ is as defined in any one of Clauses 167-179, and 246-250; T² is as defined in any one of Clauses 1, 49-69, and 251-268; T³ is as defined in any one of Clauses 1, 70-89, and 269-280; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 299. A compound of Clause 298, or a salt, hydrate, or solvate thereof; wherein R₄₈ is in an axial position.
Clause 300. A compound of any one of Clauses 1-296, or a salt, hydrate, or solvate thereof; wherein said compound has a structure according to Formula (E): 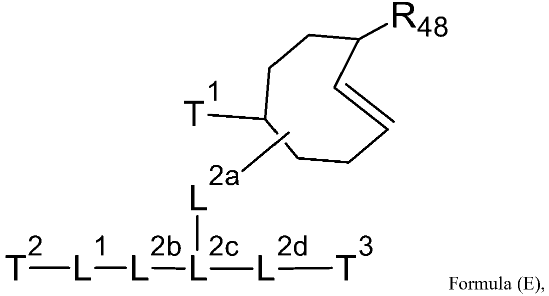 wherein R₄₈ is as defined in any one of Clauses 147-166, and 281-295; T¹ is as defined in any one of Clauses 167-179, and 246-250; T² is as defined in any one of Clauses 1, 49-69, and 251-268; T³ is as defined in any one of Clauses 1, 70-89, and 269-280; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 301. A compound of Clause 300, or a salt, hydrate, or solvate thereof; wherein R₄₈ is in an axial position.
Clause 302. A compound of any one of Clauses 1-301, or a salt, hydrate, or solvate thereof; wherein said compound has a structure according to Formula (F): 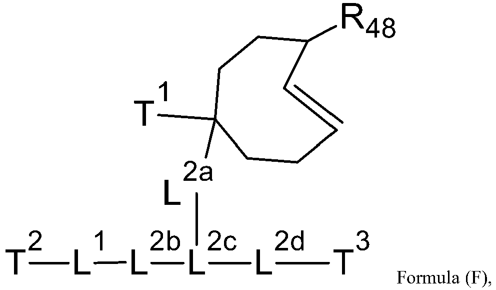 wherein R₄₈ is as defined in any one of Clauses 147-166, and 281-295; T¹ is as defined in any one of Clauses 167-179, and 246-250; T² is as defined in any one of Clauses 1, 49-69, and 251-268; T³ is as defined in any one of Clauses 1, 70-89, and 269-280; L¹ is as defined in any one of Clauses 1-29, and 229-234; L^{2a} is as defined in any one of Clauses 90-99, and 235-239; L^{2d} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 303. A compound of Clause 302, or a salt, hydrate, or solvate thereof; wherein R₄₈ is in an axial position.
Clause 304. A compound of any one of Clauses 1-302, or a salt, hydrate, or solvate thereof; wherein said compound has a structure according to Formula (G): 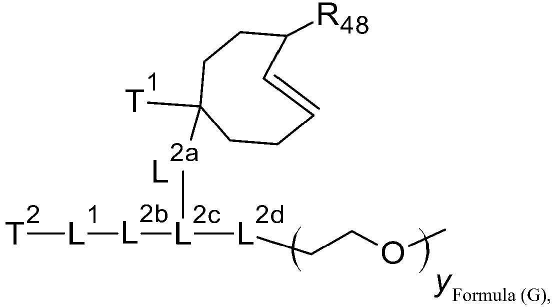 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 305. A compound of Clause 304, or a salt, hydrate, or solvate thereof; wherein R₄₈ is in an axial position.
Clause 306. A compound of Clause 296, or a salt, hydrate, or solvate thereof; wherein said compound has a structure according to Formula (2): 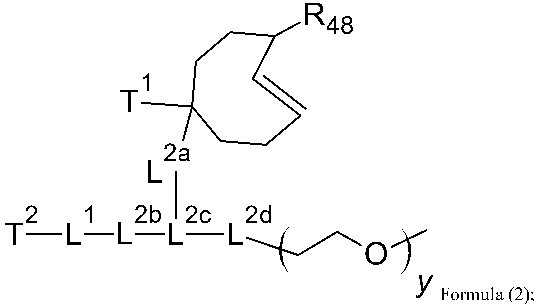 wherein
   y is an integer in a range of from 1 to 50; preferably y is an integer in a range of from 10 to 40, more preferably in a range of from 12 to 37, even more preferably in a range of from 15 to 35, more preferably still in a range of from 20 to 30, and most preferably in a range of from 23 to 25.
Clause 307. A compound of Clause 306, or a salt, hydrate, or solvate thereof; wherein R₄₈ is in an axial position.
Clause 308. A compound of any one of Clauses 1-296, or a salt, hydrate, or solvate thereof; wherein said compound has a structure according to Formula (H): 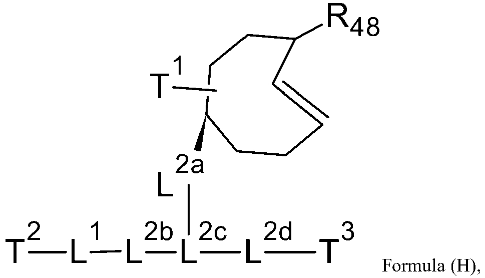 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 309. A compound of Clause 308, or a salt, hydrate, or solvate thereof; wherein R₄₈ is in an axial position.
Clause 310. A compound of any one of Clauses 1-296, or a salt, hydrate, or solvate thereof; wherein said compound has a structure according to Formula (I): 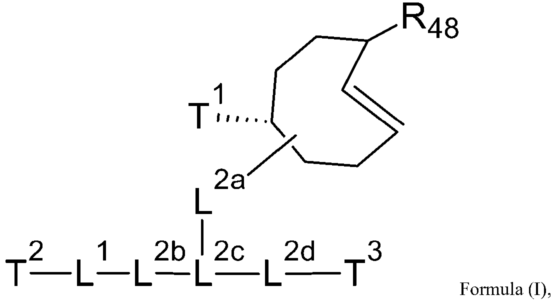 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 311. A compound of Clause 310, or a salt, hydrate, or solvate thereof; wherein R₄₈ is in an axial position.
Clause 312. A compound of any one of Clauses 1-296, or a salt, hydrate, or solvate thereof; wherein said compound has a structure according to Formula (J): 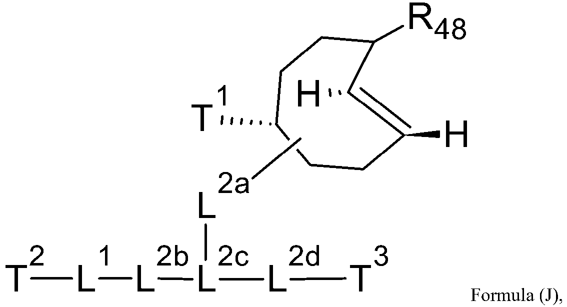 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 313. A compound of Clause 312, or a salt, hydrate, or solvate thereof; wherein R₄₈ is in an axial position.
Clause 314. A compound of any one of Clauses 312-313, or a salt, hydrate, or solvate thereof; wherein T¹ is -OH.
Clause 315. A compound of any one of Clauses 312-314, or a salt, hydrate, or solvate thereof; wherein T¹ is -OH and R₄₈ is in an axial position.
Clause 316. A compound of Clause 315, or a salt, hydrate, or solvate thereof; wherein R₄₈ is
   -O-C(O)-C^{A}, wherein C^{A} is a drug, preferably C^{A} is monomethyl auristatin E (MMAE), exatecan, or an exatecan derivative.
Clause 317. A compound of Clause 316, or a salt, hydrate, or solvate thereof; wherein R₄₈ is: 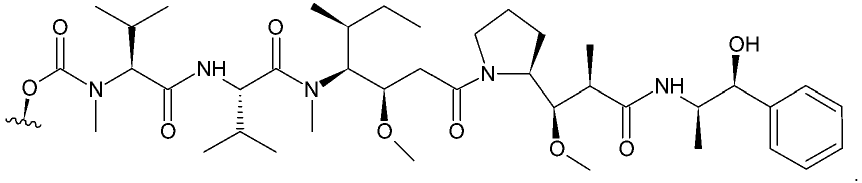
Clause 318. A compound of any one of Clauses 1-296, or a salt, hydrate, or solvate thereof; wherein said compound has a structure according to Formula (K): 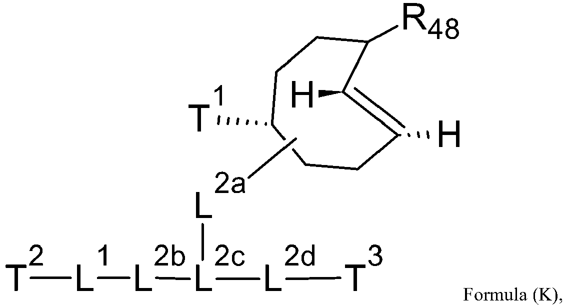 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 319. A compound of Clause 318, or a salt, hydrate, or solvate thereof; wherein R₄₈ is in an axial position.
Clause 320. A compound of any one of Clauses 318-319, or a salt, hydrate, or solvate thereof; wherein T¹ is -OH.
Clause 321. A compound of any one of Clauses 318-320, or a salt, hydrate, or solvate thereof; wherein T¹ is -OH and R₄₈ is in an axial position.
Clause 322. A compound of Clause 321, or a salt, hydrate, or solvate thereof; wherein R₄₈ is
   -O-C(O)-C^{A}, wherein C^{A} is a drug, preferably C^{A} is monomethyl auristatin E (MMAE), exatecan, or an exatecan derivative.
Clause 323. A compound of Clause 322, or a salt, hydrate, or solvate thereof; wherein R₄₈ is: 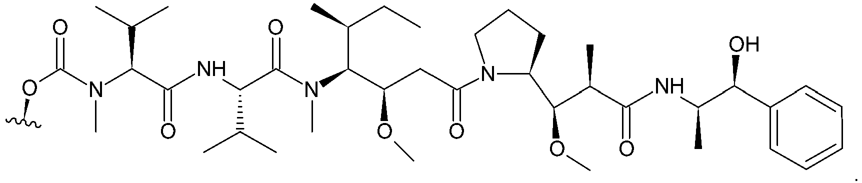
Clause 324. A compound of any one of Clauses 1-323, or a salt, hydrate, or solvate thereof; wherein said compound has a structure according to Formula (L): 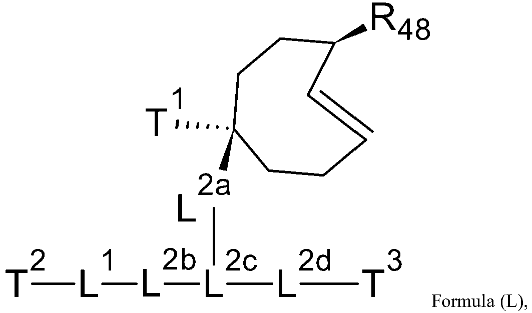 wherein R₄₈ is as defined in any one of Clauses 147-166, and 281-295; T¹ is as defined in any one of Clauses 167-179, and 246-250; T² is as defined in any one of Clauses 1, 49-69, and 251-268; y is as defined in any one of Clauses 271, and 273-278; L¹ is as defined in any one of Clauses 1-29, and 229-234; L^{2a} is as defined in any one of Clauses 90-99, and 235-239; L^{2b} is as defined in any one of Clauses 90, 91, 100-107, and 235-239; L^{2c} is as defined in any one of Clauses 90, 91, 116-128, and 240-245; and L^{2d} is as defined in any one of Clauses 90, 91, 108-115, and 235-239.
Clause 325. A compound of Clause 324, or a salt, hydrate, or solvate thereof; wherein T¹ is -OH.
Clause 326. A compound of Clause 325, or a salt, hydrate, or solvate thereof; wherein R₄₈ is
   -O-C(O)-C^{A}, wherein C^{A} is a drug, preferably C^{A} is monomethyl auristatin E (MMAE), exatecan, or an exatecan derivative.
Clause 327. A compound of Clause 326, or a salt, hydrate, or solvate thereof; wherein R₄₈ is: 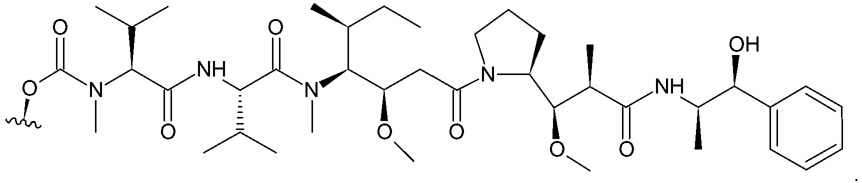
Clause 328. A compound of any one of Clauses 324-327 or a salt, hydrate, or solvate thereof; wherein L¹ is selected from the group consisting of linear or branched C₄-C₁₂ alkylene, C₃-C₈ (hetero)cycloalkylene, C₆-C₁₂ arylene, and C₄-C₁₁ heteroarylene.
Clause 329. A compound of any one of Clauses 324-328 or a salt, hydrate, or solvate thereof; wherein L¹ is a linear or branched C₄-C₁₂ alkylene.
Clause 330. A compound of any one of Clauses 324-329 or a salt, hydrate, or solvate thereof; wherein L¹ is a linear or branched C₄-C₁₀ alkylene.
Clause 331. A compound of any one of Clauses 324-330 or a salt, hydrate, or solvate thereof; wherein L¹ is L¹ is a linear C₅-C₆ alkylene.
Clause 332. A compound of any one of Clauses 324-331 or a salt, hydrate, or solvate thereof; wherein L¹ is a linear C₅ alkylene.
Clause 333. A compound of any one of Clauses 324-332 or a salt, hydrate, or solvate thereof; wherein L¹ is a linear, unsubstituted C₅ alkylene.
Clause 334. A compound of any one of Clauses 324-333 or a salt, hydrate, or solvate thereof; wherein L^{2a}, L^{2b}, and L^{2d} are each independently a linker.
Clause 335. A compound of any one of Clauses 324-334 or a salt, hydrate, or solvate thereof; wherein L^{2a}, L^{2b}, and L^{2d} are each independently a linker containing at most twenty atoms.
Clause 336. A compound of any one of Clauses 324-335 or a salt, hydrate, or solvate thereof; wherein L^{2a}, L^{2b}, and L^{2d} are each independently selected from the group consisting of
   -C(O)NL^{2T}-, -NL^{2T}C(O)-, -O-, -S-, -NL^{2T}-, -N=N-, and -C(O)-; wherein L^{2T} is hydrogen or methyl.
Clause 337. A compound of any one of Clauses 324-336 or a salt, hydrate, or solvate thereof; wherein L^{2T} is hydrogen.
Clause 338. A compound of any one of Clauses 324-337 or a salt, hydrate, or solvate thereof; wherein L^{2a}, L^{2b}, and L^{2d} are each independently selected from the group consisting of
   -C(O)NH-, and -NHC(O)-.
Clause 339. A compound of any one of Clauses 324-338 or a salt, hydrate, or solvate thereof; wherein L^{2c} is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl.
Clause 340. A compound of any one of Clauses 324-339 or a salt, hydrate, or solvate thereof; wherein L^{2c} is C₁-C₈ (hetero)alkanetriyl.
Clause 341. A compound of any one of Clauses 324-340 or a salt, hydrate, or solvate thereof; wherein L^{2c} is C₁-C₈ alkanetriyl.
Clause 342. A compound of any one of Clauses 324-341 or a salt, hydrate, or solvate thereof; wherein L^{2c} is C₄-C₆ alkanetriyl.
Clause 343. A compound of any one of Clauses 324-342 or a salt, hydrate, or solvate thereof; wherein L^{2c} is C₅ alkanetriyl.
Clause 344. A compound of any one of Clauses 324-343 or a salt, hydrate, or solvate thereof; wherein L^{2c} is >CH-CH₂-CH₂-CH₂-CH₂-.
Clause 345. A compound of any one of Clauses 324-344 or a salt, hydrate, or solvate thereof; wherein T¹ is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃; each T^{1A} is independently selected from the group consisting of hydrogen, (hetero)alkyl, (hetero)alkenyl, (hetero)alkynyl, (hetero)aryl, and an amino acid residue;
   preferably each T^{1A} is independently selected from the group consisting of hydrogen, C₁-C₆ (hetero)alkyl, C₁-C₆ (hetero)alkenyl, C₁-C₆ (hetero)alkynyl, C₂-C₅ heteroaryl, phenyl, and an amino acid residue; even more preferably each T^{1A} is independently selected from the group consisting of hydrogen, C₁-C₄ (hetero)alkyl, C₁-C₄ (hetero)alkenyl, C₁-C₄ (hetero)alkynyl, C₃-C₅ heteroaryl, phenyl, an aspartic acid residue, a glutamic acid residue, and a glycine residue; even more preferably each T^{1A} is independently selected from the group consisting of hydrogen, C₁-C₃ alkyl, an aspartic acid residue, a glutamic acid residue, and a glycine residue; and most preferably T^{1A} is hydrogen.
Clause 346. A compound of any one of Clauses 324-345 or a salt, hydrate, or solvate thereof; wherein T¹ is -OT^{1A}.
Clause 347. A compound of any one of Clauses 324-346 or a salt, hydrate, or solvate thereof; wherein T^{1A} is hydrogen or methyl.
Clause 348. A compound of any one of Clauses 324-347 or a salt, hydrate, or solvate thereof; wherein T^{1A} is hydrogen.
Clause 349. A compound of any one of Clauses 324-348 or a salt, hydrate, or solvate thereof; wherein T¹ is -OH.
Clause 350. A compound of any one of Clauses 324-349 or a salt, hydrate, or solvate thereof; wherein T² is a bioconjugation moiety or a group -L³-C^{B}; wherein L³ is a residue of a bioconjugation moiety, and C^{B} is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof.
Clause 351. A compound of Clause 350 or a salt, hydrate, or solvate thereof; wherein the bioconjugation moiety is selected from the group consisting of N-maleimidyl, halogenated N-alkylamido, sulfonyloxy N-alkylamido, vinyl sulfone, (activated) carboxylic acids, active ester, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (oxa)norbomadiene, (imino)sydnones, methylsulfonyl phenyloxadiazole, aminooxy, 2-amino benzamidoxime, ethynylphosphonamidates, reactive in the Pictet-Spengler ligation and hydrazine- Pictet-Spengler (HIPS) ligation, DNA intercalators, tetrazine, *trans-*cyclooctene, and photocrosslinkers.
Clause 352. A compound of Clause 351 or a salt, hydrate, or solvate thereof; wherein the bioconjugation moiety is selected from the group consisting of N-maleimidyl, halogenated N-alkylamido, sulfonyloxy N-alkylamido, vinyl sulfone, carboxylic acids, benzenesulfonyl halides, ester, carbonate, sulfonyl halide, thiol, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl, carbonyl halide, allenamide, 1,2-quinone, isothiocyanate, isocyanate, aldehyde, triazine, squaric acids, 2-imino-2-methoxyethyl, (oxa)norbornene, (oxa)norbomadiene, (imino)sydnones, methylsulfonyl phenyloxadiazole, aminooxy, 2-amino benzamidoxime, and ethynylphosphonamidates.
Clause 353. A compound of Clause 352 or a salt, hydrate, or solvate thereof; wherein the bioconjugation moiety is N-maleimidyl.
Clause 354. A compound of any one of Clauses 324-353 or a salt, hydrate, or solvate thereof; wherein T² is a bioconjugation moiety.
Clause 355. A compound of Clause 354 or a salt, hydrate, or solvate thereof; wherein T² is N-maleimidyl.
Clause 356. A compound of Clause 355 or a salt, hydrate, or solvate thereof; wherein T² is 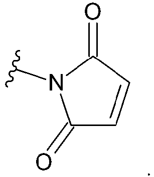
Clause 357. A compound of any one of Clauses 324-353 or a salt, hydrate, or solvate thereof; wherein T² is a group -L³-C^{B}.
Clause 358. A compound of any one of Clauses 324-353, and 357, or a salt, hydrate, or solvate thereof; wherein L³ is a residue of a maleimidyl moiety or a residue of an N-hydroxysuccinimidyl moiety.
Clause 359. A compound of any one of Clauses 324-353, and 357-358, or a salt, hydrate, or solvate thereof; wherein L³ is a residue of a maleimidyl moiety.
Clause 360. A compound of any one of Clauses 324-353, and 357-359, or a salt, hydrate, or solvate thereof; wherein T² is is selected from the group consisting of 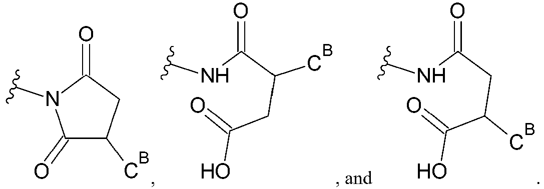
Clause 361. A compound of any one of Clauses 324-353, and 357-360, or a salt, hydrate, or solvate thereof; wherein C^{B} is a protein.
Clause 362. A compound of any one of Clauses 324-353, and 357-361, or a salt, hydrate, or solvate thereof; wherein C^{B} is an antibody or a diabody.
Clause 363. A compound of any one of Clauses 324-353, and 357-362, or a salt, hydrate, or solvate thereof; wherein C^{B} is a diabody.
Clause 364. A compound of any one of Clauses 324-353, and 357-363, or a salt, hydrate, or solvate thereof; wherein C^{B} is AVP0458 consisting of two monomers, wherein each of the two monomers has an amino acid sequence according to SEQ ID NO: 1.
Clause 365. A compound of any one of Clauses 324-353, and 357-364, or a salt, hydrate, or solvate thereof; wherein C^{B} is linked to the remainder of T² via a sulfur atom or nitrogen atom, wherein the sulfur atom or nitrogen atom is part of C^{B}.
Clause 366. A compound of any one of Clauses 324-353, and 357-365, or a salt, hydrate, or solvate thereof; wherein C^{B} is linked to the remainder of T² via a sulfur atom, wherein the sulfur atom is part of C^{B}.
Clause 367. A compound of any one of Clauses 324-353, and 357-365, or a salt, hydrate, or solvate thereof; wherein C^{B} is linked to the remainder of T² via a sulfur atom that is part of C^{B}, wherein the sulfur atom is part of a cysteine residue.
Clause 368. A compound of any one of Clauses 324-353, and 357-367, or a salt, hydrate, or solvate thereof; wherein T³ is a polymer.
Clause 369. A compound of any one of Clauses 324-353, and 357-368, or a salt, hydrate, or solvate thereof; wherein T³ is a polymer comprising a polyethylene glycol moiety.
Clause 370. A compound of any one of Clauses 324-353, and 357-369, or a salt, hydrate, or solvate thereof; wherein T³ comprises a moiety -(CH₂CH₂-O-)_{y}-T⁴, wherein y is an integer in a range of from 1 to 50, and T⁴ is according to Radical Group 1, Radical Group 3, Radical Group 4, or Radical Group 5 as defined herein; preferably y is an integer in a range of from 10 to 40, more preferably in a range of from 12 to 37, even more preferably in a range of from 15 to 35, more preferably still in a range of from 20 to 30, even more preferably in a range of from 23 to 25, and most preferably y is 24.
Clause 371. A compound of any one of Clauses 324-370, or a salt, hydrate, or solvate thereof; wherein T³ is a moiety - (CH₂CH₂-O-)_{y}-T⁴.
Clause 372. A compound of any one of Clauses 324-371, or a salt, hydrate, or solvate thereof; wherein y is an integer in a range of from 10 to 40.
Clause 373. A compound of any one of Clauses 324-372, or a salt, hydrate, or solvate thereof; wherein y is an integer in a range of from 12 to 37.
Clause 374. A compound of any one of Clauses 324-373, or a salt, hydrate, or solvate thereof; wherein y is an integer in a range of from 15 to 35.
Clause 375. A compound of any one of Clauses 324-374, or a salt, hydrate, or solvate thereof; wherein y is an integer in a range of from 20 to 30.
Clause 376. A compound of any one of Clauses 324-375, or a salt, hydrate, or solvate thereof; wherein y is an integer in a range of from 23 to 25.
Clause 377. A compound of any one of Clauses 324-376, or a salt, hydrate, or solvate thereof; wherein y is 24.
Clause 378. A compound of any one of Clauses 324-377, or a salt, hydrate, or solvate thereof; wherein T⁴ is methyl.
Clause 379. A compound of any one of Clauses 324-378, or a salt, hydrate, or solvate thereof; wherein T³ is a moiety - (CH₂CH₂-O-)₂₄-CH₃.
Clause 380. A compound of any one of Clauses 324-379, or a salt, hydrate, or solvate thereof; wherein R₄₈ is selected from the group consisting of -OH, -O-acetyl, -O-C₁₋₄ alkyl, halogen, active carbonate, and a releasable group.
Clause 381. A compound of any one of Clauses 324-380, or a salt, hydrate, or solvate thereof; wherein R₄₈ is a releasable group.
Clause 382. A compound of any one of Clauses 324-381, or a salt, hydrate, or solvate thereof; wherein said releasable group is -(Y¹-C(-Y²))ᵢ-(S^{P})ⱼ-C^{A}; wherein each of Y¹ and Y² are independently selected from O, and S; C^{A} is Construct A, which is a payload; S^{P} is a linker; j is 0 or 1; i is 0 or 1; if i is 0, -(S^{P})ⱼ-C^{A} is connected to the remainder of the compound via O or S, that is part of -(S^{P})ⱼ-C^{A}; if i is 1, -(S^{P})ⱼ-C^{A} is connected to -C(=Y²)- via O, S, secondary N, or a tertiary N, that is part of -(S^{P})ⱼ-C^{A}.
Clause 383. A compound of any one of Clauses 324-382, or a salt, hydrate, or solvate thereof; wherein said releasable group is -(O-C(-O))ᵢ-(S^{P})ⱼ-C^{A}.
Clause 384. A compound of any one of Clauses 324-383, or a salt, hydrate, or solvate thereof; wherein said releasable group is -O-C(=O)-(S^{P})ⱼ-C^{A}.
Clause 385. A compound of any one of Clauses 324-384, or a salt, hydrate, or solvate thereof; wherein S^{P} is according to Radical Group 2.
Clause 386. A compound of any one of Clauses 324-385, or a salt, hydrate, or solvate thereof; wherein S^{P} is a self-immolative linker.
Clause 387. A compound of any one of Clauses 324-386, or a salt, hydrate, or solvate thereof; wherein said releasable group is -O-C(-O)-C^{A}.
Clause 388. A compound of any one of Clauses 324-387, or a salt, hydrate, or solvate thereof; wherein C^{A} is linked to the moiety -O-C(=O)- via a secondary or tertiary nitrogen atom that is part of C^{A}, forming a carbamate.
Clause 389. A compound of any one of Clauses 324-388, or a salt, hydrate, or solvate thereof; wherein C^{A} is a drug, preferably C^{A} is monomethyl auristatin E (MMAE), exatecan, or an exatecan derivative.
Clause 390. A compound of any one of Clauses 324-389, or a salt, hydrate, or solvate thereof; wherein C^{A} is monomethyl auristatin E (MMAE).
Clause 391. A compound of any one of Clauses 324-390, or a salt, hydrate, or solvate thereof; wherein C^{A} is monomethyl auristatin E (MMAE) linked to the moiety -O-C(=O)- via a secondary or tertiary nitrogen atom that is part of MMAE, forming a carbamate.
Clause 392. A compound of any one of Clauses 324-391, or a salt, hydrate, or solvate thereof; wherein C^{A} is monomethyl auristatin E (MMAE) linked to the moiety -O-C(=O)- via a tertiary nitrogen atom that is part of MMAE, forming a carbamate.
Clause 393. A compound of any one of Clauses 324-392, or a salt, hydrate, or solvate thereof; wherein said compound is of Formula (M): 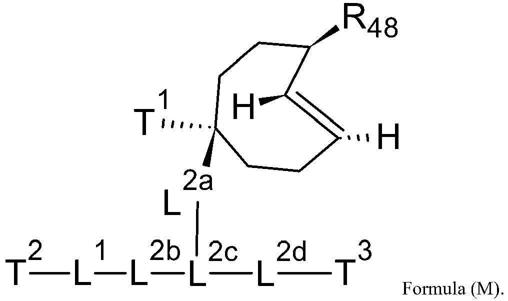
Clause 394. A compound of Clause 393, or a salt, hydrate, or solvate thereof; wherein T¹ is
   -OH.
Clause 395. A compound of any one of Clauses 393-394, or a salt, hydrate, or solvate thereof; wherein R₄₈ is -O-C(O)-C^{A}, wherein C^{A} is a drug, preferably C^{A} is monomethyl auristatin E (MMAE), exatecan, or an exatecan derivative.
Clause 396. A compound of any one of Clauses 393-395, or a salt, hydrate, or solvate thereof; wherein T¹ is -OH, and R₄₈ is - O-C(O)-C^{A}, wherein C^{A} is a drug, preferably C^{A} is monomethyl auristatin E (MMAE), exatecan, or an exatecan derivative.
Clause 397. A compound of any one of Clauses 393-396, or a salt, hydrate, or solvate thereof; wherein R₄₈ is: 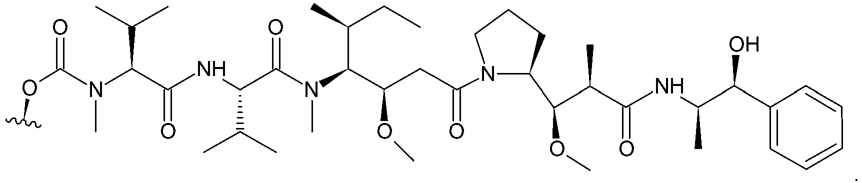
Clause 398. A compound of any one of Clauses 393-397, or a salt, hydrate, or solvate thereof; wherein T¹ is -OH, and R₄₈ is: 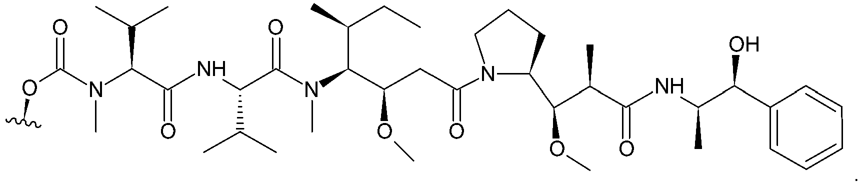
Clause 399. A compound of any one of Clauses 324-392, or a salt, hydrate, or solvate thereof; wherein said compound is of Formula (N): 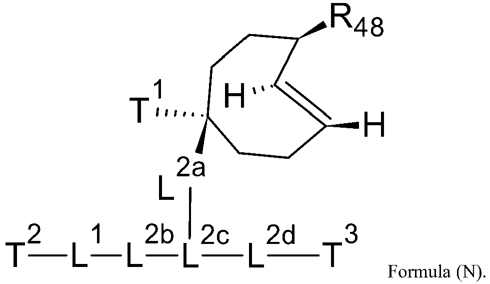
Clause 400. A compound of Clause 399, or a salt, hydrate, or solvate thereof; wherein T¹ is
   -OH.
Clause 401. A compound of any one of Clauses 399-400, or a salt, hydrate, or solvate thereof; wherein R₄₈ is -O-C(O)-C^{A}, wherein C^{A} is a drug, preferably C^{A} is monomethyl auristatin E (MMAE), exatecan, or an exatecan derivative.
Clause 402. A compound of any one of Clauses 399-401, or a salt, hydrate, or solvate thereof; wherein T¹ is -OH, and R₄₈ is - O-C(O)-C^{A}, wherein C^{A} is a drug, preferably C^{A} is monomethyl auristatin E (MMAE), exatecan, or an exatecan derivative.
Clause 403. A compound of any one of Clauses 399-402, or a salt, hydrate, or solvate thereof; wherein R₄₈ is: 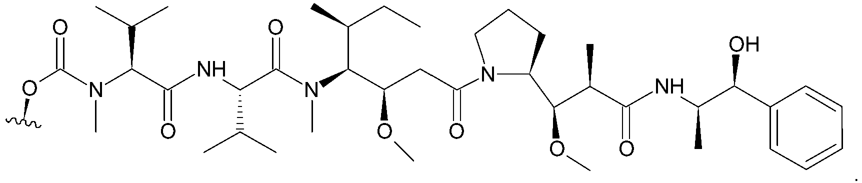
Clause 404. A compound of any one of Clauses 399-403, or a salt, hydrate, or solvate thereof; wherein T¹ is -OH, and R₄₈ is: 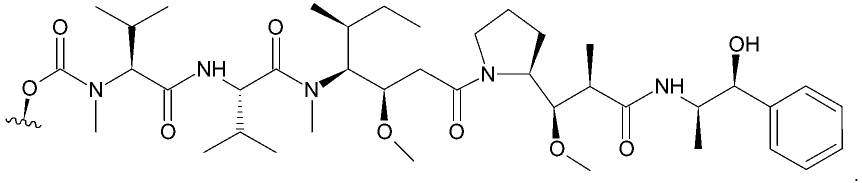
Clause 405. A compound of any one of Clauses 324-392, or a salt, hydrate, or solvate thereof; wherein said compound is of Formula (O): 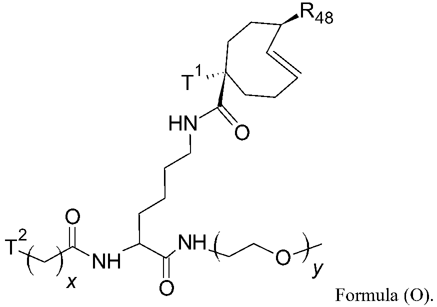
Clause 406. A compound of Clause 405, or a salt, hydrate, or solvate thereof; wherein T¹ is
   -OH.
Clause 407. A compound of any one of Clauses 405-406, or a salt, hydrate, or solvate thereof; wherein R₄₈ is -O-C(O)-C^{A}, wherein C^{A} is a drug, preferably C^{A} is monomethyl auristatin E (MMAE), exatecan, or an exatecan derivative.
Clause 408. A compound of any one of Clauses 405-407, or a salt, hydrate, or solvate thereof; wherein T¹ is -OH, and R₄₈ is - O-C(O)-C^{A}, wherein C^{A} is a drug, preferably C^{A} is monomethyl auristatin E (MMAE), exatecan, or an exatecan derivative.
Clause 409. A compound of any one of Clauses 405-408, or a salt, hydrate, or solvate thereof; wherein R₄₈ is: 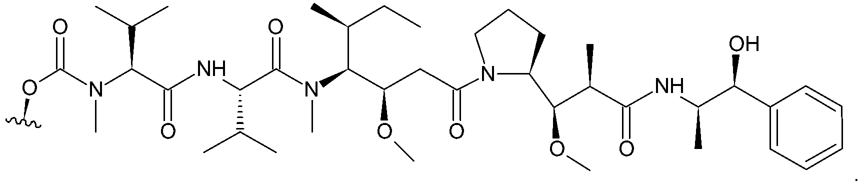
Clause 410. A compound of any one of Clauses 405-409, or a salt, hydrate, or solvate thereof; wherein T¹ is -OH, and R₄₈ is: 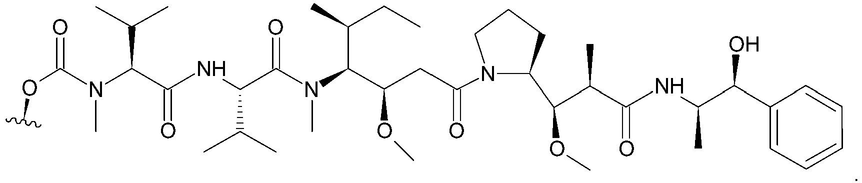
Clause 411. A compound of any one of Clauses 324-392, anc 405-410 or a salt, hydrate, or solvate thereof; wherein said compound is of Formula (3): 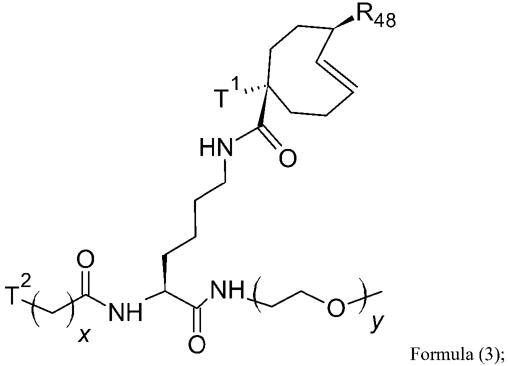 wherein
   y is as defined in Clause 306; x is an integer in a range of from 4 to 12; preferably x is an integer in a range of from 4 to 8, more preferably x is an integer in a range of from 4 to 6.
Clause 412. A compound of any one of Clauses 324-411, or a salt, hydrate, or solvate thereof; wherein said compound is of Formula (P): 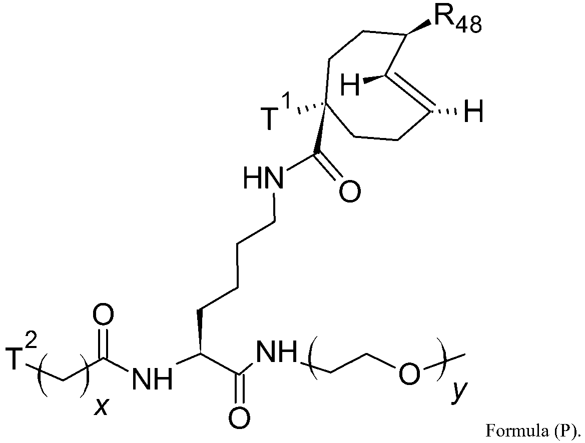
Clause 413. A compound of Clause 412, or a salt, hydrate, or solvate thereof; wherein T¹ is
   -OH.
Clause 414. A compound of any one of Clauses 412-413, or a salt, hydrate, or solvate thereof; wherein R₄₈ is -O-C(O)-C^{A}, wherein C^{A} is a drug, preferably C^{A} is monomethyl auristatin E (MMAE), exatecan, or an exatecan derivative.
Clause 415. A compound of any one of Clauses 412-414, or a salt, hydrate, or solvate thereof; wherein T¹ is -OH, and R₄₈ is - O-C(O)-C^{A}, wherein C^{A} is a drug, preferably C^{A} is monomethyl auristatin E (MMAE), exatecan, or an exatecan derivative.
Clause 416. A compound of any one of Clauses 412-414, or a salt, hydrate, or solvate thereof; wherein R₄₈ is: 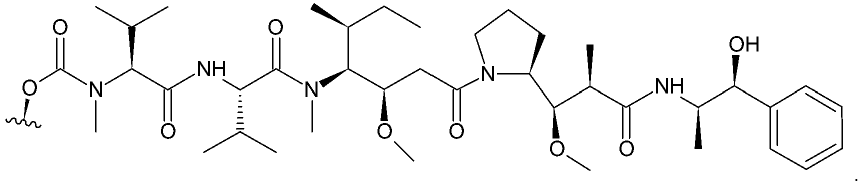
Clause 417. A compound of any one of Clauses 412-416, or a salt, hydrate, or solvate thereof; wherein T¹ is -OH, and R₄₈ is: 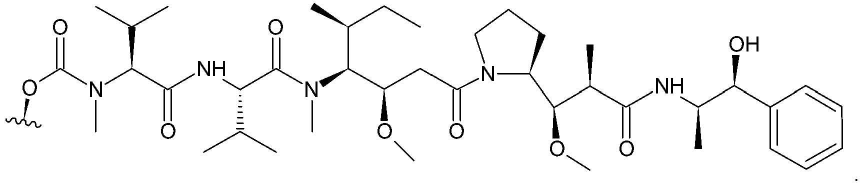
Clause 418. A compound of any one of Clauses 412-417, or a salt, hydrate, or solvate thereof; wherein x is an integer of from 3 to 8.
Clause 419. A compound of any one of Clauses 412-418, or a salt, hydrate, or solvate thereof; wherein x is an integer of from 4 to 6.
Clause 420. A compound of any one of Clauses 412-419, or a salt, hydrate, or solvate thereof; wherein x is 5.
Clause 421. A compound of any one of Clauses 412-420, or a salt, hydrate, or solvate thereof; wherein y is an integer of from 12 to 37.
Clause 422. A compound of any one of Clauses 412-420, or a salt, hydrate, or solvate thereof; wherein y is an integer of from 20 to 30.
Clause 423. A compound of any one of Clauses 412-422, or a salt, hydrate, or solvate thereof; wherein y is an integer of from 23 to 25.
Clause 424. A compound of any one of Clauses 412-423, or a salt, hydrate, or solvate thereof; wherein y is 24.
Clause 425. A compound of any one of Clauses 324-411, or a salt, hydrate, or solvate thereof; wherein said compound is of Formula (Q): 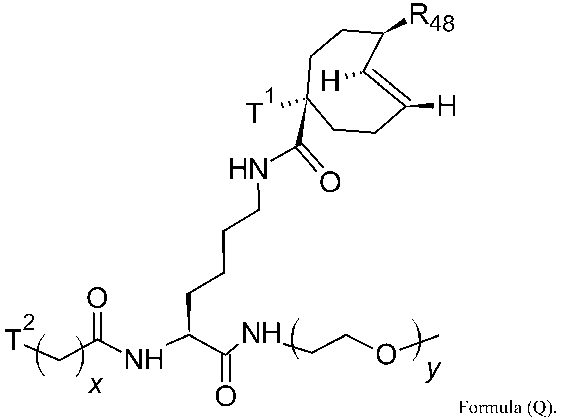
Clause 426. A compound of Clause 425, or a salt, hydrate, or solvate thereof; wherein T¹ is
   -OH.
Clause 427. A compound of any one of Clauses 425-426, or a salt, hydrate, or solvate thereof; wherein R₄₈ is -O-C(O)-C^{A}, wherein C^{A} is a drug, preferably C^{A} is monomethyl auristatin E (MMAE), exatecan, or an exatecan derivative.
Clause 428. A compound of any one of Clauses 425-427, or a salt, hydrate, or solvate thereof; wherein T¹ is -OH, and R₄₈ is - O-C(O)-C^{A}, wherein C^{A} is a drug, preferably C^{A} is monomethyl auristatin E (MMAE), exatecan, or an exatecan derivative.
Clause 429. A compound of any one of Clauses 425-428, or a salt, hydrate, or solvate thereof; wherein R₄₈ is: 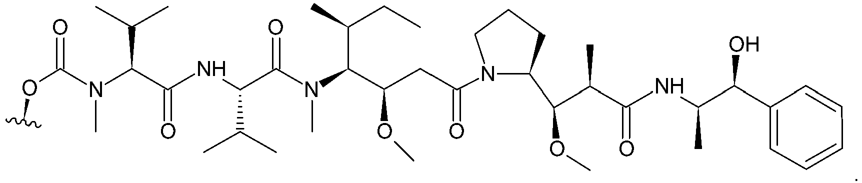
Clause 430. A compound of any one of Clauses 425-429, or a salt, hydrate, or solvate thereof; wherein T¹ is -OH, and R₄₈ is: 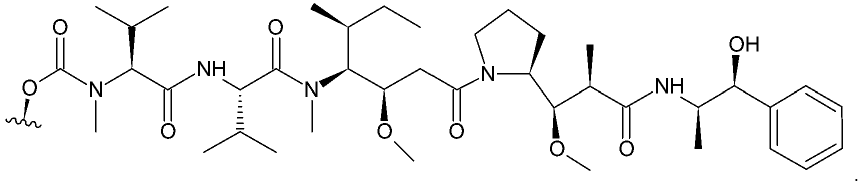
Clause 431. A compound of any one of Clauses 425-430, or a salt, hydrate, or solvate thereof; wherein x is an integer of from 3 to 8.
Clause 432. A compound of any one of Clauses 425-431, or a salt, hydrate, or solvate thereof; wherein x is an integer of from 4 to 6.
Clause 433. A compound of any one of Clauses 425-432, or a salt, hydrate, or solvate thereof; wherein x is 5.
Clause 434. A compound of any one of Clauses 425-433, or a salt, hydrate, or solvate thereof; wherein y is an integer of from 12 to 37.
Clause 435. A compound of any one of Clauses 425-434, or a salt, hydrate, or solvate thereof; wherein y is an integer of from 20 to 30.
Clause 436. A compound of any one of Clauses 425-435, or a salt, hydrate, or solvate thereof; wherein y is an integer of from 23 to 25.
Clause 437. A compound of any one of Clauses 425-436, or a salt, hydrate, or solvate thereof; wherein y is 24.
Clause 438. A compound of Clause 1, or a salt, hydrate, or solvate thereof; wherein said compound is: 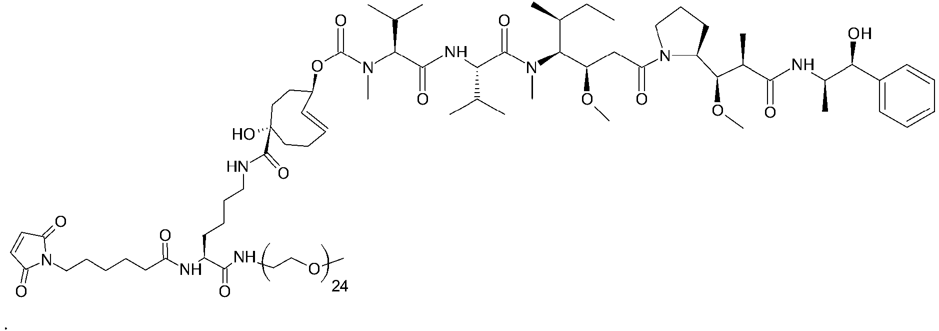
Clause 439. A compound of Clause 1, or a salt, hydrate, or solvate thereof; wherein said compound is: 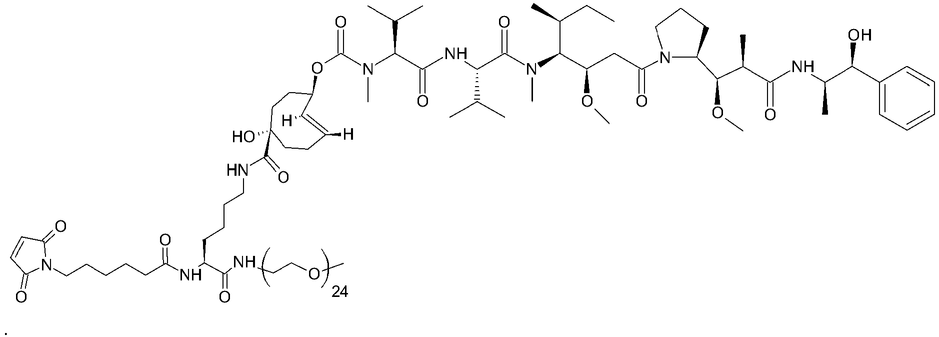
Clause 440. A compound of Clause 1, or a salt, hydrate, or solvate thereof; wherein said compound is: 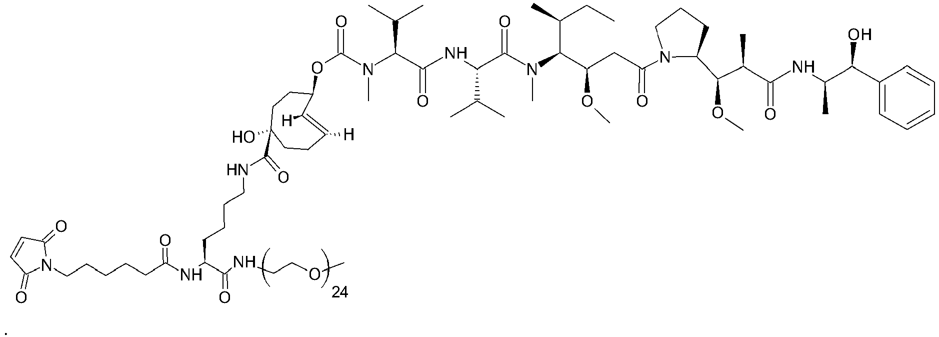
Clause 441. A compound of Clause 1, or a salt, hydrate, or solvate thereof; wherein said compound is: 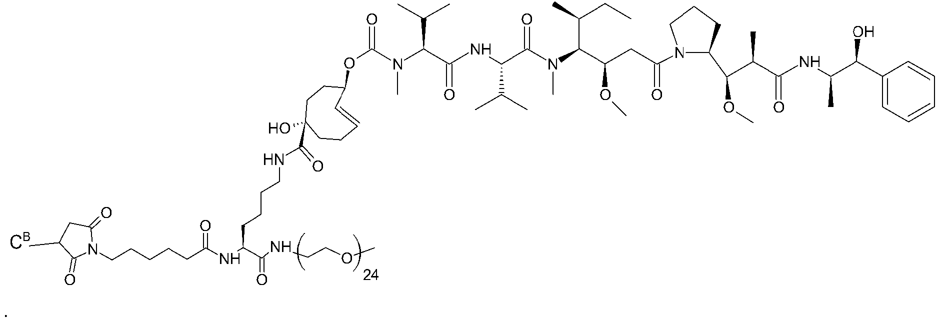
Clause 442. A compound of Clause 441, or a salt, hydrate, or solvate thereof; wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine residue.
Clause 443. A compound of Clause 1, or a salt, hydrate, or solvate thereof; wherein said compound is: 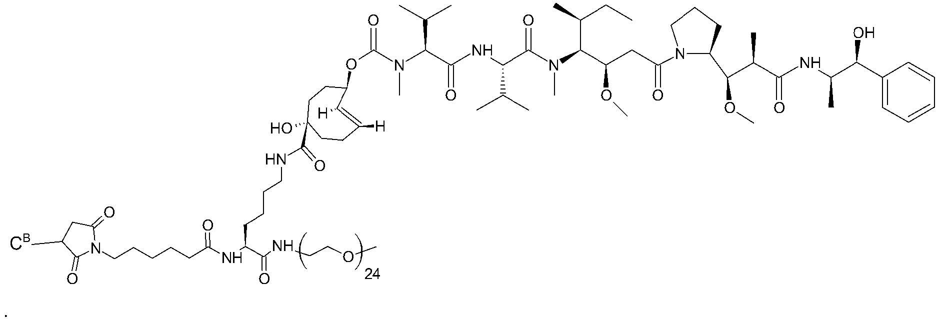
Clause 444. A compound of Clause 443, or a salt, hydrate, or solvate thereof; wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine residue.
Clause 445. A compound of Clause 1, or a salt, hydrate, or solvate thereof; wherein said compound is: 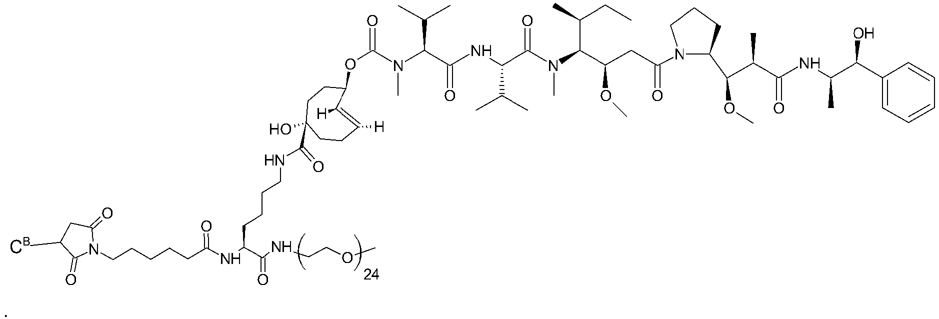
Clause 446. A compound of Clause 445, or a salt, hydrate, or solvate thereof; wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine residue.
Clause 447. A conjugate, or a salt, hydrate, or solvate thereof, wherein the conjugate comprises a protein conjugated to at least one compound according to any one of Clauses 1-446, wherein T² is a residue of a bioconjugation moiety, and said protein and said compound are conjugated via T².
Clause 448. A conjugate of Clause 447, or a salt, hydrate, or solvate thereof; wherein
   the protein is a diabody or an antibody.
Clause 449. A conjugate of any one of Clauses 447-448, or a salt, hydrate, or solvate thereof; wherein the protein is a diabody.
Clause 450. A conjugate of any one of Clauses 447-449, or a salt, hydrate, or solvate thereof; wherein the protein is AVP0458 consisting of two monomers, wherein each of the two monomers has an amino acid sequence according to SEQ ID NO: 1.
Clause 451. A conjugate of any one of Clauses 447-450, or a salt, hydrate, or solvate thereof;
   wherein the protein is conjugated to at most 12 of said compounds.
Clause 452. A conjugate of any one of Clauses 447-451, or a salt, hydrate, or solvate thereof;
   wherein the protein is conjugated to at most 8 of said compounds.
Clause 453. A conjugate of any one of Clauses 447-452, or a salt, hydrate, or solvate thereof;
   wherein the protein is conjugated to at most 4 of said compounds.
Clause 454. A conjugate of any one of Clauses 447-452, or a salt, hydrate, or solvate thereof;
   wherein the protein is conjugated to about 4 of said compounds.
Clause 455. A conjugate of any one of Clauses 447-454, or a salt, hydrate, or solvate thereof;
   wherein said protein and said compound are conjugated via T² and a residue of a sulfhydryl of said protein, a residue of a hydroxyl of said protein, or a residue of an amine of said protein.
Clause 456. A conjugate of any one of Clauses 447-455, or a salt, hydrate, or solvate thereof;
   wherein said protein and said compound are conjugated via T² and a residue of a sulfhydryl of said protein.
Clause 457. A conjugate of any one of Clauses 447-456, or a salt, hydrate, or solvate thereof;
   wherein T² is a residue of a maleimidyl moiety or a residue of an N-hydroxysuccinimidyl moiety.
Clause 458. A conjugate of any one of Clauses 447-457, or a salt, hydrate, or solvate thereof;
   wherein T² is a residue of a maleimidyl moiety.
Clause 459. A conjugate of any one of Clauses 447-458, or a salt, hydrate, or solvate thereof;
   wherein said protein and said compound are conjugated via T² and a residue of a sulfhydryl of said protein; and T² is a residue of a maleimidyl moiety.
Clause 460. A conjugate of any one of Clauses 447-459, or a salt, hydrate, or solvate thereof;
   wherein said compound is as defined in any one of Clauses 1-181.
Clause 461. A conjugate of any one of Clauses 447-459, or a salt, hydrate, or solvate thereof;
   wherein said compound is as defined in any one of Clauses 182-227.
Clause 462. A conjugate of any one of Clauses 447-459, or a salt, hydrate, or solvate thereof;
   wherein said compound is as defined in any one of Clauses 228-295.
Clause 463. A conjugate of any one of Clauses 447-459, or a salt, hydrate, or solvate thereof;
   wherein said compound is as defined in any one of Clauses 296-297.
Clause 464. A conjugate of any one of Clauses 447-459, or a salt, hydrate, or solvate thereof;
   wherein said compound is as defined in any one of Clauses 298-299.
Clause 465. A conjugate of any one of Clauses 447-459, or a salt, hydrate, or solvate thereof;
   wherein said compound is as defined in any one of Clauses 300-301.
Clause 466. A conjugate of any one of Clauses 447-459, or a salt, hydrate, or solvate thereof;
   wherein said compound is as defined in any one of Clauses 302-303.
Clause 467. A conjugate of any one of Clauses 447-459, or a salt, hydrate, or solvate thereof;
   wherein said compound is as defined in any one of Clauses 304-305.
Clause 468. A conjugate of any one of Clauses 447-459, or a salt, hydrate, or solvate thereof;
   wherein said compound is as defined in any one of Clauses 306-307.
Clause 469. A conjugate of any one of Clauses 447-459, or a salt, hydrate, or solvate thereof;
   wherein said compound is as defined in any one of Clauses 308-309.
Clause 470. A conjugate of any one of Clauses 447-459, or a salt, hydrate, or solvate thereof;
   wherein said compound is as defined in any one of Clauses 310-311.
Clause 471. A conjugate of any one of Clauses 447-459, or a salt, hydrate, or solvate thereof;

   wherein said compound is as defined in any one of Clauses 312-317.
Clause 472. A conjugate of any one of Clauses 447-459, or a salt, hydrate, or solvate thereof;
   wherein said compound is as defined in any one of Clauses 318-323.
Clause 473. A conjugate of any one of Clauses 447-459, or a salt, hydrate, or solvate thereof;
   wherein said compound is as defined in any one of Clauses 324-392.
Clause 474. A conjugate of any one of Clauses 447-459, or a salt, hydrate, or solvate thereof;
   wherein said compound is as defined in any one of Clauses 393-404.
Clause 475. A conjugate of any one of Clauses 447-459, or a salt, hydrate, or solvate thereof;
   wherein said compound is as defined in any one of Clauses 405-410.
Clause 476. A conjugate of any one of Clauses 447-459, or a salt, hydrate, or solvate thereof;
   wherein said compound is as defined in Clause 411.
Clause 477. A conjugate of any one of Clauses 447-459, or a salt, hydrate, or solvate thereof;
   wherein said compound is as defined in any one of Clauses 412-424.
Clause 478. A conjugate of any one of Clauses 447-459, or a salt, hydrate, or solvate thereof;
   wherein said compound is as defined in any one of Clauses 425-437.
Clause 479. A conjugate of any one of Clauses 447-478, or a salt, hydrate, or solvate thereof;
   wherein the conjugate is 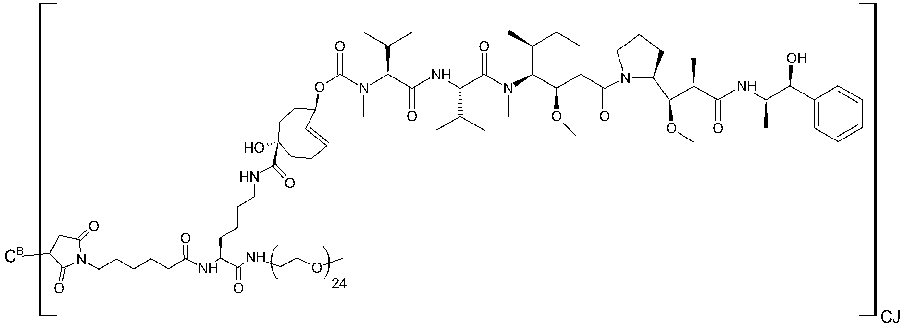
   wherein CJ is in a range of from 1 to 12, wherein preferably CJ is of from 2 to 10, more preferably of from 2.5 to 8, even more preferably of from 3 to 6, and most preferably of from 3.5 to 4.
Clause 480. A conjugate of Clause 479, or a salt, hydrate, or solvate thereof; wherein C^{B} is a protein.
Clause 481. A conjugate of Clause 480, or a salt, hydrate, or solvate thereof; wherein the protein is an antibody or a diabody. Clause 482. A conjugate of Clause 481, or a salt, hydrate, or solvate thereof; wherein the protein is a diabody.
Clause 483. A conjugate of Clause 482, or a salt, hydrate, or solvate thereof; wherein the protein is AVP0458 consisting of two monomers, wherein each of the two monomers has an amino acid sequence according to SEQ ID NO: 1.
Clause 484. A conjugate of any one of Clauses 479-483, or a salt, hydrate, or solvate thereof; wherein CJ is of from 2 to 10.
Clause 485. A conjugate of any one of Clauses 479-484, or a salt, hydrate, or solvate thereof; wherein CJ is of from 2.5 to 8.
Clause 486. A conjugate of any one of Clauses 479-485, or a salt, hydrate, or solvate thereof; wherein CJ is of from 3 to 6.
Clause 487. A conjugate of any one of Clauses 479-486, or a salt, hydrate, or solvate thereof; wherein CJ is of from 3.5 to 4.
Clause 488. A conjugate of any one of Clauses 479-487, or a salt, hydrate, or solvate thereof; wherein CJ is about 4.
Clause 489. A conjugate of any one of Clauses 479-488, or a salt, hydrate, or solvate thereof; wherein C^{B} is linked to each maleimidyl group via a sulfur atom.
Clause 490. A conjugate of any one of Clauses 479-489, or a salt, hydrate, or solvate thereof; wherein the sulfur atom is part of a cysteine residue.
Clause 491. A conjugate of any one of Clauses 447-490, or a salt, hydrate, or solvate thereof;
   wherein the conjugate is 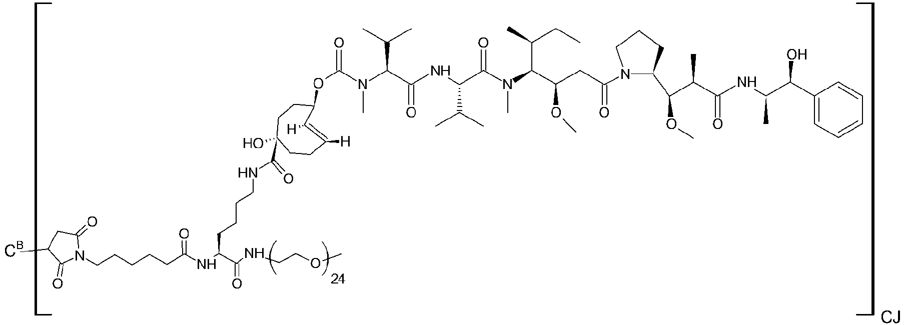
   wherein CJ is in a range of from 1 to 12.
Clause 492. A conjugate of Clause 491, or a salt, hydrate, or solvate thereof;
   wherein C^{B} is a protein.
Clause 493. A conjugate of Clause 492, or a salt, hydrate, or solvate thereof; wherein the protein is an antibody or a diabody.
Clause 494. A conjugate of Clause 493, or a salt, hydrate, or solvate thereof; wherein the protein is a diabody.
Clause 495. A conjugate of Clause 494, or a salt, hydrate, or solvate thereof; wherein the protein is AVP0458 consisting of two monomers, wherein each of the two monomers has an amino acid sequence according to SEQ ID NO: 1.
Clause 496. A conjugate of any one of Clauses 491-495, or a salt, hydrate, or solvate thereof;
   wherein CJ is of from 2 to 10.
Clause 497. A conjugate of any one of Clauses 491-496, or a salt, hydrate, or solvate thereof;
   wherein CJ is of from 2.5 to 8.
Clause 498. A conjugate of any one of Clauses 491-497, or a salt, hydrate, or solvate thereof;
   wherein CJ is of from 3 to 6.
Clause 499. A conjugate of any one of Clauses 491-498, or a salt, hydrate, or solvate thereof;
   wherein CJ is of from 3.5 to 4.
Clause 500. A conjugate of any one of Clauses 491-499, or a salt, hydrate, or solvate thereof;
   wherein CJ is about 4.
Clause 501. A conjugate of any one of Clauses 491-500, or a salt, hydrate, or solvate thereof;
   wherein C^{B} is linked to each maleimidyl group via a sulfur atom.
Clause 502. A conjugate of any one of Clauses 491-501, or a salt, hydrate, or solvate thereof;
   wherein the sulfur atom is part of a cysteine residue.
Clause 503. A conjugate of any one of Clauses 447-490, or a salt, hydrate, or solvate thereof;
   wherein the conjugate is 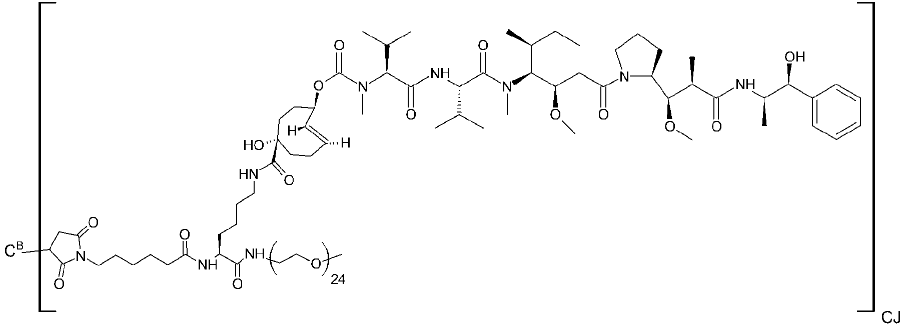
   wherein CJ is in a range of from 1 to 12.
Clause 504. A conjugate of Clause 503, or a salt, hydrate, or solvate thereof;
   wherein C^{B} is a protein.
Clause 505. A conjugate of Clause 504, or a salt, hydrate, or solvate thereof; wherein the protein is an antibody or a diabody.
Clause 506. A conjugate of Clause 505, or a salt, hydrate, or solvate thereof; wherein the protein is a diabody.
Clause 507. A conjugate of Clause 506, or a salt, hydrate, or solvate thereof; wherein the protein is AVP0458 consisting of two monomers, wherein each of the two monomers has an amino acid sequence according to SEQ ID NO: 1.
Clause 508. A conjugate of any one of Clauses 503-507, or a salt, hydrate, or solvate thereof;
   wherein CJ is of from 2 to 10.
Clause 509. A conjugate of any one of Clauses 503-508, or a salt, hydrate, or solvate thereof;
   wherein CJ is of from 2.5 to 8.
Clause 510. A conjugate of any one of Clauses 503-509, or a salt, hydrate, or solvate thereof;
   wherein CJ is of from 3 to 6.
Clause 511. A conjugate of any one of Clauses 503-510, or a salt, hydrate, or solvate thereof;
   wherein CJ is of from 3.5 to 4.
Clause 512. A conjugate of any one of Clauses 503-511, or a salt, hydrate, or solvate thereof;
   wherein CJ is about 4.
Clause 513. A conjugate of any one of Clauses 503-512, or a salt, hydrate, or solvate thereof;
   wherein C^{B} is linked to each maleimidyl group via a sulfur atom.
Clause 514. A conjugate of any one of Clauses 503-513, or a salt, hydrate, or solvate thereof;
   wherein the sulfur atom is part of a cysteine residue.
Clause 515. A composition comprising:
   (a) a compound according to any one of Clauses 1 to 446, or the salt, hydrate, or solvate thereof; and/or
   (b) the conjugate according to any one of Clauses 447 to 514, or the salt, hydrate, or solvate thereof.
Clause 516. A composition of Clause 515, wherein the composition is a pharmaceutical composition.
Clause 517. A composition of any one of Clauses 515-516, wherein said composition comprises a compound according to any one of Clauses 1 to 446, or the salt, hydrate, or solvate thereof.
Clause 518. A composition of any one of Clauses 515-517, wherein said composition comprises the conjugate according to any one of Clauses 447 to 514, or the salt, hydrate, or solvate thereof.
Clause 519. A composition of any one of Clauses 515-518, wherein said composition comprises: (a) a compound according to any one of Clauses 1 to 446, or the salt, hydrate, or solvate thereof; and (b) the conjugate according to any one of Clauses 447 to 514, or the salt, hydrate, or solvate thereof.
Clause 520. A composition of any one of Clauses 515-519, wherein said composition comprises
   (a) a compound according to any one of Clauses 1 to 446, or the salt, hydrate, or solvate thereof; and
   (b) the enantiomer of said compound, or the salt, hydrate, or solvate thereof.
Clause 521. A composition of Clause 520, wherein said composition comprises said compound and said enantiomer in a weight ratio of from 1:10 to 10:1, preferably of from 1:8 to 8:1, more preferably of from 1:7 to 7:1, even more preferably of from 1:6 to 6:1, more preferably still of from 1:5 to 5:1, even more preferably still of from 1:4 to 4:1, yet more preferably of from 1:3 to 3:1, even more preferably of from 1:2 to 2:1, more preferably still of from 1:1.5 to 1.5:1, and most preferably about 1:1.
Clause 522. A composition of Clause 521, wherein said composition is a racemic mixture of said compound and said enantiomer.
Clause 523. A composition of any one of Clauses 515-522, wherein said composition further comprises a carrier.
Clause 524. A composition of any one of Clauses 515-523, wherein said composition further comprises a pharmaceutically acceptable carrier.
Clause 525. A combination of
   (A1) a compound according to any one of Clauses 1 to 446, or the salt, hydrate, or solvate thereof;
   (A2) a conjugate according to any one of Clauses 447 to 514, or the salt, hydrate, or solvate thereof; and/or
   (A3) a composition according to any one of Clauses 515 to 524: with
   (B) a diene or a salt, solvate, or hydrate thereof.
Clause 526. The combination according to Clause 525 of (A1) and (B).
Clause 527. The combination according to Clause 525 of (A2) and (B).
Clause 528. The combination according to Clause 525 of (A3) and (B).
Clause 529. The combination according to Clause 525 of (A1), (A2), and (B).
Clause 530. The combination according to Clause 525 of (A1), (A3), and (B).
Clause 531. The combination according to Clause 525 of (A2), (A3), and (B).
Clause 532. The combination according to Clause 525 of (A1), (A2), (A3), and (B).
Clause 533. The combination of any one of Clauses 525-532, wherein the diene is a tetrazine.
Clause 534. The combination of any one of Clauses 525-535, wherein the diene is selected from the group consisting of: 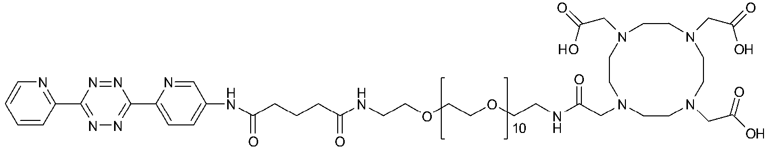 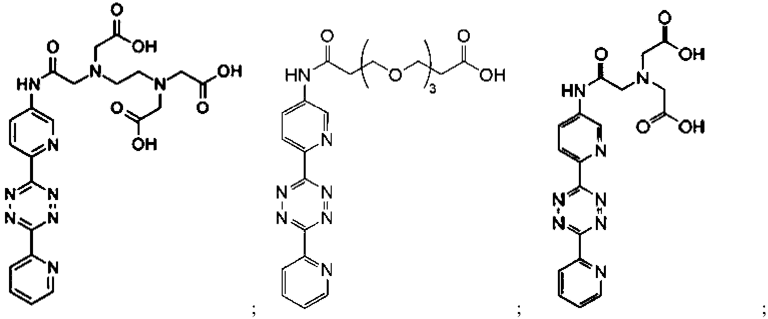 and 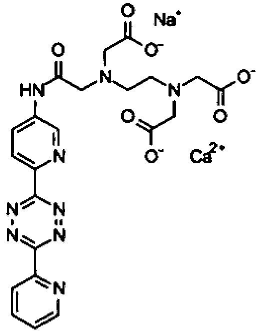 or a salt, hydrate, and/or solvate thereof.
Clause 535. The combination of Clause 534, wherein the diene is: 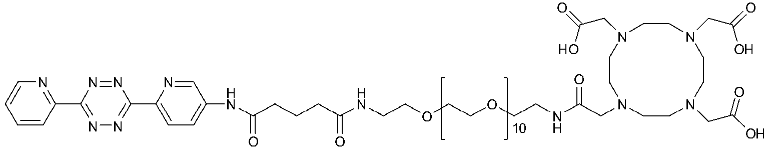 or a salt, hydrate, and/or solvate thereof.
Clause 536. The combination of Clause 534, wherein the diene is: 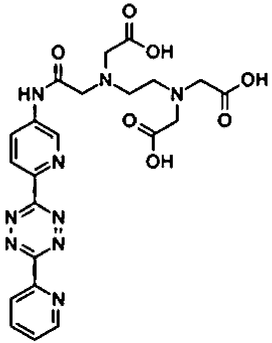 or a salt, hydrate, and/or solvate thereof.
Clause 537. The combination of Clause 534, wherein the diene is: 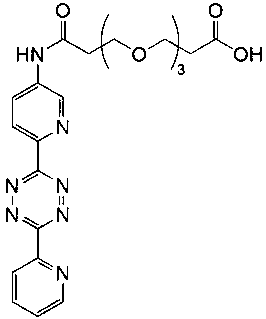 or a salt, hydrate, and/or solvate thereof.
Clause 538. The combination of Clause 534, wherein the diene is: 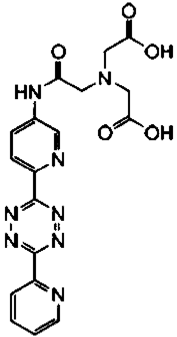 or a salt, hydrate, and/or solvate thereof.
Clause 539. The combination of Clause 534, wherein the diene is: 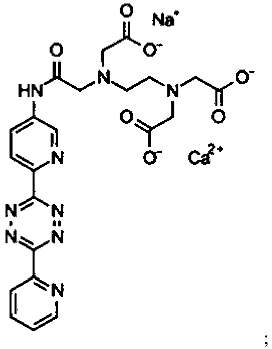 or a salt, hydrate, and/or solvate thereof.
Clause 540. The compound according to any one of Clauses 1 to 446, or the salt, hydrate, or solvate thereof; the conjugate according to any one of Clauses 447 to 514, or the salt, hydrate, or solvate thereof; the composition according to any one of Clauses 515 to 524; or the combination according to any one of Clauses 525 to 539; for use as a medicament.
Clause 541. The compound according to any one of Clauses 1 to 446, or the salt, hydrate, or solvate thereof; for use as a medicament.
Clause 542. The conjugate according to any one of Clauses 447 to 514, or the salt, hydrate, or solvate thereof; for use as a medicament.
Clause 543. The composition according to any one of Clauses 515 to 524 for use as a medicament.
Clause 544. The combination according to any one of Clauses 525 to 539 for use as a medicament.
Clause 545. The compound according to any one of Clauses 1 to 446, or the salt, hydrate, or solvate thereof; the conjugate according to any one of Clauses 447 to 514, or the salt, hydrate, or solvate thereof; the composition according to any one of Clauses 515 to 524; or the combination according to any one of Clauses 525 to 539; for use in the treatment of a disease in a subject, preferably the subject is a human; preferably the disease is cancer.
Clause 546. The compound or the salt, hydrate, or solvate thereof; conjugate or the salt, hydrate, or solvate thereof; the composition; or the combination; for use according to Clause 545, wherein the subject is a human.
Clause 547. The compound or the salt, hydrate, or solvate thereof; conjugate or the salt, hydrate, or solvate thereof; the composition; or the combination; for use according to any one of Clauses 545-546, wherein the disease is cancer.
Clause 548. The compound according to any one of Clauses 1 to 446, or the salt, hydrate, or solvate thereof; for use in the treatment of a disease in a subject, preferably the subject is a human; preferably the disease is cancer.
Clause 549. The conjugate according to any one of Clauses 447 to 514, or the salt, hydrate, or solvate thereof; for use in the treatment of a disease in a subject, preferably the subject is a human; preferably the disease is cancer.
Clause 550. The composition according to any one of Clauses 515 to 524 for use in the treatment of a disease in a subject, preferably the subject is a human; preferably the disease is cancer.
Clause 551. The combination according to any one of Clauses 525 to 539; for use in the treatment of a disease in a subject, preferably the subject is a human; preferably the disease is cancer.
Clause 552. A method of treating a disease in a subject, wherein said method comprises the step of administering to said subject:
   (a) the compound according to any one of Clauses 1 to 446, or the salt, hydrate, or solvate thereof;
   (b) the conjugate according to any one of Clauses 447 to 514, or the salt, hydrate, or solvate thereof;
   (c) the composition according to any one of Clauses 515 to 524; and/or
   (d) the combination according to any one of Clauses 525 to 539;
   preferably the subject is a human; preferably the disease is cancer.
Clause 553. Use of
   (a) a compound according to any one of Clauses 1 to 446, or the salt, hydrate, or solvate thereof;
   (b) a conjugate according to any one of Clauses 447 to 514, or the salt, hydrate, or solvate thereof;
   (c) a composition according to any one of Clauses 515 to 524; and/or
   (d) a combination according to any one of Clauses 525 to 539;
      for the manufacture of a medicament for the treatment of a disease in a subject;
      preferably the subject is a human; preferably the disease is cancer.
Clause 554. Use of a compound according to any one of Clauses 1 to 446, or the salt, hydrate, or solvate thereof; for the manufacture of a medicament for the treatment of a disease in a subject; preferably the subject is a human; preferably the disease is cancer.
Clause 555. Use of a conjugate according to any one of Clauses 447 to 514, or the salt, hydrate, or solvate thereof; for the manufacture of a medicament for the treatment of a disease in a subject; preferably the subject is a human; preferably the disease is cancer.
Clause 556. Use of a composition according to any one of Clauses 515 to 524 for the manufacture of a medicament for the treatment of a disease in a subject; preferably the subject is a human; preferably the disease is cancer.
Clause 557. Use of a combination according to any one of Clauses 525 to 539 for the manufacture of a medicament for the treatment of a disease in a subject; preferably the subject is a human; preferably the disease is cancer.
Clause 558. A non-therapeutic method for reacting:
   (ia) the compound according to any one of Clauses 1 to 446, or the salt, hydrate, or solvate thereof;
   (iia) the conjugate according to any one of Clauses 447 to 514, or the salt, hydrate, or solvate thereof; and/or
   (iiia) the composition according to any one of Clauses 515 to 524;
   with a diene or a salt, solvate, or hydrate thereof,
   wherein said method comprises the step of contacting (ia), (iia), or (iiia) with said diene or salt, solvate, or hydrate thereof; preferably said non-therapeutic method is an *in vitro* method; and preferably said diene is a tetrazine.
Clause 559. A non-therapeutic method for reacting (ia) the compound according to any one of Clauses 1 to 446, or the salt, hydrate, or solvate thereof; with a diene or a salt, solvate, or hydrate thereof, wherein said method comprises the step of contacting (ia) with said diene or salt, solvate, or hydrate thereof; preferably said non-therapeutic method is an *in vitro* method; and preferably said diene is a tetrazine.
Clause 560. A non-therapeutic method for reacting (iia) the conjugate according to any one of Clauses 447 to 514, or the salt, hydrate, or solvate thereof; with a diene or a salt, solvate, or hydrate thereof, wherein said method comprises the step of contacting (iia) with said diene or salt, solvate, or hydrate thereof; preferably said non-therapeutic method is an *in vitro* method; and preferably said diene is a tetrazine.
Clause 561. A non-therapeutic method for reacting (iiia) the composition according to any one of Clauses 515 to 524; with a diene or a salt, solvate, or hydrate thereof, wherein said method comprises the step of contacting (iiia) with said diene or salt, solvate, or hydrate thereof; preferably said non-therapeutic method is an *in vitro* method; and preferably said diene is a tetrazine.
Clause 562. A non-therapeutic use of:
   (a) the compound according to any one of Clauses 1 to 446, or the salt, hydrate, or solvate thereof;
   (b) the conjugate according to any one of Clauses 447 to 514, or the salt, hydrate, or solvate thereof;
   (c) the composition according to any one of Clauses 515 to 524; and/or
   (d) the combination according to any one of Clauses 525 to 539;
   in a click reaction.
Clause 563. A non-therapeutic use of the compound according to any one of Clauses 1 to 446, or the salt, hydrate, or solvate thereof; in a click reaction.
Clause 564. A non-therapeutic use of the conjugate according to any one of Clauses 447 to 514, or the salt, hydrate, or solvate thereof; in a click reaction.
Clause 565. A non-therapeutic use of the composition according to any one of Clauses 515 to 524; in a click reaction.
Clause 566. A non-therapeutic use of the combination according to any one of Clauses 525 to 539; in a click reaction.
Clause 567. A non-therapeutic use of any one of Clauses 562 to 566, wherein the click reaction is between a compound according to any one of Clauses 1 to 446, or the salt, hydrate, or solvate thereof; and a diene; wherein preferably the diene is a tetrazine.
Clause 568. A method for synthesizing a compound of any one of Clauses 1-446, wherein said method comprises coupling a compound of Formula (R) to a compound of Formula (S): 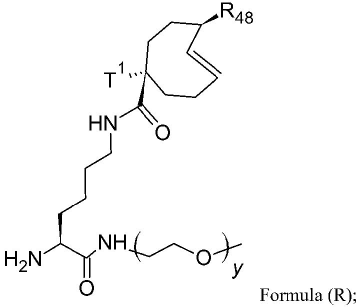 wherein R₄₈ is as defined in any one of Clauses 147-166, and 281-295, T¹ is as defined in any one of Clauses 167-179, and 246-250, and y is as defined in any one of Clauses 271, and 273-278; 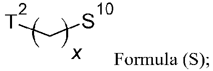 wherein T² is as defined in any one of Clauses 1, 49-69, and 251-268; x is as defined in any one of Clauses 216-218; and wherein S¹⁰ is -COOH or an active ester.
Clause 569. A method of Clause 568, wherein T² is a bioconjugation moiety.
Clause 570. A method of Clause 568, wherein T² is: 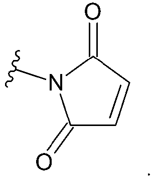
Clause 571. A method of any one of Clauses 568-570, wherein T¹ is -OH.
Clause 572. A method of any one of Clauses 568-571, wherein R₄₈ is -O-C(O)-C^{A}, wherein C^{A} is a drug, preferably C^{A} is monomethyl auristatin E (MMAE), exatecan, or an exatecan derivative.
Clause 573. A method of any one of Clauses 568-572, wherein R₄₈ is: 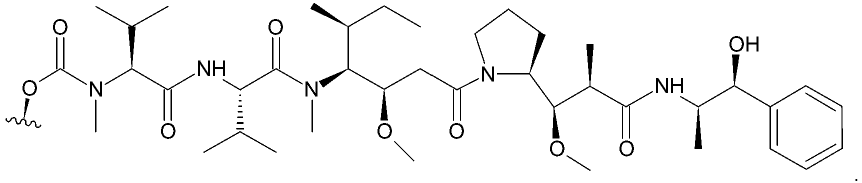
Clause 574. A method of any one of Clauses 568-573, wherein x is an integer of from 4 to 6.
Clause 575. A method of any one of Clauses 568-574, wherein x is 5.
Clause 576. A method of any one of Clauses 568-575, wherein y is an integer in a range of from 10 to 40.
Clause 577. A method of any one of Clauses 568-576, wherein y is an integer in a range of from 15 to 35.
Clause 578. A method of any one of Clauses 568-577, wherein y is an integer in a range of from 20 to 30.
Clause 579. A method of any one of Clauses 568-578, wherein y is an integer in a range of from 23 to 25.
Clause 580. A method of any one of Clauses 568-579, wherein y is 24.
Clause 581. A method of any one of Clauses 568-580, wherein in Formula (S), S¹⁰ is -COOH.
Clause 582. A method of Clause 581, wherein the compound of Formula (S) is contacted with at least one coupling reagent, preferably in the presence of a base.
Clause 583. A method of Clause 582, wherein the at least one coupling reagent is selected from the group consisting of dicyclohexylcarbodiimide (DCC), diisopropylcarbodiimide (DIC), ethyl-(N',N'-dimethylamino)propylcarbodiimide hydrochloride (EDC), 1-hydroxybenzotriazole (HOBt), 4-(N,N-dimethylamino)pyridine (DMAP), (benzotriazol-1-yloxy)tris(dimethylamino)phosphonium hexafluorophosphate (BOP), (benzotriazol-1-yloxy)tripyrrolidinophosphonium hexafluorophosphate, (7-azabenzotriazol-1-yloxy)tripyrrolidinophosphonium hexafluorophosphate (PyAOP), bromotripyrrolidinophosphonium hexafluorophosphate (PyBrOP), O-(Benzotriazol-1-yl)-N,N,N',N'-tetramethyluronium hexafluorophosphate (HBTU), O-(Benzotriazol-1-yl)-N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)-N,N,N',N'-tetramethyluronium tetrafluoroborate (TATU),
   O-(6-Chlorobenzotriazol-1-yl)-N,N,N',N'-tetramethyluronium hexafluorophosphate (HCTU), O-[(Ethoxycarbonyl)cyanomethylenamino]-N,N,N',N'-tetramethyluronium tetrafluoroborate (TOTU), (1-Cyano-2-ethoxy-2-oxoethylidenaminooxy)dimethylamino-morpholino-carbenium hexafluorophosphate (COMU), O-(N-Succinimidyl)-1,1,3,3-tetramethyl-uronium tetrafluoroborate (TSTU), O-(5-Norbornene-2,3-dicarboximido)-N,N,N',N'-tetramethyluronium tetrafluoroborate (TNTU), O-(1,2-Dihydro-2-oxo-1-pyridyl-N,N,N',N'-tetramethyluronium tetrafluoroborate (TPTU), N,N,N',N'-Tetramethyl-O-(3,4-dihydro-4-oxo-1,2,3-benzotriazin-3-yl)uronium tetrafluoroborate (TDBTU), 3-(Diethylphosphoryloxy)-1,2,3-benzotriazin-4(3H)-one (DEPBT), carbonyldiimidazole (CDI), N,N,N',N'-tetramethylchloroform-amidinium hexafluorophosphate (TCFH), thionyl chloride, oxalyl chloride, cyanuric chloride, cyanuric fluoride, phosphorous trichloride, phosphorous pentachloride, N-hydroxysuccinimide, N-hydroxysulfosuccinimide, and combinations thereof.
Clause 584. A method of any one of Clauses 568-580, wherein the active ester is selected from the group consisting of - C(O)O-N-succinimidyl, -C(O)O-pentafluorophenyl, -C(O)O-tetrafluorophenyl, -C(O)O-4-nitrophenyl, and -C(O)Cl; preferably the active ester is
   -C(O)O-N-succinimidyl, or -C(O)O-pentafluorophenyl.
Clause 585. A method of any one of Clauses 568-580, and 584, wherein in Formula (S), S¹⁰ is an active ester.
Clause 586. A method of any one of Clauses 568-585, wherein the coupling is carried out in the presence of a base.
Clause 587. A method of Clause 586, wherein the coupling is carried out in the presence of a non-nucleophilic base.
Clause 588. A method of any one of Clauses 568-587, wherein the coupling is carried out at a temperature of from -20°C to 80°C, preferably of from 0°C to 60°C, more preferably of from 4°C to 50°C, more preferably still of from 10°C to 40°C, and most preferably of from 15°C to 30°C.
Clause 589. A method of any one of Clauses 568-587, wherein the coupling is carried out in the presence of a solvent, wherein preferably the solvent is an organic solvent.
Clause 590. A method for synthesizing a compound of any one of Clauses 1-446, wherein said method comprises coupling a compound of Formula (T) to a compound of Formula (U): 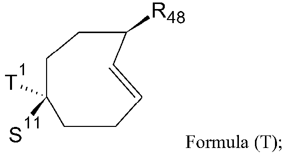 wherein R₄₈ is as defined in any one of Clauses 147-166, and 281-295; T¹ is as defined in any one of Clauses 167-179, and 246-250; and S¹¹ is -COOH or an active ester; 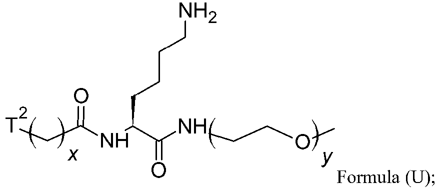 wherein T² is as defined in any one of Clauses 1, 49-69, and 251-268; x is as defined in any one of Clauses 216-218; and y is as defined in any one of Clauses 271, and 273-278.
Clause 591. A method of Clause 590, wherein T² is a bioconjugation moiety.
Clause 592. A method of Clause 591, wherein T² is: 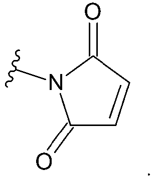
Clause 593. A method of any one of Clauses 590-592, wherein T¹ is -OH.
Clause 594. A method of any one of Clauses 590-593, wherein R₄₈ is -O-C(O)-C^{A}, wherein C^{A} is a drug, preferably C^{A} is monomethyl auristatin E (MMAE), exatecan, or an exatecan derivative.
Clause 595. A method of any one of Clauses 590-594, wherein R₄₈ is: 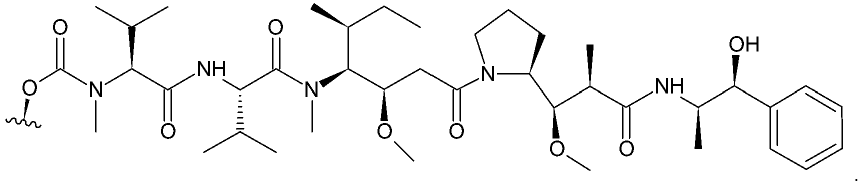
Clause 596. A method of any one of Clauses 590-595, wherein x is an integer of from 4 to 6.
Clause 597. A method of any one of Clauses 590-596, wherein x is 5.
Clause 598. A method of any one of Clauses 590-597, wherein y is an integer in a range of from 10 to 40.
Clause 599. A method of any one of Clauses 590-598, wherein y is an integer in a range of from 15 to 35.
Clause 600. A method of any one of Clauses 590-599, wherein y is an integer in a range of from 20 to 30.
Clause 601. A method of any one of Clauses 590-600, wherein y is an integer in a range of from 23 to 25.
Clause 602. A method of any one of Clauses 590-601, wherein y is 24.
Clause 603. A method of any one of Clauses 590-602, wherein in Formula (T), S¹¹ is -COOH.
Clause 604. A method of Clause 603, wherein the compound of Formula (T) is contacted with at least one coupling reagent, preferably in the presence of a base.
Clause 605. A method of Clause 604, wherein the at least one coupling reagent is selected from the group consisting of dicyclohexylcarbodiimide (DCC), diisopropylcarbodiimide (DIC), ethyl-(N',N'-dimethylamino)propylcarbodiimide hydrochloride (EDC), 1-hydroxybenzotriazole (HOBt), 4-(N,N-dimethylamino)pyridine (DMAP), (benzotriazol-1-yloxy)tris(dimethylamino)phosphonium hexafluorophosphate (BOP), (benzotriazol-1-yloxy)tripyrrolidinophosphonium hexafluorophosphate, (7-azabenzotriazol-1-yloxy)tripyrrolidinophosphonium hexafluorophosphate (PyAOP), bromotripyrrolidinophosphonium hexafluorophosphate (PyBrOP), O-(Benzotriazol-1-yl)-N,N,N',N'-tetramethyluronium hexafluorophosphate (HBTU), O-(Benzotriazol-1-yl)-N,N,N',N'-tetramethyluronium tetrafluoroborate (TBTU), O-(7-Azabenzotriazol-1-yl)-N,N,N',N'-tetramethyluronium hexafluorophosphate (HATU), O-(7-Azabenzotriazol-1-yl)-N,N,N',N'-tetramethyluronium tetrafluoroborate (TATU),
   O-(6-Chlorobenzotriazol-1-yl)-N,N,N',N'-tetramethyluronium hexafluorophosphate (HCTU), O-[(Ethoxycarbonyl)cyanomethylenamino]-N,N,N',N'-tetramethyluronium tetrafluoroborate (TOTU), (1-Cyano-2-ethoxy-2-oxoethylidenaminooxy)dimethylamino-morpholino-carbenium hexafluorophosphate (COMU), O-(N-Succinimidyl)-1,1,3,3-tetramethyl-uronium tetrafluoroborate (TSTU), O-(5-Norbornene-2,3-dicarboximido)-N,N,N',N'-tetramethyluronium tetrafluoroborate (TNTU), O-(1,2-Dihydro-2-oxo-1-pyridyl-N,N,N',N'-tetramethyluronium tetrafluoroborate (TPTU), N,N,N',N'-Tetramethyl-O-(3,4-dihydro-4-oxo-1,2,3-benzotriazin-3-yl)uronium tetrafluoroborate (TDBTU), 3-(Diethylphosphoryloxy)-1,2,3-benzotriazin-4(3H)-one (DEPBT), carbonyldiimidazole (CDI), N,N,N',N'-tetramethylchloroform-amidinium hexafluorophosphate (TCFH), thionyl chloride, oxalyl chloride, cyanuric chloride, cyanuric fluoride, phosphorous trichloride, phosphorous pentachloride, N-hydroxysuccinimide, N-hydroxysulfosuccinimide, and combinations thereof.
Clause 606. A method of any one of Clauses 590-602, wherein the active ester is selected from the group consisting of - C(O)O-N-succinimidyl, -C(O)O-pentafluorophenyl, -C(O)O-tetrafluorophenyl, -C(O)O-4-nitrophenyl, and -C(O)Cl; preferably the active ester is
   -C(O)O-N-succinimidyl, or -C(O)O-pentafluorophenyl, most preferably the active ester is -C(O)O-pentafluorophenyl.
Clause 607. A method of any one of Clauses 590-602, and 606, wherein in Formula (T), S¹¹ is an active ester.
Clause 608. A method of any one of Clauses 590-603, wherein the coupling is carried out in the presence of a base.
Clause 609. A method of Clause 608, wherein the coupling is carried out in the presence of a non-nucleophilic base.
Clause 610. A method of any one of Clauses 590-609, wherein the coupling is carried out at a temperature of from -20°C to 80°C, preferably of from 0°C to 60°C, more preferably of from 4°C to 50°C, more preferably still of from 10°C to 40°C, and most preferably of from 15°C to 30°C.
Clause 611. A method of any one of Clauses 590-610, wherein the coupling is carried out in the presence of a solvent, wherein preferably the solvent is an organic solvent.
Clause 612. A method for synthesizing a conjugate of any one of Clauses 447-514, wherein said method comprises the step of coupling a protein to a compound of any one of Clauses 1-446, or a salt, hydrate, or solvate thereof; wherein in said compound T² is a bioconjugation moiety; wherein preferably in said protein disulfide bonds have been reduced.
Clause 613. A method of Clause 612, wherein the protein, wherein the protein is an antibody or a diabody.
Clause 614. A method of any one of Clauses 612-613, wherein the protein is a diabody.
Clause 615. A method of any one of Clauses 612-614, wherein the protein is AVP0458 consisting of two monomers, wherein each of the two monomers has an amino acid sequence according to SEQ ID NO: 1.
Clause 616. A method of any one of Clauses 612-615, wherein T² is 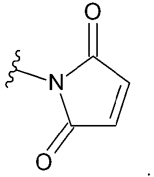
Clause 617. A method of any one of Clauses 612-616, wherein the protein has been contacted with a reducing agent prior to the coupling.
Clause 618. A method of any one of Clauses 612-617, wherein the coupling is carried out in the presence of a reducing agent.
Clause 619. A method of any one of Clauses 617 or 618, wherein the reducing agent is selected from the group consisting of dithiothreitol (DTT), and tris-2-carboxyethylphosphine hydrochloride (TCEP).
Clause 620. A method of Clause 619, wherein the reducing agent is DTT.
Clause 621. A method of any one of Clauses 612-620, wherein the coupling is carried out at a temperature of from 0°C to 40°C, more preferably of from 1°C to 30°C, more preferably still of from 2°C to 20°C, and most preferably of from 4°C to 10°C.
Clause 622. A method of any one of Clauses 612-621, wherein the coupling is carried out at a temperature of about 4°C.
Clause 623. A method of any one of Clauses 612-622, wherein the coupling is carried out in an aqueous solution.
Clause 624. A method of Clause 623, wherein the aqueous solution is an aqueous buffer solution.
Clause 625. A method of any one of Clauses 612-624, wherein the coupling is carried out at a pH of from 6.0 to 8.5, preferably of from 6.2 to 8.0, more preferably of from 6.4 to 7.8, even more preferably of from 6.5 to 7.4, and most preferably of from 6.6 to 7.0.
Clause 626. A method of any one of Clauses 612-625, wherein the coupling is carried out at a pH of about 6.8.

### List of Numbered Embodiments

Numbered Embodiment 1. A compound having a structure according to Formula (1): 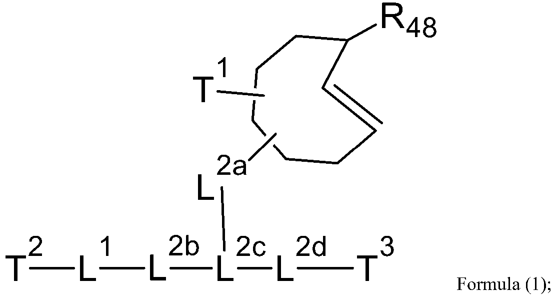 wherein
   **L¹** is selected from the group consisting of linear or branched C₄-C₁₂ alkylene, C₃-C₈ (hetero)cycloalkylene, C₆-C₁₂ arylene, and C₄-C₁₁ heteroarylene; **L^{2a}, L^{2b},** and **L^{2d}** are each independently a linker; **L^{2c}** is selected from the group consisting of C₁-Cs (hetero)alkanetriyl, C₅-C₆ (hetero)arenetriyl, C₃-C₇ cycloalkanetriyl, and C₂-C₇ heterocycloalkanetriyl; **T¹** is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃; each **T^{1A}** is independently selected from the group consisting of hydrogen, (hetero)alkyl, (hetero)alkenyl, (hetero)alkynyl, (hetero)aryl, and an amino acid residue; **T² is** a bioconjugation moiety or a group -L³-C^{B}; wherein **L³** is a residue of a bioconjugation moiety, and **C^{B}** is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof;
   **T³** is a polymer; and **R₄₈** is selected from the group consisting of -OH, -O-acetyl, -O-C₁₋₄ alkyl, halogen, active carbonate, and a releasable group; and preferably L¹ is linear or branched C₄-C₁₂ alkylene, more preferably L¹ is linear or branched C₄-C₁₀ alkylene, and most preferably L¹ is linear C₅-C₆ alkylene; preferably L^{2a}, L^{2b}, and L^{2d} are each independently a linker containing at most twenty atoms; more preferably L^{2a}, L^{2b}, and L^{2d} are each independently selected from the group consisting of -C(O)NL^{2T}-, -NL^{2T}C(O)-, -O-, -S-, -NL^{2T}-, -N=N-, and -C(O)-; wherein L^{2T} is hydrogen or methyl, preferably L^{2T} is hydrogen; preferably L^{2c} is C₁-C₈ (hetero)alkanetriyl, more preferably L^{2c} is C₁-C₈ alkanetriyl, and most preferably L^{2c} is C₄-C₆ alkanetriyl; preferably T¹ is -OT^{1A}; and most preferably T¹ is -OH; preferably T^{1A} is hydrogen or methyl, more preferably T^{1A} is hydrogen; preferably T² is maleimidyl, N-hydroxysuccinimidyl, or -L³-C^{B}; preferably L³ is a residue of a maleimidyl moiety or a residue of an N-hydroxysuccinimidyl moiety; preferably C^{B} is a protein, more preferably C^{B} is an antibody or a diabody, even more preferably C^{B} is a diabody, and most preferably C^{B} is AVP0458 consisting of two monomers, wherein each of the two monomers has an amino acid sequence according to SEQ ID NO: 1; preferably T³ is a polymer comprising a polyethylene glycol moiety; and preferably R₄₈ is a releasable group.
Numbered Embodiment 2. The compound according to Numbered Embodiment 1, wherein said compound is according to Formula (2): 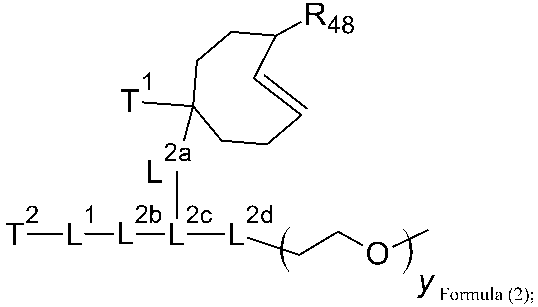 wherein y is an integer in a range of from 1 to 50; preferably y is an integer in a range of from 10 to 40, more preferably in a range of from 12 to 37, even more preferably in a range of from 15 to 35, more preferably still in a range of from 20 to 30, and most preferably in a range of from 23 to 25.
Numbered Embodiment 3. The compound according to any one of the preceding Numbered Embodiments, wherein said compound is according to Formula (3): 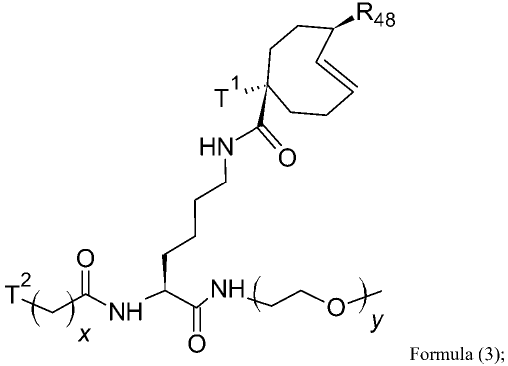 wherein y is as defined in Numbered Embodiment 2; x is an integer in a range of from 4 to 12; preferably x is an integer in a range of from 4 to 8, more preferably x is an integer in a range of from 4 to 6.
Numbered Embodiment 4. The compound according to any one of the preceding Numbered Embodiments, wherein R⁴⁸ is a releasable group, and said releasable group is -O-CO-C^{A}; wherein C^{A} is a drug; preferably the drug is linked to the moiety - O-CO- via a secondary or tertiary nitrogen atom that is part of the drug, forming a carbamate; preferably the drug is monomethyl auristatin E (MMAE).
Numbered Embodiment 5. The compound according to any one of the preceding Numbered Embodiments, wherein T² is selected from the group consisting of 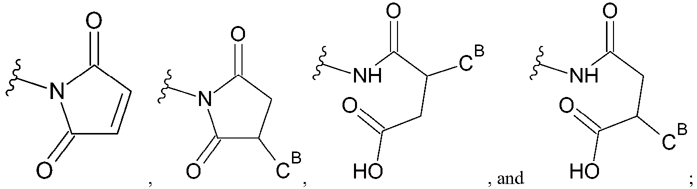 wherein
   C^{B} is a protein; preferably C^{B} is an antibody or a diabody, more preferably a diabody, and most preferably AVP0458 consisting of two monomers, wherein each of the two monomers has an amino acid sequence according to SEQ ID NO: 1; preferably C^{B} is linked to the remainder of T² via S or N that is part of C^{B}, more preferably S.
Numbered Embodiment 6. The compound according to any one of the preceding Numbered Embodiments, wherein said compound is: 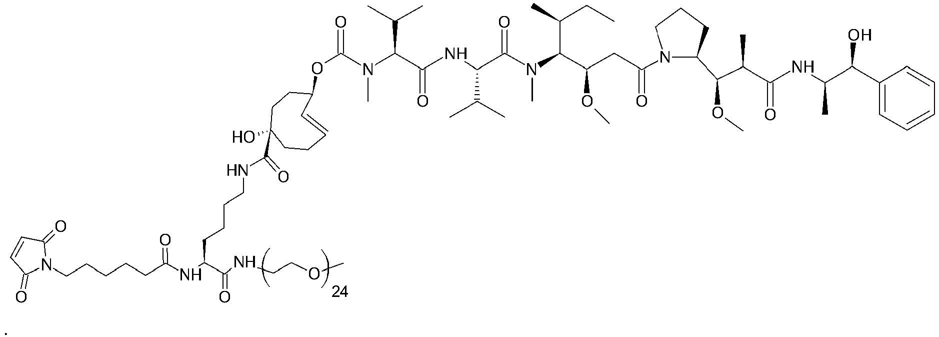
Numbered Embodiment 7. The compound according to any one of the preceding Numbered Embodiments, wherein said compound is 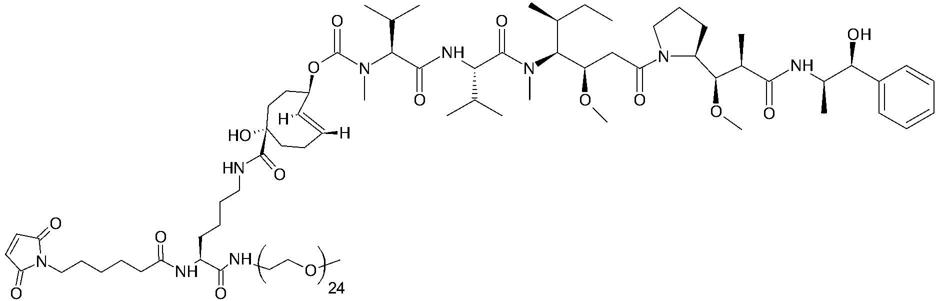 or 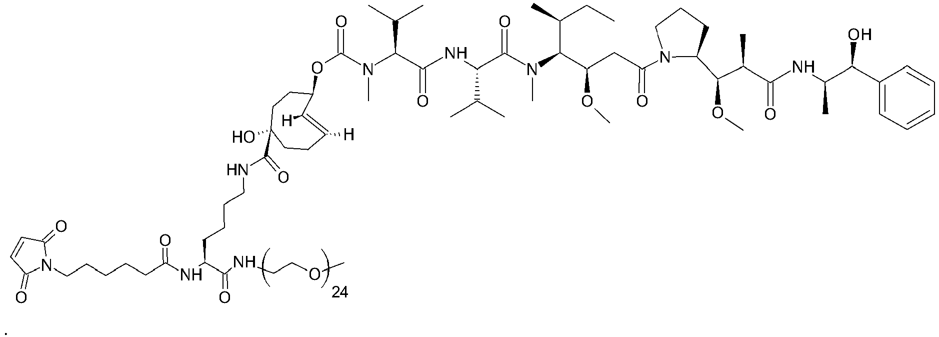
Numbered Embodiment 8. The compound according to any one of Numbered Embodiments 1 to 5, wherein said compound is: 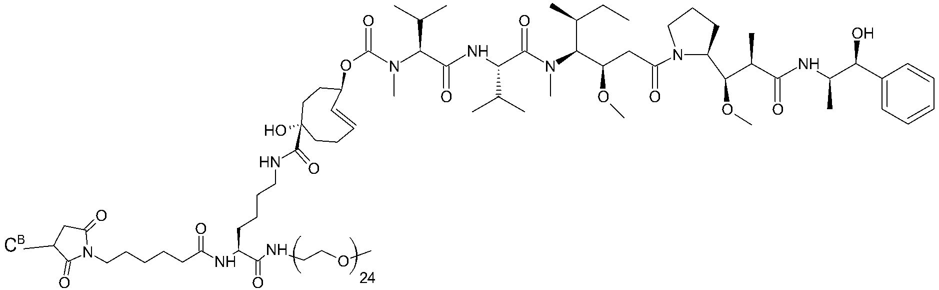 wherein C^{B} is AVP0458 consisting of two monomers, wherein each of the two monomers has an amino acid sequence according to SEQ ID NO: 1; preferably C^{B} is linked to the maleimidyl group via a sulfur atom that is part of C^{B}, preferably the sulfur atom is part of a cysteine.
Numbered Embodiment 9. The compound according to Numbered Embodiment 8, wherein said compound is: 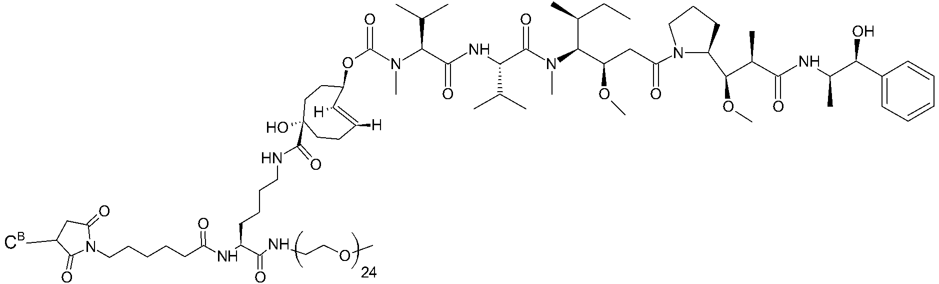 or 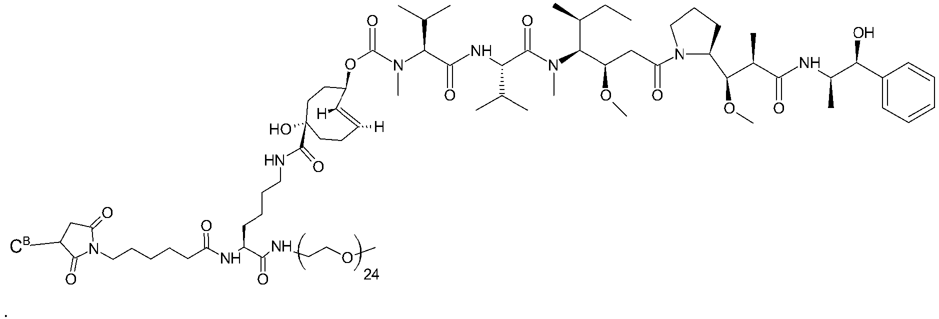
Numbered Embodiment 10. A compound comprising an eight-membered non-aromatic cyclic mono-alkenylene moiety, wherein said moiety comprises a non-vinylic carbon atom, wherein said non-vinylic carbon atom is substituted with at least one structure according to Formula (A): 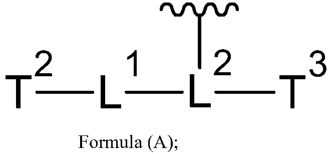 wherein
   L¹ and L² are each independently a linker; and
   T² and T³ are organic moieties.
Numbered Embodiment 11. A conjugate comprising a protein conjugated to at least one compound according to Formula (1) as defined in any one of Numbered Embodiments 1 to 9, wherein L¹, L^{2a}, L^{2b}, L^{2c}, L^{2d}, T¹, T³, and R⁴⁸ are as defined in any one of Numbered Embodiments 1 to 9, and wherein T² is a residue of a bioconjugation moiety, and said protein and said compound are conjugated via T²; preferably the protein is a diabody or an antibody; more preferably the protein is a diabody; and most preferably the protein is AVP0458 consisting of two monomers, wherein each of the two monomers has an amino acid sequence according to SEQ ID NO: 1; preferably the protein is conjugated to at most 12 of said compounds; more preferably the protein is conjugated to at most 8 of said compounds, most preferably the protein is conjugated to at most 4 of said compounds; preferably said protein and said compound are conjugated via T² and a residue of a sulfhydryl of said protein, a residue of a hydroxyl of said protein, or a residue of an amine of said protein; more preferably said protein and said compound are conjugated via T² and a residue of a sulfhydryl of said protein; preferably T² is a residue of a maleimidyl moiety or a residue of an N-hydroxysuccinimidyl moiety; more preferably T² is a residue of a maleimidyl moiety.
Numbered Embodiment 12. The conjugate according to Numbered Embodiment 11, wherein the conjugate is 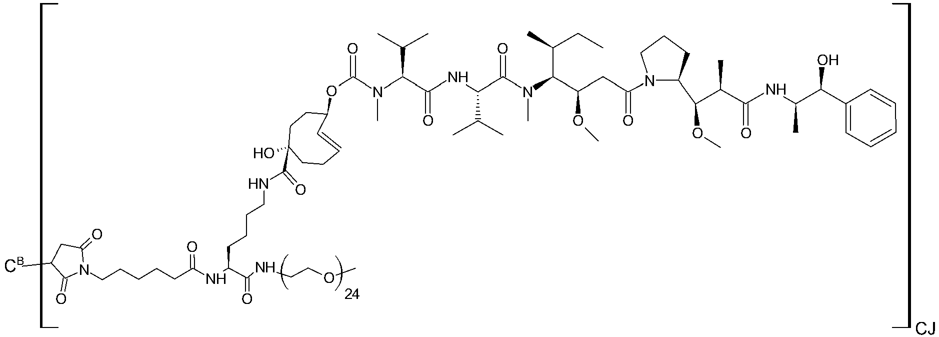 wherein CJ is in a range of from 1 to 12; wherein C^{B} is AVP0458 consisting of two monomers, wherein each of the two monomers has an amino acid sequence according to SEQ ID NO: 1; preferably CJ is of from 2 to 10, more preferably of from 2.5 to 8, even more preferably of from 3 to 6, even more preferably still of from 3.5 to 4, and most preferably about 4; preferably C^{B} is linked to each maleimidyl group via a sulfur atom, preferably the sulfur atom is part of a cysteine.
Numbered Embodiment 13. The conjugate according to Numbered Embodiment 12, wherein the conjugate is 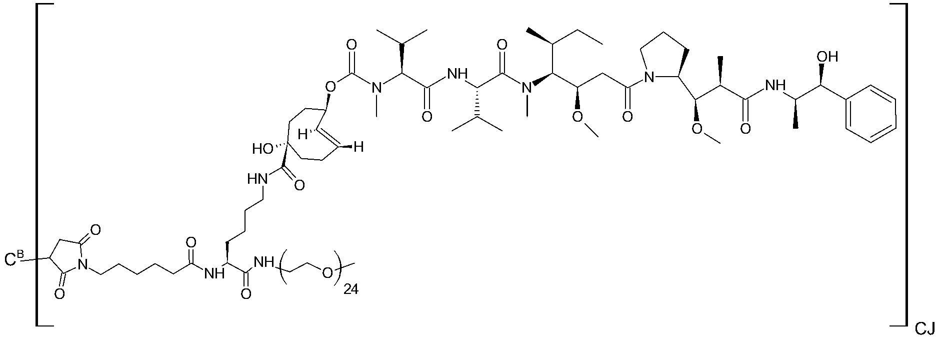 or 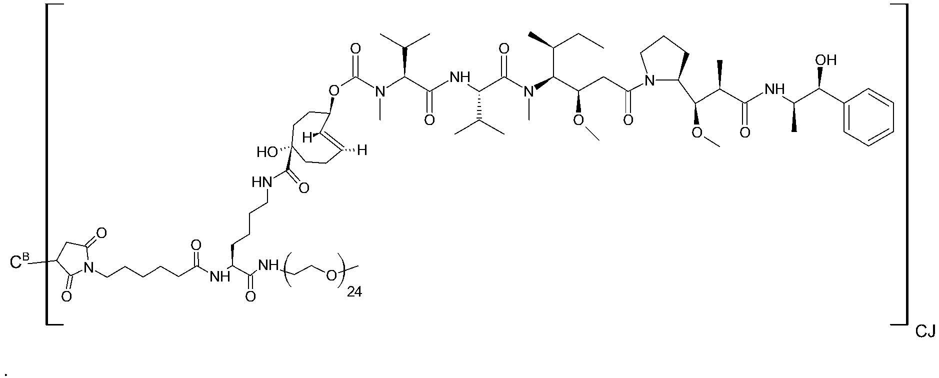
Numbered Embodiment 14. A composition comprising: (a) a compound according to any one of Numbered Embodiments 1 to 10; and/or (b) the conjugate according to any one of Numbered Embodiments 11 to 13; preferably the composition is a pharmaceutical composition.
Numbered Embodiment 15. A composition according to Numbered Embodiment 14, wherein said composition comprises: (a) a compound according to any one of Numbered Embodiments 1 to 10; and (b) the enantiomer of said compound; preferably said composition is a racemic mixture of (a) and (b).
Numbered Embodiment 16. A combination of (A1) a compound according to any one of Numbered Embodiments 1 to 10; (A2) a conjugate according to any one of Numbered Embodiments 11 to 13; and/or (A3) a composition according to Numbered Embodiment 14 or 15; with (B) a diene; preferably the diene is a tetrazine.
Numbered Embodiment 17. The combination according to Numbered Embodiment 16, wherein the diene is selected from the group consisting of: 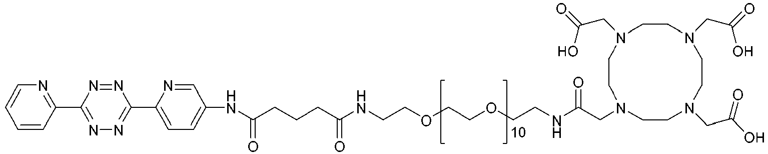 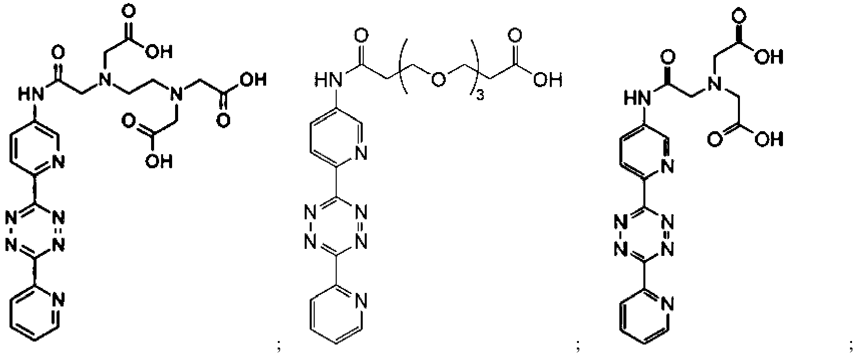 and 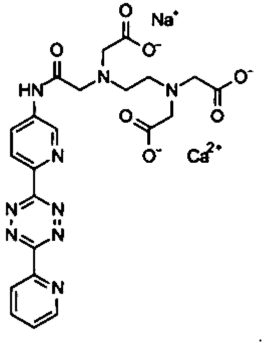
Numbered Embodiment 18. The compound according to any one of Numbered Embodiments 1 to 10; the conjugate according to any one of Numbered Embodiments 11 to 13; the composition according to any one of Numbered Embodiments 14 to 15; or the combination according to any one of Numbered Embodiments 16 to 17; for use as a medicament.
Numbered Embodiment 19. The compound according to any one of Numbered Embodiments 1 to 10; the conjugate according to any one of Numbered Embodiments 11 to 13; the composition according to any one of Numbered Embodiments 14 to 15; or the combination according to any one of Numbered Embodiments 16 to 17; for use in the treatment of a disease in a subject, preferably the subject is a human; preferably the disease is cancer.
Numbered Embodiment 20. A method of treating a disease in a subject, wherein said method comprises the step of administering to said subject: (a) the compound according to any one of Numbered Embodiments 1 to 10; (b) the conjugate according to any one of Numbered Embodiments 11 to 13; (c) the composition according to any one of Numbered Embodiments 14 to 15; and/or (d) the combination according to any one of Numbered Embodiments 16 to 17; preferably the subject is a human; preferably the disease is cancer.
Numbered Embodiment 21. A non-therapeutic method for reacting: (ia) the compound according to any one of Numbered Embodiments 1 to 10; (iia) the conjugate according to any one of Numbered Embodiments 11 to 13; and/or (iiia) the composition according to any one of Numbered Embodiments 14 to 15; with a diene, wherein said method comprises the step of contacting (ia), (iia), or (iiia) with said diene, preferably said non-therapeutic method is an *in vitro* method; and preferably said diene is a tetrazine.
Numbered Embodiment 22. A non-therapeutic use of: (a) the compound according to any one of Numbered Embodiments 1 to 10; (b) the conjugate according to any one of Numbered Embodiments 11 to 13; (c) the composition according to any one of Numbered Embodiments 14 to 15; and/or (d) the combination according to any one of Numbered Embodiments 16 to 17; in a click reaction.
Numbered Embodiment 23. A method for synthesizing a compound according to any one of Numbered Embodiments 1 to 10; wherein said method comprises (A) coupling a compound of Formula (R) to a compound of Formula (S): 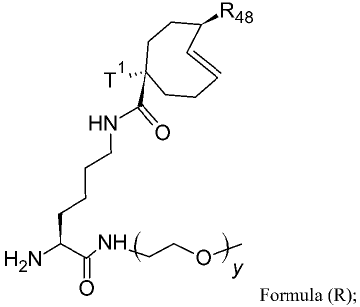 wherein R₄₈, T¹, and y are as defined in any one of Numbered Embodiments 1 to 10; 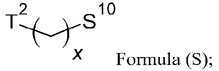 wherein T², and x, are as defined in any one of Numbered Embodiments 1 to 10, and S¹⁰ is - COOH or an active ester, preferably S¹⁰ is -COOH;
   or
   (B) coupling a compound of Formula (T) to a compound of Formula (U): 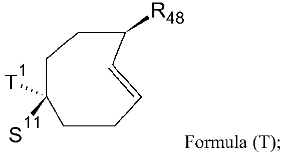 wherein R₄₈, and T¹ are as defined in any one of Numbered Embodiments 1 to 10; and S¹¹ is -COOH or an active ester, preferably S¹¹ is an active ester; 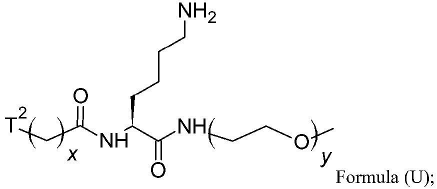 wherein T², x, and y are as defined in any one of Numbered Embodiments 1 to 10.
Numbered Embodiment 24. A method for synthesizing a conjugate according to any one of Numbered Embodiments 11 to 13; wherein said method comprises the step of coupling a protein to a compound according to any one of Numbered Embodiments 1 to 10; wherein in said compound T² is a bioconjugation moiety; wherein preferably in said protein disulfide bonds have been reduced.

### Examples

### Example 1: Synthesis

### Example 1a: compounds 1 and 2

Intermediate compound **3** was obtained in three steps with an overall yield of 29% using commercially available starting materials:

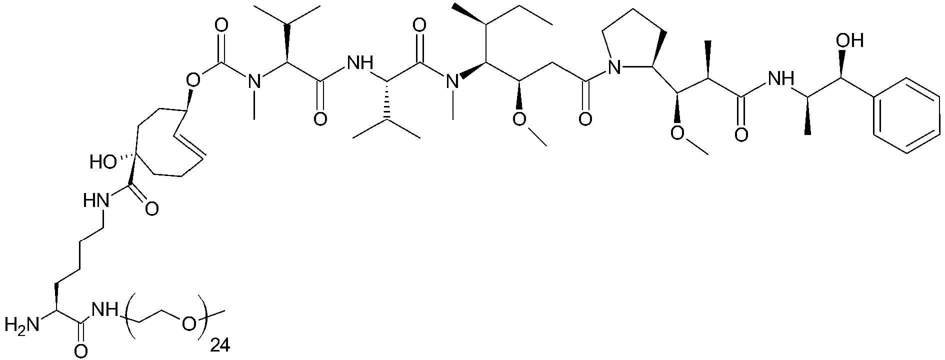

### Compound 3.

Compounds **4** and **5** were synthesized by coupling compound **3** to maleimide-PEG4-COOH or maleimide-C5-COOH, respectively, using conventional coupling reagents. This afforded the desired products, viz. compounds **4** and **5,** in high yield.

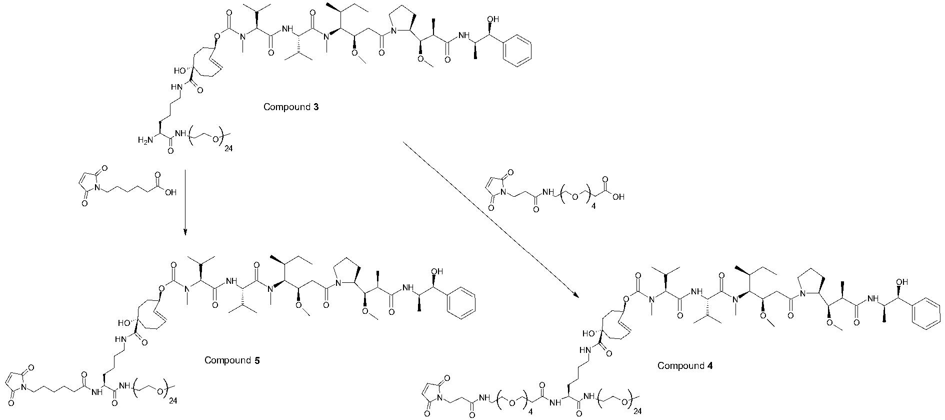

Compound **4** or **5,** respectively, was conjugated to diabody AVP0458 to afford compound **1** or **2** following the optimized procedure by Rossin *et al.,* Nature Communications (2018)9:1484.

Briefly, 10 mg AVP0458 was reacted with 6 mM DTT for 3 h at room temperature on a roller bench, followed by purification via PD-10 pre-equilibrated with 0.1 M phosphate buffer pH 6.8, containing 2 mM EDTA (PB-EDTA buffer). The purified diabody solution was then split in two aliquots which were then added with 30 eq of compound **4** or compound **5,** respectively, dissolved in dry DMSO at 10 mM concentration. The two reaction mixtures were incubated for 1h at room temperature on a roller bench followed by overnight incubation at +4°C. The two resulting conjugates ADCs (compound **1,** conjugate of AVP0458 and compound **4;** and compound **2,** conjugate of AVP0458 and compound **5)** were then purified from the crude mixtures by SEC (Superdex75 10/300 column eluted with PBS at 0.8 mL/min) followed by concentration via Amicon Ultra-4 (30 kDa MW cut-off). UV measurements on the final solutions showed 75-80% recovery of diabody. SDS-PAGE analysis of the two ADC solutions showed the presence of one species with the expected increase in MW with respect to that of the monomer in AVP0458. Further analysis using mass spectrometry showed a complete reaction between the four cysteine residues in AVP0458 with the respective TCOs, confirming the production of two conjugates with a DAR of 4.

Compound **1** has the following structure:

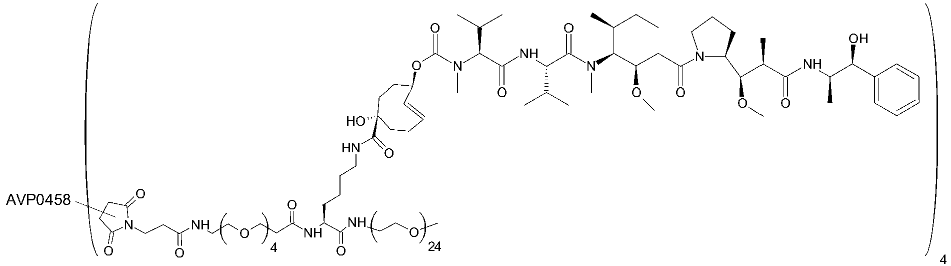

Compound **2** has the following structure:

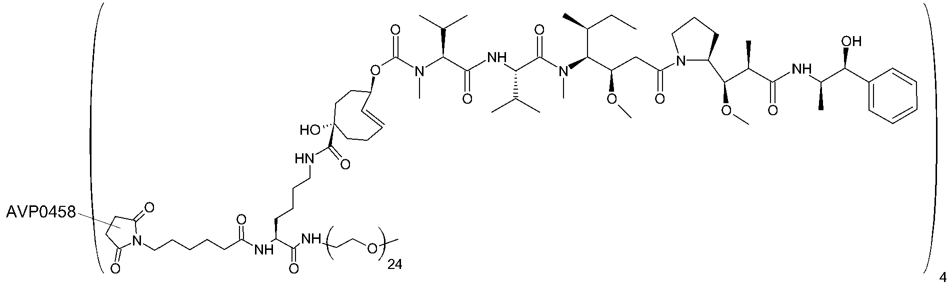

### Example 1b: reference compounds 14a and 14b and claimed compounds 15a and 15b

Reference compounds **14a** and **14b** were synthesized by conjugating compound **11a** or **11b**, respectively, to the diabody AVP0458. Likewise, claimed compounds **15a** and **15b** were synthesized by conjugating compound 13a or 13b, respectively, to the diabody AVP0458.

Below, first the synthesis of **11a** and **11b** is described, and then the synthesis of **13a** and **13b**. Thereafter, the conjugation is described of **11a**, **11b**, **13a,** or **13b** to AVP0458 to yield **14a, 14b, 15a,** or **15b.**

### Example lb-i: synthesis of compounds 11a and 11b

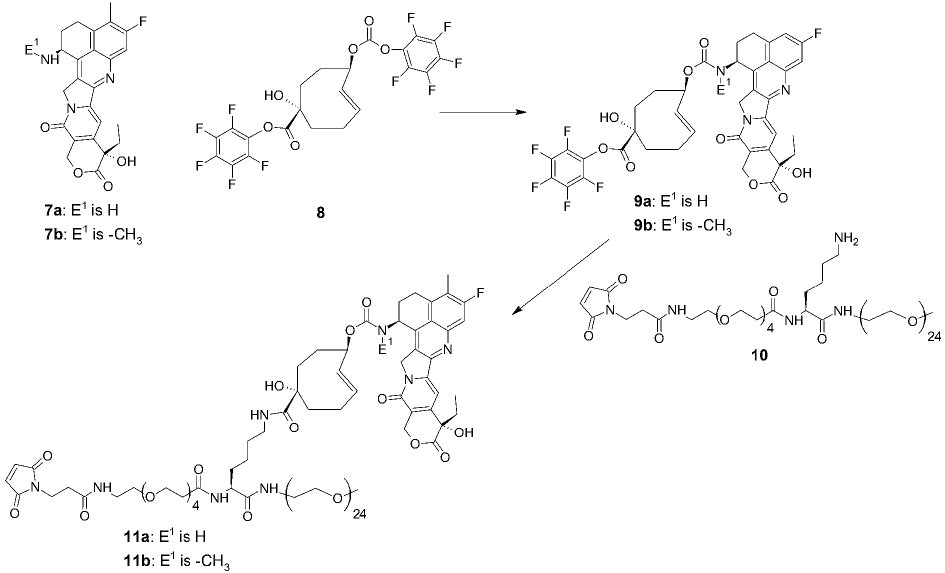

Compound **11a** was prepared in several steps *in situ.* To a suspension of exatecan mesylate (7) (287 mg, 0.54 mmol, MedChemExpress) in 6 mL of anhydrous dimethylformamide (DMF) in a glass vial was added compound **8** (506 mg, 0.90 mmol) and diethylamine (DIEA; 313 µL, 1.80 mmol). The mixture was stirred at room temperature in the dark for 2 h, at which point LC-MS analysis indicated complete consumption of **7a** and formation of intermediate **9a**. The excess of compound **8** was quenched by addition of N-isopropylmethylamine (73 mg, 1.0 mmol) and stirring at room temperature in the dark for 2 h. To this reaction mixture was added a solution of compound **10** (trifluoroacetic acid salt, 1555 mg, 0.90 mmol) and DIEA (312 µL, 1.80 mmol) in 4 mL of anhydrous DMF. The reaction mixture was stirred at room temperature in the dark for 2 h. The mixture was purified by preparative RP-HPLC (HPLC conditions: solvent A (0.05% TFA in water), solvent B (acetonitrile), 10 to 65% of B over 30 min at a rate of 50 mL/min, elution time 24 min). The collected fractions were analyzed by HPLC/LC-MS. The pure fractions were lyophilized in the dark to give compound **11a**. (234 mg, 19 % yield from 7). LCMS m/z 1122.7 ((M+2H)/2).

Compound **11b** is prepared in several steps *in situ.* To N-methyl exatecan **(7b,** 1 eq) in 6 mL of anhydrous dimethylformamide (DMF) in a glass vial is added compound **8** (1 eq) and diethylamine (4 eq). The mixture is stirred at room temperature in the dark for 11 days. To this reaction mixture is added a solution of compound **10** (trifluoroacetic acid salt, 1 eq) and DIEA (4 eq) in 4 mL of anhydrous DMF. The reaction mixture is stirred at room temperature in the dark for 2 h. The mixture is purified by preparative RP-HPLC (HPLC conditions: solvent A (0.05% TFA in water), solvent B (acetonitrile), 10 to 65% of B over 30 min at a rate of 50 mL/min. The collected fractions are analyzed by HPLC/LC-MS and pure fractions are lyophilized to give compound **11b.**

### Example 1b-ii: synthesis of compounds 13a and 13b

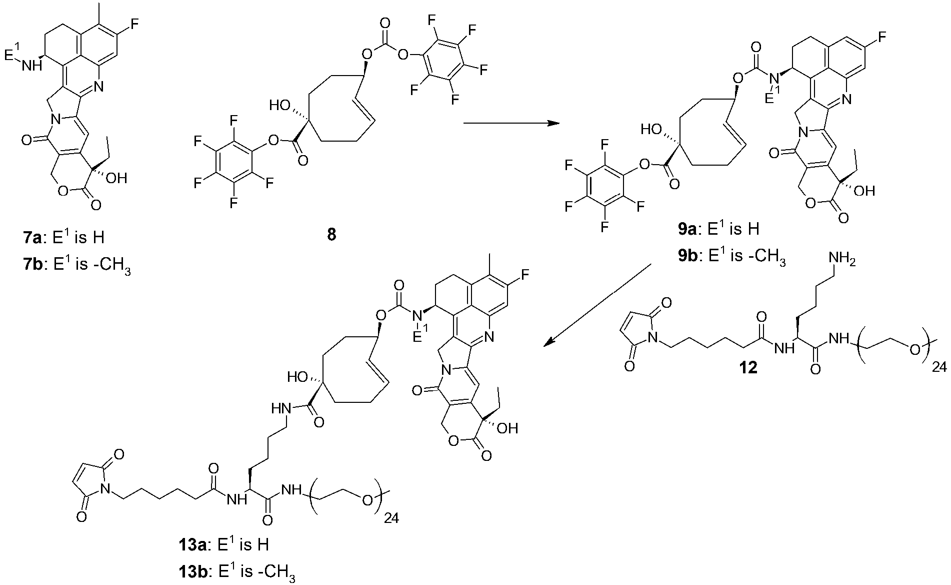

Compound **13a** was prepared in several steps in situ. To a suspension of exatecan mesylate **(7a)** (287 mg, 0.54 mmol, MedChemExpress) in 6 mL of anhydrous DMF in a glass vial was added compound **8** (506 mg, 0.90 mmol) and DIEA (313 µL, 1.80 mmol). The mixture was stirred at room temperature in the dark for 2 h, at which point LC-MS analysis indicated complete consumption of **7a** and formation of intermediate **9a.** The excess of compound **8** was quenched by addition of N-isopropylmethylamine (73 mg, 1.0 mmol) and stirring at room temperature in the dark for 2 h. To this reaction mixture was added a solution of compound **12** (HCl salt, 1300 mg, 0.90 mmol, Biomatrik) and DIEA (156 µL, 0.90 mmol) in 4 mL of anhydrous DMF. The reaction mixture was stirred at room temperature in the dark for 2 h. The mixture was purified by preparative RP-HPLC (HPLC conditions: solvent A (0.05% TFA in water), solvent B (acetonitrile), 10 to 65% of B over 30 min at a rate of 50 mL/min, elution time 26 min). The collected fractions were analyzed by HPLC/LC-MS. The pure fractions were lyophilized in the dark to give compound **13a.** (280 mg, 25 % yield from **7).** LCMS m/z 1020.3 ((M+2H)/2).

Compound **13b** is prepared in several steps *in situ.* To N-methyl exatecan **(7b,** 1 eq) in 6 mL of anhydrous dimethylformamide (DMF) in a glass vial is added compound **8** (1 eq) and diethylamine (4 eq). The mixture is stirred at room temperature in the dark for 11 days. To this reaction mixture is added a solution of compound **12** (trifluoroacetic acid salt, 1 eq) and diethylamine (4 eq) in 4 mL of anhydrous DMF. The reaction mixture is stirred at room temperature in the dark for 2 h. The mixture is purified by preparative RP-HPLC (HPLC conditions: solvent A (0.05% TFA in water), solvent B (acetonitrile), 10 to 65% of B over 30 min at a rate of 50 mL/min. The collected fractions are analyzed by HPLC/LC-MS and pure fractions are lyophilized to give compound **13b.**

### Example 1b-iii: synthesis of compounds 14a, 14b, 15a, and 15b

Compound **11a, 11b**, **13a,** or **13b** was conjugated to diabody AVP0458 to afford compound **14a, 14b, 15a,** or **15b,** respectively, following the optimized procedure by Rossin et al., Nature Communications (2018)9:1484.

Briefly, 2 mg AVP0458 was reacted with 6 mM DTT for 2 h at room temperature on a roller bench, followed by purification via PD-10 pre-equilibrated with 0.1 M phosphate buffer pH 6.8, containing 2 mM ETDA (PB-EDTA buffer). The purified diabody solution was then split in four aliquots which were then added with 24 eq of compound **11a, 11b**, **13a,** or **13b,** respectively, dissolved in dry DMSO at 10 mM concentration. The four reaction mixtures were incubated for 1h at room temperature on a roller bench followed by overnight incubation at +4°C. The four resulting conjugates ADCs (compounds **14a, 14b, 15a,** and **15b,** conjugates of AVP0458 and compound **11a, 11b**, **13a,** or **13b,** respectively) were then purified from the crude mixtures by SEC (Superdex75 10/300 column eluted with PBS at 0.8 mL/min) followed by concentration via Amicon Ultra-4 (30 kDa MW cut-off). UV measurements on the final solutions showed 60-78% recovery of diabody. SDS-PAGE analysis of the two ADC solutions showed the presence of one species with the expected increase in MW with respect to that of the monomer in AVP0458. Further analysis using mass spectrometry showed a complete reaction between the four cysteine residues in AVP0458 with the respective TCOs, confirming the production of two conjugates with a DAR of 4.

### Conjugates 14a, 14b, 15a, and 15b have the following structures:

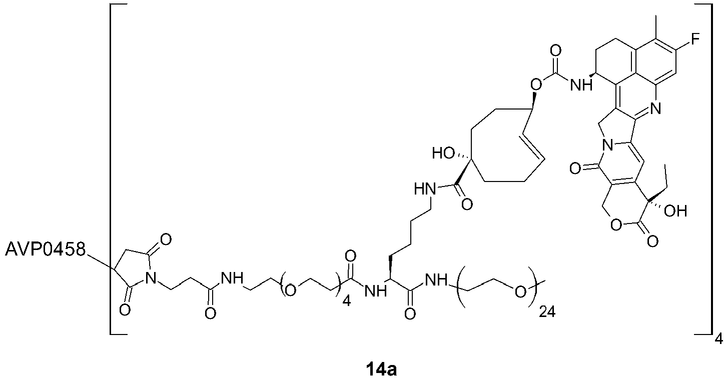

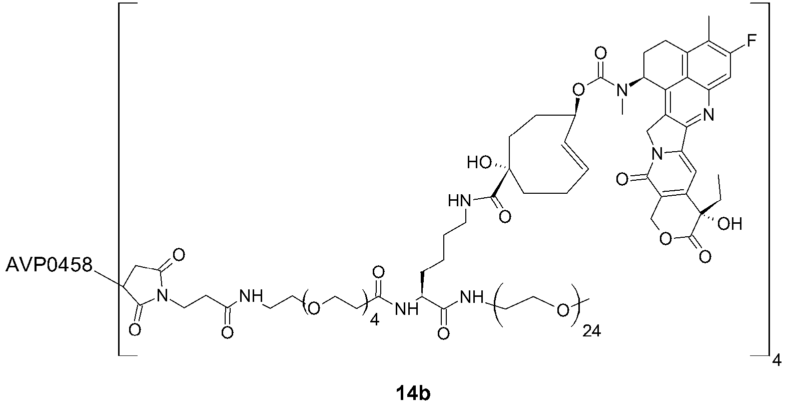

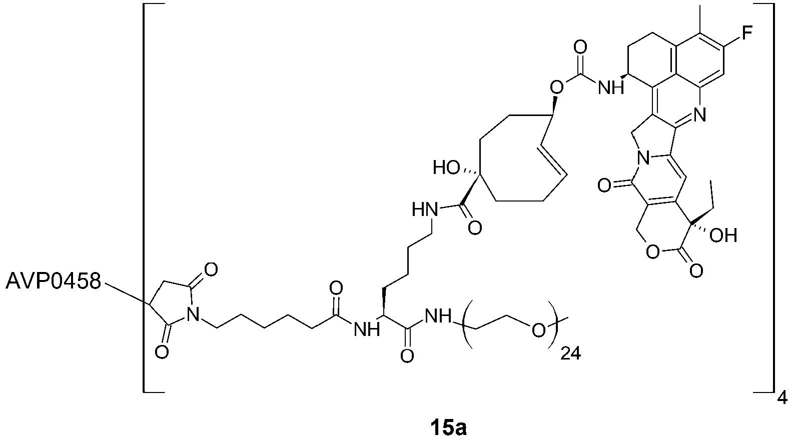

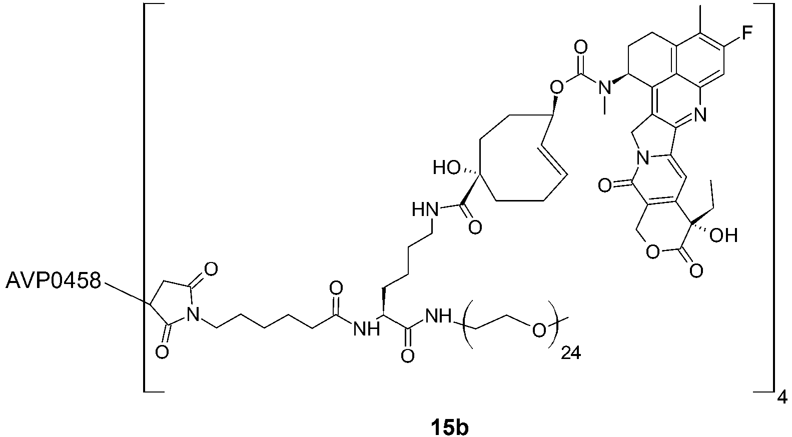

### Example 2: in vivo blood clearance in tumor-free mice

Six groups of tumor-free mice (n = 3 or 4 per group) were injected ¹²⁵I-labeled compound **1, 2, 14a, 15a, 14b,** or **15b** in a dosage of 5 mg/kg (for compounds **1, 2, 14a,** and **15a)** or 1 mg/kg (for compounds **14b** and **15b).** Blood samples (ca 50 µL) were withdrawn from the vena saphena at various times up to 72h post injection.

For experiments using compounds **1, 2, 14a,** or **15a,** after gamma-counting plasma isolated from blood was reacted *ex vivo* with an excess of tetrazine **6** for at least 1h at 37°C.

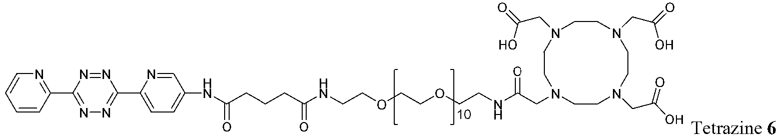

Then, the samples were analyzed by SEC on a Superdex75 10/300 column eluted with PBS at 0.8 mL/min. The eluates were collected in 1 ml fractions which were then measured by gamma-counting using a dual-isotope protocol with crossover correction.

From the above exeperiments, the amount of compound **1, 2, 14a, 15a, 14b,** or **15b** in the blood of the mice 48 hours after injection could be determined, as well as the respective half-lives in blood for said compounds. The results are shown in Table 1.

**Table 1. In vivo blood clearance in tumor-free mice.**

| | **Compound left in blood of mice 48h post-injection** (in %ID/g) | **Half-lives of compounds in blood of mice** (in hours) |
|---|---|---|
| Compound **1** (reference) | 1.14 ± 0.15 | 4.22 |
| Compound **2** | 0.82 ± 0.06 | 4.20 |
| Compound **14a** (reference) | 1.36 ± 0.19 | 5.22 |
| Compound **15a** | 0.92 ± 0.16 | 4.82 |
| Compound **14b** (reference) | 1.02 ± 0.40 | 4.98 |
| Compound **15b** | 0.85 ± 0.09 | 4.53 |

Thus, compound **2** advantageously and surprisingly has a faster clearance rate than compound 1. Likewise, compounds **15a** and **15b** advantageously and surprisingly have faster clearance rates than compounds **14a** and **14b,** respectively. Furthermore, it was observed that at least compounds **1, 2, 14a,** and **15a** showed high *in vivo* TCO stability.

### Example 3: in vivo tumor and off-target binding of in tumour-bearing mice

Below, the *in vivo* tumor binding and off-target binding in tumor-bearing mice of compounds **1, 2, 14a,** and **15a** is described. The protocol for compounds **1** and **2** are discussed first, and then the protocol for compounds **14a** and **15a.** Thereafter, the results are presented in Table 1.

### Example 3-i: protocol for compounds 1 and 2

Two groups of mice (n=5) bearing LS174T xenografts were injected compound **1** (reference) or compound **2** (2 mg/kg) followed 49h later by ¹¹¹In-labeled tetrazine **6** (10 eq with respect to ADC). Three hours post tetrazine **6** injection, the mice were euthanized and blood, tumors and other tissues were harvested, weighed and counted (together with standards) in a gamma counter with dual isotope protocol with crossover correction. The ¹²⁵I counts were used to calculate the amounts of compounds 1 and 2 in the various tissues (as %ID/g).

### Example 3-ii: protocol for compounds 14a and 15a

Two groups of mice (n=4) bearing LS174T xenografts were injected compound **14a** (reference) or compound **15a** (2 mg/kg) The mice were euthanized 52 h post-ADC injection and blood, tumors and other tissues were harvested, weighed and counted together with standards. The ¹²⁵I counts were used to calculate the amounts of compounds 1 and 2 in the various tissues (as %ID/g).

The results of Example 3 are shown in Tables 2 and 3.

**Table 2. Biodistribution of compounds in tumor-bearing mice.**

| | **Amount of compound (in % ID/g)** | | | |
|---|---|---|---|---|
| | **Compound 1** (reference) | **Compound 2** (claimed) | **Compound 14a** (reference) | **Compound 15a** (claimed) |
| **Tumor** | 18.42 ± 5.14 | 23.11 ± 6.21^{a} | 44.15 ± 2.02 | 55.34 ± 6.97 |
| **Blood** | 0.77 ± 0.12 | 0.52 ± 0.10 | 0.75 ± 0.14 | 0.87 ± 0.46^{b} |
| **Heart** | 0.22 ± 0.04 | 0.15 ± 0.03 | 0.33 ± 0.06 | 0.33 ± 0.12 |
| **Lung** | 0.59 ± 0.06 | 0.44 ± 0.06 | 0.90 ± 0.17 | 1.03 ± 0.30 |
| **Liver** | 0.43 ± 0.20 | 0.38 ± 0.22 | 1.46 ± 0.73 | 1.69 ± 0.73 |
| **Spleen** | 0.27 ± 0.08 | 0.21 ± 0.07 | 0.71 ± 0.31 | 0.64 ± 0.20 |
| **Kidney** | 0.60 ± 0.08 | 0.57 ± 0.14 | 0.93 ± 0.25 | 0.64 ± 0.20 |
| **Muscle** | 0.08 ± 0.02 | 0.06 ± 0.01 | 0.10 ± 0.02 | 0.11 ± 0.04 |
| **Bone** | 0.11 ± 0.01 | 0.10 ± 0.02 | 0.19 ± 0.04 | 0.14 ± 0.10 |

| | | | | |
|---|---|---|---|---|
| ^{a} n = 4 (one mouse did not develop a tumour). ^{b} the individual values are 0.45, 0.64, 0.87, and 1.51 (possible outlier). Without the possible outlier, the mean value is 0.66 ± 0.21 %ID/g. | | | | |

**Table 3. Biodistribution of compounds in tumor-bearing mice.**

| | **Amount of compound (in % ID/organ)** | | | |
|---|---|---|---|---|
| | **Compound 1** (reference) | **Compound 2** (claimed) | **Compound 14a** (reference) | **Compound 15a** (claimed) |
| **Stomach full** | 0.04 ± 0.01 | 0.03 ± 0.01 | 0.10 ± 0.04 | 0.07 ± 0.01 |
| **Small intestine full** | 0.27 ± 0.03 | 0.20 ± 0.04 | 0.42 ± 0.12 | 0.40 ± 0.14 |
| **Large intestine full** | 0.17 ± 0.03 | 0.16 ± 0.04 | 0.42 ± 0.08 | 0.33 ± 0.14 |
| **Thyroid** | 0.48 ± 0.11 | 0.42 ± 0.07 | 1.12 ± 0.58 | 0.44 ± 0.20 |

From Tables 2 and 3 it is clear that compound **2** shows a higher uptake in tumour and a lower off-target uptake than reference compound **1.** Likewise, compound **15a** shows a higher uptake in tumour and a lower off-target uptake than reference compound **14a.** These results are highly advantageous, since the higher the tumour/off target ratio, the lower the extent of unwanted side-effects are usually observed.

### Example 4: further in vitro and in vivo properties of compound 2

Other properties of compound **2** were tested as well:
*1. In vitro* metabolism studies were carried out using compound **1** or **2** in the presence or absence of acidified human liver S9 fraction for up to 24 hours. From these studies, it was shown that compound **2** has a better metabolism profile than compound **1.**
2. *An in vitro* reaction of compound **2** with a standard tetrazine yielded quantitative MMAE release after 24 hours of incubation.
3. At most 0.8% MMAE release was detected after 6 days of incubating compound **2** in mouse plasma in the absence of a tetrazine or any other trigger.

## Claims

1. A compound according to Formula (2): 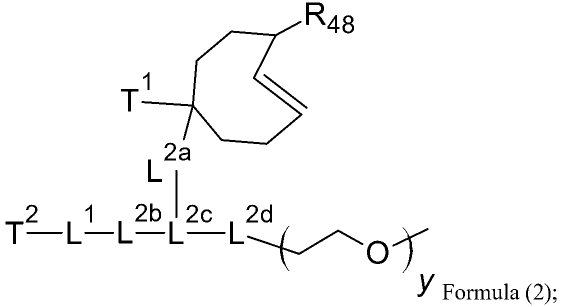 wherein
**L¹** is a linear C₄-C₈ alkylene; **T¹** is -OH; **R₄₈** is -O-C(O)-C^{A}; wherein C^{A} is a drug, wherein the drug is linked to the moiety -O-C(O)- via a secondary or tertiary nitrogen atom that is part of the drug, forming a carbamate; **L^{2a}, L^{2b},** and **L^{2d}** are each independently selected from the group consisting of -C(O)NH-, and -NHC(O)-; **L^{2c}** is C₄-C₆ alkanetriyl; **T²** is maleimidyl; and y is an integer in a range of from 15 to 35.

2. The compound according to claim 1, wherein the drug is selected from the group consisting of monomethyl auristatin E, exatecan, N-methyl-exatecan, duocarmycins, pyrrolobenzodiazepines, and pyrrolobenzodiazepine dimers.

3. The compound according to any one of claims 1 to 2, wherein L¹ is an unsubstituted, linear C₅ alkylene, y is an integer in a range of from 23 to 25, the drug is monomethyl auristatin E, T¹ is in an axial position, and R₄₈ is in an axial position.

4. A conjugate comprising a protein conjugated to at least one compound according to any one of claims 1 to 3, wherein said protein and said compound are conjugated via T².

5. The conjugate according to claim 4, wherein the conjugate is 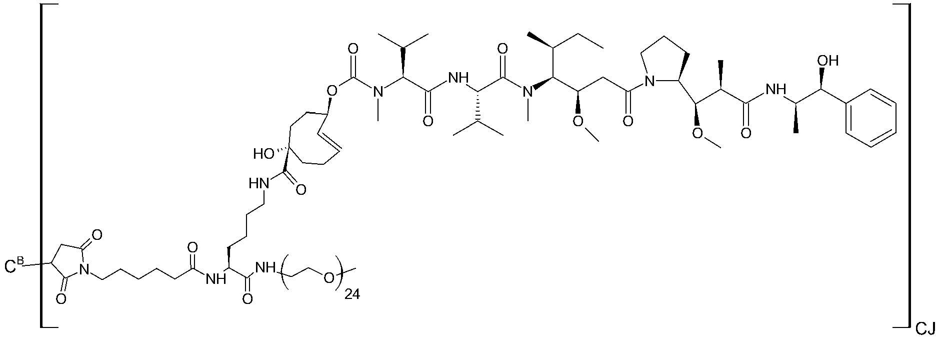 wherein CJ is in a range of from 1 to 12, and C^{B} is said protein.

6. The conjugate according to claim 5, wherein the conjugate is

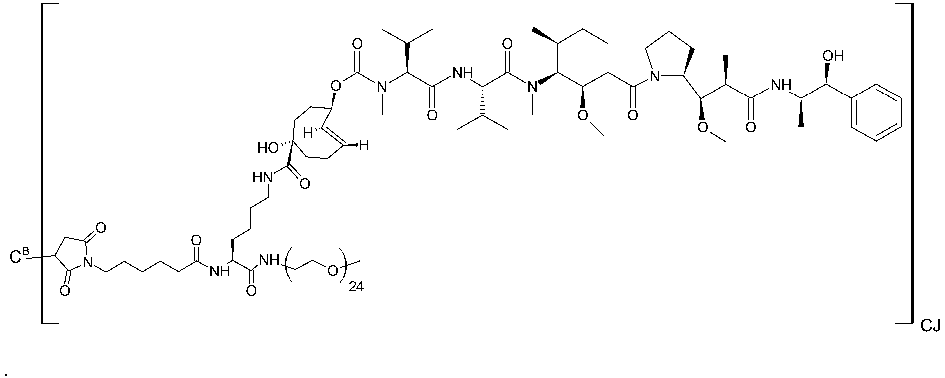

7. The conjugate according to any one of claims 4 to 6, wherein the protein is AVP0458 consisting of two monomers, wherein each of the two monomers has an amino acid sequence according to SEQ ID NO: 1.

8. The conjugate according to claim 4, wherein the protein is AVP0458 consisting of two monomers, wherein each of the two monomers has an amino acid sequence according to SEQ ID NO: 1, wherein the at least one compound is a compound according to claim 3, and wherein the protein is conjugated to at most 4 of said compounds.

9. A pharmaceutical composition comprising the conjugate according to any one of claims 4 to 8.

10. A combination of the conjugate according to any one of claims 4 to 8, and/or the pharmaceutical composition according to claim 9; with a tetrazine.

11. The combination according to claim 10, wherein the tetrazine is

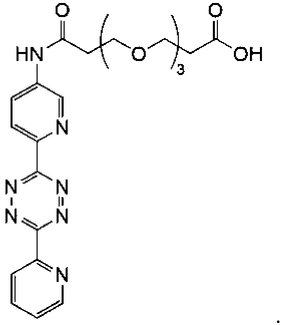

12. The conjugate according to any one of claims 4 to 8; the pharmaceutical composition according to claim 9; or the combination according to any one of claims 10 to 11; for use as a medicament.

13. The conjugate according to any one of claims 4 to 8; the pharmaceutical composition according to claim 9; or the combination according to any one of claims 10 to 11; for use in the treatment of a disease in a subject; wherein the subject is a human; and the disease is cancer.

14. A method for synthesizing a compound according to any one of claims 1 to 3; wherein said method comprises
(A) coupling a compound of Formula (R) to a compound of Formula (S): 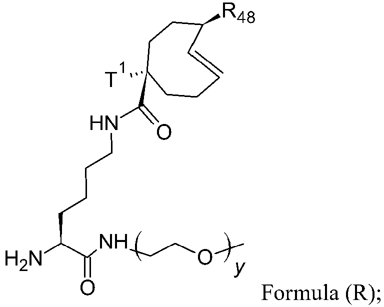 wherein R₄₈, T¹, and y are as defined in any one of claims 1 to 3; 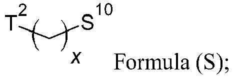 wherein T² is as defined in any one of claims 1 to 3, x is an integer in a range of from 4 to 8; and S¹⁰ is -COOH or an active ester, preferably S¹⁰ is -COOH;
or
(B) coupling a compound of Formula (T) to a compound of Formula (U): 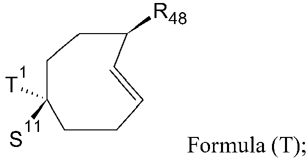 wherein R₄₈, and T¹ are as defined in any one of claims 1 to 3; and S¹¹ is -COOH or an active ester, preferably S¹¹ is an active ester; 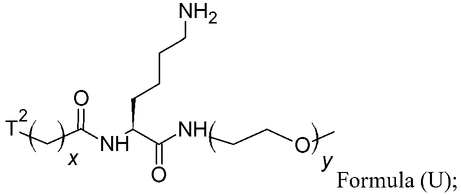 wherein T², and y are as defined in any one of claims 1 to 3; and x is an integer in a range of from 4 to 8.

15. A method for synthesizing a conjugate according to any one of claims 4 to 8; wherein said method comprises the step of coupling a protein to a compound according to any one of claims 1 to 3; wherein preferably in said protein disulfide bonds have been reduced.
